(19) Europäisches Patentamt / European Patent Office / Office européen des brevets

(11) **EP 3 686 279 B1**

(12) **EUROPEAN PATENT SPECIFICATION**

(45) Date of publication and mention
of the grant of the patent:
**04.01.2023 Bulletin 2023/01**

(51) International Patent Classification (IPC):
*C12N 9/22* *(2006.01)*    *C12N 9/96* *(2006.01)*
*C12N 15/63* *(2006.01)*    *C12N 15/10* *(2006.01)*

(21) Application number: **19216733.6**

(22) Date of filing: **17.08.2015**

(52) Cooperative Patent Classification (CPC):
**C12N 9/22; C12N 9/96; C12N 15/102**

(54) **GENOME EDITING USING CAS9 NICKASES**

GENOMBEARBEITUNG MIT CAS9-NICKASEN

ÉDITION DU GÉNOME À L'AIDE DE NICKASES CAS9

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.08.2014 US 201462038358 P
17.06.2015 US 201562180699 P**

(43) Date of publication of application:
**29.07.2020 Bulletin 2020/31**

(62) Document number(s) of the earlier application(s) in
accordance with Art. 76 EPC:
**15756300.8 / 3 180 426**

(73) Proprietors:
 • **The Broad Institute, Inc.
 Cambridge, MA 02142 (US)**
 • **Massachusetts Institute of Technology
 Cambridge MA 02142 (US)**
 • **President and Fellows of Harvard College
 Cambridge, MA 02138 (US)**

(72) Inventors:
 • **ZHANG, Feng
 Cambridge, MA 02139 (US)**
 • **RAN, Fei
 Boston, MA 02116 (US)**

(74) Representative: **Icely, Dominic Michael
 Impact Intellectual Property LLP
 1 Sans Walk
 London EC1R 0LT (GB)**

(56) References cited:
 **WO-A1-2013/141680    WO-A1-2014/093635
 WO-A1-2014/093694    WO-A1-2014/204724**

**WO-A1-2014/204725**

 • **S. W. CHO ET AL: "Analysis of off-target effects
 of CRISPR/Cas-derived RNA-guided
 endonucleases and nickases", GENOME
 RESEARCH, vol. 24, no. 1, 19 November 2013
 (2013-11-19), pages 132-141, XP055227885, ISSN:
 1088-9051, DOI: 10.1101/gr.162339.113**
 • **RAN F ANN ET AL: "Double Nicking by
 RNA-Guided CRISPR Cas9 for Enhanced
 Genome Editing Specificity", CELL, vol. 154, no.
 6, 12 September 2013 (2013-09-12), pages
 1380-1389, XP028716272, ISSN: 0092-8674, DOI:
 10.1016/J.CELL.2013.08.021**
 • **RAN F ANN ET AL: "Genome engineering using
 the CRISPR-Cas9 system", NATURE
 PROTOCOLS, NATURE PUBLISHING GROUP,
 GB, vol. 8, no. 11, 1 November 2013 (2013-11-01),
 pages 2281-2308, XP009174668, ISSN: 1750-2799**
 • **BIN SHEN ET AL: "Efficient genome modification
 by CRISPR-Cas9 nickase with minimal off-target
 effects", NATURE METHODS, vol. 11, no. 4, 2
 March 2014 (2014-03-02), pages 399-402,
 XP055227888, GB ISSN: 1548-7091, DOI:
 10.1038/nmeth.2857**
 • **PRASHANT MALI ET AL: "CAS9 transcriptional
 activators for target specificity screening and
 paired nickases for cooperative genome
 engineering", NATURE BIOTECHNOLOGY, vol.
 31, no. 9, 1 August 2013 (2013-08-01) , pages
 833-838, XP055186073, ISSN: 1087-0156, DOI:
 10.1038/nbt.2675**

EP 3 686 279 B1

• HSU PATRICK D ET AL: "Development and Applications of CRISPR-Cas9 for Genome Engineering", CELL, vol. 157, no. 6, 5 June 2014 (2014-06-05), pages 1262-1278, XP028849523, ISSN: 0092-8674, DOI: 10.1016/J.CELL.2014.05.010

• WOONG Y HWANG ET AL: "Efficient genome editing in zebrafish using a CRISPR-Cas system", NATURE BIOTECHNOLOGY, vol. 31, no. 3, 29 January 2013 (2013-01-29), pages 227-229, XP055086625, ISSN: 1087-0156, DOI: 10.1038/nbt.2501

• WANG HAOYI ET AL: "One-Step Generation of Mice Carrying Mutations in Multiple Genes by CRISPR/Cas-Mediated Genome Engineering", CELL, CELL PRESS, US, vol. 153, no. 4, 2 May 2013 (2013-05-02), pages 910-918, XP028538358, ISSN: 0092-8674, DOI: 10.1016/J.CELL.2013.04.025

• LI JIAN-FENG ET AL: "Multiplex and homologous recombination-mediated genome editing in Arabidopsis and Nicotiana benthamiana using guide RNA and Cas9", NATURE BIOTECHNOLOGY, vol. 31, no. 8, 23 June 2013 (2013-06-23), pages 688-691, XP055133339, ISSN: 1087-0156, DOI: 10.1038/nbt.2654

• TREVINO ALEXANDRO E ET AL: "Genome editing using Cas9 nickases", METHODS IN ENZYMOLOGY, ACADEM. PRESS, USA, vol. 546, 1 January 2014 (2014-01-01), pages 161-174, XP009187160, ISSN: 1557-7988, DOI: 10.1016/B978-0-12-801185-0.00008-8

Remarks:

The complete document including Reference Table(s) and the Sequence Listing(s) can be downloaded from the EPO website

**Description**

RELATED APPLICATIONS

This application claims priority to US provisional patent application Serial No. 62/038,358 filed August 17, 2014 and US provisional patent application Serial No. 62/180,699 filed June 17, 2015.

All documents or applications cited therein during their prosecution ("appln cited documents") and all documents cited or referenced in the appln cited documents, and all documents cited or referenced herein ("herein cited documents"), and all documents cited or referenced in herein cited documents, together with any manufacturer's instructions, descriptions, product specifications, and product sheets for any products mentioned herein or in any document cited by reference herein, and may be employed in the practice of the invention. More specifically, all referenced documents are cited to the same extent as if each individual document was specifically and individually indicated to be cited.

STATEMENT AS TO FEDERALLY SPONSORED RESEARCH

This invention was made with government support under Grant No. MH100706 awarded by the National Institutes of Health. The government has certain rights in the invention.

FIELD OF THE INVENTION

The present invention generally relates to the delivery, engineering and optimization of systems, methods and compositions used for the control of gene expression involving sequence targeting, such as genome perturbation or gene-editing, that relate to Clustered Regularly Interspaced Short Palindromic Repeats (CRISPR) and components thereof.

In particular, the present invention relates to the preparation, testing, and application of mutated Cas9 enzymes capable of inducing single-strand nicks for precision mammalian genome engineering.

BACKGROUND OF THE INVENTION

Recent advances in genome sequencing techniques and analysis methods have significantly accelerated the ability to catalog and map genetic factors associated with a diverse range of biological functions and diseases. Precise genome targeting technologies are needed to enable systematic reverse engineering of causal genetic variations by allowing selective perturbation of individual genetic elements, as well as to advance synthetic biology, biotechnological, and medical applications. Although genome-editing techniques such as designer zinc fingers, transcription activator-like effectors (TALEs), or homing meganucleases are available for producing targeted genome perturbations, there remains a need for new genome engineering technologies that are affordable, easy to set up, scalable, and amenable to targeting multiple positions within the eukaryotic genome.

Targeted, rapid, and efficient genome editing using the RNA-guided Cas9 system is enabling the systematic interrogation of genetic elements in a variety of cells and organisms and holds enormous potential as next-generation gene therapies (Hsu, Lander, & Zhang, 2014). In contrast to other DNA targeting systems based on zinc-finger proteins (ZFPs) (Klug, 2010) and transcription activator-like effectors (TALEs) (Boch & Bonas, 2010), which rely on protein domains to confer DNA-binding specificity, Cas9 forms a complex with a small guide RNA that directs the enzyme to its DNA target via Watson-Crick base pairing. Consequently, the system is simple and fast to design and requires only the production of a short oligonucleotide to direct DNA binding to any locus.

The type II microbial CRISPR (clustered regularly interspaced short palindromic repeats) system (Chylinski, Makarova, Charpentier, & Koonin, 2014), which is the simplest among the three known CRISPR types (Barrangou & Marraffini, 2014; Gasiunas, Sinkunas, & Siksnys, 2014; Wiedenheft, Sternberg, & Doudna, 2012), consists of the CRISPR-associated (Cas) genes and a series of non-coding repetitive elements (direct repeats) interspaced by short variable sequences (spacers). These short approximate 30 bp spacers are often derived from foreign genetic elements such as phages and conjugating plasmids, and they constitute the basis for an adaptive immune memory of those invading elements (Barrangou et al., 2007). The corresponding sequences on the phage genomes and plasmids are called protospacers, and each protospacer is flanked by a short protospacer-adjacent motif (PAM), which plays a critical role in the target search and recognition mechanism of Cas9. The CRISPR array is transcribed and processed into short RNA molecules known as CRISPR RNAs (crRNA) that, together with a second short transactivating RNA (tracrRNA) (Deltcheva et al., 2011), complex with Cas9 to facilitate target recognition and cleavage (Deltcheva et al., 2011; Garneau et al., 2010). Additionally, the crRNA and tracrRNA can be fused into a single guide RNA (sgRNA) to facilitate Cas9 targeting (Jinek et al., 2012).

The Cas9 enzyme from Streptococcus pyogenes (SpCas9), which requires a 5'-NGG PAM (Mojica, Diez-Villasenor, Garcia-Martinez, & Almendros, 2009), has been widely used for genome editing applications (Hsu et al., 2014). In order to target any desired genomic locus of interest that fulfills the PAM requirement, the enzyme can be "programmed" merely by altering the 20-bp guide sequence of the sgRNA. Additionally, the simplicity of targeting lends itself to easy multiplexing such as simultaneous editing of several loci by including multiple sgRNAs (Cong et al., 2013; Wang et al., 2013).

Like other designer nucleases, Cas9 facilitates genome editing by inducing double-strand breaks (DSBs) at its target site, which in turn stimulates endogenous DNA damage repair pathways that lead to edited DNA: homology directed repair (HDR), which requires a homologous template for recombination but repairs DSBs with high fidelity, and non-homologous end-joining (NHEJ), which functions without a template and frequently produces insertions or deletions (indels) as a consequence of repair. Exogenous HDR templates can be designed and introduced along with Cas9 and sgRNA to promote exact sequence alteration at a target locus; however, this process typically occurs only in dividing cells and at low efficiency.

Certain applications - e.g. therapeutic genome editing in human stem cells - demand editing that is not only efficient, but also highly specific. Nucleases with off-target DSB activity could induce undesirable mutations with potentially deleterious effects, an unacceptable outcome in most clinical settings. The remarkable ease of targeting Cas9 has enabled extensive off-target binding and mutagenesis studies employing deep sequencing (Fu et al., 2013; Hsu et al., 2013; Pattanayak et al., 2013) and chromatin immunoprecipitation (ChIP) in human cells (Kuscu, Arslan, Singh, Thorpe, & Adli, 2014; Wu et al., 2014). As a result, an increasingly complete picture of the off-target activity of the enzyme is emerging. Cas9 will tolerate some mismatches between its guide and a DNA substrate, a characteristic that depends strongly on the number, position (PAM proximal or distal) and identity of the mismatches. Off-target binding and cleavage may further depend on the organism being edited, the cell type, and epigenetic contexts.

These specificity studies, together with direct investigations of the catalytic mechanism of Cas9, have stimulated homology- and structure-guided engineering to improve its targeting specificity. The wild-type enzyme makes use of two conserved nuclease domains, HNH and RuvC, to cleave DNA by nicking the sgRNA-complimentary and non-complimentary strands, respectively. A "nickase" mutant (Cas9n) can be generated by alanine substitution at key catalytic residues within these domains - SpCas9 D10A inactivates RuvC (Jinek et al., 2012), while N863A has been found to inactivate HNH (Nishimasu et al., 2014). Though an H840A mutation was also reported to convert Cas9 into a nicking enzyme, this mutant has reduced levels of activity in mammalian cells compared with N863A (Nishimasu et al., 2014).

Because single stranded nicks are generally repaired via the non-mutagenic base-excision repair pathway (Dianov & Hubscher, 2013), Cas9n mutants can be leveraged to mediate highly specific genome engineering. A single Cas9n-induced nick can stimulate HDR at low efficiency in some cell types, while two nicking enzymes, appropriately spaced and oriented at the same locus, effectively generate DSBs, creating 3' or 5' overhangs along the target as opposed to a blunt DSB as in the wild-type case (Mali et al., 2013; Ran et al., 2013). The on-target modification efficiency of the double-nicking strategy is comparable to wild-type, but indels at predicted off-target sites are reduced below the threshold of detection by Illumina deep sequencing (Ran et al., 2013).

Despite this progress in Cas9 directed genetic engineering technologies, the efficiency of successful gene modifications, in particular in the context of HDR, is still at low levels, and improved strategies for increasing HDR efficiency for Cas9 directed genetic engineering are needed.

## SUMMARY OF THE INVENTION AND OF THE PRESENT DISCLOSURE

According to the present invention, there is provided a non-naturally occurring or engineered composition and a repair template for use in therapy according to claim 1. In a second aspect, there is provided an *ex vivo* method of modifying a cell of an organism by manipulation of a first and a second target sequence on opposite strands of a DNA duplex in a genomic locus of interest in a cell by promoting homology directed repair according to claim 2. Any embodiments or aspects falling outside the clams are provided as disclosure only. The CRISPR-Cas system does not require the generation of customized proteins to target specific sequences but rather a single Cas enzyme can be programmed by a short RNA molecule to recognize a specific DNA target. Adding the CRISPR-Cas system to the repertoire of genome sequencing techniques and analysis methods may significantly simplify the methodology and accelerate the ability to catalog and map genetic factors associated with a diverse range of biological functions and diseases. To utilize the CRISPR-Cas system effectively for genome editing without deleterious effects, it is critical to understand aspects of engineering and optimization of these genome engineering tools, which are aspects of the claimed invention.

There exists a pressing need for alternative and robust systems and techniques for sequence targeting with a wide array of applications. Aspects of this invention address this need and provide related advantages. An exemplary CRISPR complex comprises a mutated CRISPR enzyme complexed with a guide sequence hybridized to a target sequence within the target polynucleotide. The guide sequence is linked to a tracr mate sequence, which in turn hybridizes to a tracr sequence.

In one aspect, the disclosure provides methods for using one or more elements of a CRISPR system. The CRISPR complex of the invention provides an effective means for modifying a target polynucleotide. The CRISPR complex of the invention has a wide variety of utilities including modifying (e.g., deleting, inserting, translocating, inactivating, activating) a target polynucleotide in a multiplicity of cell types. In one aspect, the cell is a eukaryotic cell. In one aspect, the cell is a prokaryotic cell. As such the CRISPR complex of the invention has a broad spectrum of applications in, e.g., gene or genome editing, gene therapy, drug discovery, drug screening, disease diagnosis, and prognosis. An exemplary CRISPR complex comprises a CRISPR enzyme complexed with a guide sequence hybridized to a target sequence within the target polynucleotide. In one aspect, the guide sequence is linked to a tracr mate sequence, which in turn hybridizes to a tracr sequence. Aspects of the invention relate to Cas9 enzymes having improved target specificity in a CRISPR-Cas9 system, having guide RNAs with optimal activity, with Cas9 enzymes that are smaller in length than wild-type Cas9 enzymes (and nucleic acid molecules coding therefor), and chimeric Cas9 enzymes, as well as methods of improving the target specificity of a Cas9 enzyme or of designing a CRISPR-Cas9 system comprising designing or preparing guide RNAs having optimal activity and/or selecting or preparing a Cas9 enzyme having a smaller size or length than wild-type Cas9 whereby packaging a nucleic acid coding such construct into a delivery vector is advantageous as there is less coding therefor in the delivery vector than for wild-type Cas9, and/or generating chimeric Cas9 enzymes.

Also provided are uses of the present sequences, vectors, enzymes or systems, in medicine. Also provided are uses of the same in gene or genome editing.

The CRISPR enzyme is a nickase. The nickase is a modified Cas9 comprising a mutation at N863A (according to the numbering found in SpCas9 from *S. pyogenes*) or at N580 (according to the numbering found in SaCas9 from *S. aureus*) or at a residue which is equivalent or corresponding to those residues in orthologs of *S. pyogenes* or *S. aureus.* In particular, mutation of the residue to A (alanine) is preferred in some embodiments of the disclosure, but any catalytically inactive mutation at these residues should suffice. Surprisingly, Applicants found that the use of this mutation in a dual nickase system suppresses NHEJ and instead promotes HDR through the generation of 3' overhangs in the nicked duplex DNA.

Since Cas9n (D10A) and Cas9H840A nick opposite strands of DNA as previously shown, substitution of Cas9n with Cas9H840A with a given sgRNA pair should result in the inversion of the overhang type. For example, a pair of sgRNAs that will generate a 5' overhang with Cas9n should in principle generate the corresponding 3' overhang instead. Therefore, sgRNA pairs that lead to the generation of a 3' overhang with Cas9n might be used with Cas9H840A to generate a 5' overhang. Unexpectedly, Applicants tested Cas9H840A with a set of sgRNA pairs designed to generate both 5' and 3' overhangs (offset range from -278 to +58 bp), but were unable to observe indel formation. Surprisingly, HDR was observed with the present mutated Cas9 nickase (N863A) creating 3'-overhangs. Thus, from the instant invention, one can use 3' overhang, e.g., paired 3' overhangs to obtain HDR. In SaCas9 Applicants have identified that the mutation corresponding to N863A in SpCas9 is N580A. It may have the advantage of being a more predictable mutation for protein function than the H840A equivalent, which may change binding behavior.

In the disclosure, the Cas9 enzyme comprises a mutation and may be used as a generic DNA binding protein (e.g. the mutated Cas9 may or may not function as a double stranded nuclease or as a single stranded nickase; can function as merely a binding protein; but advantageously, the Cas9 is a nickase); and the so-mutated Cas9 may be with or without fusion to a functional domain or protein domain. The mutation concerns the catalytic domain HNH at residue N863; the Cas9 enzyme is, a SpCas9 protein comprising the mutation N863A, or any mutated ortholog having a mutation corresponding to SpCas9N863A. In one aspect of the disclosure, the mutated Cas9 enzyme may be fused to a protein domain or functional domain, e.g., such as a transcriptional activation domain. In one aspect, the transcriptional activation domain may be VP64. In another aspect the protein domain or functional domain can be, for example, a FokI domain. Other aspects of the disclosure relate to the mutated Cas 9 enzyme being fused to domains which include but are not limited to a transcriptional repressor, a recombinase, a transposase, a histone remodeler, a DNA methyltransferase, a cryptochrome, a light inducible/controllable domain or a chemically inducible/controllable domain. Further functional domains are also described herein.

In a further embodiment, the disclosure provides for methods to generate mutant tracrRNA and direct repeat sequences or mutant chimeric guide sequences that allow for enhancing performance of these RNAs in cells. Aspects of the disclosure also provide for selection of said sequences.

Aspects of the disclosure also provide for methods of simplifying the cloning and delivery of components of the CRISPR complex. In the preferred embodiment of the disclosure, a suitable promoter, such as the U6 promoter, is amplified with a DNA oligo and positioned contiguous to and upstream of a sequence encoding the guide RNA. The resulting PCR product can then be transfected into cells to drive expression of the guide RNA. Aspects of the disclosure also relate to the guide RNA being transcribed *in vitro* or ordered from a synthesis company and directly transfected.

In one aspect, the disclosure provides for methods to improve activity by using a more active polymerase. In one aspect, a T7 promoter may be inserted contiguous to and upstream of a sequence encoding a guide RNA. In a preferred embodiment, the expression of guide RNAs under the control of the T7 promoter is driven by the expression of the T7 polymerase in the cell. In an advantageous embodiment, the cell is a eukaryotic cell. In a preferred embodiment the eukaryotic cell is a human cell. In a more preferred embodiment the human cell is a patient specific cell.

In one aspect, the disclosure provides for methods of reducing the toxicity of Cas enzymes. The Cas9 enzyme is a nickase. In one embodiment, the Cas9 is delivered into the cell in the form of mRNA. This allows for the transient expression of the enzyme thereby reducing toxicity. In another embodiment, the Cas9 is delivered into the cell in the nucleotide construct that encodes and expresses the Cas9 enzyme. In another embodiment, the disclosure also provides for methods of expressing Cas9 under the control of an inducible promoter the constructs used therein. In another embodiment, the Cas9 is delivered into the cell as a protein. In another and particularly preferred embodiment, the Cas9 is delivered into the cell as a protein or as a nucleotide sequence encoding it.

In another aspect, the disclosure provides for methods of improving the *in vivo* applications of the CRISPR-Cas system. An advantageous aspect of the invention provides for the selection of Cas9 homologs that are easily packaged into viral vectors for delivery. Cas9 orthologs typically share the general organization of 3-4 RuvC domains and a HNH domain. The 5' most RuvC domain cleaves the non-complementary strand, and the HNH domain cleaves the complementary strand. All notations are in reference to the guide sequence.

The catalytic residue in the 5' RuvC domain is identified through homology comparison of the Cas9 of interest with other Cas9 orthologs (from S. pyogenes type II CRISPR locus, S. thermophilus CRISPR locus 1, S. thermophilus CRISPR locus 3, and Franciscilla novicida type II CRISPR locus), and the conserved Asp residue is mutated to alanine to convert Cas9 into a complementary-strand nicking enzyme. In the present invention, the conserved Asparagine residue (e.g. N863 in S. pyogenes (Sp) Cas9) in the HNH domain is mutated to Alanine to convert Cas9 into a non-complementary-strand nicking enzyme (e.g. SpCas9 N863A).

The present invention further encompasses any mutated ortholog that corresponds to SpCas9N863A. In some embodiments, the ortholog is *Staphyloccus aureus* so that the Cas9 is that from or derived from *Staphyloccus aureus* (referred to as SaCas9). In some embodiments, the *Staphyloccus aureus* is *Staphyloccus aureus* subspecies *aureus.* In some embodiments, the mutation corresponding to N863A in SpCas9 is N580A in *Staphyloccus aureus* or *Staphyloccus aureus* subspecies *aureus.*

The CRISPR enzyme is a mutated type II CRISPR enzyme. This type II CRISPR enzyme is a mutated Cas9 enzyme. A Cas enzyme may be identified Cas9 as this can refer to the general class of enzymes that share homology to the largest or biggest nuclease with multiple nuclease domains from the type II CRISPR system. Most preferably, the Cas9 enzyme is from, or is derived from, SpCas9 or SaCas9. By derived, Applicants mean that the derived enzyme is largely based, in the sense of having a high degree of sequence homology with, a wildtype enzyme, but that it has been mutated (modified) in some way as described herein.

It will be appreciated that the terms Cas and CRISPR enzyme are generally used herein interchangeably, unless otherwise apparent. As mentioned above, many of the residue numberings used herein refer to the Cas9 enzyme from the type II CRISPR locus in *Streptococcus pyogenes* (annotated alternatively as SpCas9 or spCas9). However, it will be appreciated that this invention includes many more Cas9s from other species of microbes, such as SpCas9 derived from *S. pyogenes*, SaCas9 derived from *S. aureus,* St1Cas9 derived from *S. thermophilus* and so forth. Further examples are provided herein. Thus, although numerous references are made herein to a Cas or CRISPR enzyme, it will be appreciated that these apply equally to any Cas9 ortholog that functions as required herein. In particular, however, mention of a Cas or CRISPR enzyme applies equally to SpCas9 or SaCas9, and visa versa, unless otherwise apparent.

An example of a codon optimized sequence, in this instance optimized for humans (i.e. being optimized for expression in humans) is provided herein, see the SaCas9 human codon optimized sequence. Whilst this is preferred, it will be appreciated that other examples are possible and codon optimization for a host species other than human, or for codon optimization for specific organs such as the brain, is known.

In further embodiments, the disclosure provides for methods of enhancing the function of Cas9 by generating chimeric Cas9 proteins. These methods may comprise fusing N-terminal fragments of one Cas9 homolog with C-terminal fragments of another Cas9 homolog. These methods also allow for the selection of new properties displayed by the chimeric proteins. Chimeras of SpCas9 and SaCas9 are preferred, in some embodiments.

It will be appreciated that in the present methods, where the organism is an animal or a plant, the modification may occur *ex vivo* or *in vitro,* for instance in a cell culture or a sample from the organism, and in some instances not *in vivo.* In other embodiments, it may occur *in vivo.*

Any or all of the polynucleotide sequence encoding a CRISPR enzyme, guide sequence, tracr mate sequence or tracr sequence may be RNA, DNA or a combination of RNA and DNA. In one aspect, the polynucleotides comprising the sequence encoding a CRISPR enzyme, the guide sequence, tracr mate sequence or tracr sequence are RNA. In one aspect, the polynucleotides comprising the sequence encoding a CRISPR enzyme, the guide sequence, tracr mate sequence or tracr sequence are DNA. In one aspect, the polynucleotides are a mixture of DNA and RNA, wherein some of the polynucleotides comprising the sequence encoding one or more of the CRISPR enzyme, the guide sequence, tracr mate sequence or tracr sequence are DNA and some of the polynucleotides are RNA. In one aspect, the polynucleotide comprising the sequence encoding the CRISPR enzyme is a DNA and the guide sequence, tracr mate sequence or tracr sequence are RNA. The one or more polynucleotides comprising the sequence encoding a CRISPR enzyme, the guide sequence, tracr mate sequence or tracr sequence may be delivered via electroporation, encapsulation in or attachment to particles, nanoparticles, exosomes, or microvesicles; or a via attachment to, for example, a gold particle and fired using a so-called "gene-gun."

It will be appreciated that where reference is made to a polynucleotide, where that polynucleotide is RNA and is said to 'comprise' a feature such as a tracr mate sequence, the RNA sequence includes the feature. Where the polynucleotide is DNA and is said to comprise a feature such a tracr mate sequence, the DNA sequence is or can be transcribed into the RNA that comprises the feature at issue. Where the feature is a protein, such as the CRISPR enzyme, the DNA or RNA sequence referred to is, or can be, translated (and in the case of DNA transcribed first). Furthermore, in cases where an RNA encoding the CRISPR enzyme is provided to a cell, it is understood that the RNA is capable of being translated by the cell into which it is delivered.

In one aspect, the disclosure provides a non-naturally occurring or engineered composition comprising:

I. two or more CRISPR-Cas system polynucleotide sequences comprising

    (a) a first guide sequence capable of hybridizing to a first target sequence in a polynucleotide locus,
    (b) a second guide sequence capable of hybridizing to a second target sequence in a polynucleotide locus,
    (c) a tracr mate sequence, and
    (d) a tracrRNA sequence, and

II. a Type II Cas9 enzyme or a second polynucleotide sequence encoding it,
wherein the Type II Cas9 enzyme is or comprises a SpCas9 enzyme comprising the mutation N863 or N863A, SaCas9 enzyme comprising the mutation N580 or N580A or an ortholog thereof, having a mutation corresponding to SpCas9N863 or N863A,

    wherein when transcribed, the first and the second tracr mate sequences hybridize to the first and second tracrRNA sequences respectively and the first and the second guide sequences direct sequence-specific binding of a first and a second CRISPR complex to the first and second target sequences respectively,
    wherein the first CRISPR complex comprises the Cas9 enzyme complexed with (1) the first guide sequence that is hybridizable to the first target sequence, and (2) the first tracr mate sequence that is hybridized to the first tracrRNA sequence,
    wherein the second CRISPR complex comprises the Cas9 enzyme complexed with (1) the second guide sequence that is hybridizable to the second target sequence, and (2) the second tracr mate sequence that is hybridized to the second tracrRNA sequence, and
    wherein the first guide sequence directs cleavage of one strand of the DNA duplex near the first target sequence and the second guide sequence directs cleavage of the other strand near the second target sequence inducing a double strand break, thereby modifying the organism or the non-human or non-animal organism, and wherein the first guide sequence directing cleavage of one strand of the DNA duplex near the first target sequence and the second guide sequence directing cleavage of the other strand near the second target sequence result in 3' overhangs.

In a preferred embodiment, components I and II are operably linked to one or more regulatory elements. In a preferred embodiment, component (I) comprises a CRISPR-Cas system polynucleotide sequence which comprises the guide sequence, the tracr mate sequence and the tracrRNA sequence. In a preferred embodiment, component (I) comprises a first regulatory element operably linked to the guide sequence and the tracr mate sequence, and a third regulatory element operably linked to the tracrRNA sequence.

In a preferred embodiment, the composition comprises a delivery system operably configured to deliver CRISPR-Cas complex components or polynucleotide sequences comprising or encoding said components to a cell. In a preferred embodiment, the delivery system comprises a vector system comprising one or more vectors, and wherein components I and II are located on the same or different vectors of the system. In a preferred embodiment the one or more vectors comprise one or more viral vectors. In a preferred embodiment the one or more viral vectors comprise one or more retrovirus, lentivirus, adenovirus, adeno-associated virus or herpes simplex virus vectors.

In a preferred embodiment, the delivery system comprises a nanoparticle, liposome, exosome, yeast system, microvesicle, or gene gun.

In a preferred embodiment, the composition comprises one or more functional domains. In a preferred embodiment, the one or more functional domain comprises a transcriptional activator domain. In a preferred embodiment the functional domain comprises VP64 or KRAB, SID or SID4X, or a recombinase, a transposase, a histone remodeler, a DNA methyltransferase, a cryptochrome, a light inducible/controllable domain or a chemically inducible/controllable domain.

In a preferred embodiment, the vector composition comprises a single vector.

In a preferred embodiment the cell is a eukaryotic cell. In a preferred embodiment the one or more vectors are operably configured to direct expression of CRISPR transcripts when introduced into a eukaryotic cell.

In a preferred embodiment the nucleotide sequence encoding the SaCas9 is codon optimized for expression in a eukaryotic cell.

In a preferred embodiment one or more of the regulatory elements comprises a tissue-specific promoter. In a preferred embodiment the tissue-specific promoter directs expression of CRISPR transcripts in muscle, neuron, bone, skin, blood, liver, pancreas, or lymphocytes.

In a preferred embodiment the target sequence is adjacent to a Protospacer Adjacent Motif (PAM) recognized by the Cas9 enzyme.

In a preferred embodiment the target sequence is flanked at its 3' end by 5'-NRG (where N is any Nucleotide) for SpCas9 or NNGRR for SaCas9.

In a preferred embodiment the guide sequence is capable of hybridizing to a target sequence in a eukaryotic cell.

In a preferred embodiment the tracrRNA sequence is 30 or more nucleotides in length. In a preferred embodiment the tracrRNA is 50 or more nucleotides in length.

In a preferred embodiment the SaCas9 enzyme further comprises one or more nuclear localization sequences (NLSs).

In an aspect, the disclosure provides an *in vivo, ex vivo* or *in vitro* host cell or cell line comprising or modified by the composition or enzyme as described herein, or progeny thereof. In a preferred embodiment, the host cell, cell line or progeny thereof is a stem cell or stem cell line.

In an aspect, the disclosure provides an *in vivo* or *ex vivo* method of modifying an organism by manipulation of one or more target sequences at genomic loci of interest comprising delivering to the organism the composition described herein.

In an aspect, the invention provides an *ex vivo* method of modifying a cell of an organism by manipulation of one or more target sequences at genomic loci of interest comprising delivering to the cell a non-naturally occurring or engineered composition comprising a vector composition operably encoding a composition as described herein. In a preferred embodiment the organism is a plant or algae.

In an aspect, the disclosure provides a composition or enzyme as described herein for use in medicine or for use in therapy.

In an aspect, the disclosure provides use of the composition or enzyme as described herein:

- in the preparation of a medicament;
- in the preparation of a medicament for *ex vivo* gene or genome editing; or

In an aspect, the invention provides a composition for use, method or the use as described herein to correct ocular defects that arise from genetic mutations.

Accordingly, in certain embodiments the disclosure provides a non-naturally occurring or engineered composition comprising: I. two or more CRISPR-Cas system polynucleotide sequences comprising (a) a first guide sequence capable of hybridizing to a first target sequence in a polynucleotide locus, (b) a second guide sequence capable of hybridizing to a second target sequence in a polynucleotide locus, (c) a tracr mate sequence, and (d) a tracrRNA sequence, and II. a Type II Cas9 enzyme or a second polynucleotide sequence encoding it, wherein the Type II Cas9 enzyme is or comprises a SpCas9 enzyme comprising the mutation N863 or N863A, SaCas9 enzyme comprising the mutation N580 or N580An or an ortholog thereof, having a mutation corresponding to SpCas9N863 or N863A, wherein when transcribed, the first and the second tracr mate sequences hybridize to the first and second tracrRNA sequences respectively and the first and the second guide sequences direct sequence-specific binding of a first and a second CRISPR complex to the first and second target sequences respectively, wherein the first CRISPR complex comprises the Cas9 enzyme complexed with (1) the first guide sequence that is hybridizable to the first target sequence, and (2) the first tracr mate sequence that is hybridized to the first tracrRNA sequence, wherein the second CRISPR complex comprises the Cas9 enzyme complexed with (1) the second guide sequence that is hybridizable to the second target sequence, and (2) the second tracr mate sequence that is hybridized to the second tracrRNA sequence, and wherein the first guide sequence directs cleavage of one strand of the DNA duplex near the first target sequence and the second guide sequence directs cleavage of the other strand near the second target sequence inducing a double strand break, thereby modifying the organism or the non-human or non-animal organism, and wherein the first guide sequence directing cleavage of one strand of the DNA duplex near the first target sequence and the second guide sequence directing cleavage of the other strand near the second target sequence result in 3' overhangs. Advantageously, components I and II are operably linked to one or more regulatory elements. Advantageously, component (I) comprises a CRISPR-Cas system polynucleotide sequence which comprises the guide sequence, the tracr mate sequence and the tracrRNA sequence. Advantageously, component (I) comprises a first regulatory element operably linked to the guide sequence and the tracr mate sequence, and a third regulatory element operably linked to the tracrRNA sequence. Advantageously, the composition includes a delivery system operably configured to deliver CRISPR-Cas complex components or polynucleotide sequences comprising or encoding said components to a cell. Advantageously, the delivery system comprises a vector system comprising one or more vectors, and wherein components I and II are located on the same or different vectors of the system. Advantageously, the one or more vectors comprise one or more viral vectors. Advantageously, the one or more viral vectors comprise one or more retrovirus, lentivirus, adenovirus, adeno-associated virus or herpes simplex virus vectors. Advantageously, the delivery system comprises a nanoparticle, liposome, exosome, yeast system, microvesicle, or gene gun. Advantageously, the composition (e.g., the Cas9 or CRISPR enzyme) includes one or more functional domains. Advantageously, the one or more functional domain comprises a transcriptional activator domain. Advantageously, the functional domain comprises VP64 or KRAB, SID or SID4X, or a recombinase, a transposase, a histone remodeler, a DNA methyltransferase, a cryptochrome, a light inducible/controllable domain or a chemically inducible/controllable domain. Advantageously, in embodiments involving one or more vectors the composition or CRISPR-Cas system is delivered via single vector. Advantageously, the cell is a eukaryotic cell; or the one or more vectors are operably configured to direct expression of CRISPR transcripts when introduced into a eukaryotic cell. Advantageously, the nucleotide sequence encoding the SaCas9 is codon optimized for expression in a eukaryotic cell. Advantageously, the regulatory elements comprise a tissue-specific promoter. Advantageously, the tissue-specific promoter directs expression in muscle, neuron, bone, skin, blood, liver, pancreas, or lymphocytes. Advantageously, the target sequence is adjacent to a Protospacer Adjacent Motif (PAM) recognized by the Cas9 enzyme. Advantageously, the target sequence is flanked at its 3' end by 5'-NRG (where N is any Nucleotide) for SpCas9 or NNGRR for SaCas9. Advantageously, the guide sequence is capable of hybridizing to a target sequence in a eukaryotic cell. Advantageously, the tracrRNA sequence is 30 or more nucleotides in length. Advantageously, the tracrRNA is 50 or more nucleotides in length. Advantageously, the Cas9 enzyme, e.g., SaCas9 enzyme further comprises one or more nuclear localization sequences (NLSs). Advantageous aspects mentioned in this paragraph can apply mutatis mutandis to other embodiments discussed herein.

The disclosure also comprehends an *in vivo, ex vivo* or *in vitro* host cell or cell line comprising or modified by a composition or enzyme or CRISPR-Cas system discussed herein, as well as progeny thereof, e.g., a stem cell or stem cell line. The disclosure also comprehends a method of modifying an organism by manipulation of one or more target sequences at genomic loci of interest comprising delivering to the organism the composition or enzyme or CRISPR-Cas system discussed herein. The invention further provides an *in vivo* or *ex vivo* method of modifying a cell of an organism by manipulation of one or more target sequences at genomic loci of interest comprising delivering to the cell a non-naturally occurring or engineered composition comprising a vector composition operably encoding a composition or enzyme or CRISPR-Cas system discussed herein according to any herein embodiment. The organism is a plant or algae. A composition or enzyme or CRISPR-Cas system discussed herein according to any herein embodiment can be used in medicine or for use in therapy, e.g., in the preparation of a medicament; in the preparation of a medicament for *ex vivo* gene or genome editing; or in *ex vivo* gene or genome editing. The invention also comprehends a composition or enzyme or CRISPR-Cas system discussed herein according to any herein embodiment for or in use, or methods involving the use thereof or any herein-mentioned use to treat, address, minimize symptoms of, alleviate, or correct ocular defects, e.g., that arise from genetic mutations. The disclosure further comprehends products enabled by the instant invention, e.g., improved or altered cells, expression products such as improved or altered expression products or plants or non-human animals or cells having traits from the practice of the invention.

Accordingly, in certain embodiments the disclosure provides a method of modifying an organism or a non-human organism by manipulation of a first and a second target sequence on opposite strands of a DNA duplex in a genomic locus of interest in a cell comprising

delivering a non-naturally occurring or engineered composition comprising :

I. a first CRISPR-Cas system chimeric RNA (chiRNA) polynucleotide sequence, wherein the first polynucleotide sequence comprises:

(a) a first guide sequence capable of hybridizing to the first target sequence,
(b) a first tracr mate sequence, and
(c) a first tracr sequence,

II. a second CRISPR-Cas system chiRNA polynucleotide sequence, wherein the second polynucleotide sequence comprises:

(a) a second guide sequence capable of hybridizing to the second target sequence,
(b) a second tracr mate sequence, and
(c) a second tracr sequence, and

III. a polynucleotide sequence encoding a CRISPR enzyme, wherein the CRISPR enzyme is a SpCas9 protein comprising the mutation N863A, or an ortholog thereof (e.g., *S. aureus*) having a mutation corresponding to SpCas9N863A (e.g., *S. aureus* with N580A), and comprising at least one or two or or more nuclear localization sequences,

wherein (a), (b) and (c) are arranged in a 5' to 3' orientation,
wherein when transcribed, the first and the second tracr mate sequence hybridize to the first and second tracr sequence respectively and the first and the second guide sequence direct sequence-specific binding of a first and a second CRISPR complex to the first and second target sequences respectively,
wherein the first CRISPR complex comprises the CRISPR enzyme complexed with (1) the first guide sequence that is hybridizable to the first target sequence, and (2) the first tracr mate sequence that is hybridized to the first tracr sequence,
wherein the second CRISPR complex comprises the CRISPR enzyme complexed with (1) the second guide sequence that is hybridizable to the second target sequence, and (2) the second tracr mate sequence that is hybridized to the second tracr sequence,
wherein the polynucleotide sequence encoding said CRISPR enzyme is DNA or RNA, and
wherein the first guide sequence directs cleavage of one strand of the DNA duplex near the first target sequence and the second guide sequence directs cleavage of the other strand near the second target sequence inducing a double strand break, thereby modifying the organism or the non-human organism, and wherein the first guide sequence directing cleavage of one strand of the DNA duplex near the first target sequence and the second guide sequence directing cleavage of the other strand near the second target sequence result in 3' overhangs.

The present invention can therefore be considered to include a dual nickase or double nickase approach. It will be appreciated that, here and in any other aspect or embodiment of the invention, the first guide sequence directing cleavage of one strand of the DNA duplex near the first target sequence and the second guide sequence directing cleavage of the other strand near the second target sequence in order to result in 3' overhangs may allow for the following: the sequence of the first guide and PAM and the second guide and PAM are selected together and not in isolation so that they are appropriately offset. In other words, the sequence of each of the first and the second guides and PAMs are considered with respect to each other to ensure that they will result in correct positioning of the first and second CRISPR complexes on the target DNA so as to achieve (in concert with the mutant Cas9) the required 3' overhangs. This is achieved by sequence comparison of the target sites on both strands of the DNA duplex with respect to identification of suitable guide and PAM sequences on both strands and their relative positioning (i.e. resulting offset). Careful consideration is usually given to this in any case to reduce off-target effects, albeit it normally only with a single target and PAM sequence.

Preferably, the vector is a viral vector, such as a retroviral, lenti- or baculo- or preferably adeno-viral/adeno-associated viral vectors, but other means of delivery are known (such as yeast systems, microvesicles, gene guns/means of attaching vectors to gold nanoparticles) and are provided. In some embodiments, one or more of the viral or plasmid vectors may be delivered via nanoparticles, exosomes, microvesicles, or a gene-gun.

By manipulation of a target sequence, Applicants also mean the epigenetic manipulation of a target sequence. This may be of the chromatin state of a target sequence, such as by modification of the methylation state of the target sequence (i.e. addition or removal of methylation or methylation patterns or CpG islands), histone modification, increasing or reducing accessibility to the target sequence, or by promoting or reducing 3D folding, or through activation or repression of the gene (its expression) through action on the promoter, enhancer or silencer.

It will be appreciated that where reference is made to a method of modifying an organism, including a prokaryotic organism or a eukaryotic organism such as a plant or an animal, e.g., a mammal including human or a non-human mammal or organism) by manipulation of a target sequence in a genomic locus of interest, this may apply to the organism (or mammal) as a whole or just a single cell or population of cells from that organism (if the organism is multicellular). In the case of humans, for instance, Applicants envisage, *inter alia,* a single cell or a population of cells and these may preferably be modified *ex vivo* and then re-introduced. In this case, a biopsy or other tissue or biological fluid sample may be necessary. Accordingly, methods of cellular therapy are envisaged, where, for example, a single cell or a population of cells is sampled or cultured, wherein that cell or cells is or has been modified *ex vivo* as described herein, and is then re-introduced (sampled cells) or introduced (cultured cells) into the organism. Stem cells, whether embryonic or induce pluripotent or totipotent stem cells, are also particularly preferred in this regard. But, of course, *in vivo* embodiments are also envisaged.

In certain embodiments the disclosure provides a method of modifying an organism or a non-human or non-animal organism by manipulation of a first and a second target sequence on opposite strands of a DNA duplex in a genomic locus of interest in a cell comprising

delivering a non-naturally occurring or engineered composition comprising a vector system comprising one or more vectors comprising

    I. a first regulatory element operably linked to

        (a) a first guide sequence capable of hybridizing to the first target sequence, and
        (b) at least one or more tracr mate sequences,

    II. a second regulatory element operably linked to

        (a) a second guide sequence capable of hybridizing to the second target sequence, and
        (b) at least one or more tracr mate sequences,

    III. a third regulatory element operably linked to an enzyme-coding sequence encoding a CRISPR enzyme, wherein the CRISPR enzyme is a SpCas9 protein comprising the mutation N863A, or an ortholog thereof (e.g., *S. aureus*) having a mutation corresponding to SpCas9N863A (e.g., *S. aureus* with N580A), and
    IV. a fourth regulatory element operably linked to a tracr sequence,

        wherein components I, II, III and IV are located on the same or different vectors of the system,
        when transcribed, the tracr mate sequence hybridizes to the tracr sequence and the first and the second guide sequence directs sequence-specific binding of a first and a second CRISPR complex to the first and second target sequences respectively,
        wherein the first CRISPR complex comprises the CRISPR enzyme complexed with (1) the first guide sequence that is hybridizable to the first target sequence, and (2) the tracr mate sequence that is hybridized to the tracr sequence,
        wherein the second CRISPR complex comprises the CRISPR enzyme complexed with (1) the second guide sequence that is hybridizable to the second target sequence, and (2) the tracr mate sequence that is hybridized to the tracr sequence,
        wherein the polynucleotide sequence encoding the CRISPR enzyme is DNA or RNA, and
        wherein the first guide sequence directs cleavage of one strand of the DNA duplex near the first target sequence and the second guide sequence directs cleavage of the other strand near the second target sequence inducing a double strand break, thereby modifying the organism or the non-human organism, and wherein the first guide sequence directing cleavage of one strand of the DNA duplex near the first target sequence and the second guide sequence directing cleavage of other strand near the second target sequence result in 3' overhangs.

Some methods of the invention can include inducing expression. In some methods of the invention the organism or subject is a eukaryote, including e.g., a plant or an animal (including mammal, including human) or a non-human eukaryote or a non-human animal or a non-human mammal. In some methods of the invention the organism or subject is a plant. In some methods of the invention the organism or subject is a mammal or a non-human mammal. In some methods of the invention the organism or subject is algae. In some methods of the invention the viral vector is an AAV. In some methods of the invention the viral vector is a retrovirus or lentivirus-derived vector. In some methods of the invention the viral vector is an Agrobacterium Ti or Ri plasmid for use in plants. In the methods of the invention the CRISPR enzyme is a mutated Cas9 nickase. In some methods of the invention the expression of the guide sequence is under the control of a T7 promoter that is driven by the expression of T7 polymerase. In some methods of the invention the expression of the guide sequence is under the control of a U6 promoter.

By manipulation of a target sequence, Applicants mean the alteration of the target sequence, which may include the epigenetic manipulation of a target sequence. This epigenetic manipulation may be of the chromatin state of a target sequence, such as by modification of the methylation state of the target sequence (i.e., addition or removal of methylation or methylation patterns or CpG islands), histone modification, increasing or reducing accessibility to the target sequence, or by promoting or reducing 3D folding.

It will be appreciated that where reference is made to a method of modifying an organism or a non-human organism by manipulation of a target sequence in a genomic locus of interest, this may apply to the organism as a whole or just a single cell or population of cells from that organism (if the organism is multicellular). In the case of humans, for instance, Applicants envisage, *inter alia,* a single cell or a population of cells and these may preferably be modified *ex vivo* and then re-introduced. In this case, a biopsy or other tissue or biological fluid sample may be necessary. Stem cells are also particularly preferred in this regard. But, of course, *in vivo* embodiments are also envisaged.

Some methods of the invention can include inducing expression. In some methods of the invention the organism or subject is a eukaryote, including e.g., a plant or an animal (including mammal, including human) or a non-human eukaryote or a non-human animal. In some methods of the invention the organism or subject is a plant. In some methods of the invention the organism or subject is a mammal or a non-human mammal. In some methods of the invention the organism or subject is algae. In some methods of the invention the viral vector is an AAV. In some methods of the invention the viral vector is a retroviral or lentiviral vector. In some methods of the invention the viral vector is a tobacco mosaic virus vector. In the invention the CRISPR enzyme is a mutated Cas9 nickase. In some methods of the invention the expression of the guide sequence is under the control of the T7 promoter is driven by the expression of T7 polymerase. In some methods of the invention the expression of the guide sequence is under the control of a U6 promoter.

The invention in some embodiments comprehends a method of delivering a CRISPR enzyme comprising delivering to a cell mRNA encoding the CRISPR enzyme. The CRISPR enzyme is a mutated Cas9.

The invention in some embodiments comprehends a method of preparing the AAV vector of the invention comprising transfecting one or more plasmid(s) containing or consisting essentially of nucleic acid molecule(s) coding for the AAV into AAV-infectable cells, and supplying AAV rep and/or cap obligatory for replication and packaging of the AAV. In some embodiments the AAV rep and/or cap obligatory for replication and packaging of the AAV are supplied by transfecting the cells with helper plasmid(s) or helper virus(es). In some embodiments the helper virus is a poxvirus, adenovirus, herpesvirus or baculovirus. In some embodiments the poxvirus is a vaccinia virus. In some embodiments the cells are mammalian cells. And in some embodiments the cells are insect cells and the helper virus is baculovirus.

In plants, pathogens are often host-specific. For example, *Fusarium oxysporum* f. sp. *lycopersici* causes tomato wilt but attacks only tomato, and *F. oxysporum f. dianthii Puccinia graminis* f. sp. *tritici* attacks only wheat. Plants have existing and induced defenses to resist most pathogens. Mutations and recombination events across plant generations lead to genetic variability that gives rise to susceptibility, especially as pathogens reproduce with more frequency than plants. In plants there can be non-host resistance, e.g., the host and pathogen are incompatible. There can also be Horizontal Resistance, e.g., partial resistance against all races of a pathogen, typically controlled by many genes and Vertical Resistance, e.g., complete resistance to some races of a pathogen but not to other races, typically controlled by a few genes. In a Gene-for-Gene level, plants and pathogens evolve together, and the genetic changes in one balance changes in other. Accordingly, using Natural Variability, breeders combine most useful genes for Yield, Quality, Uniformity, Hardiness, Resistance. The sources of resistance genes include native or foreign Varieties, Heirloom Varieties, Wild Plant Relatives, and Induced Mutations, e.g., treating plant material with mutagenic agents. Using the present disclosure, plant breeders are provided with a new tool to induce mutations. Accordingly, one skilled in the art can analyze the genome of sources of resistance genes, and in Varieties having desired characteristics or traits employ the present invention to induce the rise of resistance genes, with more precision than previous mutagenic agents and hence accelerate and improve plant breeding programs.

The invention comprehends a composition for use in medicine, as defined in the claims. In the invention the CRISPR enzyme comprises a mutation in the catalytic HNH domain (N863A). The CRISPR enzyme is a Cas9 nickase. The invention comprehends in some embodiments a composition of the invention or a CRISPR enzyme thereof (including or alternatively mRNA encoding the CRISPR enzyme), wherein the target sequence is flanked at its 3' end by a 5'-motif termed a proto-spacer adjacent motif (PAM), especially where the Cas9 is (or is derived from) *S. pyogenes* or *S. aureus* Cas9. For example, a suitable PAM is 5'-NRG or 5'-NNGRR or 5'-NNGRRT (where N is any Nucleotide) for SpCas9 or SaCas9 enzymes (or derived enzymes), respectively, as mentioned below.

It will be appreciated that SpCas9 or SaCas9 are those from or derived from *S. pyogenes* or *S. aureus* Cas9.

In some methods of the invention any or all of the polynucleotide sequences encoding the CRISPR enzyme, the first and the second guide sequence, the first and the second tracr mate sequence or the first and the second tracr sequence, is/are RNA. In further embodiments of the invention the polynucleotides comprising the sequence encoding the CRISPR enzyme, the first and the second guide sequence, the first and the second tracr mate sequence or the first and the second tracr sequence, is/are RNA and are delivered via nanoparticles, exosomes, microvesicles, or a gene-gun. In certain embodiments of the invention, the first and second tracr mate sequence share 100% identity and/or the first and second tracr sequence share 100% identity. In the invention the CRISPR enzyme is a mutated Cas9 nickase, e.g. mutated SpCas9. In the invention the CRISPR enzyme comprises a mutation in one of the catalytic domains, wherein the mutation is N863A in the HNH domain.

In the embodiments of the invention the 3' overhang is at most 150, 100 or 25 base pairs or at least 15, 10 or 1 base pairs. In preferred embodiments the 3' overhang is 1-100 basepairs.

The invention in some embodiments comprehends a method of modifying a genomic locus of interest by introducing into a cell containing and expressing a double stranded DNA molecule encoding the gene product an engineered, non-naturally occurring CRISPR-Cas system comprising SpCas9 protein comprising the mutation N863A, or an ortholog thereof (e.g., *S. aureus*) having a mutation corresponding to SpCas9N863A (e.g., *S. aureus* with N580A), and two guide RNAs that target a first strand and a second strand of the DNA molecule respectively, whereby the guide RNAs target the DNA molecule encoding the gene product and the Cas protein nicks each of the first strand and the second strand of the DNA molecule encoding the gene product; wherein the Cas protein and the two guide RNAs do not naturally occur together; and wherein the Cas protein nicking each of the first strand and the second strand of the DNA molecule encoding the gene product result in 3' overhangs.

In some methods of the invention any or all of the polynucleotide sequence encoding the CRISPR enzyme, the first and the second guide sequence, the first and the second tracr mate sequence or the first and the second tracr sequence, is/are RNA. In further embodiments of the invention the first and second tracr mate sequence share 100% identity and/or the first and second tracr sequence share 100% identity. In the invention the CRISPR enzyme is a mutated Cas9 nickase, e.g. mutated SpCas9 (N863A) or SaCas9 (N580A).

Although Alanine is preferred as the replacement residue in the mutant, other alternatives are available, provided that they are catalytically inactive and so retain the nickase function of the Cas9 and result in the 3' overhang. Suitable guidance is given below, but preferred alternatives to Alanine, may include, in some embodiments, other small and non-polar amino acids. These may include Glycine, Valine, Leucine or Isoleucine, so in SpCas9 this would translate to N863G, N863V, N863L or N863I; and in SaCas9 would translate to N580G, N580V, N580L or N580I.

In a further embodiment of the invention, one or more of the viral vectors are delivered via nanoparticles, exosomes, microvesicles, or a gene-gun.

The invention in some embodiments comprehends a method of modifying a genomic locus of interest by minimizing off-target modifications by introducing into a cell containing and expressing a double stranded DNA molecule encoding the gene product an engineered, non-naturally occurring CRISPR-Cas system comprising a mutated Cas protein having one mutation and two guide RNAs that target a first strand and a second strand of the DNA molecule respectively, whereby the guide RNAs target the DNA molecule encoding the gene product and the Cas protein nicks each of the first strand and the second strand of the DNA molecule encoding the gene product, whereby expression of the gene product is altered; and, wherein the Cas protein and the two guide RNAs do not naturally occur together. The Cas9 mutant is N863A SpCas9 or orthologs having corresponding mutations.

Aspects of the invention relate to the expression of the gene product being decreased or a template polynucleotide being further introduced into the DNA molecule encoding the gene product or an intervening sequence being excised by allowing the two 3' overhangs to reanneal and ligate or the activity or function of the gene product being altered or the expression of the gene product being increased. In an embodiment of the invention, the gene product is a protein. The excision of the intervening sequence can have precision through the use of the 3' overhangs. Thus, the invention envisions an intervening sequence being precisely excised by allowing the two 3' overhangs to reanneal and ligate or the activity or function of the gene product being altered or the expression of the gene product being increased.

The disclosure also comprehends an engineered, non-naturally occurring CRISPR-Cas system comprising a Cas protein having one mutation and two guide RNAs that target a first strand and a second strand respectively of a double stranded DNA molecule encoding a gene product in a cell, whereby the guide RNAs target the DNA molecule encoding the gene product and the Cas protein nicks each of the first strand and the second strand of the DNA molecule encoding the gene product, whereby expression of the gene product is altered; and, wherein the mutated Cas protein and the two guide RNAs do not naturally occur together. The Cas9 mutant is preferably the N863 SpCas9, N863A SpCas9, N580 SaCas9, or N580A SaCas9, or orthologs having corresponding mutations.

In aspects of the invention the guide RNAs may comprise a guide sequence fused to a tracr mate sequence and a tracr sequence, i.e. a chimeric guide. In other embodiments, the guide RNA is not a chimeric guide. For example, the guide RNA may comprise a guide sequence fused (at its 3' end) to (the 5' end of) a tracr mate sequence, with the tracrRNA being provided separately.

It will be appreciated that the terms tracrRNA and tracr sequence can be used interchangeably herein.

Aspects of the invention relate to the expression of the gene product being decreased or a template polynucleotide being further introduced into the DNA molecule encoding the gene product or an intervening sequence being excised by allowing the two 3' overhangs to reanneal and ligate or the activity or function of the gene product being altered or the expression of the gene product being increased. In an embodiment of the invention, the gene product is a protein. The excision of the intervening sequence can have precision through the use of the 3' overhangs. Thus, the invention envisions an intervening sequence being precisely excised by allowing the two 3' overhangs to reanneal and ligate or the activity or function of the gene product being altered or the expression of the gene product being increased.

The invention also comprehends an engineered, non-naturally occurring CRISPR-Cas system comprising SpCas9 protein comprising the mutation N863A, or an ortholog thereof having a mutation corresponding to SpCas9 N863A (e.g., *S. aureus* N580A), and two guide RNAs that target a first strand and a second strand respectively of a double stranded DNA molecule encoding a gene product in a cell, whereby the guide RNAs target the DNA molecule encoding the gene product and the Cas protein nicks each of the first strand and the second strand of the DNA molecule encoding the gene product; and, wherein the Cas protein and the two guide RNAs do not naturally occur together; and wherein the Cas protein nicking each of the first strand and the second strand of the DNA molecule encoding the gene product results in 3' overhangs.

The disclosure also comprehends an engineered, non-naturally occurring vector system comprising one or more vectors comprising:

a) a first regulatory element operably linked to each of two CRISPR-Cas system guide RNAs that target a first strand and a second strand respectively of a double stranded DNA molecule encoding a gene product,
b) a second regulatory element operably linked to a polynucleotide sequence encoding SpCas9 protein comprising the mutation N863A, or an ortholog thereof having a mutation corresponding to SpCas9N863A (e.g., *S. aureus* N580A),

wherein components (a) and (b) are located on same or different vectors of the system,
whereby the guide RNAs target the DNA molecule encoding the gene product and the Cas protein nicks each of the first strand and the second strand of the DNA molecule encoding the gene product; and, wherein the Cas protein and the two guide RNAs do not naturally occur together; wherein the Cas protein nicking each of the first strand and the second strand of the DNA molecule encoding the gene product results in 3' overhangs.

Aspects of the disclosure provide for methods of modifying an organism comprising a first and a second target sequence on opposite strands of a DNA duplex in a genomic locus of interest in a cell by promoting homology directed repair comprising

delivering a non-naturally occurring or engineered composition comprising :

I. a first CRISPR-Cas system chimeric RNA (chiRNA) polynucleotide sequence, wherein the first polynucleotide sequence comprises:

(a) a first guide sequence capable of hybridizing to the first target sequence,
(b) a first tracr mate sequence, and
(c) a first tracr sequence,

II. a second CRISPR-Cas system chiRNA polynucleotide sequence, wherein the second polynucleotide sequence comprises:

(a) a second guide sequence capable of hybridizing to the second target sequence,
(b) a second tracr mate sequence, and
(c) a second tracr sequence, and

III. a polynucleotide sequence encoding a CRISPR enzyme, wherein the CRISPR enzyme is a SpCas9 protein comprising the mutation N863A, or an ortholog thereof having a mutation corresponding to SpCas9N863A (e.g., *S. aureus* N580A), comprising at least one or two or or more nuclear localization sequences,
IV. a repair template comprising a synthesized or engineered single-stranded oligonucleotide,

wherein (a), (b) and (c) are arranged in a 5' to 3' orientation,
wherein when transcribed, the first and the second tracr mate sequence hybridize to the first and second tracr sequence respectively and the first and the second guide sequence directs sequence-specific binding of a first and a second CRISPR complex to the first and second target sequences respectively,
wherein the first CRISPR complex comprises the CRISPR enzyme complexed with (1) the first guide sequence that is hybridizable to the first target sequence, and (2) the first tracr mate sequence that is hybridized to the first tracr sequence,
wherein the second CRISPR complex comprises the CRISPR enzyme complexed with (1) the second guide sequence that is hybridizable to the second target sequence, and (2) the second tracr mate sequence that is hybridized to the second tracr sequence,
wherein the polynucleotide sequence encoding the CRISPR enzyme is DNA or RNA,
wherein the first guide sequence directs cleavage of one strand of the DNA duplex near the first target sequence and the second guide sequence directs cleavage of the other strand near the second target sequence inducing a double strand break; wherein the first guide sequence directing cleavage of one strand of the DNA duplex near the first target sequence and the second guide sequence directing cleavage of the other strand near the second target sequence result in 3' overhangs and wherein the repair template is introduced into the DNA duplex by homologous recombination, whereby the organism is modified.

In embodiments of the invention the repair template may further comprise a restriction endonuclease restriction site. In further embodiments, the first guide sequence directing cleavage of one strand of the DNA duplex near the first target sequence and the second guide sequence directing cleavage of other strand near the second target sequence results in a 3' overhang. In preferred embodiments of the invention, the 3' overhang is 1-100 base pairs. In other aspects, any or all of the polynucleotide sequence encoding the mutated CRISPR enzyme, the first and the second guide sequence, the first and the second tracr mate sequence or the first and the second tracr sequence, is/are RNA. In yet further aspects the polynucleotides comprising the sequence encoding the CRISPR enzyme, the first and the second guide sequence, the first and the second tracr mate sequence or the first and the second tracr sequence, is/are RNA and are delivered via nanoparticles, exosomes, microvesicles, or a gene-gun. In further embodiments of the invention the first and second tracr mate sequence share 100% identity and/or the first and second tracr sequence share 100% identity. In the embodiments of the invention the mutated CRISPR enzyme is a mutated Cas9 enzyme, e.g. mutated SpCas9 (N863A) or *S. aureus* N580A.

Aspects of the disclosure also provide for methods of modifying a DNA duplex at a locus of interest in a cell, the method comprising delivering to the cell:

I. a first polynucleotide comprising:

(a) a first guide sequence capable of hybridizing to a first target sequence,
(b) a first tracr mate sequence, and
(c) a first tracr sequence;

II. a second polynucleotide comprising:

(a) a second guide sequence capable of hybridizing to a second target sequence,
(b) a second tracr mate sequence, and
(c) a second tracr sequence;

and

III. a third polynucleotide comprising a sequence encoding a CRISPR enzyme, wherein the CRISPR enzyme is a SpCas9 protein comprising the mutation N863A, or an ortholog thereof having a mutation corresponding to SpCas9N863A (e.g., *S. aureus* N580A), and one or two or more nuclear localization sequences

wherein (a), (b) and (c) in said first and second polynucleotides are arranged in a 5' to 3' orientation;
wherein the first target sequence is on a first strand of the DNA duplex and the second target sequence is on the opposite strand of the DNA duplex, and when the first and second guide sequences are hybridized to said target sequences in the duplex, the 5' ends of the first polynucleotide and the second polynucleotide are offset relative to each other by at least one base pair of the duplex;
wherein when transcribed, the first and the second tracr mate sequences hybridize to the first and second tracr sequences, respectively, and the first and the second guide sequences direct sequence-specific binding of a first and a second CRISPR complex to the first and second target sequences respectively,
wherein the first CRISPR complex comprises the CRISPR enzyme complexed with (1) the first guide sequence that is hybridizable to the first target sequence, and (2) the first tracr mate sequence that is hybridized to the first tracr sequence,
wherein the second CRISPR complex comprises the CRISPR enzyme complexed with (1) the second guide sequence that is hybridizable to the second target sequence, and (2) the second tracr mate sequence that is hybridized to the second tracr sequence,
and wherein said first strand of the DNA duplex is cleaved near said first target sequence, and said opposite strand of the DNA duplex is cleaved near said second target sequence, resulting in a double strand break with 3' overhangs.

In some embodiments of the invention the repair template is a synthesized or engineered double-stranded oligonucleotide duplex or in other embodiments the repair template is generated from a piece of DNA that is introduced into the cell and is enzymatically processed. This enzymatic processing may be carried out by endogenous enzymes or by enzymes (e.g. restriction endonucleases, nucleases or a pair of nickases) that have been introduced into the cell so the compatible overhangs are generated on the repair template.

In one aspect, the disclosure provides a method of modifying a target polynucleotide in a eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a mutated CRISPR enzyme complexed with a guide sequence hybridized to a target sequence within said target polynucleotide, wherein said guide sequence is linked to a tracr mate sequence which in turn hybridizes to a tracr sequence. In some embodiments, said cleavage comprises cleaving one or two strands at the location of the target sequence by said mutated CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expressed from a gene comprising the target sequence. In some embodiments, the method further comprises delivering one or more vectors to said eukaryotic cell, wherein the one or more vectors drive expression of one or more of: the mutated CRISPR enzyme, the guide sequence linked to the tracr mate sequence, and the tracr sequence. In some embodiments, said vectors are delivered to the eukaryotic cell in a subject. In some embodiments, said modifying takes place in said eukaryotic cell in a cell culture. In some embodiments, the method further comprises isolating said eukaryotic cell from a subject prior to said modifying. In some embodiments, the method further comprises returning said eukaryotic cell and/or cells derived therefrom to said subject.

In one aspect, the disclosure provides a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a mutated CRISPR enzyme complexed with a guide sequence hybridized to a target sequence within said polynucleotide, wherein said guide sequence is linked to a tracr mate sequence which in turn hybridizes to a tracr sequence. In some embodiments, the method further comprises delivering one or more vectors to said eukaryotic cells, wherein the one or more vectors drive expression of one or more of: the mutated CRISPR enzyme, the guide sequence linked to the tracr mate sequence, and the tracr sequence.

In one aspect, the disclosure provides a method of generating a model eukaryotic cell comprising a mutated disease gene. In some embodiments, a disease gene is any gene associated an increase in the risk of having or developing a disease. In some embodiments, the method comprises (a) introducing one or more vectors into a eukaryotic cell, wherein the one or more vectors drive expression of one or more of: a CRISPR enzyme, a guide sequence linked to a tracr mate sequence, and a tracr sequence; and (b) allowing a CRISPR complex to bind to a target polynucleotide to effect cleavage of the target polynucleotide within said disease gene, wherein the CRISPR complex comprises the CRISPR enzyme complexed with (1) the guide sequence that is hybridizable to the target sequence within the target polynucleotide, and (2) the tracr mate sequence that is hybridized to the tracr sequence, thereby generating a model eukaryotic cell comprising a mutated disease gene. In some embodiments, said cleavage comprises cleaving one or two strands at the location of the target sequence by said CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expression from a gene comprising the target sequence.

In one aspect the disclosure provides for a method of selecting one or more prokaryotic cell(s) by introducing one or more mutations in a gene in the one or more prokaryotic cell (s), the method comprising: introducing one or more vectors into the prokaryotic cell (s), wherein the one or more vectors drive expression of one or more of: a CRISPR enzyme, a guide sequence linked to a tracr mate sequence, a tracr sequence, and an editing template; wherein the editing template comprises the one or more mutations that abolish CRISPR enzyme cleavage; allowing homologous recombination of the editing template with the target polynucleotide in the cell(s) to be selected; allowing a CRISPR complex to bind to a target polynucleotide to effect cleavage of the target polynucleotide within said gene, wherein the CRISPR complex comprises the CRISPR enzyme complexed with (1) the guide sequence that is hybridizable to the target sequence within the target polynucleotide, and (2) the tracr mate sequence that is hybridized to the tracr sequence, wherein binding of the CRISPR complex to the target polynucleotide induces cell death, thereby allowing one or more prokaryotic cell(s) in which one or more mutations have been introduced to be selected. In a preferred embodiment, the CRISPR enzyme is Cas9. In another aspect of the disclosure the cell to be selected may be a eukaryotic cell. Aspects of the invention allow for selection of specific cells without requiring a selection marker or a two-step process that may include a counter-selection system.

In one aspect, the disclosure provides for methods of modifying a target polynucleotide in a eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence.

In other embodiments, this disclosure provides a method of modifying expression of a polynucleotide in a eukaryotic cell. The method comprises increasing or decreasing expression of a target polynucleotide by using a CRISPR complex that binds to the polynucleotide.

Where desired, to effect the modification of the expression in a cell, one or more vectors comprising a tracr sequence, a guide sequence linked to the tracr mate sequence, a sequence encoding a CRISPR enzyme is delivered to a cell. In some methods, the one or more vectors comprises a regulatory element operably linked to an enzyme-coding sequence encoding said CRISPR enzyme comprising a nuclear localization sequence; and a regulatory element operably linked to a tracr mate sequence and one or more insertion sites for inserting a guide sequence upstream of the tracr mate sequence. When expressed, the guide sequence directs sequence-specific binding of a CRISPR complex to a target sequence in a cell. Typically, the CRISPR complex comprises a CRISPR enzyme complexed with (1) the guide sequence that is hybridizable to the target sequence, and (2) the tracr mate sequence that is hybridized to the tracr sequence.

In some methods, a target polynucleotide can be inactivated to effect the modification of the expression in a cell. For example, upon the binding of a CRISPR complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein is not produced.

In some embodiments, the mutated CRISPR enzyme has a mutation in the catalytic HNH domain (N863A in Sp or N580A in Sa or a corresponding mutation in an ortholog), wherein when transcribed, the tracr mate sequence hybridizes to the tracr sequence and the guide sequence directs sequence-specific binding of a CRISPR complex to the target sequence, and wherein the enzyme further comprises a functional domain. In some embodiments, the mutated Cas9 enzyme may be fused to a protein domain, e.g., such as a transcriptional activation domain. In one aspect, a transcriptional activation domain is VP64. In some embodiments, a transcription repression domains is KRAB. In some embodiments, a transcription repression domain is SID, or concatemers of SID (i.e. SID4X). In some embodiments, an epigenetic modifying enzyme is provided. In some embodiments, an activation domain is provided, which may be the P65 activation domain. Further functional domains are also described herein.

The disclosure also provides a method of modifying a DNA duplex at a locus of interest in a cell, the method comprising delivering to the cell a vector system comprising one or more vectors comprising:

I. a first polynucleotide sequence comprising a regulatory element operably linked to

(a) a first guide sequence capable of hybridizing to a first target sequence, and
(b) at least one or more tracr mate sequences,

II. a second polynucleotide sequence comprising a second regulatory element operably linked to

(a) a second guide sequence capable of hybridizing to a second target sequence, and
(b) at least one or more tracr mate sequences,

III. a third polynucleotide sequence comprising a third regulatory element operably linked to a sequence encoding a CRISPR enzyme, wherein the CRISPR enzyme is a SpCas9 protein comprising the mutation N863A, or an ortholog thereof having a mutation corresponding to SpCas9N863A (e.g., *S. aureus* N580A), and
IV. a fourth polynucleotide sequence comprising a fourth regulatory element operably linked to a tracr sequence,

wherein components I, II, III and IV are located on the same or different vectors of the system
wherein the first target sequence is on a first strand of the DNA duplex and the second target sequence is on the opposite strand of the DNA duplex, and when the first and second guide sequences are hybridized to said target sequences in the duplex, the 5' ends of the first polynucleotide and the second polynucleotide are offset relative to each other by at least one base pair of the duplex;
wherein when transcribed, the first and the second tracr mate sequences hybridize to a tracr sequence, and the first and the second guide sequences direct sequence-specific binding of a first and a second CRISPR complex to the first and second target sequences respectively,
wherein the first CRISPR complex comprises the CRISPR enzyme complexed with (1) the first guide sequence that is hybridizable to the first target sequence, and (2) the first tracr mate sequence that is hybridized to a tracr sequence,
wherein the second CRISPR complex comprises the CRISPR enzyme complexed with (1) the second guide sequence that is hybridizable to the second target sequence, and (2) the second tracr mate sequence that is hybridized to a tracr sequence,
and wherein said first strand of the DNA duplex is cleaved near said first target sequence, and said opposite strand of the DNA duplex is cleaved near said second target sequence, resulting in a double strand break with 3' overhangs.

Advantageously in inventive methods, any or all of the polynucleotide sequence encoding the mutated CRISPR enzyme, the first and the second guide sequence, the first and the second tracr mate sequence or the first and the second tracr sequence, is/are RNA, and optionally wherein any or all of I, II and III are delivered via nanoparticles, exosomes, microvesicles, or a gene-gun. In inventive methods advantageously the first and second tracr mate sequence can share 100% identity and/or the first and second tracr sequence share 100% identity. For instance, each of I, II and III can be provided in a vector, optionally wherein each is provided in the same or a different vector. The locus of interest in inventive methods can comprises a gene and wherein said method results in a change in the expression of said gene, or in a change in the activity or function of the gene product. For instance, the gene product can be a protein, and/or wherein said change in expression, activity or function is a reduction in said expression, activity or function.

Inventive methods can further comprise delivery of templates, such as repair templates, which may be dsODN or ssODN, see below. Delivery of templates may be via the cotemporaneous or separate from delivery of any or all the CRISPR enzyme, guide, tracr mate or tracrRNA and via the same delivery mechanism or different. In some embodiments, it is preferred that the template is delivered together with the guide, tracr mate and/or tracrRNA and, preferably, also the CRISPR enzyme. An example may be an AAV vector where the CRISPR enzyme is SaCas9 (with the N580 mutation).

Inventive methods can further comprise: (a) delivering to the cell a double-stranded oligodeoxynucleotide (dsODN) comprising overhangs complimentary to the overhangs created by said double strand break, wherein said dsODN is integrated into the locus of interest; or -(b) delivering to the cell a single-stranded oligodeoxynucleotide (ssODN), wherein said ssODN acts as a template for homology directed repair of said double strand break. Inventive methods can be for the prevention or treatment of disease in an individual, optionally wherein said disease is caused by a defect in said locus of interest. Methods of the disclosure can be conducted *in vivo* in the individual or *ex vivo* on a cell taken from the individual, optionally wherein said cell is returned to the individual.

The disclosure also provides a kit or composition comprising:

> I. a first polynucleotide comprising:
>
>> (a) a first guide sequence capable of hybridizing to a first target sequence,
>> (b) a first tracr mate sequence, and
>> (c) a first tracr sequence;
>
> II. a second polynucleotide comprising:
>
>> (a) a second guide sequence capable of hybridizing to a second target sequence,
>> (b) a second tracr mate sequence, and
>> (c) a second tracr sequence;
>
> and
> III. a third polynucleotide comprising a sequence encoding a CRISPR enzyme, wherein the CRISPR enzyme is a SpCas9 protein comprising the mutation N863A, or an ortholog thereof having a mutation corresponding to SpCas9N863A (e.g., *S. aureus* N580A), and one or two or more nuclear localization sequences
>
>> wherein (a), (b) and (c) in said first and second polynucleotides are arranged in a 5' to 3' orientation;
>> wherein the first target sequence is on a first strand of a DNA duplex and the second target sequence is on the opposite strand of the DNA duplex, and when the first and second guide sequences are hybridized to said target sequences in the duplex, the 5' ends of the first polynucleotide and the second polynucleotide are offset relative to each other by at least one base pair of the duplex,
>> and optionally wherein each of I, II and III is provided in the same or a different vector; and wherein the first guide sequence directing cleavage of one strand of the DNA duplex near the first target sequence and the second guide sequence directing cleavage of the other strand near the second target sequence result in 3' overhangs.

The disclosure also provides use of a kit or composition according of the invention in a method of the disclosure. The disclosure also provides use of a kit or composition of the disclosure in the manufacture of a medicament, optionally wherein said medicament is for the prevention or treatment of a disease caused by a defect in said locus of interest.

An example of a codon optimized sequence, in this instance optimized for humans (i.e. being optimized for expression in humans), is provided herein, see the SaCas9 human codon optimized sequence. Whilst this is preferred, it will be appreciated that other examples are possible and codon optimization for a host species other than human, or for codon optimization for specific organs such as the brain, is known.

In one aspect, delivery is in the form of a vector. In one aspect the vector may be a viral vector, such as a retro-, lenti- or baculo- or preferably adeno-viral/adeno-associated viral vectors, but other means of delivery are known (such as yeast systems, microvesicles, gene guns/means of attaching vectors to gold nanoparticles) and are provided. A vector may mean not only a viral or yeast system (for instance, where the nucleic acids of interest may be operably linked to and under the control (in terms of expression, such as to ultimately provide a processed RNA) a promoter), but also direct delivery of nucleic acids into a host cell. While in herein methods the vector may be a viral vector and this is advantageously an AAV, other viral vectors as herein discussed can be employed. For example, baculoviruses may be used for expression in insect cells. These insect cells may, in turn be useful for producing large quantities of further vectors, such as AAV vectors adapted for delivery of the present invention. Also envisaged is a method of delivering the present mutated CRISPR enzyme comprising delivering to a cell mRNA encoding the mutated CRISPR enzyme. It will be appreciated that the CRISPR enzyme is truncated, comprised of less than one thousand amino acids or less than four thousand amino acids, is a nuclease or nickase, is codon-optimized comprises one or more mutations, and/or comprises a chimeric CRISPR enzyme, or the other options as herein discussed. AAV viral vectors are preferred, especially for delivery of SaCas9 mutants.

In certain embodiments, the target sequence is flanked or followed, at its 3' end, by a PAM suitable for the CRISPR enzyme, typically a Cas and in particular a Cas9.

For example, a suitable PAM is 5'-NRG for SpCas9 (or derived enzymes), or 5'-NNGRR or 5'-NNGRRT for SaCas9 enzymes (or derived enzymes)..

It will be appreciated that SpCas9 or SaCas9 are those from or derived from *S. pyogenes* or *S. aureus* Cas9, including *S. aureus* subspecies *aureus.*

Accordingly, in certain embodiments or aspects of the disclosure is said to provide a method of modifying an organism (or a non-human organism), for example by manipulation of a first and a second target sequence on opposite strands of a DNA duplex in a genomic locus of interest in a cell comprising delivering a non-naturally occurring or engineered composition. In such embodiments or aspects, it will be appreciated that the organism is not an animal. In some embodiments or aspects, it will be appreciated that provided is a composition for use in a method of genetic or genome engineering or for use in a method of modifying an organism (or a non-human or non-animal organism), for example by manipulation of a first and a second target sequence on opposite strands of a DNA duplex in a genomic locus of interest in a cell. The use here may, in some embodiments, be by (or comprise) delivering the composition.

The term "non-naturally occurring or engineered" for example in respect of a composition is optional and, where present, may, in certain embodiments, be substituted or removed.

The current invention is based on several technical effects, which are, inter alia, generally defined by one or more of: optimized double nicking; generation of 3' overhangs; inhibition of NHEJ; and improved HDR efficiency.

The current invention is based the technical effect of improved HDR efficiency using SpCas9N863A mutant or an ortholog thereof having a mutation corresponding to SpCas9N863A, such as *S. aureus* N580A. Specifically, the improved HDR efficiency is the result of inhibition of NHEJ events and thus a bias (i.e. increase) in HDR events.

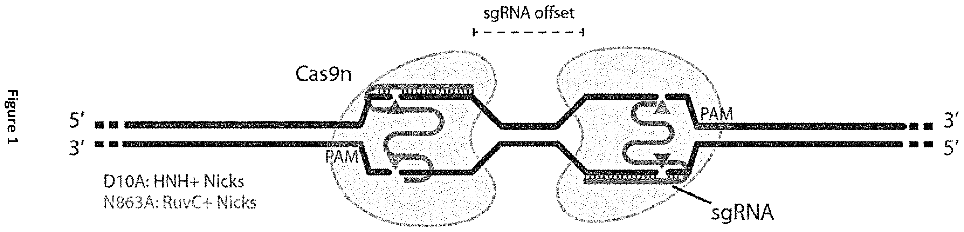

The current invention is also based on the technical effect of optimized double nicking due to optimal target sequence selection so that the 5' PAM sequences face away from one another. PAMs facing away from each are shown in Figure 1. Each Cas9 is recruited to a genomic locus by a guide sequence binding to a target sequence in the genome. The target sequence and the PAM are found nearby but on opposite stands of the DNA (and hence the PAMs face away from each other). The PAMs associate with PI (PAM Interacting) domains on the Cas9. The guide sequences are selected with a view to the positioning of the PAM as the PAM is also crucial to effective Cas9 recruitment. Thus, the PAMs (and so the guide sequences) are therefore optimally selected in the present nicking (i.e. double nicking) system such that the PAMs are distal to each other. This is distal in terms of the opposite to proximal. PAMs may be considered to face way or be distal to each other if the number of nucleotides between them is at least 10, at least 15, at least 20, at least 25, at least 30, at least 35, or at least 40 or more nucleotides between them. This may be counted in the 3' to 5' direction from one PAM, along its DNA strand until one is opposite the other PAM (or until one comes to the complementary sequence corresponding to the other PAM (on the other DNA strand)). In Figure 1 it can be seen that the PAMs are found in this distal arrangement. As such, the guide offset is preferably positive. It would be negative if the PAMs were proximal to each other, for example where the two Cas9s in Figure 1 were swapped around (subject to at least one set of guide and PAM changes). In other words, again with reference to Figure 1, with each sgRNA arranged 5' to 3' on opposite strands, the PAMs face away from each other as from left to right one strand goes 3' to 5' and the other goes 5' to 3'. As such, the 3' ends of each PAM point away from each other, i.e. are distal to (furthest away from) each other, whilst the 5' ends of each PAM point towards each other, i.e. are proximal (nearest to each other). If the PAMs did not face away from each other, the 5' ends of each PAM would point towards each other, i.e. would be distal to each other, whilst the 3' ends of each PAM would point towards each other, i.e. be proximal. Put another way, the PAMs face away if the 3' end of one PAM points away from the 3' end of the other PAM, whilst the 5' end of one PAM points towards the 5' end of the other PAM

Optimal 3' overhang lengths are described herein, but range from 1 to 10, 20, 30, 40, 50, 60, 70, 80, 90, or 100, such as 1 to 3, 4, 5, 6, 7, 8, 9, 10, 11, 12, 13,14, 15, 16, 17, 18, 19, 20, 21, 22, 23, 24, 25, 26, 27, 28, 29, 30, 31, 32, 33, 34, 35, 36, 37, 38, 39, 40, 41, 42 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 nucleotides on each 3' overhanging end. The offset between the 5' end of each of guide pair is, in some embodiments 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 nucleotides. Ranges of around 15-60, 16-60, 17-60, 18-60, 19-60, 20-60, 21-60, 22-60, 23-60, 24-60, 25-60, 15-55, 16-55, 17-55, 18-55, 19-55, 20-55, 21-55, 22-55, 23-55, 24-, 25-55, 35-60, 15-40, 16-40, 17-40, 18-40, 19-40, 20-40, 21-40, 22-40, 23-40, 24-40, 25-40, 15-45, 16-45, 17-45, 18-45, 19-45, 20-45, 21-45, 22-45, 23-45, 24-45, 25-45, 30-50, 35-55, and especially 35-45 are also preferred in some embodiments.

In some embodiments, phenotypic alteration is preferably the result of genome modification when a genetic disease is targeted, especially in methods of therapy and preferably where a repair template is provided to correct or alter the phenotype.

In some embodiments diseases that may be targeted include those concerned with disease-causing splice defects.

In some embodiments, cellular targets include Hemopoietic Stem/Progenitor Cells (CD34+); Human T cells; and Eye (retinal cells) - for example photoreceptor precursor cells.

In some embodiments Gene targets include: Human Beta Globin - HBB (for treating Sickle Cell Anemia, including by stimulating gene-conversion (using closely related HBD gene as an endogenous template)); CD3 (T-Cells); and CEP920 - retina (eye).

In some embodiments disease targets also include: cancer; Sickle Cell Anemia (based on a point mutation); HIV; Beta-Thalassemia; and ophthalmic disease - for example Leber Congenital Amaurosis (LCA)-causing Splice Defect.

In some embodiments delivery methods include: Cationic Lipid Mediated "direct" delivery of Enzyme-Guide complex (RiboNucleoProtein) and electroporation of plasmid DNA.

Further, the current invention is based on the technical effect that nuclease activity of the SpCas9N863A mutant or an ortholog having a mutation corresponding to SpCas9N863A (e.g., *S. aureus* N580A) always results in strand cleavage in the non-complementary strand. Under these conditions, 3' overhangs are generated if the target sequences (as defined by the sgRNA) on the individual strands are arranged such that the corresponding 5' PAM sequences (located immediately 3' to the target sequences) face away from one another (are on opposite strands, e.g., as illustrated and discussed herein). Such an ortholog can be a mutated *S. aureus* Cas9, i.e. *S. aureus* N580, especially N580A.

Cas9n N863A (Sp) or N580A (Sa), or orthologs having corresponding mutations, selectively nick the non-complimentary strand, see for example the nicks represented with yellow triangles in Figure 1.

Guide offset, for example sgRNA offset, is preferably defined as the distance between the 5' (or PAM-distal) end of each sgRNA.

Further, the current invention is based on a technical effect that 3' overhangs result in inhibition of NHEJ. The technical effect of directed generation of 3' overhangs is improved HDR efficiency as such overhangs results in inhibition of NHEJ events and thus a bias (i.e. increase) in HDR events (i.e. improved HDR efficiency and/or reduced indel formation).

An improved HDR efficiency is considered a higher frequency of HDR events (and/or reduced indel formation) as a result of double nickase activity resulting from either the use of SpCas9N863A mutant or an ortholog having a mutation corresponding to SpCas9N863A (e.g., *S. aureus* N580A) as compared to double nickase activity resulting from a SpCas9 which does not comprise the N863A mutation or an ortholog not comprising a corresponding mutation to SpCas9N863A (e.g., *S. aureus* N580A).

By performing the methods of the invention of modifying an organism or a genomic locus of interest, the skilled person inevitably arrives at minimized off-target modifications. The compositions of the invention arrive at minimized off-target modifications when used.

In some aspects and embodiments, a single type Cas9 nickase may be delivered, for example an SpCas9 or an SaCas9 nickase. This results in the target DNA being bound by either two (2) SpCas9s or two (2) SaCas9s. However, it is also envisaged that the different Cas9 orthologs may be used, one Cas9 ortholog on the coding strand of the DNA and another ortholog Cas9 on the non-coding or opposite DNA strand. For instance, a SpCas9 could be used on one strand and a SaCas9 could be used on another strand. Alternatively, a SpCas9 could be used on one strand and an ortholog Cas9 could be used on another strand, or a SaCas9 could be used on one strand and an ortholog Cas9 could be used on another strand. Using dual, but different Cas9 will require delivery or constitutional expression of an additional Cas9. However, it may be advantageous to do so as the two different ortholog Cas9s require different PAMs and may also have different guide requirements, thus allowing a greater deal of control for the user, especially if one or both of the two orthologs is controllable, e.g. inducible; and more especially if each of the two orthologs is separately controllable or inducible, e.g., each is controlled or induced via a different trigger (although each could be controlled or induced by the same trigger).

Guidance is provided below in respect of guide length (the spacer or guide sequence). In some embodiments, for Sp, optimal guide length can vary as low as Keith Joung's 17-nucleotide 'tru-guide.' In some embodiments, for Sa, the optimal guide length may be 20 or 21 or 22 or 23 or 24 nucleotides in length (Ran 2015).

Also provided is a host cell or cell line. This may be an *in vivo, ex vivo* or *in vitro* host cell or cell line. The host cell or cell line may, in some embodiments, comprise or have been modified by the composition or enzyme according to the present invention. Also provided are progeny of said host cell or cell line. In some embodiments, the cells of the host cell, cell line or progeny are stem cells or a stem cell line.

Methods, products and uses described herein may be used for non-therapeutic purposes. Furthermore, any of the methods described herein may be applied *in vitro* and *ex vivo.*

In relation to the guides in general, but specifically in respect of the sgRNA and the CRISPR complex formed therewith, it is preferable that the guide has one or more of the following features. In some embodiments, the tracr sequence has one or more hairpins and is 30 or more nucleotides in length, more preferably 40 or more nucleotides in length, or more preferably 50 or more nucleotides in length. In some embodiments, the guide sequence is between 10 to 30 nucleotides in length. In some embodiments, the CRISPR/Cas enzyme is a Type II Cas9 enzyme. In some embodiments, the tracr sequence has one or more hairpins and is 30 or more nucleotides in length, more preferably 40 or more nucleotides in length, or more preferably 50 or more nucleotides in length, the guide sequence is between 10 to 30 nucleotides in length and the CRISPR/Cas enzyme is a Type II Cas9 enzyme.

BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

**Figure 1:** Diagram of Cas9n enzymes in a double nicking configuration. Offset nicking with the D10A mutant, which retains only the catalytic activity of the HNH nuclease domain, generates 5' overhang products in the target genome by nicking the sgRNA-complimentary DNA strand (nicks represented with red triangles). Alternatively, Cas9n N863A selectively nicks the non-complimentary strand (nicks represented with yellow triangles). sgRNA offset is defined as the distance between the 5' (or PAM-distal) end of each sgRNA. The PAM sequences, represented in green, are present in the target genome but not the sgRNA.

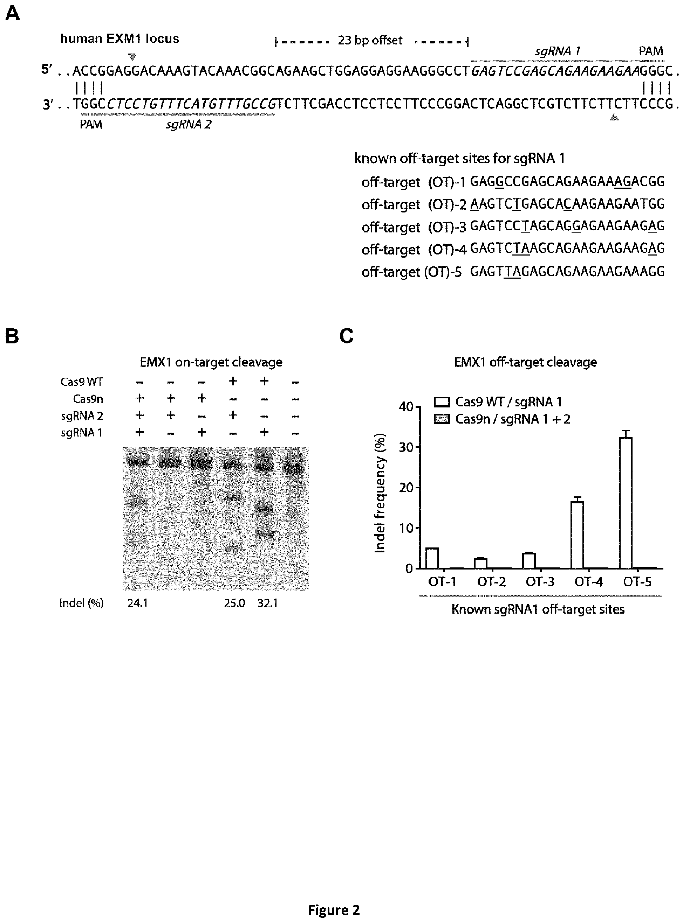

**Figure 2:** Double nicking reduces off-target modification. (A) Diagram of a Cas9n D10A double nicking sgRNA pair designed for the human EMX1 locus. Guide sequences are shown in blue, demonstrating a 23 bp offset. The PAM is shown in pink, and nicking sites are represented by red triangles. Five known genomic off-target sites (Hsu et al., 2013) for sgRNA1 are listed. (B) Example SURVEYOR results showing modification of the EMX1 locus by Cas9 WT and Cas9n along with sgRNA 1 and/or 2. (C) Deep sequencing quantification of off-target modifications at five known off-target sites by Cas9 WT and sgRNA 1 or Cas9n with sgRNAs 1 and 2.

**Figure 3:** General design of ssODN HDR templates. The ssODN consists of an insertion sequence (red) flanked by homology arms on the left and right sides (at least 40 bp each). The homology between the ssODN and its targeting region is indicated by black dashes. Double nicking Cas9 target sites are shown in blue, and their corresponding PAM sequences are shown in pink. Nicking sites are represented by red triangles.

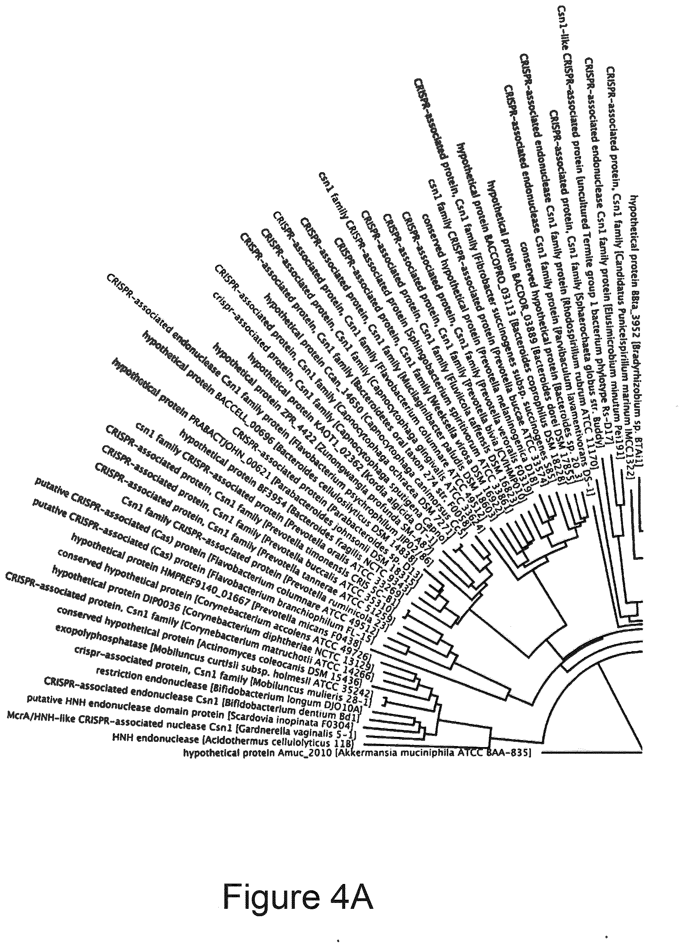

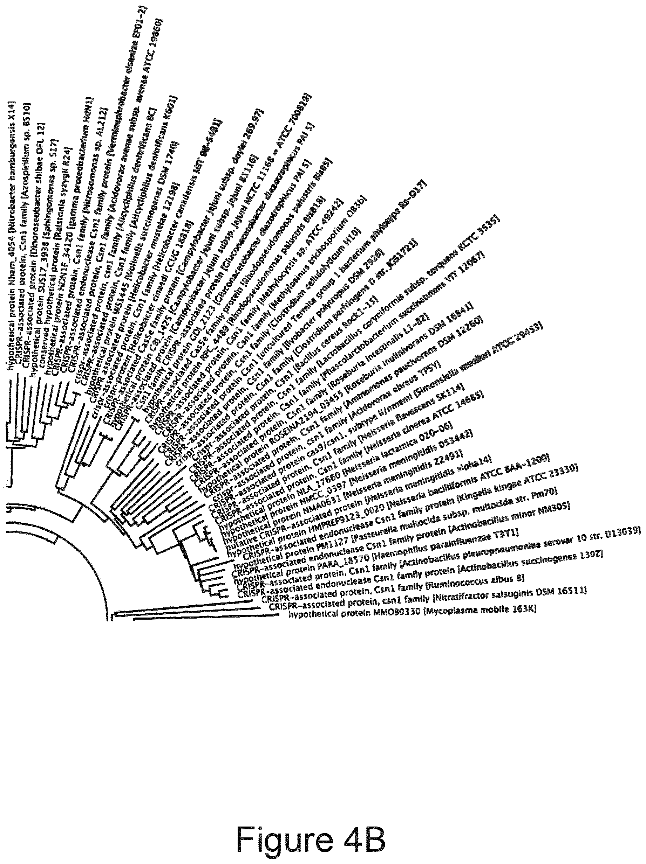

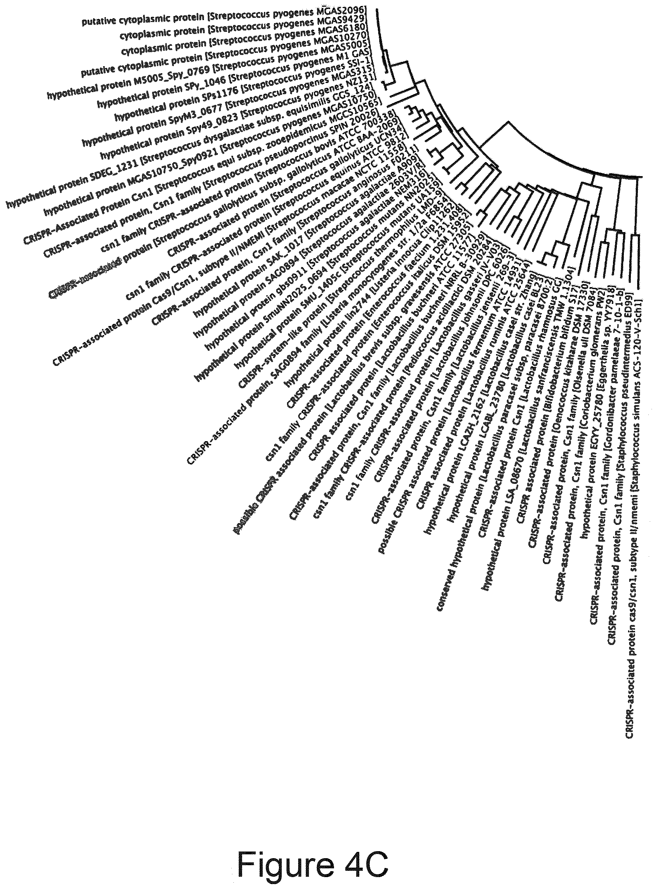

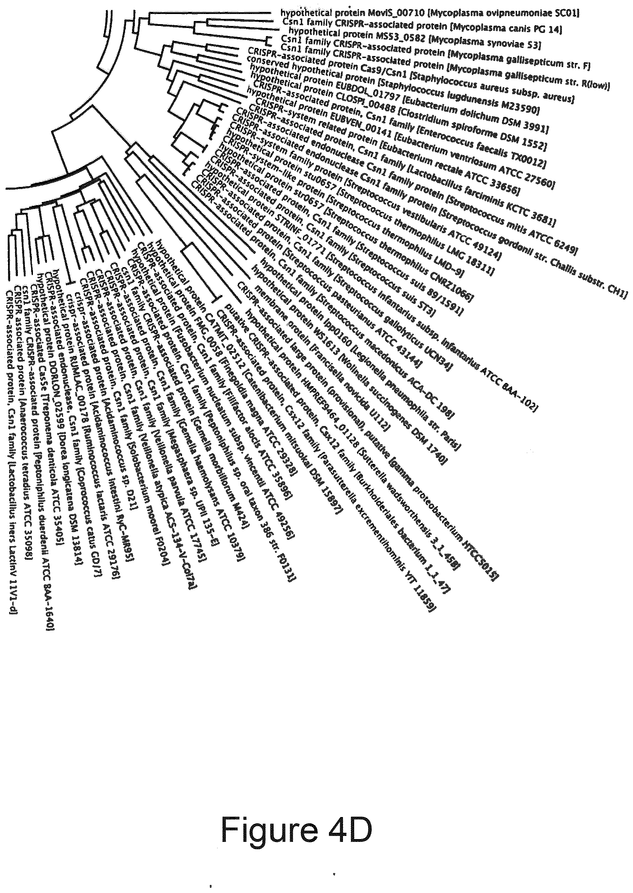

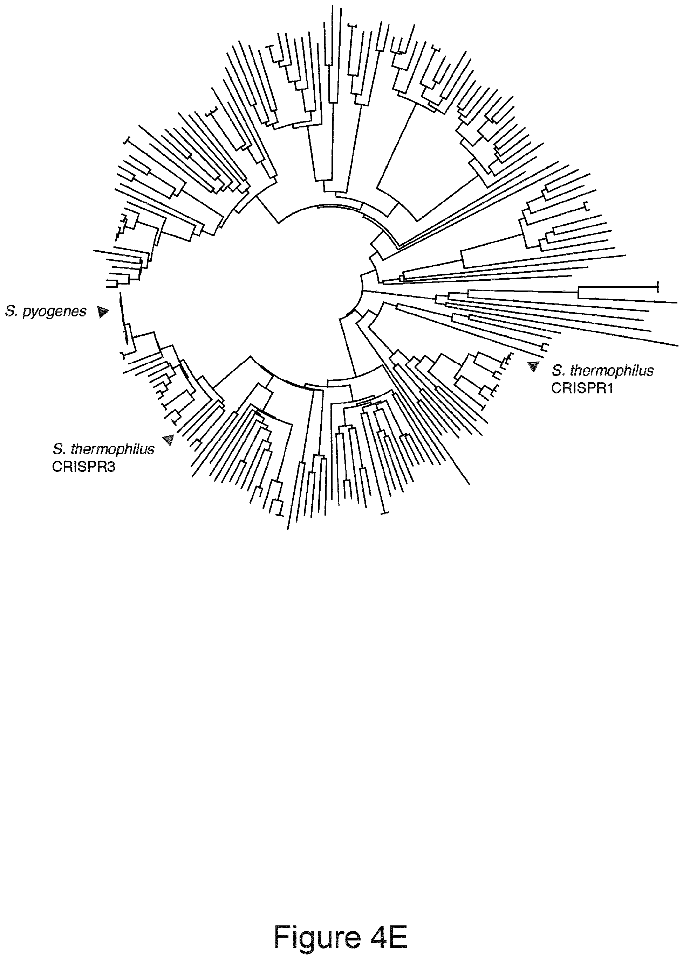

**Figure 4A-E:** Circular depiction of the phylogenetic analysis revealing five families of Cas9s, including three groups of large Cas9s (~1400 amino acids) and two of small Cas9s (~1100 amino acids); circular depiction of CRISPR families.

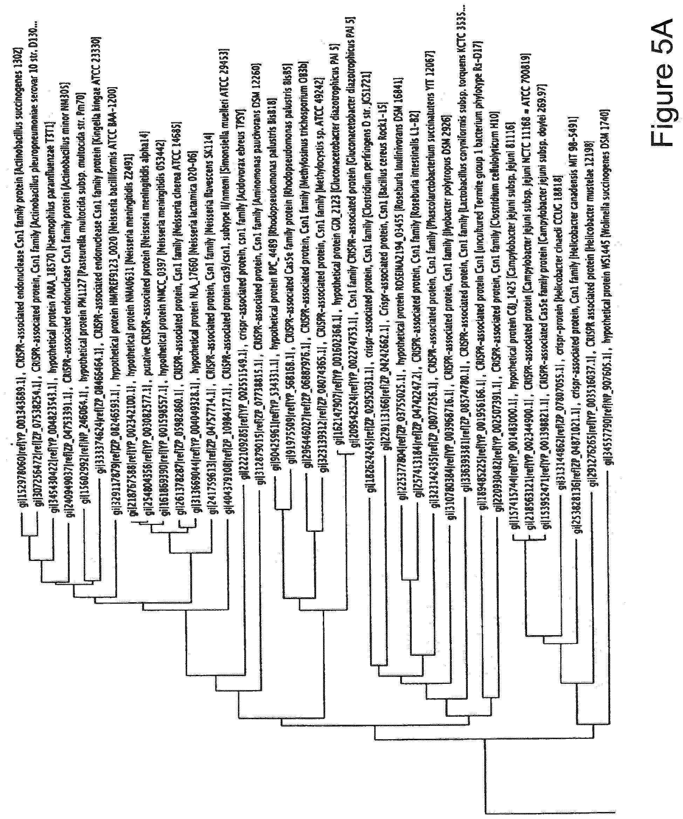

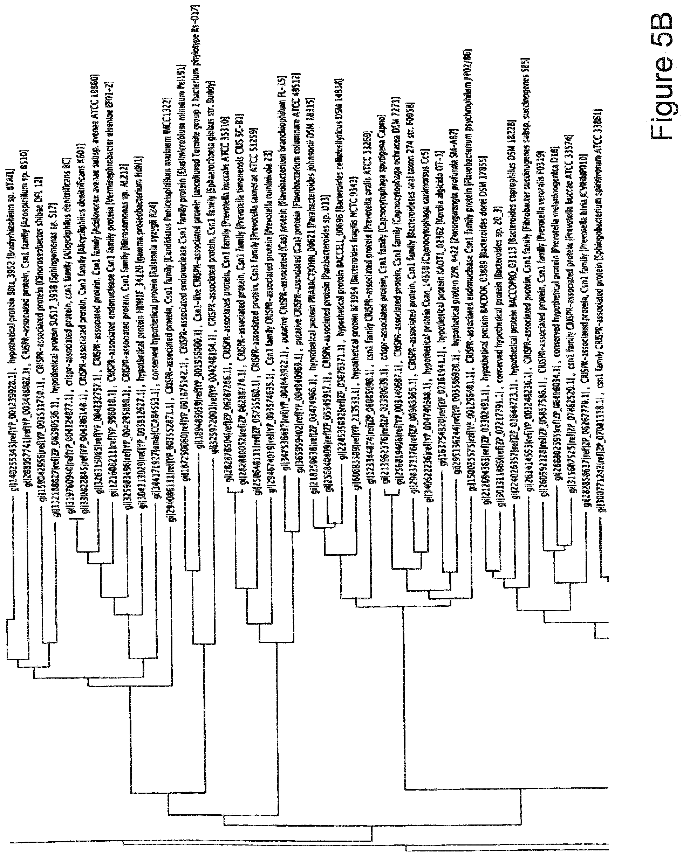

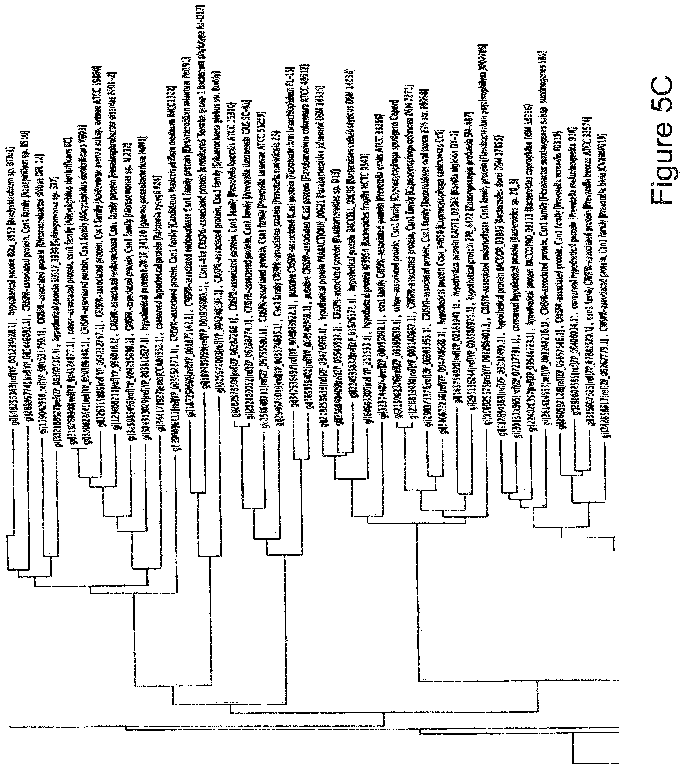

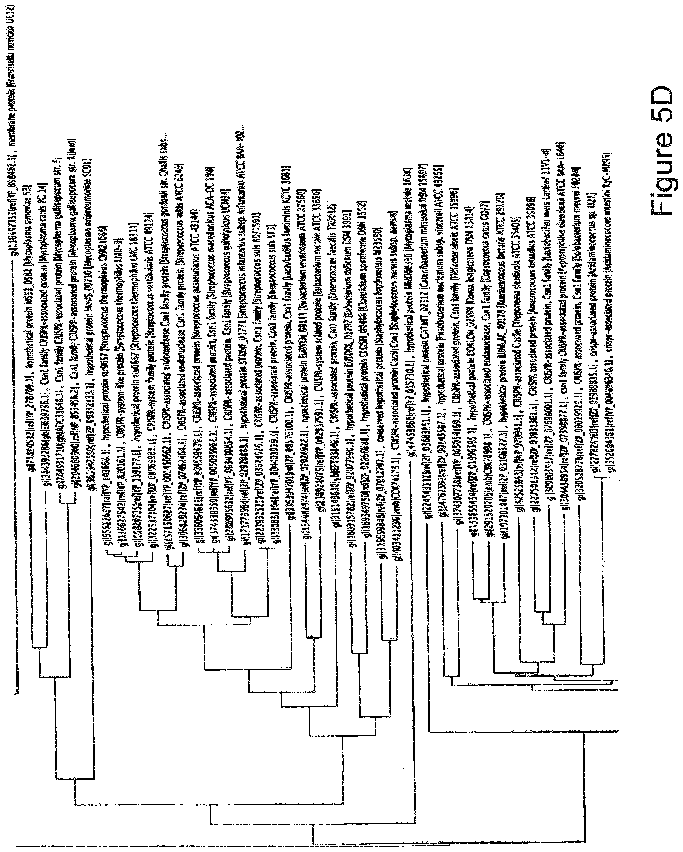

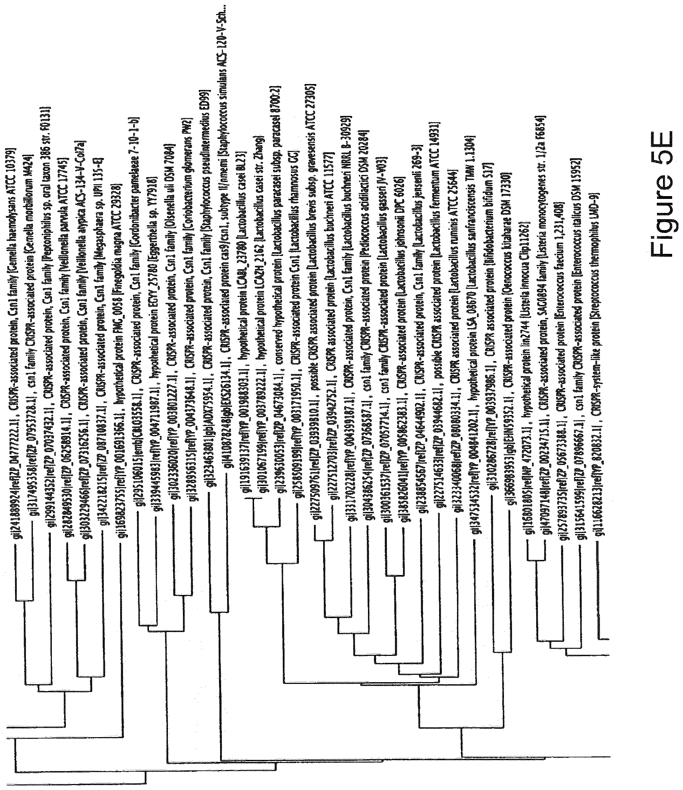

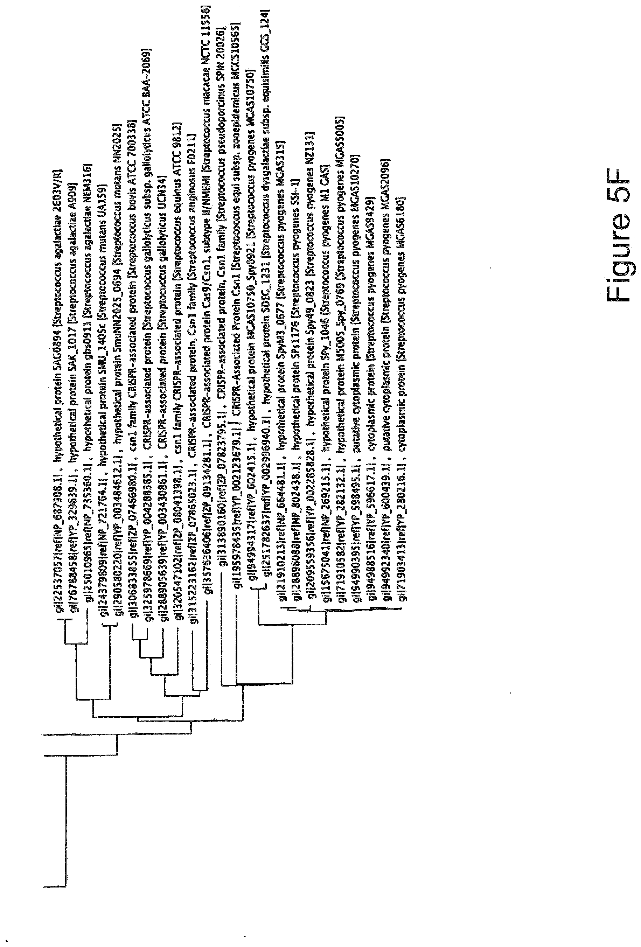

**Figure 5A-F:** Linear depiction of the phylogenetic analysis revealing five families of Cas9s, including three groups of large Cas9s (~1400 amino acids) and two of small Cas9s (~1100 amino acids).

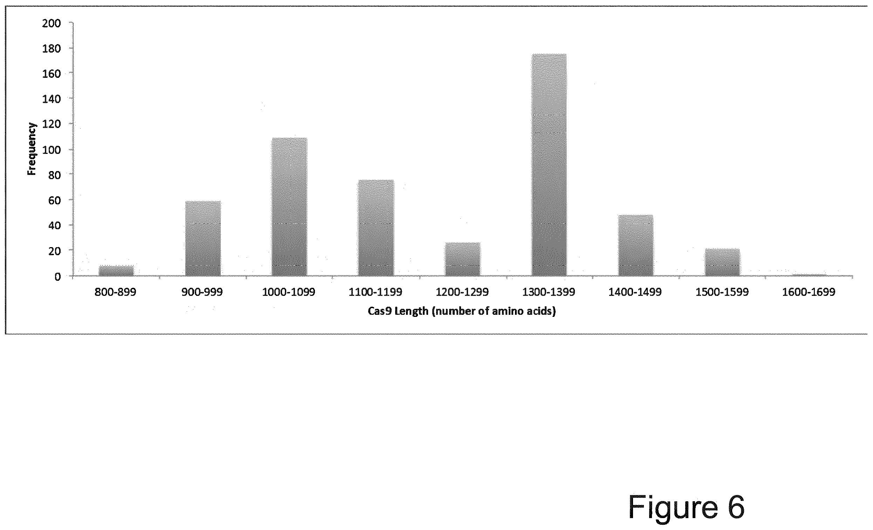

**Figure 6:** Graph representing the length distribution of Cas9 orthologs.

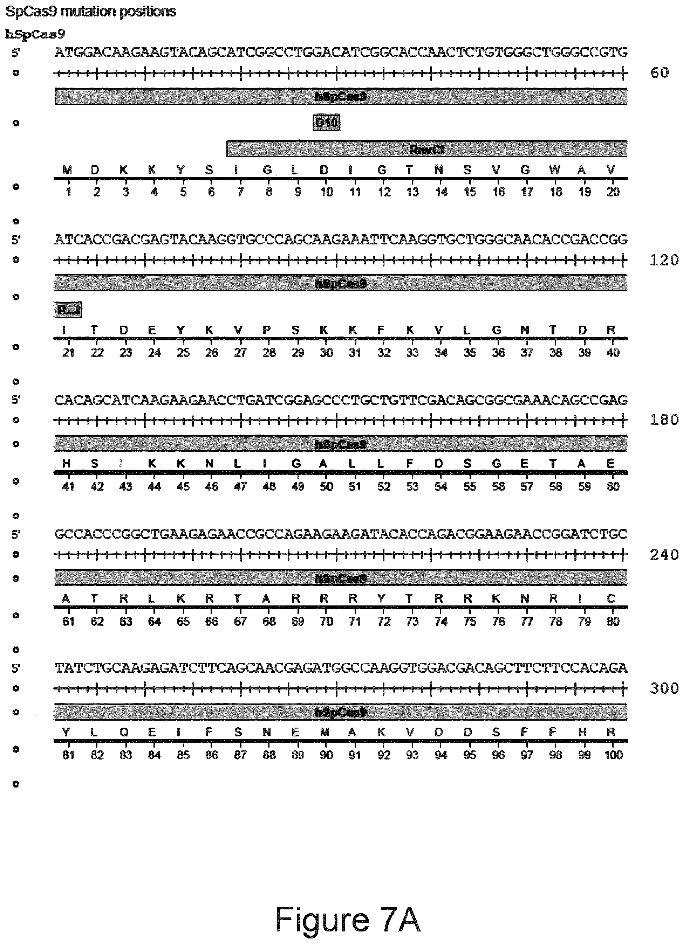

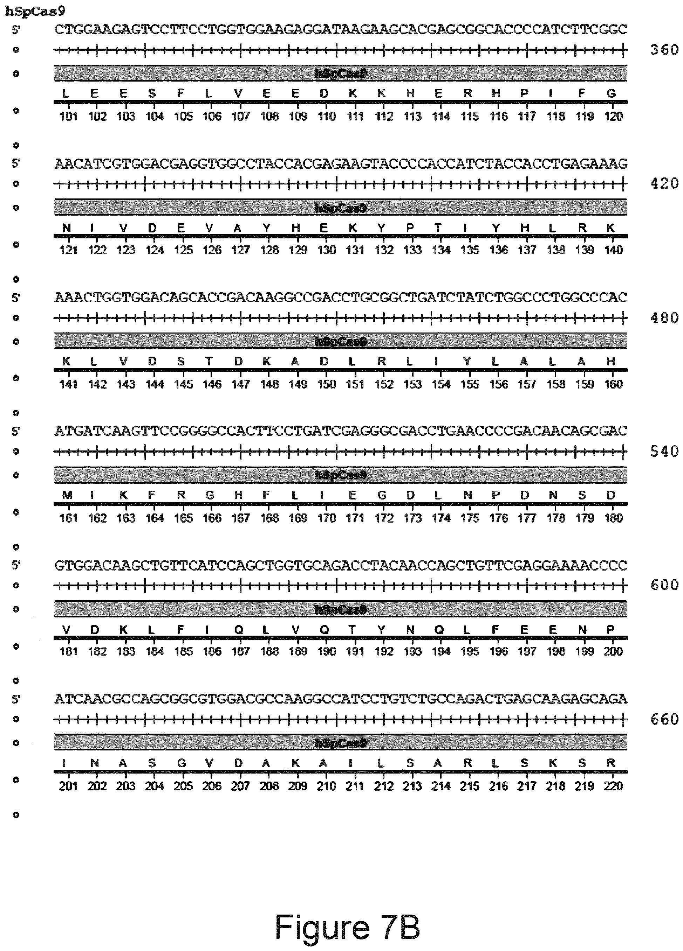

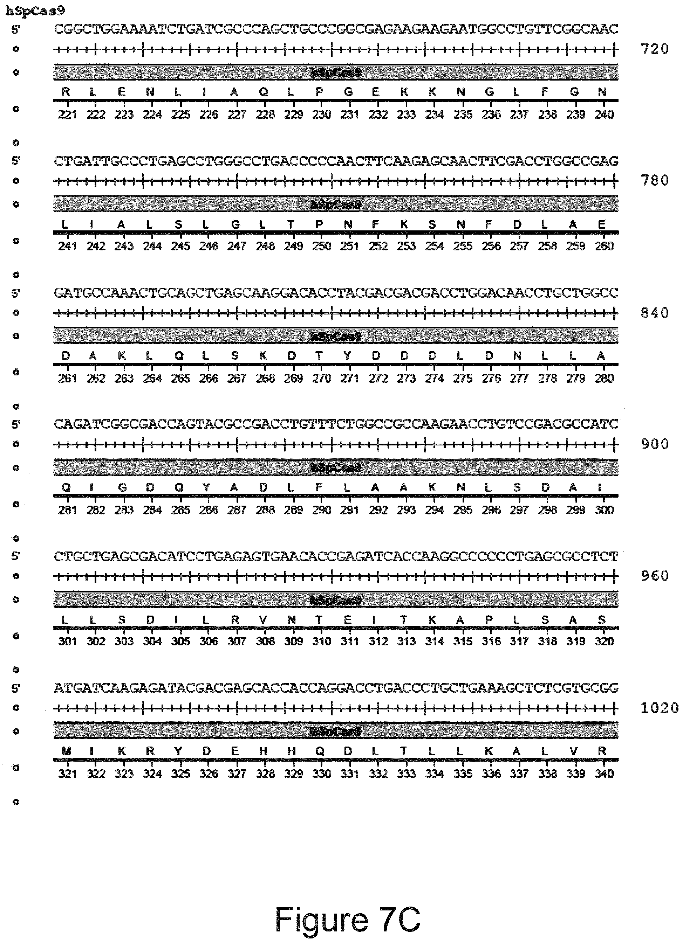

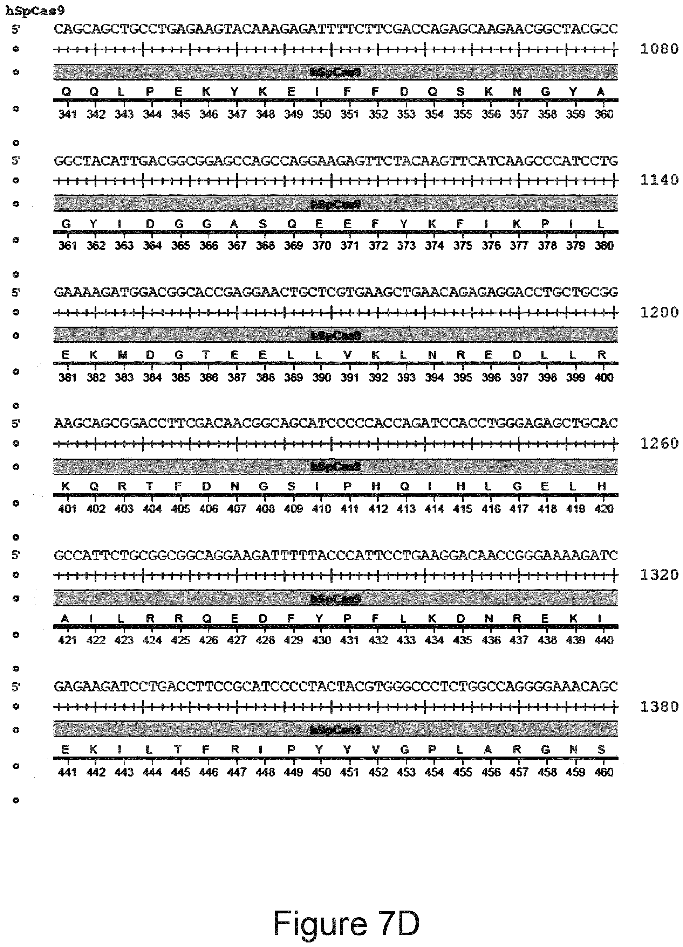

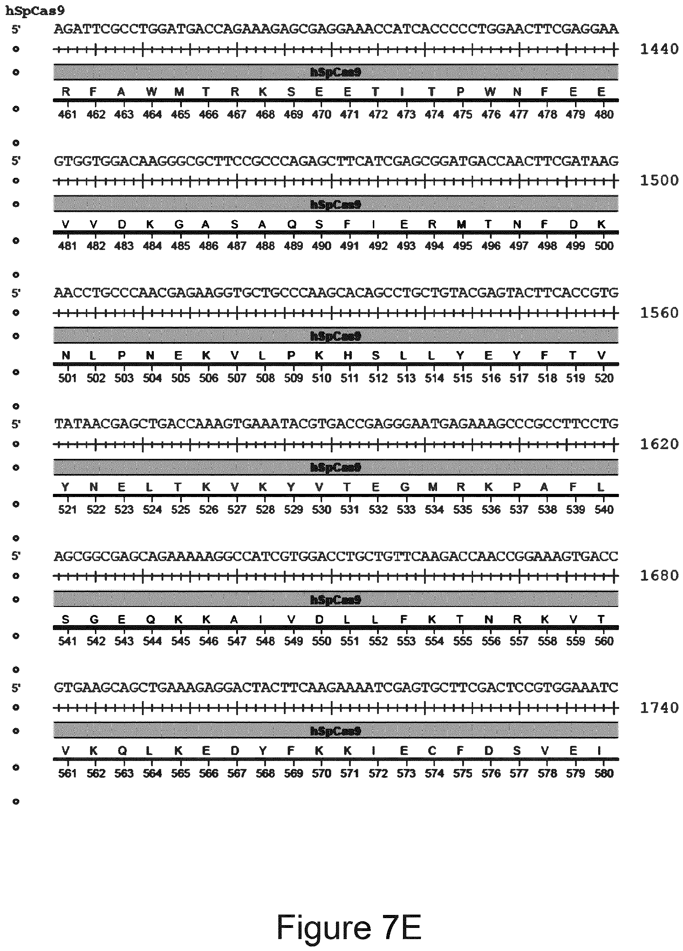

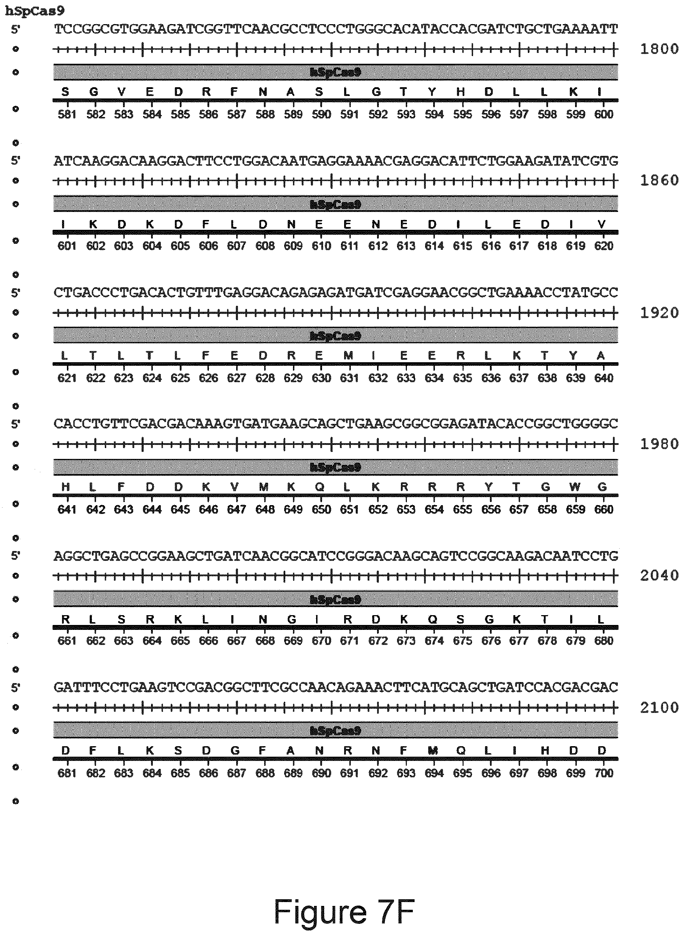

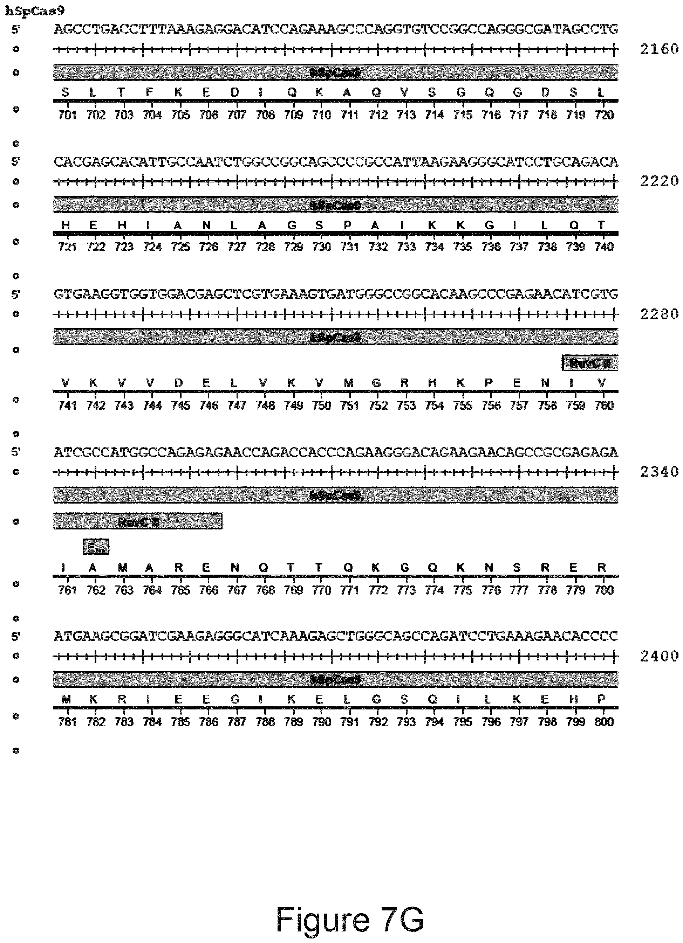

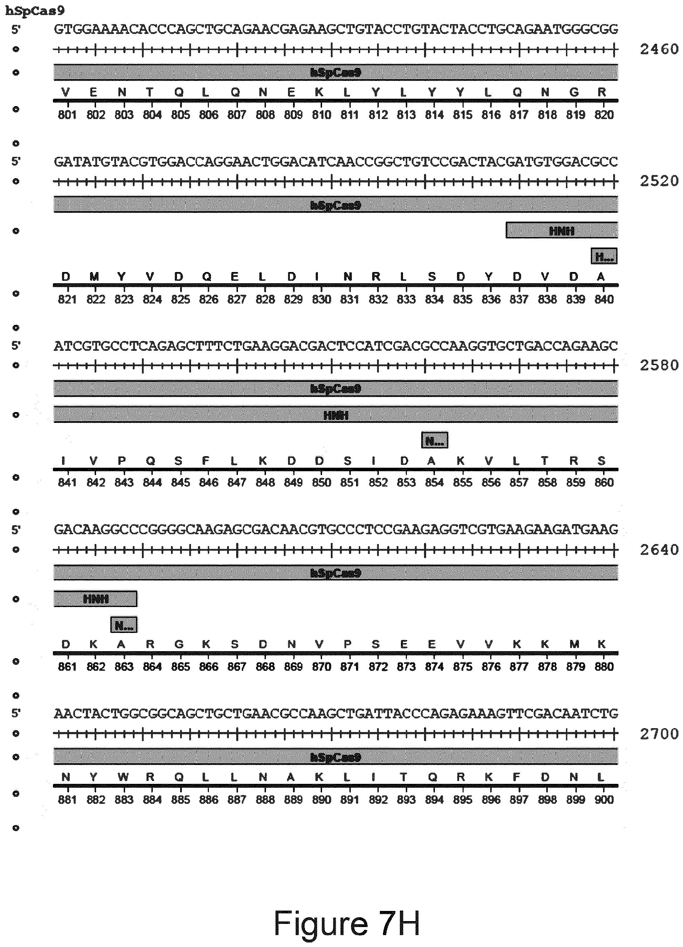

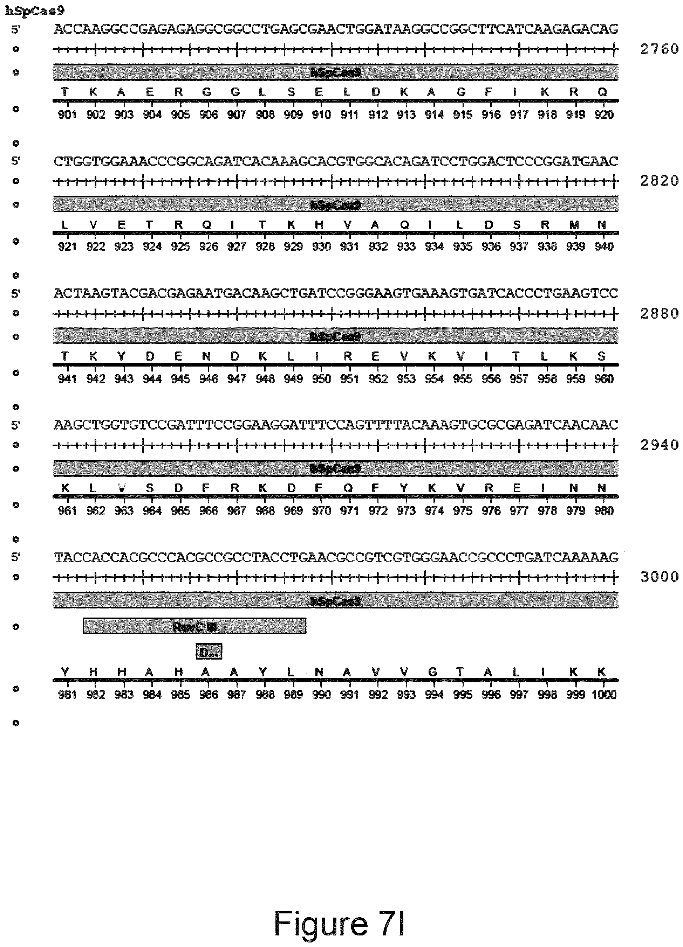

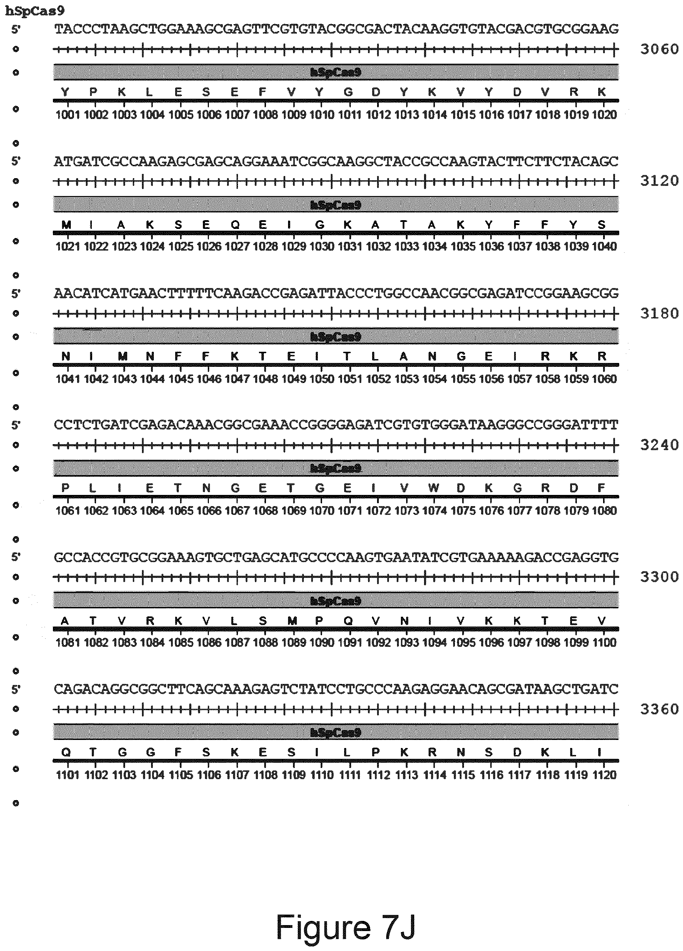

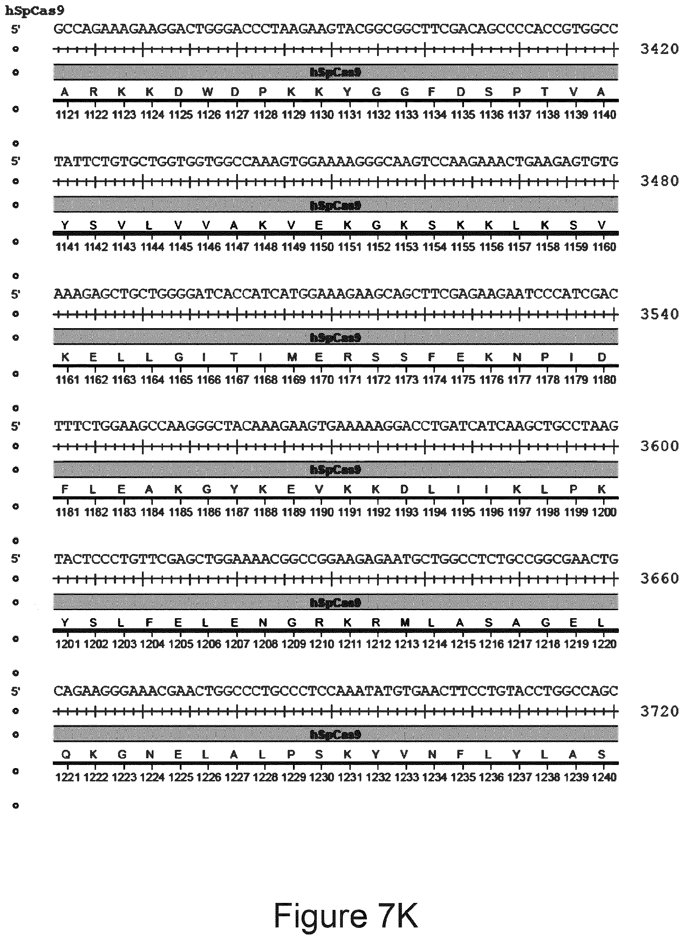

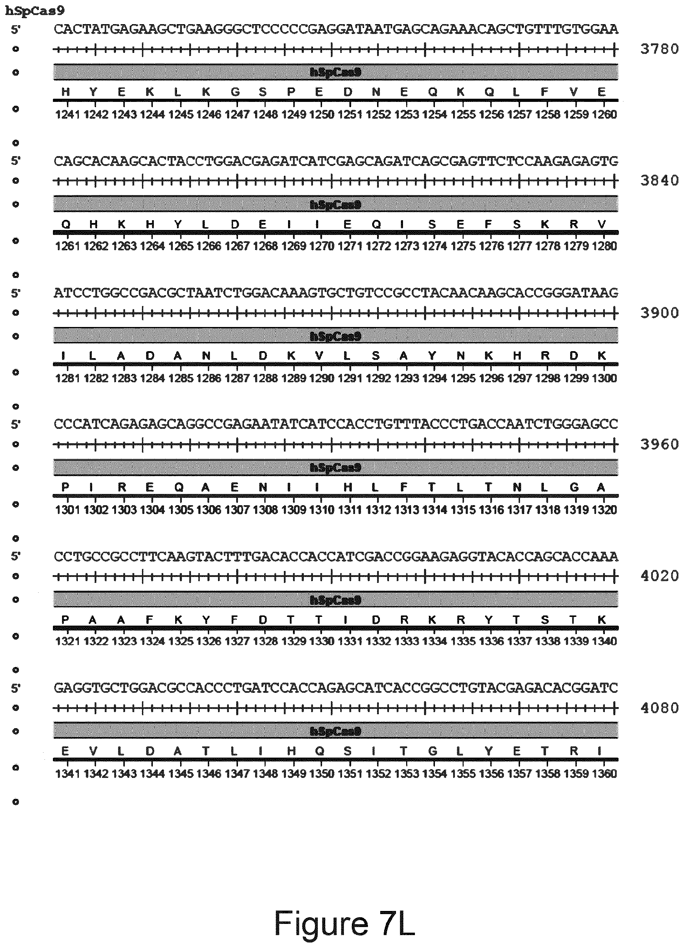

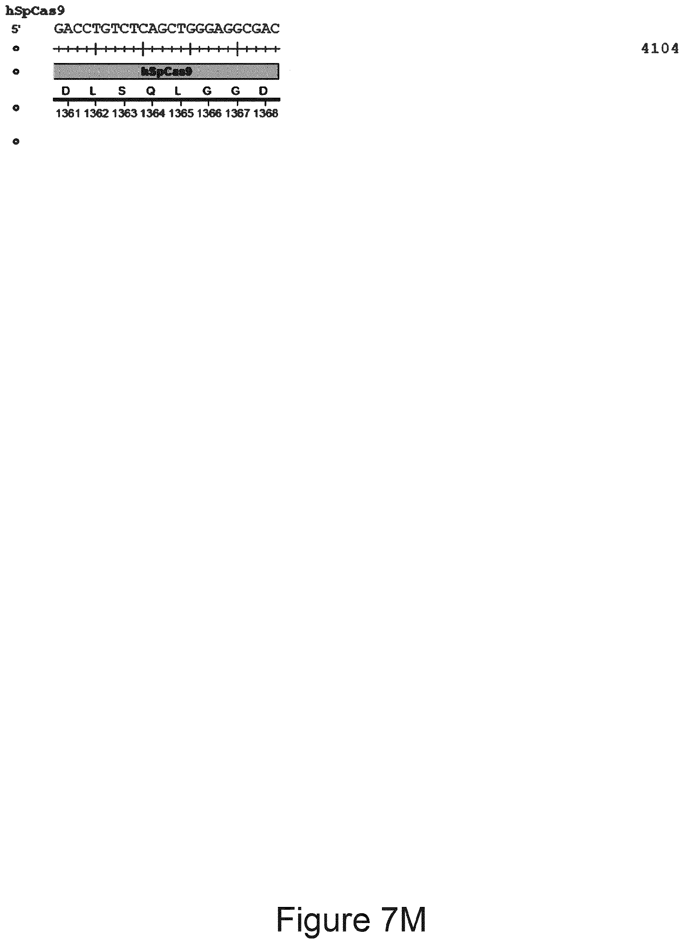

**Figure 7A-M:** Sequence of SpCas9 gene where the mutation points are located within the sequence.

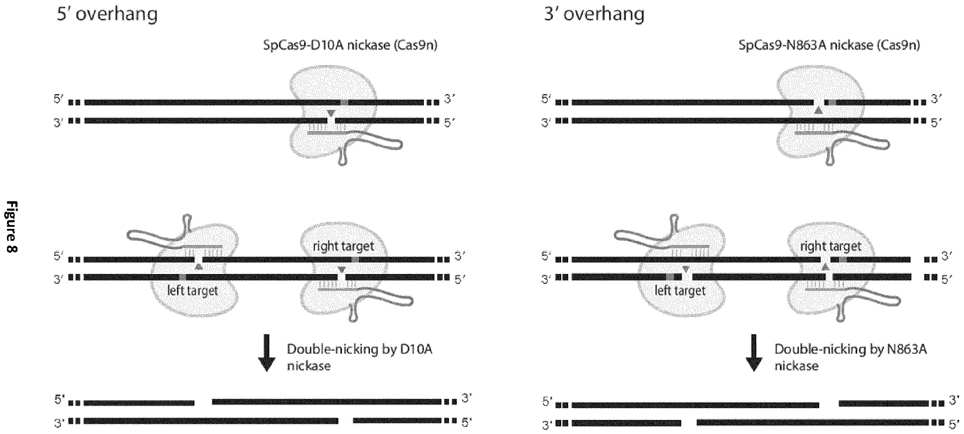

**Figure 8** illustrates both 5' and 3' overhangs in single nickase and dual nickase systems with SpCas9, but this applies equally to SaCas9 and other orthologues.

**Figure 9A-B** illustrates the result of an experiment showing nickase activity of D10A and N580A mutants of SaCas9. Panel A illustrates the sequence of the target locus for 5 gRNAs annotated in gray and shows activity of the mutant enzymes with the indicated guides. NHEJ % on the Y axis represents on-target cleavage rates as measured by TOPO sequencing. Panel B shows wild type S.aureus Cas9 with the indicated gRNAs targeting five different loci. NHEJ % on the Y axis represents on-target cleavage rates as measured by T7E1 assay.

DETAILED DESCRIPTION OF THE INVENTION AND OF THE PRESENT DISCLOSURE

With respect to general information on CRISPR-Cas Systems, components thereof, and delivery of such components, including methods, materials, delivery vehicles, vectors, particles, AAV, and making and using thereof, including as to amounts and formulations, all useful in the practice of the instant invention, reference is made to: US Patents Nos.

8,999,641, 8,993,233, 8,945,839, 8,932,814, 8,906,616, 8,895,308, 8,889,418, 8,889,356, 8,871,445, 8,865,406, 8,795,965, 8,771,945 and 8,697,359; US Patent Publications US 2014-0310830 (US APP. Ser. No. 14/105,031), US 2014-0287938 A1 (U.S. App. Ser. No. 14/213,991), US 2014-0273234 A1 (U.S. App. Ser. No. 14/293,674), US2014-0273232 A1 (U.S. App. Ser. No. 14/290,575), US 2014-0273231 (U.S. App. Ser. No. 14/259,420), US 2014-0256046 A1 (U.S. App. Ser. No. 14/226,274), US 2014-0248702 A1 (U.S. App. Ser. No. 14/258,458), US 2014-0242700 A1 (U.S. App. Ser. No. 14/222,930), US 2014-0242699 A1 (U.S. App. Ser. No. 14/183,512), US 2014-0242664 A1 (U.S. App. Ser. No. 14/104,990), US 2014-0234972 A1 (U.S. App. Ser. No. 14/183,471), US 2014-0227787 A1 (U.S. App. Ser. No. 14/256,912), US 2014-0189896 A1 (U.S. App. Ser. No. 14/105,035), US 2014-0186958 (U.S. App. Ser. No. 14/105,017), US 2014-0186919 A1 (U.S. App. Ser. No. 14/104,977), US 2014-0186843 A1 (U.S. App. Ser. No. 14/104,900), US 2014-0179770 A1 (U.S. App. Ser. No. 14/104,837) and US 2014-0179006 A1 (U.S. App. Ser. No. 14/183,486), US 2014-0170753 (US App Ser No 14/183,429); European Patents EP 2 784 162 B1 and EP 2 771 468 B1; European Patent Applications EP 2 771 468 (EP13818570.7), EP 2 764 103 (EP13824232.6), and EP 2 784 162 (EP14170383.5); and PCT Patent Publications PCT Patent Publications WO 2014/093661 (PCT/US2013/074743), WO 2014/093694 (PCT/US2013/074790), WO 2014/093595 (PCT/US2013/074611), WO 2014/093718 (PCT/US2013/074825), WO 2014/093709 (PCT/US2013/074812), WO 2014/093622 (PCT/US2013/074667), WO 2014/093635 (PCT/US2013/074691), WO 2014/093655 (PCT/US2013/074736), WO 2014/093712 (PCT/US2013/074819), WO2014/093701 (PCT/US2013/074800), WO2014/018423 (PCT/US2013/051418), WO 2014/204723 (PCT/US2014/041790), WO 2014/204724 (PCT/US2014/041800), WO 2014/204725 (PCT/US2014/041803), WO 2014/204726 (PCT/US2014/041804), WO 2014/204727 (PCT/US2014/041806), WO 2014/204728 (PCT/US2014/041808), WO 2014/204729 (PCT/US2014/041809). Reference is also made to US provisional patent applications 61/758,468; 61/802,174; 61/806,375; 61/814,263; 61/819,803 and 61/828,130, filed on January 30, 2013; March 15, 2013; March 28, 2013; April 20, 2013; May 6, 2013 and May 28, 2013 respectively. Reference is also made to US provisional patent application 61/836,123, filed on June 17, 2013. Reference is additionally made to US provisional patent applications 61/835,931, 61/835,936, 61/836,127, 61/836, 101, 61/836,080 and 61/835,973, each filed June 17, 2013. Further reference is made to US provisional patent applications 61/862,468 and 61/862,355 filed on August 5, 2013; 61/871,301 filed on August 28, 2013; 61/960,777 filed on September 25, 2013 and 61/961,980 filed on October 28, 2013. Reference is yet further made to: PCT Patent applications Nos: PCT/US2014/041803, PCT/US2014/041800, PCT/US2014/041809, PCT/US2014/041804 and PCT/US2014/041806, each filed June 10, 2014 6/10/14; PCT/US2014/041808 filed June 11, 2014; and PCT/US2014/62558 filed October 28, 2014, and US Provisional Patent Applications Serial Nos.: 61/915,150, 61/915,301, 61/915,267 and 61/915,260, each filed December 12, 2013; 61/757,972 and 61/768,959, filed on January 29, 2013 and February 25, 2013; 61/835,936, 61/836,127, 61/836,101, 61/836,080, 61/835,973, and 61/835,931, filed June 17, 2013; 62/010,888 and 62/010,879, both filed June 11, 2014; 62/010,329 and 62/010,441, each filed June 10, 2014; 61/939,228 and 61/939,242, each filed February 12, 2014; 61/980,012, filed April 15,2014; 62/038,358, filed August 17, 2014; 62/054,490, 62/055,484, 62/055,460 and 62/055,487, each filed September 25, 2014; and 62/069,243, filed October 27, 2014. Reference is also made to US provisional patent applications Nos. 62/055,484, 62/055,460, and 62/055,487, filed September 25, 2014; US provisional patent application 61/980,012, filed April 15, 2014; and US provisional patent application 61/939,242 filed February 12, 2014. Reference is made to PCT application designating, inter alia, the United States, application No. PCT/US14/41806, filed June 10, 2014. Reference is made to US provisional patent application 61/930,214 filed on January 22, 2014. Reference is made to US provisional patent applications 61/915,251; 61/915,260 and 61/915,267, each filed on December 12, 2013. Reference is made to US provisional patent application USSN 61/980,012 filed April 15, 2014. Reference is made to PCT application designating, inter alia, the United States, application No. PCT/US14/41806, filed June 10, 2014. Reference is made to US provisional patent application 61/930,214 filed on January 22, 2014. Reference is made to US provisional patent applications 61/915,251; 61/915,260 and 61/915,267, each filed on December 12, 2013.

Mention is also made of US application 62/091,455, filed, 12-Dec-14, PROTECTED GUIDE RNAS (PGRNAS); US application 62/096,708, 24-Dec-14, PROTECTED GUIDE RNAS (PGRNAS); US application 62/091,462, 12-Dec-14, DEAD GUIDES FOR CRISPR TRANSCRIPTION FACTORS; US application 62/096,324, 23-Dec-14, DEAD GUIDES FOR CRISPR TRANSCRIPTION FACTORS; US application 62/091,456, 12-Dec-14, ESCORTED AND FUNCTIONALIZED GUIDES FOR CRISPR-CAS SYSTEMS; US application 62/091,461, 12-Dec-14, DELIVERY, USE AND THERAPEUTIC APPLICATIONS OF THE CRISPR-CAS SYSTEMS AND COMPOSITIONS FOR GENOME EDITING AS TO HEMATOPOETIC STEM CELLS (HSCs); US application 62/094,903, 19-Dec-14, UNBIASED IDENTIFICATION OF DOUBLE-STRAND BREAKS AND GENOMIC REARRANGEMENT BY GENOME-WISE INSERT CAPTURE SEQUENCING; US application 62/096,761, 24-Dec-14, ENGINEERING OF SYSTEMS, METHODS AND OPTIMIZED ENZYME AND GUIDE SCAFFOLDS FOR SEQUENCE MANIPULATION; US application 62/098,059, 30-Dec-14, RNA-TARGETING SYSTEM; US application 62/096,656, 24-Dec-14, CRISPR HAVING OR ASSOCIATED WITH DESTABILIZATION DOMAINS; US application 62/096,697, 24-Dec-14, CRISPR HAVING OR ASSOCIATED WITH AAV; US application 62/098,158, 30-Dec-14, ENGINEERED CRISPR COMPLEX INSERTIONAL TARGETING SYSTEMS; US application 62/151,052, 22-Apr-15, CELLUAR TARGETING FOR EXTRACELLULAR EXOSOMAL REPORTING; US application 62/054,490, 24-Sep-14, DELIVERY, USE AND THERAPEUTIC APPLICATIONS OF THE CRISPR-CAS SYSTEMS AND COMPOSITIONS FOR TARGETING DISORDERS AND DISEASES USING PARTICLE DELIVERY COMPONENTS; US application 62/055,484, 25-Sep-14, SYSTEMS, METHODS AND COMPOSITIONS FOR SEQUENCE MANIPULATION WITH OPTIMIZED FUNCTIONAL CRISPR-CAS SYSTEMS; US application 62/087,537, 4-Dec-14, SYSTEMS, METHODS AND COMPOSITIONS FOR SEQUENCE MANIPULATION WITH OPTIMIZED FUNCTIONAL CRISPR-CAS SYSTEMS; US application 62/054,651, 24-Sep-14, DELIVERY, USE AND THERAPEUTIC APPLICATIONS OF THE CRISPR-CAS SYSTEMS AND COMPOSITIONS FOR MODELING COMPETITION OF MULTIPLE CANCER MUTATIONS IN VIVO; US application 62/067,886, 23-Oct-14, DELIVERY, USE AND THERAPEUTIC APPLICATIONS OF THE CRISPR-CAS SYSTEMS AND COMPOSITIONS FOR MODELING COMPETITION OF MULTIPLE CANCER MUTATIONS IN VIVO; US application 62/054,675, 24-Sep-14, DELIVERY, USE AND THERAPEUTIC APPLICATIONS OF THE CRISPR-CAS SYSTEMS AND COMPOSITIONS IN NEURONAL CELLS/TISSUES; US application 62/054,528, 24-Sep-14, DELIVERY, USE AND THERAPEUTIC APPLICATIONS OF THE CRISPR-CAS SYSTEMS AND COMPOSITIONS IN IMMUNE DISEASES OR DISORDERS; US application 62/055,454, 25-Sep-14, DELIVERY, USE AND THERAPEUTIC APPLICATIONS OF THE CRISPR-CAS SYSTEMS AND COMPOSITIONS FOR TARGETING DISORDERS AND DISEASES USING CELL PENETRATION PEPTIDES (CPP); US application 62/055,460, 25-Sep-14, MULTIFUNCTIONAL-CRISPR COMPLEXES AND/OR OPTIMIZED ENZYME LINKED FUNCTIONAL-CRISPR COMPLEXES; US application 62/087,475, 4-Dec-14, FUNCTIONAL SCREENING WITH OPTIMIZED FUNCTIONAL CRISPR-CAS SYSTEMS; US application 62/055,487, 25-Sep-14, FUNCTIONAL SCREENING WITH OPTIMIZED FUNCTIONAL CRISPR-CAS SYSTEMS; US application 62/087,546, 4-Dec-14, MULTIFUNCTIONAL CRISPR COMPLEXES AND/OR OPTIMIZED ENZYME LINKED FUNCTIONAL-CRISPR COMPLEXES; and US application 62/098,285, 30-Dec-14, CRISPR MEDIATED IN VIVO MODELING AND GENETIC SCREENING OF TUMOR GROWTH AND METASTASIS.

Also with respect to general information on CRISPR-Cas Systems, mention is made of the following:

➢ Multiplex genome engineering using CRISPR/Cas systems. Cong, L., Ran, F.A., Cox, D., Lin, S., Barretto, R., Habib, N., Hsu, P.D., Wu, X., Jiang, W., Marraffini, L.A., & Zhang, F. Science Feb 15;339(6121):819-23 (2013);

➢ RNA-guided editing of bacterial genomes using CRISPR-Cas systems. Jiang W., Bikard D., Cox D., Zhang F, Marraffini LA. Nat Biotechnol Mar;31(3):233-9 (2013);

➢ One-Step Generation of Mice Carrying Mutations in Multiple Genes by CRISPR/Cas-Mediated Genome Engineering. Wang H., Yang H., Shivalila CS., Dawlaty MM., Cheng AW., Zhang F., Jaenisch R. Cell May 9;153(4):910-8 (2013);

➢ Optical control of mammalian endogenous transcription and epigenetic states. Konermann S, Brigham MD, Trevino AE, Hsu PD, Heidenreich M, Cong L, Platt RJ, Scott DA, Church GM, Zhang F. Nature. 2013 Aug 22;500(7463):472-6. doi: 10.1038/Nature12466. Epub 2013 Aug 23;

➢ Double Nicking by RNA-Guided CRISPR Cas9 for Enhanced Genome Editing Specificity. Ran, FA., Hsu, PD., Lin, CY., Gootenberg, JS., Konermann, S., Trevino, AE., Scott, DA., Inoue, A., Matoba, S., Zhang, Y., & Zhang, F. Cell Aug 28. pii: S0092-8674(13)01015-5. (2013);

➢ DNA targeting specificity of RNA-guided Cas9 nucleases. Hsu, P., Scott, D., Weinstein, J., Ran, FA., Konermann, S., Agarwala, V., Li, Y., Fine, E., Wu, X., Shalem, O., Cradick, TJ., Marraffini, LA., Bao, G., & Zhang, F. Nat Biotechnol doi:10.1038/nbt.2647 (2013);

➢ Genome engineering using the CRISPR-Cas9 system. Ran, FA., Hsu, PD., Wright, J., Agarwala, V., Scott, DA., Zhang, F. Nature Protocols Nov;8(11):2281-308. (2013);

➢ Genome-Scale CRISPR-Cas9 Knockout Screening in Human Cells. Shalem, O., Sanjana, NE., Hartenian, E., Shi, X., Scott, DA., Mikkelson, T., Heckl, D., Ebert, BL., Root, DE., Doench, JG., Zhang, F. Science Dec 12. (2013). [Epub ahead of print];

➢ Crystal structure of cas9 in complex with guide RNA and target DNA. Nishimasu, H., Ran, FA., Hsu, PD., Konermann, S., Shehata, SI., Dohmae, N., Ishitani, R., Zhang, F., Nureki, O. Cell Feb 27. (2014). 156(5):935-49;

➢ Genome-wide binding of the CRISPR endonuclease Cas9 in mammalian cells. Wu X., Scott DA., Kriz AJ., Chiu AC., Hsu PD., Dadon DB., Cheng AW., Trevino AE., Konermann S., Chen S., Jaenisch R., Zhang F., Sharp PA. Nat Biotechnol. (2014) Apr 20. doi: 10.1038/nbt.2889,

➢ CRISPR-Cas9 Knockin Mice for Genome Editing and Cancer Modeling, Platt et al., Cell 159(2): 440-455 (2014) DOI: 10.1016/j.cell.2014.09.014,

➢ Development and Applications of CRISPR-Cas9 for Genome Engineering, Hsu et al, Cell 157, 1262-1278 (June 5, 2014) (Hsu 2014),

➢ Genetic screens in human cells using the CRISPR/Cas9 system, Wang et al., Science. 2014 January 3; 343(6166): 80-84. doi:10.1126/science.1246981,

➢ Rational design of highly active sgRNAs for CRISPR-Cas9-mediated gene inactivation, Doench et al., Nature Biotechnology published online 3 September 2014; doi:10.1038/nbt.3026, and

➢ In vivo interrogation of gene function in the mammalian brain using CRISPR-Cas9, Swiech et al, Nature Biotechnology published online 19 October 2014; doi:10.1038/nbt.3055,

➢ Konermann et al., "Genome-scale transcription activation by an engineered CRISPR-Cas9 complex," doi:10.1038/nature14136,

➢ Zetsche et al., "A split-Cas9 architecture for inducible genome editing and transcription modulation," Nature Biotechnology 33:139-142, DOI:10.1038/nbt.3149 (Published online 02 February 2015),

➢ Sidi Chen et al., "Genome-wide CRISPR Screen in a Mouse Model of Tumor Growth and Metastasis," Cell 160, 1246-1260, March 12, 2015 (multiplex screen in mouse), and

➢ Ran et al., "In vivo genome editing using Staphylococcus aureus Cas9," Nature 520, 186-191 (09 April 2015) doi:10.1038/nature14299 (Published online 01 April 2015), may be considered in the practice of the instant invention, and discussed briefly below:

➢ Cong *et al.* engineered type II CRISPR-Cas systems for use in eukaryotic cells based on both *Streptococcus thermophilus* Cas9 and also *Streptoccocus pyogenes* Cas9 and demonstrated that Cas9 nucleases can be directed by short RNAs to induce precise cleavage of DNA in human and mouse cells. Their study further showed that Cas9 as converted into a nicking enzyme can be used to facilitate homology-directed repair in eukaryotic cells with minimal mutagenic activity. Additionally, their study demonstrated that multiple guide sequences can be encoded into a single CRISPR array to enable simultaneous editing of several at endogenous genomic loci sites within the mammalian genome, demonstrating easy programmability and wide applicability of the RNA-guided nuclease technology. This ability to use RNA to program sequence specific DNA cleavage in cells defined a new class of genome engineering tools. These studies further showed that other CRISPR loci are likely to be transplantable into mammalian cells and can also mediate mammalian genome cleavage. Importantly, it can be envisaged that several aspects of the CRISPR-Cas system can be further improved to increase its efficiency and versatility.

➢ Jiang *et al.* used the clustered, regularly interspaced, short palindromic repeats (CRISPR)-associated Cas9 endonuclease complexed with dual-RNAs to introduce precise mutations in the genomes of *Streptococcus pneumoniae* and *Escherichia coli.* The approach relied on dual-RNA:Cas9-directed cleavage at the targeted genomic site to kill unmutated cells and circumvents the need for selectable markers or counter-selection systems. The study reported reprogramming dual-RNA:Cas9 specificity by changing the sequence of short CRISPR RNA (crRNA) to make single- and multinucleotide changes carried on editing templates. The study showed that simultaneous use of two crRNAs enabled multiplex mutagenesis. Furthermore, when the approach was used in combination with recombineering, in *S. pneumoniae,* nearly 100% of cells that were recovered using the described approach contained the desired mutation, and in *E. coli,* 65% that were recovered contained the mutation.

➢ Konermann *et al.* addressed the need in the art for versatile and robust technologies that enable optical and chemical modulation of DNA-binding domains based CRISPR Cas9 enzyme and also Transcriptional Activator Like Effectors

➢ The Cas9 nuclease from the microbial CRISPR-Cas system is targeted to specific genomic loci by a guide sequence, which can tolerate certain mismatches to the DNA target and thereby promote undesired off-target mutagenesis. To address this, Ran *et al.* described an approach that combined a Cas9 nickase mutant with paired guide RNAs to introduce targeted double-strand breaks. Because individual nicks in the genome are repaired with high fidelity, simultaneous nicking *via* appropriately offset guide RNAs is required for double-stranded breaks and extends the number of specifically recognized bases for target cleavage. The authors demonstrated that using paired nicking can reduce off-target activity by 50- to 1,500-fold in cell lines and to facilitate gene knockout in mouse zygotes without sacrificing on-target cleavage efficiency. This versatile strategy enables a wide variety of genome editing applications that require high specificity.

➢ Hsu *et al.* characterized SpCas9 targeting specificity in human cells to inform the selection of target sites and avoid off-target effects. The study evaluated >700 guide RNA variants and SpCas9-induced indel mutation levels at >100 predicted genomic off-target loci in 293T and 293FT cells. The authors that SpCas9 tolerates mismatches between guide RNA and target DNA at different positions in a sequence-dependent manner, sensitive to the number, position and distribution of mismatches. The authors further showed that SpCas9-mediated cleavage is unaffected by DNA methylation and that the dosage of SpCas9 and sgRNA can be titrated to minimize off-target modification. Additionally, to facilitate mammalian genome engineering applications, the authors reported providing a web-based software tool to guide the selection and validation of target sequences as well as off-target analyses.

➢ Ran *et al.* described a set of tools for Cas9-mediated genome editing *via* non-homologous end joining (NHEJ) or homology-directed repair (HDR) in mammalian cells, as well as generation of modified cell lines for downstream functional studies. To minimize off-target cleavage, the authors further described a double-nicking strategy using the Cas9 nickase mutant with paired guide RNAs. The protocol provided by the authors experimentally derived guidelines for the selection of target sites, evaluation of cleavage efficiency and analysis of off-target activity. The studies showed that beginning with target design, gene modifications can be achieved within as little as 1-2 weeks, and modified clonal cell lines can be derived within 2-3 weeks.

➢ Shalem *et al.* described a new way to interrogate gene function on a genome-wide scale. Their studies showed that delivery of a genome-scale CRISPR-Cas9 knockout (GeCKO) library targeted 18,080 genes with 64,751 unique guide sequences enabled both negative and positive selection screening in human cells. First, the authors showed use of the GeCKO library to identify genes essential for cell viability in cancer and pluripotent stem cells. Next, in a melanoma model, the authors screened for genes whose loss is involved in resistance to vemurafenib, a therapeutic that inhibits mutant protein kinase BRAF. Their studies showed that the highest-ranking candidates included previously validated genes NF1 and MED12 as well as novel hits NF2, CUL3, TADA2B, and TADA1. The authors observed a high level of consistency between independent guide RNAs targeting the same gene and a high rate of hit confirmation, and thus demonstrated the promise of genome-scale screening with Cas9.

➢ Nishimasu *et al.* reported the crystal structure of *Streptococcus pyogenes* Cas9 in complex with sgRNA and its target DNA at 2.5 A° resolution. The structure revealed a bilobed architecture composed of target recognition and nuclease lobes, accommodating the sgRNA:DNA heteroduplex in a positively charged groove at their interface. Whereas the recognition lobe is essential for binding sgRNA and DNA, the nuclease lobe contains the HNH and RuvC nuclease domains, which are properly positioned for cleavage of the complementary and non-complementary strands of the target DNA, respectively. The nuclease lobe also contains a carboxyl-terminal domain responsible for the interaction with the protospacer adjacent motif (PAM). This high-resolution structure and accompanying functional analyses have revealed the molecular mechanism of RNA-guided DNA targeting by Cas9, thus paving the way for the rational design of new, versatile genome-editing technologies.

➢ Wu *et al.* mapped genome-wide binding sites of a catalytically inactive Cas9 (dCas9) from *Streptococcus pyogenes* loaded with single guide RNAs (sgRNAs) in mouse embryonic stem cells (mESCs). The authors showed that each of the four sgRNAs tested targets dCas9 to between tens and thousands of genomic sites, frequently characterized by a 5-nucleotide seed region in the sgRNA and an NGG protospacer adjacent motif (PAM). Chromatin inaccessibility decreases dCas9 binding to other sites with matching seed sequences; thus 70% of off-target sites are associated with genes. The authors showed that targeted sequencing of 295 dCas9 binding sites in mESCs transfected with catalytically active Cas9 identified only one site mutated above background levels. The authors proposed a two-state model for Cas9 binding and cleavage, in which a seed match triggers binding but extensive pairing with target DNA is required for cleavage.

➢ Hsu 2014 is a review article that discusses generally CRISPR-Cas9 history from yogurt to genome editing, including genetic screening of cells. The general teachings of Hsu 2014 do not involve the specific aspects, e.g., models, animals, of the instant invention.

➢ Konermann et al., "Genome-scale transcription activation by an engineered CRISPR-Cas9 complex," doi:10.1038/nature14136 (ability to attach multiple effector domains, e.g., transcriptional activator, functional and epigenomic regulators at appropriate positions on guide such as stem or tetraloop with and without linkers).

➢ Zetsche et al., "A split-Cas9 architecture for inducible genome editing and transcription modulation," Nature Biotechnology 33:139-142, DOI:10.1038/nbt.3149 (Published online 02 February 2015) (ability to control assembly of Cas9 for activation).

➢ Sidi Chen et al., "Genome-wide CRISPR Screen in a Mouse Model of Tumor Growth and Metastasis," Cell 160, 1246-1260, March 12, 2015 (multiplex screen in mouse).

➢ Tsai et al, "Dimeric CRISPR RNA-guided FokI nucleases for highly specific genome editing," Nature Biotechnology 32(6): 569-77 (2014) (FokI nucleases).

➢ Ran et al., "In vivo genome editing using Staphylococcus aureus Cas9," Nature 520, 186-191 (09 April 2015) doi:10.1038/nature14299 (Published online 01 April 2015) (relating to SaCas9 and that one cannot extrapolate from biochemical assays). With regard to SaCas9, the optimal guide length for Sa may be 21-24 nucleotides in length; and a PAM may be NNGRRT, although NNGRR may also be considered.

Aspects of the invention, as related to SaCas9 systems and other orthologus CRISPR-Cas systems, may be practiced using structural and functional comparisons to an SpCas9 system as used in the methods and systems further described in International Patent Application PCT/US14/70068 titled "CRISPR-CAS SYSTEMS AND METHODS FOR ALTERING EXPRESSION OF GENE PRODUCTS, STRUCTURAL INFORMATION AND INDUCIBLE MODULAR CAS ENZYMES" filed on December 12, 2014, which claims priority to US provisional patent application Serial Nos. 61/915,267, filed Dec 12 2013 and US 61/939,228 filed on February 12, 2014.

Homology modeling: Corresponding residues in other Cas9 orthologs can be identified by the methods of Zhang et al., 2012 (Nature; 490(7421): 556-60) and Chen et al., 2015 (PLoS Comput Biol; 11(5): e1004248)-a computational protein-protein interaction (PPI) method to predict interactions mediated by domain-motif interfaces. PrePPI (Predicting PPI), a structure based PPI prediction method, combines structural evidence with non-structural evidence using a Bayesian statistical framework. The method involves taking a pair a query proteins and using structural alignment to identify structural representatives that correspond to either their experimentally determined structures or homology models.

Structural alignment is further used to identify both close and remote structural neighbours by considering global and local geometric relationships. Whenever two neighbors of the structural representatives form a complex reported in the Protein Data Bank, this defines a template for modelling the interaction between the two query proteins. Models of the complex are created by superimposing the representative structures on their corresponding structural neighbour in the template. This approach is further described in Dey et al., 2013 (Prot Sci; 22: 359-66).

Any of the embodiments of the foregoing literature, patents, patent publications, and patent applications pertaining to CRISPR-Cas can be used in the practice of the instant invention, e.g., any embodiment of the foregoing literature, patents, patent publications, and patent applications pertaining to CRISPR-Cas can be used with any of the overhang(s) inventions herein. The invention relates to improved methods for the design and testing of nickase reagents for high-precision mammalian genome editing using, in particular, homology directed repair (HDR), including target selection, sgRNA construction, transfection, detection of Cas9-induced indel mutations using the SURVEYOR nuclease assay, and design and quantification of homology-directed insertions.

The RNA-guided, sequence-specific endonuclease Cas9 has been widely adopted as genome engineering tool due to its efficiency and ease of use. Derived from the microbial CRISPR (clustered regularly interspaced short palindromic repeats) type II adaptive immune system, Cas9 has now been successfully engineered for genome editing applications in a variety of animal and plant species. To reduce potential off-target mutagenesis by wild-type Cas9, homology- and structure- guided mutagenesis of Streptococcus pyogenes Cas9 catalytic domains has produced "nicking" enzymes (Cas9n) capable of inducing single-strand nicks rather than double-strand breaks (DSBs). Since nicks are generally repaired with high fidelity in eukaryotic cells, Cas9n can be leveraged to mediate highly specific genome editing, either via non-homologous end joining or homology-directed repair.

Specifically, a technical effect of the current invention can be that 3' overhang products by N863A-mediated double nicking increases HDR efficiency.

Cas9 mediates genome editing through targeted introduction of a DNA double strand break (DSB). The DSB is recognized by endogenous repair machineries and can lead to genome editing. Two main pathways of endogenous repair systems are available: NHEJ and HDR. These two pathways are in competition with each other. In order for HDR to proceed, the broken DNA ends need to be resected to generate a 3' overhang, which will in turn inhibit NHEJ.

Using the D10A Cas9 nickase 5' overhangs may be generated, which can be processed by both NHEJ and HR. Using the N863A Cas9 nickase 3' overhang may be generated, which partially inhibits NHEJ and therefore biases the editing outcome toward HR

HDR in mammalian cells proceeds via the generation of 3' overhangs followed by strand invasion of a homologous locus by the 3' end.

**Target selection**

The following description is a mere exemplary discussion of how target selection may proceed. Equivalent alternatives as well as protocols known in the art may also be applied. Although reference is made to SpCas9, it will be appreciated that it applies equally to SaCas9 or other orthologs as appropriate, noting especially that PAMs will tend to vary from ortholog to ortholog.

SpCas9 targets can be any 20-bp DNA sequence followed at the 3' end by 5'-NGG-3'. An online tool is available that will accept a region of interest as input and output a list of all potential sgRNA target sites within that region. Each sgRNA target site is then associated with a list of predicted genomic off-targets (http://tools.genome-engineering.org). This online tool may be used to assist in target selection, however, it is not considered essential for target selection. Target selection may also be based on known methods in the art.

The tool also generates double-nicking sgRNA pairs automatically. The most important consideration for double-nicking sgRNA design is the spacing between the two targets (Ran et al., 2013). If the "offset" between two guides is defined as the distance between the PAM-distal (5') ends of an sgRNA pair, an offset of -4 to 20 bp is ideal, though offsets as large as 100 bp can induce DSB-mediated indels. sgRNA pairs for double nicking may target opposite DNA strands.

**Plasmid** sgRNA **construction**

The following description is a mere exemplary discussion of how plasmid sgRNA construction may proceed. Equivalent alternatives as well as protocols known in the art may also be applied.

sgRNA expression vectors can be constructed by cloning target sequences into a plasmid backbone encoding, for example, a human U6 promoter-driven sgRNA expression cassette and, for example, a CBh-driven Cas9-D10A (pSpCas9n(BB), (Addgene #48873) (e.g. 20-bp target sequences). The N863A nickase can be exchanged with, for example, D10A in all cases. It is preferred to prepare this plasmid as an endotoxin-free maxiprep. The generalized oligos for use in cloning a new target into, for example, pSpCas9n(BB) are described in Table 1 and may easily be produced using routine protocols or may be purchased from any number of suppliers, for example, from Integrated DNA Technologies (IDT). Note that the PAM sequence required for target recognition by Cas9 is never present as part of the sgRNA itself.

In general, the following points may be considered:

- Clone a target sequence into an sgRNA backbone vector.
- Annealing the oligos.
- Dilute the annealed oligos.
- Set up digestion/ligation with, for example, pSpCas9n(BB), and the annealed oligos as a cloning insert.
- A negative control may be performed using the same conditions.
- Incubate the ligation.
- Transform ligation reaction into a competent strain.
- Selection of positive colonies from the transformation, inoculate and culture.
- Isolate plasmid DNA and determine the DNA concentration by spectrophotometry. These constructs may be Sanger sequence-verified to confirm correct insertion of the target sequence. For optimal transfection conditions downstream, endotoxin-free plasmid may be prepared.

## Validation of sgRNAs in Cell Lines

The following description is a mere exemplary discussion of how validation of sgRNAs in cell lines may proceed. Equivalent alternatives as well as protocols known in the art may also be applied.

In general, the following points may be considered:

- Maintain healthy cells.
- Transfect, culture/maintain, and control cultures.
- Harvest the cells for genomic DNA extraction and/or downstream analysis.

When working with different cell types, alternative transfection reagents may be compared for efficiency and toxicity. It may also be informative to titrate pSpCas9n(sgRNA) in order to find the optimal transfection concentration with highest efficacy.

## Cell harvest and DNA extraction

The following description is a mere exemplary discussion of how cell harvest and DNA extraction may proceed. Equivalent alternatives as well as protocols known in the art may also be applied.

In general, the following points may be considered:

- Harvest cells.
- Aliquot, centrifuge, and resuspend pellet to wash.
- centrifuge and resuspend.
- Extract genomic DNA, using, for example, a thermocycler protocol.
- Centrifuge the reaction product to pellet cell debris and transfer cleared supernatant into a fresh tube for further analysis.
- Determine the DNA concentration of the extraction by, for example, spectrophotometry and normalize with ddH20.

## SURVEYOR indel analysis

The following description is a mere exemplary discussion of how SURVEYOR indel analysis may proceed. Equivalent alternatives as well as protocols known in the art may also be applied.

The SURVEYOR assay (Transgenomic 706025) is a method for detecting polymorphisms and small indels. DNA samples are PCR-amplified, and the products are heated to denature and cooled slowly to form heteroduplexes. Mismatched duplexes are then cleaved by the SURVEYOR nuclease, and cleavage products are analyzed by gel electrophoresis.

In general, the following points may be considered:

- Perform PCR on genomic DNA.
- Note that, since SURVEYOR was designed to detect mutations, it is crucial to use a high-fidelity polymerase to avoid false positives.

- Run PCR product on a gel to ensure that a single product of expected size has formed.
- Purify the PCR product, measure the DNA concentration and normalize using ddH20.
- Mix normalized PCR product with Taq PCR buffer. Melt and re-hybridize the products gradually in a thermocycler.
- Mix SURVEYOR nuclease S, and SURVEYOR enhancer S with all of the annealed product from above. Perform the digestion. Samples that have mutations within the rehybridized PCR amplicons will be cleaved by SURVEYOR.

The digestion products can be mixed with an appropriate loading dye and visualized by electrophoresis on a 4-20% polyacrylamide TBE gel (see example, Figure 2B).

Genome modification rates can be estimated first by calculating the relative intensities of digestion products a and b, and the undigested band c. The frequency of cutting fcut is then given by (a+b)/(a+b+c). The following formula, based on the binomial probability distribution of duplex formation, estimates the percentage of indels in the sample.

$$\%\mathrm{indel} = (1 - \sqrt{(1 - f\_cut)})100$$

## HDR and non-HDR insertion using Cas9n

The following description is a mere exemplary discussion of how HDR and non-HDR insertion using Cas9n may proceed. Equivalent alternatives as well as protocols known in the art may also be applied.

In general, the following points may be considered:

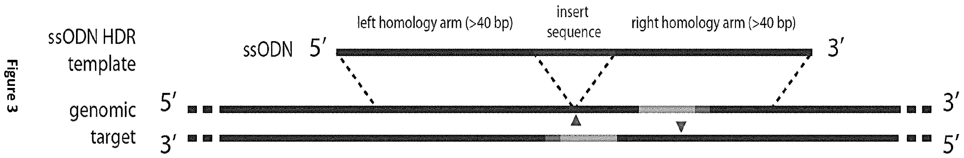

- Design of ssODN homology arms may be designed to be as long as possible, with at least 40 nucleotides of homology on either side of the sequence to be introduced. (see design example, Figure 3).
- Mix Cas9(sgRNA) plasmids with ssODN for nucleofection.

A single-stranded oligodeoxynucleotide (ssODN) has a high efficiency as a template for homologous recombination, though linearized plasmid vectors can also be used. In some cell types, a single nickase may stimulate a targeted homologous repair event in the presence of a donor template. In others, such as human embryonic stem cells, a double-stranded break mediated by double-nicking may be required to promote efficient HDR (Ran et al., 2013). The considerations for choosing double-nicking sgRNA pairs for HDR are similar to those for gene knockdown by NHEJ, with the additional requirement that one of the nicks must occur within approximately 20 bp of the HDR insertion site. In 293FT cells, double-nicking-mediated HDR can be comparably efficient to wild-type Cas9-mediated HDR.

Nicking Cas9 enzymes are well suited to generating highly precise modifications. Since HDR typically occurs at low efficiency in the best cases, we also provide pSpCas9n plasmids encoding the polycistronic 2A linker followed by GFP and puromycin markers (Addgene #48140 and 48141) in order to facilitate enrichment of modified cells.

- ssODN homology arms may be designed to be as long as possible, with at least 40 nucleotides of homology on either side of the sequence to be introduced. The Ultramer service provided by IDT allows the synthesis of oligos up to 200 bp in length. Homology templates may be diluted to 10 μM and stored at -20 °C (see design example, Figure 3).
- Delivery by Nucleofection is optimal for ssODNs. The 4D Nucleofector X Kit S (Lonza V4XC-2032) can be used for HEK293FT cells seeded in 6-well tissue culture-treated plates. The manufacturer provides an optimal protocol for nucleofection of these and other cell types. Mix 500 ng total pSpCas9n(sgRNA) plasmids with 1 μL 10 μM ssODN for nucleofection.

The technical effect of the current invention is that 3' overhang products generated by N863A-mediated double nicking increases HDR efficiency.

## Analysis of HDR and insertion events

The following description is a mere exemplary discussion of how analysis of HDR and insertion events may proceed. Equivalent alternatives as well as protocols known in the art may also be applied.

In general, the following points may be considered:

- Prepare FACS media.
- Prepare well plates for clone sorting.
- Dissociate the cells.
- Stop trypsinization, transfer the cells, and establish a single-cell suspension before proceeding.
- Centrifuge the cells, aspirate the supernatant completely, and resuspend the pellet thoroughly.

- Filter the cells to filter out cell aggregates.
- Sort single cells, using for example, FACS machine.
- Incubate and expand the cells.
- Passage of clonal populations into replica plates, disassociation, and conservation for DNA extraction
- Genotyping can be performed by, for example, PCR amplification of the locus of interest, PCR purification, and Sanger sequencing of the products.

**Troubleshooting**

The following description is a mere exemplary discussion of how troubleshooting may proceed. Equivalent alternatives as well as protocols known in the art may also be applied.

 *1. Colonies form on the negative control plate while cloning targets into pSpCas9n.*

 a. The presence of negative colonies generally indicates an incomplete restriction digestion of the backbone plasmid.
 b. A mere example for overcoming this issue includes extending the Golden Gate reaction for 20-25 cycles in order to increase the efficiency of digestion. The amount of restriction enzyme used can also be increased, though the volume of enzyme may not exceed 20% of the total reaction volume. Retransform the Cas9 backbone plasmid, isolate a new preparation of plasmid DNA, and sequence-verify the restriction site.

 *2. The transfection efficiency of Cas9 reagents is low.*

 a. Low transfection efficiency may be the norm for some cell lines, and especially primary cells or stem cell lines.
 b. A mere example for overcoming this issue includes enrichment of cell populations for transfected cells by using pSpCas9n(BB)-GFP or pSpCas9n(BB)-Puro plasmids to FACS on GFP fluorescence or perform antibiotic selection.

 *3. Double nicking does not produce indels.*

 a. The individual double nicking sgRNAs may be tested with the wild-type context to ensure that each of them functions separately as a valid Cas9 guide.
 b. Check the spacing of the sgRNA pair. Double nicking performs optimally when the guides are spaced 20 bp apart or less, and the guides may be oriented such that their respective 5 ' PAM sequences face away from each other.

 *4. Efficiency of HDR is* low.

 a. Silent mutations may be introduced within the target site on the ssODN to prevent cleavage of the successfully recombined genomic site.

The invention relates to the engineering and optimization of systems, methods and compositions used for the control of gene expression involving sequence targeting, such as genome perturbation or gene-editing, that relate to the CRISPR-Cas system and components thereof. In the embodiments of the invention, the Cas enzyme is a mutated Cas9 (N863A).

An advantage of the present methods is that the CRISPR system avoids off-target binding and its resulting side effects. This is achieved using systems arranged to have a high degree of sequence specificity for the target DNA.

Cas9 optimization may be used to enhance function or to develop new functions, one can generate chimeric Cas9 proteins. Chimeric Cas9 proteins can be made by combining fragments from different Cas9 homologs. For example, two example chimeric Cas9 proteins from the Cas9s described herein. For example, Applicants fused the N-term of St1Cas9 (fragment from this protein is in bold) with C-term of SpCas9. The benefit of making chimeric Cas9s include any or all of:

- reduced toxicity;
- improved expression in eukaryotic cells;
- enhanced specificity;
- reduced molecular weight of protein, make protein smaller by combining the smallest domains from different Cas9 homologs; and/or

- altering the PAM sequence requirement.

As mentioned above, transgenic animals are also provided, as are transgenic plants, especially crops and algae. The transgenic may be useful in applications outside of providing a disease model. These may include food of feed production through expression of, for instance, higher protein, carbohydrate, nutrient or vitamins levels than would normally be seen in the wildtype. In this regard, transgenic plants, especially pulses and tubers, and animals, especially mammals such as livestock (cows, sheep, goats and pigs), but also poultry and edible insects, are preferred.

Transgenic algae or other plants such as rape may be particularly useful in the production of vegetable oils or biofuels such as alcohols (especially methanol and ethanol), for instance. These may be engineered to express or overexpress high levels of oil or alcohols for use in the oil or biofuel industries.

In terms of *in vivo* delivery, AAV is advantageous over other viral vectors for a couple of reasons:

○ Low toxicity (this may be due to the purification method not requiring ultracentrifugation of cell particles that can activate the immune response)

○ Low probability of causing insertional mutagenesis because it doesn't integrate into the host genome.

AAV has a packaging limit of 4.5 or 4.75 Kb. This means that Cas9 as well as a promoter and transcription terminator have to be all fit into the same viral vector. Constructs larger than 4.5 or 4.75 Kb will lead to significantly reduced virus production. SpCas9 is quite large, the gene itself is over 4.1 Kb, which makes it difficult for packing into AAV. Therefore embodiments of the invention include utilizing homologs of Cas9 that are shorter. For example:

| Species | Cas9 Size |
| --- | --- |
| Corynebacter diphtheriae | 3252 |
| Eubacterium ventriosum | 3321 |
| Streptococcus pasteurianus | 3390 |
| Lactobacillus farciminis | 3378 |
| Sphaerochaeta globus | 3537 |
| Azospirillum B510 | 3504 |
| Gluconacetobacter diazotrophicus | 3150 |
| Neisseria cinerea | 3246 |
| Roseburia intestinalis | 3420 |
| Parvibaculum lavamentivorans | 3111 |
| Staphylococcus aureus | 3159 |
| Nitratifractor salsuginis DSM 16511 | 3396 |
| Campylobacter lari CF89-12 | 3009 |
| Streptococcus thermophilus LMD-9 | 3396 |

These species are therefore, in general, preferred Cas9 species, especially SaCas9 as mentioned. Applicants have shown delivery and *in vivo* mouse brain Cas9 expression data.

Two ways to package Cas9 coding nucleic acid molecules, e.g., DNA, into viral vectors to mediate genome modification *in vivo* are preferred:

To achieve NHEJ-mediated gene knockout:

Single virus vector:
Vector containing two or more expression cassettes:

Promoter-Cas9 coding nucleic acid molecule -terminator
Promoter-gRNA1-terminator
Promoter-gRNA2-terminator
Promoter-gRNA(N)-terminator (up to size limit of vector)

Double virus vector:

Vector 1 containing one expression cassette for driving the expression of Cas9 Promoter-Cas9 coding nucleic acid molecule-terminator

Vector 2 containing one more expression cassettes for driving the expression of one or more guideRNAs
Promoter-gRNA1-terminator
Promoter-gRNA(N)-terminator (up to size limit of vector)

To mediate homology-directed repair.
In addition to the single and double virus vector approaches described above, an additional vector is used to deliver a homology-direct repair template.

Promoter used to drive Cas9 coding nucleic acid molecule expression can include:

AAV ITR can serve as a promoter: this is advantageous for eliminating the need for an additional promoter element (which can take up space in the vector). The additional space freed up can be used to drive the expression of additional elements (gRNA, etc.). Also, ITR activity is relatively weaker, so can be used to reduce toxicity due to over expression of Cas9.

For ubiquitous expression, can use promoters: CMV, CAG, CBh, PGK, SV40, Ferritin heavy or light chains, etc.
For brain expression, can use promoters: SynapsinI for all neurons, CaMKIIalpha for excitatory neurons, GAD67 or GAD65 or VGAT for GABAergic neurons, etc.
For liver expression, can use Albumin promoter
For lung expression, can use SP-B
For endothelial cells, can use ICAM
For hematopoietic cells can use IFNbeta or CD45
For Osteoblasts can use OG-2
Promoter used to drive guide RNA can include:

Pol III promoters such as U6 or H1
Use of Pol II promoter and intronic cassettes to express gRNA.

As to AAV, the AAV can be AAV1, AAV2, AAV5 or any combination thereof. One can select the AAV of the AAV with regard to the cells to be targeted; e.g., one can select AAV serotypes 1, 2, 5 or a hybrid or capsid AAV1, AAV2, AAV5 or any combination thereof for targeting brain or neuronal cells; and one can select AAV4 for targeting cardiac tissue. AAV8 is useful for delivery to the liver. The above promoters and vectors are preferred individually.

RNA delivery is also a useful method of *in vivo* delivery. It is possible to deliver Cas9 and gRNA (and, for instance, HR repair template) into cells using liposomes or nanoparticles. Thus delivery of the CRISPR enzyme, such as a Cas9 and/or delivery of the RNAs of the invention may be in RNA form and via microvesicles, liposomes or nanoparticles. For example, Cas9 mRNA and gRNA can be packaged into liposomal particles for delivery in vivo. Liposomal transfection reagents such as Invivofectamine from Life Technologies and other reagents on the market can effectively deliver RNA molecules into the liver.

Enhancing NHEJ or HR efficiency is also helpful for delivery. It is preferred that NHEJ efficiency is enhanced by co-expressing end-processing enzymes such as Trex2 (Dumitrache et al. Genetics. 2011 August; 188(4): 787-797). It is preferred that HR efficiency is increased by transiently inhibiting NHEJ machineries such as Ku70 and Ku86. HR efficiency can also be increased by co-expressing prokaryotic or eukaryotic homologous recombination enzymes such as RecBCD, RecA.

Various means of delivery are described herein, and further discussed in this section.

## Delivery

Vector delivery, e.g., plasmid, viral delivery: The CRISPR enzyme, for instance a Cas9, and/or any of the present RNAs, for instance a guide RNA, can be delivered using any suitable vector, e.g., plasmid or viral vectors, such as adeno associated virus (AAV), lentivirus, adenovirus or other viral vector types, or combinations thereof. Cas9 and one or more guide RNAs can be packaged into one or more vectors, e.g., plasmid or viral vectors. In some embodiments, the vector, e.g., plasmid or viral vector is delivered to the tissue of interest by, for example, an intramuscular injection, while other times the delivery is via intravenous, transdermal, intranasal, oral, mucosal, or other delivery methods. Such delivery may be either via a single dose, or multiple doses. One skilled in the art understands that the actual dosage to be delivered herein may vary greatly depending upon a variety of factors, such as the vector choice, the target cell, organism, or tissue, the general condition of the subject to be treated, the degree of transformation/modification sought, the administration route, the administration mode, the type of transformation/modification sought, etc.

Such a dosage may further contain, for example, a carrier (water, saline, ethanol, glycerol, lactose, sucrose, calcium phosphate, gelatin, dextran, agar, pectin, peanut oil, sesame oil, etc.), a diluent, a pharmaceutically-acceptable carrier (e.g., phosphate-buffered saline), a pharmaceutically-acceptable excipient, and/or other compounds known in the art. The dosage may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include microcrystalline cellulose, carboxymethylcellulose sodium, polysorbate 80, phenylethyl alcohol, chlorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991).

In an embodiment herein the delivery is via an adenovirus, which may be at a single booster dose containing at least $1 \times 10^5$ particles (also referred to as particle units, pu) of adenoviral vector. In an embodiment herein, the dose preferably is at least about $1 \times 10^6$ particles (for example, about $1 \times 10^6$-$1 \times 10^{12}$ particles), more preferably at least about $1 \times 10^7$ particles, more preferably at least about $1 \times 10^8$ particles (e.g., about $1 \times 10^8$-$1 \times 10^{11}$ particles or about $1 \times 10^8$-$1 \times 10^{12}$ particles), and most preferably at least about $1 \times 10^0$ particles (e.g., about $1 \times 10^9$-$1 \times 10^{10}$ particles or about $1 \times 10^9$-$1 \times 10^{12}$ particles), or even at least about $1 \times 10^{10}$ particles (e.g., about $1 \times 10^{10}$-$1 \times 10^{12}$ particles) of the adenoviral vector. Alternatively, the dose comprises no more than about $1 \times 10^{14}$ particles, preferably no more than about $1 \times 10^{13}$ particles, even more preferably no more than about $1 \times 10^{12}$ particles, even more preferably no more than about $1 \times 10^{11}$ particles, and most preferably no more than about $1 \times 10^{10}$ particles (e.g., no more than about $1 \times 10^9$ articles). Thus, the dose may contain a single dose of adenoviral vector with, for example, about $1 \times 10^6$ particle units (pu), about $2 \times 10^6$ pu, about $4 \times 10^6$ pu, about $1 \times 10^7$ pu, about $2 \times 10^7$ pu, about $4 \times 10^7$ pu, about $1 \times 10^8$ pu, about $2 \times 10^8$ pu, about $4 \times 10^8$ pu, about $1 \times 10^9$ pu, about $2 \times 10^9$ pu, about $4 \times 10^9$ pu, about $1 \times 10^{10}$ pu, about $2 \times 10^{10}$ pu, about $4 \times 10^{10}$ pu, about $1 \times 10^{11}$ pu, about $2 \times 10^{11}$ pu, about $4 \times 10^{11}$ pu, about $1 \times 10^{12}$ pu, about $2 \times 10^{12}$ pu, or about $4 \times 10^{12}$ pu of adenoviral vector. See, for example, the adenoviral vectors in U.S. Patent No. 8,454,972 B2 to Nabel, et. al., granted on June 4, 2013;, and the dosages at col 29, lines 36-58 thereof. In an embodiment herein, the adenovirus is delivered via multiple doses.

In an embodiment herein, the delivery is via an AAV. A therapeutically effective dosage for in vivo delivery of the AAV to a human is believed to be in the range of from about 20 to about 50 ml of saline solution containing from about $1 \times 10^{10}$ to about $1 \times 10^{10}$ functional AAV/ml solution. The dosage may be adjusted to balance the therapeutic benefit against any side effects. In an embodiment herein, the AAV dose is generally in the range of concentrations of from about $1 \times 10^5$ to $1 \times 10^{50}$ genomes AAV, from about $1 \times 10^8$ to $1 \times 10^{20}$ genomes AAV, from about $1 \times 10^{10}$ to about $1 \times 10^{16}$ genomes, or about $1 \times 10^{11}$ to about $1 \times 10^{16}$ genomes AAV. A human dosage may be about $1 \times 10^{13}$ genomes AAV. Such concentrations may be delivered in from about 0.001 ml to about 100 ml, about 0.05 to about 50 ml, or about 10 to about 25 ml of a carrier solution. Other effective dosages can be readily established by one of ordinary skill in the art through routine trials establishing dose response curves. See, for example, U.S. Patent No. 8,404,658 B2 to Hajjar, et al., granted on March 26, 2013, at col. 27, lines 45-60.

In an embodiment herein the delivery is via a plasmid. In such plasmid compositions, the dosage should be a sufficient amount of plasmid to elicit a response. For instance, suitable quantities of plasmid DNA in plasmid compositions can be from about 0.1 to about 2 mg, or from about 1 μg to about 10 μg per 70 kg individual. Plasmids of the invention will generally comprise (i) a promoter; (ii) a sequence encoding a CRISPR enzyme, operably linked to said promoter; (iii) a selectable marker; (iv) an origin of replication; and (v) a transcription terminator downstream of and operably linked to (ii). The plasmid can also encode the RNA components of a CRISPR complex, but one or more of these may instead be encoded on a different vector.

The doses herein are based on an average 70 kg individual. The frequency of administration is within the ambit of the medical or veterinary practitioner (e.g., physician, veterinarian), or scientist skilled in the art. It is also noted that mice used in experiments are typically about 20g and from mice experiments one can scale up to a 70 kg individual.

In some embodiments the RNA molecules of the invention are delivered in liposome or lipofectin formulations and the like and can be prepared by methods well known to those skilled in the art. Such methods are described, for example, in U.S. Pat. Nos. 5,593,972, 5,589,466, and 5,580,859. Delivery systems aimed specifically at the enhanced and improved delivery of siRNA into mammalian cells have been developed, (see, for example, Shen et al FEBS Let. 2003, 539:111-114; Xia et al., Nat. Biotech. 2002, 20:1006-1010; Reich et al., Mol. Vision. 2003, 9: 210-216; Sorensen et al., J. Mol. Biol. 2003, 327: 761-766; Lewis et al., Nat. Gen. 2002, 32: 107-108 and Simeoni et al., NAR 2003, 31, 11: 2717-2724) and may be applied to the present invention. siRNA has recently been successfully used for inhibition of gene expression in primates (see for example. Tolentino et al., Retina 24(4):660 which may also be applied to the present invention.

Indeed, RNA delivery is a useful method of in vivo delivery. It is possible to deliver Cas9 and gRNA (and, for instance, HR repair template) into cells using liposomes or particle or nanoparticles. Thus delivery of the CRISPR enzyme, such as a Cas9 and/or delivery of the RNAs of the invention may be in RNA form and via microvesicles, liposomes or particle or nanoparticles. For example, Cas9 mRNA and gRNA can be packaged into liposomal particles for delivery in vivo. Liposomal transfection reagents such as lipofectamine from Life Technologies and other reagents on the market can effectively deliver RNA molecules into the liver.

Means of delivery of RNA also preferred include delivery of RNA via particles or nanoparticles (Cho, S., Goldberg, M., Son, S., Xu, Q., Yang, F., Mei, Y., Bogatyrev, S., Langer, R. and Anderson, D., Lipid-like nanoparticles for small interfering RNA delivery to endothelial cells, Advanced Functional Materials, 19: 3112-3118, 2010) or exosomes (Schroeder, A., Levins, C., Cortez, C., Langer, R., and Anderson, D., Lipid-based nanotherapeutics for siRNA delivery, Journal of Internal Medicine, 267: 9-21, 2010, PMID: 20059641). Indeed, exosomes have been shown to be particularly useful in delivery siRNA, a system with some parallels to the CRISPR system. For instance, El-Andaloussi S, et al. ("Exosome-mediated delivery of siRNA in vitro and in vivo." Nat Protoc. 2012 Dec;7(12):2112-26. doi: 10.1038/nprot.2012.131. Epub 2012 Nov 15.) describe how exosomes are promising tools for drug delivery across different biological barriers and can be harnessed for delivery of siRNA in vitro and in vivo. Their approach is to generate targeted exosomes through transfection of an expression vector, comprising an exosomal protein fused with a peptide ligand. The exosomes are then purify and characterized from transfected cell supernatant, then RNA is loaded into the exosomes. Delivery or administration according to the invention can be performed with exosomes, in particular but not limited to the brain. Vitamin E (a-tocopherol) may be conjugated with CRISPR Cas and delivered to the brain along with high density lipoprotein (HDL), for example in a similar manner as was done by Uno et al. (HUMAN GENE THERAPY 22:711-719 (June 2011)) for delivering short-interfering RNA (siRNA) to the brain. Mice were infused via Osmotic minipumps (model 1007D; Alzet, Cupertino, CA) filled with phosphate-buffered saline (PBS) or free TocsiBACE or Toc-siBACE/HDL and connected with Brain Infusion Kit 3 (Alzet). A brain-infusion cannula was placed about 0.5mm posterior to the bregma at midline for infusion into the dorsal third ventricle. Uno et al. found that as little as 3 nmol of Toc-siRNA with HDL could induce a target reduction in comparable degree by the same ICV infusion method. A similar dosage of CRISPR Cas conjugated to $\alpha$-tocopherol and coadministered with HDL targeted to the brain may be contemplated for humans in the present invention, for example, about 3 nmol to about 3 $\mu$mol of CRISPR Cas targeted to the brain may be contemplated. Zou et al. ((HUMAN GENE THERAPY 22:465-475 (April 2011)) describes a method of lentiviral-mediated delivery of short-hairpin RNAs targeting PKCy for in vivo gene silencing in the spinal cord of rats. Zou et al. administered about 10 $\mu$l of a recombinant lentivirus having a titer of $1 \times 10^9$ transducing units (TU)/ml by an intrathecal catheter. A similar dosage of CRISPR Cas expressed in a lentiviral vector targeted to the brain may be contemplated for humans in the present invention, for example, about 10-50 ml of CRISPR Cas targeted to the brain in a lentivirus having a titer of $1 \times 10^9$ transducing units (TU)/ml may be contemplated.

In terms of local delivery to the brain, this can be achieved in various ways. For instance, material can be delivered intrastriatally e.g. by injection. Injection can be performed stereotactically via a craniotomy.

Enhancing NHEJ or HR efficiency is also helpful for delivery. It is preferred that NHEJ efficiency is enhanced by co-expressing end-processing enzymes such as Trex2 (Dumitrache et al. Genetics. 2011 August; 188(4): 787-797). It is preferred that HR efficiency is increased by transiently inhibiting NHEJ machineries such as Ku70 and Ku86. HR efficiency can also be increased by co-expressing prokaryotic or eukaryotic homologous recombination enzymes such as RecBCD, RecA.

## Packaging and Promoters

Ways to package Cas9 coding nucleic acid molecules, e.g., DNA, into vectors, e.g., viral vectors, to mediate genome modification in vivo include:

- To achieve NHEJ-mediated gene knockout:
- Single virus vector:
- Vector containing two or more expression cassettes:
- Promoter-Cas9 coding nucleic acid molecule -terminator
- Promoter-gRNA1-terminator
- Promoter-gRNA2-terminator
- Promoter-gRNA(N)-terminator (up to size limit of vector)
- Double virus vector:
- Vector 1 containing one expression cassette for driving the expression of Cas9
- Promoter-Cas9 coding nucleic acid molecule-terminator
- Vector 2 containing one more expression cassettes for driving the expression of one or more guideRNAs
- Promoter-gRNA1-terminator
- Promoter-gRNA(N)-terminator (up to size limit of vector)

- To mediate homology-directed repair.
- In addition to the single and double virus vector approaches described above, an additional vector may be used to deliver a homology-direct repair template.

The promoter used to drive Cas9 coding nucleic acid molecule expression can include:

- AAV ITR can serve as a promoter: this is advantageous for eliminating the need for an additional promoter element (which can take up space in the vector). The additional space freed up can be used to drive the expression of additional elements (gRNA, etc.). Also, ITR activity is relatively weaker, so can be used to reduce potential toxicity due to over expression of Cas9.
- For ubiquitous expression, any of the following promoters may be used: CMV, CAG, CBh, PGK, SV40, Ferritin heavy or light chains, and so forth.

For brain or other CNS expression, can use promoters: SynapsinI for all neurons, CaMKIIalpha for excitatory neurons, GAD67 or GAD65 or VGAT for GABAergic neurons, etc. For liver expression, one can use the Albumin promoter. For lung expression, one can use the use SP-B. For endothelial cells, one can use the use ICAM. For hematopoietic cells one can use the use IFNbeta or CD45. For Osteoblasts can one can use the OG-2.
The promoter used to drive guide RNA can include:

- Pol III promoters such as U6 or H1
- Use of Pol II promoter and intronic cassettes to express gRNA

**Adeno associated virus (AAV)**

Cas9 and one or more guide RNA can be delivered using adeno associated virus (AAV), lentivirus, adenovirus or other plasmid or viral vector types, in particular, using formulations and doses from, for example, US Patents Nos. 8,454,972 (formulations, doses for adenovirus), 8,404,658 (formulations, doses for AAV) and 5,846,946 (formulations, doses for DNA plasmids) and from clinical trials and publications regarding the clinical trials involving lentivirus, AAV and adenovirus. For examples, for AAV, the route of administration, formulation and dose can be as in US Patent No. 8,454,972 and as in clinical trials involving AAV. For Adenovirus, the route of administration, formulation and dose can be as in US Patent No. 8,404,658 and as in clinical trials involving adenovirus. For plasmid delivery, the route of administration, formulation and dose can be as in US Patent No 5,846,946 and as in clinical studies involving plasmids. Doses may be based on or extrapolated to an average 70 kg individual (e.g. a male adult human), and can be adjusted for patients, subjects, mammals of different weight and species. Frequency of administration is within the ambit of the medical or veterinary practitioner (e.g., physician, veterinarian), depending on usual factors including the age, sex, general health, other conditions of the patient or subject and the particular condition or symptoms being addressed. The viral vectors can be injected into the tissue of interest. For cell-type specific genome modification, the expression of Cas9 can be driven by a cell-type specific promoter. For example, liver-specific expression might use the Albumin promoter and neuron-specific expression (e.g. for targeting CNS disorders) might use the Synapsin I promoter.
In terms of in vivo delivery, AAV is advantageous over other viral vectors for a couple of reasons:
Low toxicity (this may be due to the purification method not requiring ultra centrifugation of cell particles that can activate the immune response).
Low probability of causing insertional mutagenesis because it doesn't integrate into the host genome.
AAV has a packaging limit of 4.5 or 4.75 Kb. This means that Cas9 as well as a promoter and transcription terminator have to be all fit into the same viral vector. Constructs larger than 4.5 or 4.75 Kb will lead to significantly reduced virus production. SpCas9 is quite large, the gene itself is over 4.1 Kb, which makes it difficult for packing into AAV. Therefore embodiments of the invention include utilizing homologs of Cas9 that are shorter. For example:

| Species | Cas9 Size |
| --- | --- |
| Corynebacter diphtheriae | 3252 |
| Eubacterium ventriosum | 3321 |
| Streptococcus pasteurianus | 3390 |
| Lactobacillus farciminis | 3378 |
| Sphaerochaeta globus | 3537 |
| Azospirillum B510 | 3504 |
| Gluconacetobacter diazotrophicus | 3150 |
| Neisseria cinerea | 3246 |

(continued)

| Species | Cas9 Size |
|---|---|
| Roseburia intestinalis | 3420 |
| Parvibaculum lavamentivorans | 3111 |
| Staphylococcus aureus | 3159 |
| Nitratifractor salsuginis DSM 16511 | 3396 |
| Campylobacter lari CF89-12 | 3009 |
| Streptococcus thermophilus LMD-9 | 3396 |

These species are therefore, in general, preferred Cas9 species.

As to AAV, the AAV can be AAV1, AAV2, AAV5 or any combination thereof. One can select the AAV of the AAV with regard to the cells to be targeted; e.g., one can select AAV serotypes 1, 2, 5 or a hybrid capsid AAV1, AAV2, AAV5 or any combination thereof for targeting brain or neuronal cells; and one can select AAV4 for targeting cardiac tissue. AAV8 is useful for delivery to the liver. The herein promoters and vectors are preferred individually. A tabulation of certain AAV serotypes as to these cells (see Grimm, D. et al, J. Virol. 82: 5887-5911 (2008)) is as follows:

| Cell Line | AAV-1 | AAV-2 | AAV-3 | AAV-4 | AAV-5 | AAV-6 | AAV-8 | AAV-9 |
|---|---|---|---|---|---|---|---|---|
| Huh-7 | 13 | 100 | 2.5 | 0.0 | 0.1 | 10 | 0.7 | 0.0 |
| HEK293 | 25 | 100 | 2.5 | 0.1 | 0.1 | 5 | 0.7 | 0.1 |
| HeLa | 3 | 100 | 2.0 | 0.1 | 6.7 | 1 | 0.2 | 0.1 |
| HepG2 | 3 | 100 | 16.7 | 0.3 | 1.7 | 5 | 0.3 | ND |
| Hep1A | 20 | 100 | 0.2 | 1.0 | 0.1 | 1 | 0.2 | 0.0 |
| 911 | 17 | 100 | 11 | 0.2 | 0.1 | 17 | 0.1 | ND |
| CHO | 100 | 100 | 14 | 1.4 | 333 | 50 | 10 | 1.0 |
| COS | 33 | 100 | 33 | 3.3 | 5.0 | 14 | 2.0 | 0.5 |
| MeWo | 10 | 100 | 20 | 0.3 | 6.7 | 10 | 1.0 | 0.2 |
| NIH3T3 | 10 | 100 | 2.9 | 2.9 | 0.3 | 10 | 0.3 | ND |
| A549 | 14 | 100 | 20 | ND | 0.5 | 10 | 0.5 | 0.1 |
| HT1180 | 20 | 100 | 10 | 0.1 | 0.3 | 33 | 0.5 | 0.1 |
| Monocytes | 1111 | 100 | ND | ND | 125 | 1429 | ND | ND |
| Immature DC | 2500 | 100 | ND | ND | 222 | 2857 | ND | ND |
| Mature DC | 2222 | 100 | ND | ND | 333 | 3333 | ND | ND |

## Lentivirus

Lentiviruses are complex retroviruses that have the ability to infect and express their genes in both mitotic and post-mitotic cells. The most commonly known lentivirus is the human immunodeficiency virus (HIV), which uses the envelope glycoproteins of other viruses to target a broad range of cell types.

Lentiviruses may be prepared as follows. After cloning pCasES10 (which contains a lentiviral transfer plasmid backbone), HEK293FT at low passage (p=5) were seeded in a T-75 flask to 50% confluence the day before transfection in DMEM with 10% fetal bovine serum and without antibiotics. After 20 hours, media was changed to OptiMEM (serum-free) media and transfection was done 4 hours later. Cells were transfected with 10 μg of lentiviral transfer plasmid (pCasES10) and the following packaging plasmids: 5 μg of pMD2.G (VSV-g pseudotype), and 7.5ug of psPAX2 (gag/pol/rev/tat). Transfection was done in 4mL OptiMEM with a cationic lipid delivery agent (50uL Lipofectamine 2000 and 100ul Plus reagent). After 6 hours, the media was changed to antibiotic-free DMEM with 10% fetal bovine serum. These methods use serum during cell culture, but serum-free methods are preferred.

Lentivirus may be purified as follows. Viral supernatants were harvested after 48 hours. Supernatants were first cleared of debris and filtered through a 0.45um low protein binding (PVDF) filter. They were then spun in a ultracentrifuge for 2 hours at 24,000 rpm. Viral pellets were resuspended in 50ul of DMEM overnight at 4C. They were then aliquotted and immediately frozen at -80°C.

In another embodiment, minimal non-primate lentiviral vectors based on the equine infectious anemia virus (EIAV) are also contemplated, especially for ocular gene therapy (see, e.g., Balagaan, J Gene Med 2006; 8: 275 - 285). In another embodiment, RetinoStat®, an equine infectious anemia virus-based lentiviral gene therapy vector that expresses angiostatic proteins endostatin and angiostatin that is delivered via a subretinal injection for the treatment of the web form of age-related macular degeneration is also contemplated (see, e.g., Binley et al., HUMAN GENE THERAPY 23:980-991 (September 2012)) and this vector may be modified for the CRISPR-Cas system of the present invention.

In another embodiment, self-inactivating lentiviral vectors with an siRNA targeting a common exon shared by HIV tat/rev, a nucleolar-localizing TAR decoy, and an anti-CCR5-specific hammerhead ribozyme (see, e.g., DiGiusto et al. (2010) Sci Transl Med 2:36ra43) may be used/and or adapted to the CRISPR-Cas system of the present invention. A minimum of $2.5 \times 106$ CD34+ cells per kilogram patient weight may be collected and prestimulated for 16 to 20 hours in X-VIVO 15 medium (Lonza) containing 2 $\mu$mol/L-glutamine, stem cell factor (100 ng/ml), Flt-3 ligand (Flt-3L) (100 ng/ml), and thrombopoietin (10 ng/ml) (CellGenix) at a density of $2 \times 106$ cells/ml. Prestimulated cells may be transduced with lentiviral at a multiplicity of infection of 5 for 16 to 24 hours in 75-cm2 tissue culture flasks coated with fibronectin (25 mg/cm2) (RetroNectin,Takara Bio Inc.).

Lentiviral vectors have been disclosed as in the treatment for Parkinson's Disease, see, e.g., US Patent Publication No. 20120295960 and US Patent Nos. 7303910 and 7351585. Lentiviral vectors have also been disclosed for the treatment of ocular diseases, see e.g., US Patent Publication Nos. 20060281180, 20090007284, US20110117189; US20090017543; US20070054961, US20100317109. Lentiviral vectors have also been disclosed for delivery to the brain, see, e.g., US Patent Publication Nos. US20110293571; US20110293571, US20040013648, US20070025970, US20090111106 and US Patent No. US7259015.

## RNA delivery

RNA delivery: The CRISPR enzyme, for instance a Cas9, and/or any of the present RNAs, for instance a guide RNA, can also be delivered in the form of RNA. Cas9 mRNA can be generated using in vitro transcription. For example, Cas9 mRNA can be synthesized using a PCR cassette containing the following elements: T7_promoter-kozak sequence (GCCACC)-Cas9-3' UTR from beta globin-polyA tail (a string of 120 or more adenines). The cassette can be used for transcription by T7 polymerase. Guide RNAs can also be transcribed using in vitro transcription from a cassette containing T7_promoter-GG-guide RNA sequence.

To enhance expression and reduce possible toxicity, the CRISPR enzyme-coding sequence and/or the guide RNA can be modified to include one or more modified nucleoside e.g. using pseudo-U or 5-Methyl-C.

mRNA delivery methods are especially promising for liver delivery currently.

Much clinical work on RNA delivery has focused on RNAi or antisense, but these systems can be adapted for delivery of RNA for implementing the present invention. References below to RNAi etc. should be read accordingly.

## Nanoparticles

Nanoparticles are a type of particle.

CRISPR enzyme mRNA and guide RNA may be delivered simultaneously using nanoparticles or lipid envelopes.

For example, Su X, Fricke J, Kavanagh DG, Irvine DJ ("In vitro and in vivo mRNA delivery using lipid-enveloped pH-responsive polymer nanoparticles" Mol Pharm. 2011 Jun 6;8(3):774-87. doi: 10.1021/mp100390w. Epub 2011 Apr 1) describes biodegradable core-shell structured nanoparticles with a poly($\beta$-amino ester) (PBAE) core enveloped by a phospholipid bilayer shell. These were developed for in vivo mRNA delivery. The pH-responsive PBAE component was chosen to promote endosome disruption, while the lipid surface layer was selected to minimize toxicity of the polycation core. Such are, therefore, preferred for delivering RNA of the present invention.

In one embodiment, nanoparticles based on self assembling bioadhesive polymers are contemplated, which may be applied to oral delivery of peptides, intravenous delivery of peptides and nasal delivery of peptides, all to the brain. Other embodiments, such as oral absorption and ocular delivery of hydrophobic drugs are also contemplated. The molecular envelope technology involves an engineered polymer envelope which is protected and delivered to the site of the disease (see, e.g., Mazza, M. et al. ACSNano, 2013. 7(2): 1016-1026; Siew, A., et al. Mol Pharm, 2012. 9(1): 14-28; Lalatsa, A., et al. J Contr Rel, 2012. 161(2):523-36; Lalatsa, A., et al., Mol Pharm, 2012. 9(6):1665-80; Lalatsa, A., et al. Mol Pharm, 2012. 9(6):1764-74; Garrett, N.L., et al. J Biophotonics, 2012. 5(5-6):458-68; Garrett, N.L., et al. J Raman Spect, 2012. 43(5):681-688; Ahmad, S., et al. J Royal Soc Interface 2010. 7:S423-33; Uchegbu, I.F. Expert Opin Drug Deliv, 2006. 3(5):629-40; Qu, X.,et al. Biomacromolecules, 2006. 7(12):3452-9 and Uchegbu, I.F., et al. Int J Pharm, 2001. 224:185-199). Doses of about 5 mg/kg are contemplated, with single or multiple doses, depending on the target tissue.

In one embodiment, nanoparticles that can deliver RNA to a cancer cell to stop tumor growth developed by Dan Anderson's lab at MIT may be used/and or adapted to the CRISPR Cas system of the present invention. In particular, the Anderson lab developed fully automated, combinatorial systems for the synthesis, purification, characterization, and formulation of new biomaterials and nanoformulations. See, e.g., Alabi et al., Proc Natl Acad Sci USA. 2013 Aug 6;110(32):12881-6; Zhang et al., Adv Mater. 2013 Sep 6;25(33):4641-5; Jiang et al., Nano Lett. 2013 Mar 13;13(3):1059-64; Karagiannis et al., ACS Nano. 2012 Oct 23;6(10):8484-7; Whitehead et al., ACS Nano. 2012 Aug 28;6(8):6922-9 and Lee et al., Nat Nanotechnol. 2012 Jun 3;7(6):389-93.

US patent application 20110293703 relates to lipidoid compounds are also particularly useful in the administration of polynucleotides, which may be applied to deliver the CRISPR Cas system of the present invention. In one aspect, the aminoalcohol lipidoid compounds are combined with an agent to be delivered to a cell or a subject to form microparticles, nanoparticles, liposomes, or micelles. The agent to be delivered by the particles, liposomes, or micelles may be in the form of a gas, liquid, or solid, and the agent may be a polynucleotide, protein, peptide, or small molecule. The minoalcohol lipidoid compounds may be combined with other aminoalcohol lipidoid compounds, polymers (synthetic or natural), surfactants, cholesterol, carbohydrates, proteins, lipids, etc. to form the particles. These particles may then optionally be combined with a pharmaceutical excipient to form a pharmaceutical composition.

US Patent Publication No. 20110293703 also provides methods of preparing the aminoalcohol lipidoid compounds. One or more equivalents of an amine are allowed to react with one or more equivalents of an epoxide-terminated compound under suitable conditions to form an aminoalcohol lipidoid compound of the present invention. In certain embodiments, all the amino groups of the amine are fully reacted with the epoxide-terminated compound to form tertiary amines. In other embodiments, all the amino groups of the amine are not fully reacted with the epoxide-terminated compound to form tertiary amines thereby resulting in primary or secondary amines in the aminoalcohol lipidoid compound. These primary or secondary amines are left as is or may be reacted with another electrophile such as a different epoxide-terminated compound. As will be appreciated by one skilled in the art, reacting an amine with less than excess of epoxide-terminated compound will result in a plurality of different aminoalcohol lipidoid compounds with various numbers of tails. Certain amines may be fully functionalized with two epoxide-derived compound tails while other molecules will not be completely functionalized with epoxide-derived compound tails. For example, a diamine or polyamine may include one, two, three, or four epoxide-derived compound tails off the various amino moieties of the molecule resulting in primary, secondary, and tertiary amines. In certain embodiments, all the amino groups are not fully functionalized. In certain embodiments, two of the same types of epoxide-terminated compounds are used. In other embodiments, two or more different epoxide-terminated compounds are used. The synthesis of the aminoalcohol lipidoid compounds is performed with or without solvent, and the synthesis may be performed at higher temperatures ranging from 30-100 °C., preferably at approximately 50-90 °C. The prepared aminoalcohol lipidoid compounds may be optionally purified. For example, the mixture of aminoalcohol lipidoid compounds may be purified to yield an aminoalcohol lipidoid compound with a particular number of epoxide-derived compound tails. Or the mixture may be purified to yield a particular stereo- or regioisomer. The aminoalcohol lipidoid compounds may also be alkylated using an alkyl halide (e.g., methyl iodide) or other alkylating agent, and/or they may be acylated.

US Patent Publication No. 20110293703 also provides libraries of aminoalcohol lipidoid compounds prepared by the inventive methods. These aminoalcohol lipidoid compounds may be prepared and/or screened using high-throughput techniques involving liquid handlers, robots, microtiter plates, computers, etc. In certain embodiments, the aminoalcohol lipidoid compounds are screened for their ability to transfect polynucleotides or other agents (e.g., proteins, peptides, small molecules) into the cell.

US Patent Publication No. 20130302401 relates to a class of poly(beta-amino alcohols) (PBAAs) has been prepared using combinatorial polymerization. The inventive PBAAs may be used in biotechnology and biomedical applications as coatings (such as coatings of films or multilayer films for medical devices or implants), additives, materials, excipients, non-biofouling agents, micropatterning agents, and cellular encapsulation agents. When used as surface coatings, these PBAAs elicited different levels of inflammation, both in vitro and in vivo, depending on their chemical structures. The large chemical diversity of this class of materials allowed us to identify polymer coatings that inhibit macrophage activation in vitro. Furthermore, these coatings reduce the recruitment of inflammatory cells, and reduce fibrosis, following the subcutaneous implantation of carboxylated polystyrene microparticles. These polymers may be used to form polyelectrolyte complex capsules for cell encapsulation. The invention may also have many other biological applications such as antimicrobial coatings, DNA or siRNA delivery, and stem cell tissue engineering. The teachings of US Patent Publication No. 20130302401 may be applied to the CRISPR Cas system of the present invention.

In another embodiment, lipid nanoparticles (LNPs) are contemplated. An antitransthyretin small interfering RNA has been encapsulated in lipid nanoparticles and delivered to humans (see, e.g., Coelho et al., N Engl J Med 2013;369:819-29), and such a ssystem may be adapted and applied to the CRISPR Cas system of the present invention. Doses of about 0.01 to about 1 mg per kg of body weight administered intravenously are contemplated. Medications to reduce the risk of infusion-related reactions are contemplated, such as dexamethasone, acetampinophen, diphenhydramine or cetirizine, and ranitidine are contemplated. Multiple doses of about 0.3 mg per kilogram every 4 weeks for five doses are also contemplated.

LNPs have been shown to be highly effective in delivering siRNAs to the liver (see, e.g., Tabernero et al., Cancer Discovery, April 2013, Vol. 3, No. 4, pages 363-470) and are therefore contemplated for delivering RNA encoding CRISPR Cas to the liver. A dosage of about four doses of 6 mg/kg of the LNP every two weeks may be contemplated. Tabernero et al. demonstrated that tumor regression was observed after the first 2 cycles of LNPs dosed at 0.7 mg/kg, and by the end of 6 cycles the patient had achieved a partial response with complete regression of the lymph node metastasis and substantial shrinkage of the liver tumors. A complete response was obtained after 40 doses in this patient, who has remained in remission and completed treatment after receiving doses over 26 months. Two patients with RCC and extrahepatic sites of disease including kidney, lung, and lymph nodes that were progressing following prior therapy with VEGF pathway inhibitors had stable disease at all sites for approximately 8 to 12 months, and a patient with PNET and liver metastases continued on the extension study for 18 months (36 doses) with stable disease.

However, the charge of the LNP must be taken into consideration. As cationic lipids combined with negatively charged lipids to induce nonbilayer structures that facilitate intracellular delivery. Because charged LNPs are rapidly cleared from circulation following intravenous injection, ionizable cationic lipids with pKa values below 7 were developed (see, e.g., Rosin et al, Molecular Therapy, vol. 19, no. 12, pages 1286-2200, Dec. 2011). Negatively charged polymers such as RNA may be loaded into LNPs at low pH values (e.g., pH 4) where the ionizable lipids display a positive charge. However, at physiological pH values, the LNPs exhibit a low surface charge compatible with longer circulation times. Four species of ionizable cationic lipids have been focused upon, namely 1,2-dilineoyl-3-dimethylammonium-propane (DLinDAP), 1,2-dilinoleyloxy-3-N,N-dimethylaminopropane (DLinDMA), 1,2-dilinoleyloxy-keto-N,N-dimethyl-3-aminopropane (DLinKDMA), and 1,2-dilinoleyl-4-(2-dimethylaminoethyl)-[1,3]-dioxolane (DLinKC2-DMA). It has been shown that LNP siRNA systems containing these lipids exhibit remarkably different gene silencing properties in hepatocytes in vivo, with potencies varying according to the series DLinKC2-DMA>DLinKDMA>DLinDMA>>DLinDAP employing a Factor VII gene silencing model (see, e.g., Rosin et al, Molecular Therapy, vol. 19, no. 12, pages 1286-2200, Dec. 2011). A dosage of 1 μg/ml of LNP or CRISPR-Cas RNA in or associated with the LNP may be contemplated, especially for a formulation containing DLinKC2-DMA.

Preparation of LNPs and CRISPR Cas encapsulation may be used/and or adapted from Rosin et al, Molecular Therapy, vol. 19, no. 12, pages 1286-2200, Dec. 2011). The cationic lipids 1,2-dilineoyl-3-dimethylammonium-propane (DLinDAP), 1,2-dilinoleyloxy-3-N,N-dimethylaminopropane (DLinDMA), 1,2-dilinoleyloxyketo-N,N-dimethyl-3-aminopropane (DLinK-DMA), 1,2-dilinoleyl-4-(2-dimethylaminoethyl)-[1,3]-dioxolane (DLinKC2-DMA), (3-o-[2"-(methoxypolyethyleneglycol 2000) succinoyl]-1,2-dimyristoyl-sn-glycol (PEG-S-DMG), and R-3-[(ω-methoxy-poly(ethylene glycol)2000) carbamoyl]-1,2-dimyristyloxlpropyl-3-amine (PEG-C-DOMG) may be provided by Tekmira Pharmaceuticals (Vancouver, Canada) or synthesized. Cholesterol may be purchased from Sigma (St Louis, MO). The specific CRISPR Cas RNA may be encapsulated in LNPs containing DLinDAP, DLinDMA, DLinK-DMA, and DLinKC2-DMA (cationic lipid:DSPC:CHOL: PEGS-DMG or PEG-C-DOMG at 40:10:40:10 molar ratios). When required, 0.2% SP-DiOC18 (Invitrogen, Burlington, Canada) may be incorporated to assess cellular uptake, intracellular delivery, and biodistribution. Encapsulation may be performed by dissolving lipid mixtures comprised of cationic lipid:DSPC:cholesterol:PEG-c-DOMG (40:10:40:10 molar ratio) in ethanol to a final lipid concentration of 10 mmol/l. This ethanol solution of lipid may be added drop-wise to 50 mmol/l citrate, pH 4.0 to form multilamellar vesicles to produce a final concentration of 30% ethanol vol/vol. Large unilamellar vesicles may be formed following extrusion of multilamellar vesicles through two stacked 80 nm Nuclepore polycarbonate filters using the Extruder (Northern Lipids, Vancouver, Canada). Encapsulation may be achieved by adding RNA dissolved at 2 mg/ml in 50 mmol/l citrate, pH 4.0 containing 30% ethanol vol/vol drop-wise to extruded preformed large unilamellar vesicles and incubation at 31 °C for 30 minutes with constant mixing to a final RNA/lipid weight ratio of 0.06/1 wt/wt. Removal of ethanol and neutralization of formulation buffer were performed by dialysis against phosphate-buffered saline (PBS), pH 7.4 for 16 hours using Spectra/Por 2 regenerated cellulose dialysis membranes. Nanoparticle size distribution may be determined by dynamic light scattering using a NICOMP 370 particle sizer, the vesicle/intensity modes, and Gaussian fitting (Nicomp Particle Sizing, Santa Barbara, CA). The particle size for all three LNP systems may be ~70 nm in diameter. RNA encapsulation efficiency may be determined by removal of free RNA using VivaPureD MiniH columns (Sartorius Stedim Biotech) from samples collected before and after dialysis. The encapsulated RNA may be extracted from the eluted nanoparticles and quantified at 260 nm. RNA to lipid ratio was determined by measurement of cholesterol content in vesicles using the Cholesterol E enzymatic assay from Wako Chemicals USA (Richmond, VA). In conjunction with the herein discussion of LNPs and PEG lipids, PEGylated liposomes or LNPs are likewise suitable for delivery of a CRISPR-Cas system or components thereof.

Preparation of large LNPs may be used/and or adapted from Rosin et al, Molecular Therapy, vol. 19, no. 12, pages 1286-2200, Dec. 2011. A lipid premix solution (20.4 mg/ml total lipid concentration) may be prepared in ethanol containing DLinKC2-DMA, DSPC, and cholesterol at 50:10:38.5 molar ratios. Sodium acetate may be added to the lipid premix at a molar ratio of 0.75:1 (sodium acetate:DLinKC2-DMA). The lipids may be subsequently hydrated by combining the mixture with 1.85 volumes of citrate buffer (10 mmol/l, pH 3.0) with vigorous stirring, resulting in spontaneous liposome formation in aqueous buffer containing 35% ethanol. The liposome solution may be incubated at 37 °C to allow for time-dependent increase in particle size. Aliquots may be removed at various times during incubation to investigate changes in liposome size by dynamic light scattering (Zetasizer Nano ZS, Malvern Instruments, Worcestershire, UK). Once the desired particle size is achieved, an aqueous PEG lipid solution (stock = 10 mg/ml PEG-DMG in 35% (vol/vol) ethanol) may be added to the liposome mixture to yield a final PEG molar concentration of 3.5% of total lipid. Upon addition of PEG-lipids, the liposomes should their size, effectively quenching further growth. RNA may then be added to the empty liposomes at an RNA to total lipid ratio of approximately 1:10 (wt:wt), followed by incubation for 30 minutes at 37 °C to form loaded LNPs. The mixture may be subsequently dialyzed overnight in PBS and filtered with a 0.45-$\mu$m syringe filter.

Spherical Nucleic Acid (SNA™) constructs and other nanoparticles (particularly gold nanoparticles) are also contemplated as a means to delivery CRISPR-Cas system to intended targets. Significant data show that AuraSense Therapeutics' Spherical Nucleic Acid (SNA™) constructs, based upon nucleic acid-functionalized gold nanoparticles, are useful.

Literature that may be employed in conjunction with herein teachings include: Cutler et al., J. Am. Chem. Soc. 2011 133:9254-9257, Hao et al., Small. 2011 7:3158-3162, Zhang et al., ACS Nano. 2011 5:6962-6970, Cutler et al., J. Am. Chem. Soc. 2012 134:1376-1391, Young et al., Nano Lett. 2012 12:3867-71, Zheng et al., Proc. Natl. Acad. Sci. USA. 2012 109:11975-80, Mirkin, Nanomedicine 2012 7:635-638 Zhang et al., J. Am. Chem. Soc. 2012 134:16488-1691, Weintraub, Nature 2013 495:S14-S16, Choi et al., Proc. Natl. Acad. Sci. USA. 2013 110(19):7625-7630, Jensen et al., Sci. Transl. Med. 5, 209ra152 (2013) and Mirkin, et al., Small, 10:186-192.

Self-assembling nanoparticles with RNA may be constructed with polyethyleneimine (PEI) that is PEGylated with an Arg-Gly-Asp (RGD) peptide ligand attached at the distal end of the polyethylene glycol (PEG). This system has been used, for example, as a means to target tumor neovasculature expressing integrins and deliver siRNA inhibiting vascular endothelial growth factor receptor-2 (VEGF R2) expression and thereby achieve tumor angiogenesis (see, e.g., Schiffelers et al., Nucleic Acids Research, 2004, Vol. 32, No. 19). Nanoplexes may be prepared by mixing equal volumes of aqueous solutions of cationic polymer and nucleic acid to give a net molar excess of ionizable nitrogen (polymer) to phosphate (nucleic acid) over the range of 2 to 6. The electrostatic interactions between cationic polymers and nucleic acid resulted in the formation of polyplexes with average particle size distribution of about 100 nm, hence referred to here as nanoplexes. A dosage of about 100 to 200 mg of CRISPR Cas is envisioned for delivery in the self-assembling nanoparticles of Schiffelers et al.

The nanoplexes of Bartlett et al. (PNAS, September 25, 2007,vol. 104, no. 39) may also be applied to the present invention. The nanoplexes of Bartlett et al. are prepared by mixing equal volumes of aqueous solutions of cationic polymer and nucleic acid to give a net molar excess of ionizable nitrogen (polymer) to phosphate (nucleic acid) over the range of 2 to 6. The electrostatic interactions between cationic polymers and nucleic acid resulted in the formation of polyplexes with average particle size distribution of about 100 nm, hence referred to here as nanoplexes. The DOTA-siRNA of Bartlett et al. was synthesized as follows: 1,4,7,10-tetraazacyclododecane-1,4,7,10-tetraacetic acid mono(N-hydroxysuccinimide ester) (DOTA-NHSester) was ordered from Macrocyclics (Dallas, TX). The amine modified RNA sense strand with a 100-fold molar excess of DOTA-NHS-ester in carbonate buffer (pH 9) was added to a microcentrifuge tube. The contents were reacted by stirring for 4 h at room temperature. The DOTA-RNAsense conjugate was ethanol-precipitated, resuspended in water, and annealed to the unmodified antisense strand to yield DOTA-siRNA. All liquids were pretreated with Chelex-100 (Bio-Rad, Hercules, CA) to remove trace metal contaminants. Tf-targeted and nontargeted siRNA nanoparticles may be formed by using cyclodextrin-containing polycations. Typically, nanoparticles were formed in water at a charge ratio of 3 (+/-) and an siRNA concentration of 0.5 g/liter. One percent of the adamantane-PEG molecules on the surface of the targeted nanoparticles were modified with Tf (adamantane-PEG-Tf). The nanoparticles were suspended in a 5% (wt/vol) glucose carrier solution for injection.

Davis et al. (Nature, Vol 464, 15 April 2010) conducts a RNA clinical trial that uses a targeted nanoparticle-delivery system (clinical trial registration number NCT00689065). Patients with solid cancers refractory to standard-of-care therapies are administered doses of targeted nanoparticles on days 1, 3, 8 and 10 of a 21-day cycle by a 30-min intravenous infusion. The nanoparticles consist of a synthetic delivery system containing: (1) a linear, cyclodextrin-based polymer (CDP), (2) a human transferrin protein (TF) targeting ligand displayed on the exterior of the nanoparticle to engage TF receptors (TFR) on the surface of the cancer cells, (3) a hydrophilic polymer (polyethylene glycol (PEG) used to promote nanoparticle stability in biological fluids), and (4) siRNA designed to reduce the expression of the RRM2 (sequence used in the clinic was previously denoted siR2B+5). The TFR has long been known to be upregulated in malignant cells, and RRM2 is an established anti-cancer target. These nanoparticles (clinical version denoted as CALAA-01) have been shown to be well tolerated in multi-dosing studies in non-human primates. Although a single patient with chronic myeloid leukaemia has been administered siRNAby liposomal delivery, Davis et al.'s clinical trial is the initial human trial to systemically deliver siRNA with a targeted delivery system and to treat patients with solid cancer. To ascertain whether the targeted delivery system can provide effective delivery of functional siRNA to human tumours, Davis et al. investigated biopsies from three patients from three different dosing cohorts; patients A, B and C, all of whom had metastatic melanoma and received CALAA-01 doses of 18, 24 and 30 mg m$^{-2}$ siRNA, respectively. Similar doses may also be contemplated for the CRISPR Cas system of the present invention. The delivery of the invention may be achieved with nanoparticles containing a linear, cyclodextrin-based polymer (CDP), a human transferrin protein (TF) targeting ligand displayed on the exterior of the nanoparticle to engage TF receptors (TFR) on the surface of the cancer cells and/or a hydrophilic polymer (for example, polyethylene glycol (PEG) used to promote nanoparticle stability in biological fluids).

In terms of this invention, it is preferred to have one or more components of CRISPR complex, e.g., CRISPR enzyme or mRNA or guide RNA delivered using nanoparticles or lipid envelopes. Other delivery systems or vectors are may be used in conjunction with the nanoparticle aspects of the invention.

In general, a "nanoparticle" refers to any particle having a diameter of less than 1000 nm. In certain preferred embodiments, nanoparticles of the invention have a greatest dimension (e.g., diameter) of 500 nm or less. In other preferred embodiments, nanoparticles of the invention have a greatest dimension ranging between 25 nm and 200 nm. In other preferred embodiments, nanoparticles of the invention have a greatest dimension of 100 nm or less. In other preferred embodiments, nanoparticles of the invention have a greatest dimension ranging between 35 nm and 60 nm.

Nanoparticles encompassed in the present invention may be provided in different forms, e.g., as solid nanoparticles (e.g., metal such as silver, gold, iron, titanium), non-metal, lipid-based solids, polymers), suspensions of nanoparticles, or combinations thereof. Metal, dielectric, and semiconductor nanoparticles may be prepared, as well as hybrid structures (e.g., core-shell nanoparticles). Nanoparticles made of semiconducting material may also be labeled quantum dots if they are small enough (typically sub 10 nm) that quantization of electronic energy levels occurs. Such nanoscale particles are used in biomedical applications as drug carriers or imaging agents and may be adapted for similar purposes in the present invention.

Semi-solid and soft nanoparticles have been manufactured, and are within the scope of the present invention. A prototype nanoparticle of semi-solid nature is the liposome. Various types of liposome nanoparticles are currently used clinically as delivery systems for anticancer drugs and vaccines. Nanoparticles with one half hydrophilic and the other half hydrophobic are termed Janus particles and are particularly effective for stabilizing emulsions. They can self-assemble at water/oil interfaces and act as solid surfactants.

US Patent No. 8,709,843, provides a drug delivery system for targeted delivery of therapeutic agent-containing particles to tissues, cells, and intracellular compartments. The invention provides targeted particles comprising comprising polymer conjugated to a surfactant, hydrophilic polymer or lipid.

US Patent No. 6,007,845, provides particles which have a core of a multiblock copolymer formed by covalently linking a multifunctional compound with one or more hydrophobic polymers and one or more hydrophilic polymers, and conatin a biologically active material.

US Patent No. 5,855,913, provides a particulate composition having aerodynamically light particles having a tap density of less than 0.4 g/cm3 with a mean diameter of between 5 $\mu$m and 30 $\mu$m, incorporating a surfactant on the surface thereof for drug delivery to the pulmonary system.

US Patent No. 5,985,309, provides particles incorporating a surfactant and/or a hydrophilic or hydrophobic complex of a positively or negatively charged therapeutic or diagnostic agent and a charged molecule of opposite charge for delivery to the pulmonary system.

US. Patent No. 5,543,158, provides biodegradable injectable nanoparticles having a biodegradable solid core containing a biologically active material and poly(alkylene glycol) moieties on the surface.

WO2012135025 (also published as US20120251560), describes conjugated polyethyleneimine (PEI) polymers and conjugated aza-macrocycles (collectively referred to as "conjugated lipomer" or "lipomers"). In certain embodiments, it can envisioned that such conjugated lipomers can be used in the context of the CRISPR-Cas system to achieve in vitro, ex vivo and in vivo genomic perturbations to modify gene expression, including modulation of protein expression.

In one embodiment, the nanoparticle may be epoxide-modified lipid-polymer, advantageously 7C1 (see, e.g., James E. Dahlman and Carmen Barnes et al. Nature Nanotechnology (2014) published online 11 May 2014, doi:10.1038/nnano.2014.84). C71 was synthesized by reacting C15 epoxide-terminated lipids with PEI600 at a 14:1 molar ratio, and was formulated with C14PEG2000 to produce nanoparticles (diameter between 35 and 60 nm) that were stable in PBS solution for at least 40 days.

An epoxide-modified lipid-polymer may be utilized to deliver the CRISPR-Cas system of the present invention to pulmonary, cardiovascular or renal cells, however, one of skill in the art may adapt the system to deliver to other target organs. Dosage ranging from about 0.05 to about 0.6 mg/kg are envisioned. Dosages over several days or weeks are also envisioned, with a total dosage of about 2 mg/kg.

**Particle delivery systems and/or formulations:**

Several types of particle delivery systems and/or formulations are known to be useful in a diverse spectrum of biomedical applications. In general, a particle is defined as a small object that behaves as a whole unit with respect to its transport and properties. Particles are further classified according to diameter Coarse particles cover a range between 2,500 and 10,000 nanometers. Fine particles are sized between 100 and 2,500 nanometers. Ultrafine particles, or nanoparticles, are generally between 1 and 100 nanometers in size. The basis of the 100-nm limit is the fact that novel properties that differentiate particles from the bulk material typically develop at a critical length scale of under 100 nm.

As used herein, a particle delivery system/formulation is defined as any biological delivery system/formulation which includes a particle in accordance with the present invention. A particle in accordance with the present invention is any entity having a greatest dimension (e.g. diameter) of less than 100 microns ($\mu$m). In some embodiments, inventive particles have a greatest dimension of less than 10 $\mu$m. In some embodiments, inventive particles have a greatest dimension of less than 2000 nanometers (nm). In some embodiments, inventive particles have a greatest dimension of less than 1000 nanometers (nm). In some embodiments, inventive particles have a greatest dimension of less than 900 nm, 800 nm, 700 nm, 600 nm, 500 nm, 400 nm, 300 nm, 200 nm, or 100 nm. Typically, inventive particles have a greatest dimension (e.g., diameter) of 500 nm or less. In some embodiments, inventive particles have a greatest dimension (e.g., diameter) of 250 nm or less. In some embodiments, inventive particles have a greatest dimension (e.g., diameter) of 200 nm or less. In some embodiments, inventive particles have a greatest dimension (e.g., diameter) of 150 nm or less. In some embodiments, inventive particles have a greatest dimension (e.g., diameter) of 100 nm or less. Smaller particles, e.g., having a greatest dimension of 50 nm or less are used in some embodiments of the invention. In some embodiments, inventive particles have a greatest dimension ranging between 25 nm and 200 nm.

Particle characterization (including e.g., characterizing morphology, dimension, etc.) is done using a variety of different techniques. Common techniques are electron microscopy (TEM, SEM), atomic force microscopy (AFM), dynamic light scattering (DLS), X-ray photoelectron spectroscopy (XPS), powder X-ray diffraction (XRD), Fourier transform infrared spectroscopy (FTIR), matrix-assisted laser desorption/ionization time-of-flight mass spectrometry (MALDI-TOF), ultraviolet-visible spectroscopy, dual polarisation interferometry and nuclear magnetic resonance (NMR). Characterization (dimension measurements) may be made as to native particles (i.e., preloading) or after loading of the cargo (herein cargo refers to e.g., one or more components of CRISPR-Cas system e.g., CRISPR enzyme or mRNA or guide RNA, or any combination thereof, and may include additional carriers and/or excipients) to provide particles of an optimal size for delivery for any *in vitro, ex vivo* and/or *in vivo* application of the present invention. In certain preferred embodiments, particle dimension (e.g., diameter) characterization is based on measurements using dynamic laser scattering (DLS). Mention is made of US Patent No. 8,709,843; US Patent No. 6,007,845; US Patent No. 5,855,913; US Patent No. 5,985,309; US. Patent No. 5,543,158; and the publication by James E. Dahlman and Carmen Barnes et al. Nature Nanotechnology (2014) published online 11 May 2014, doi:10.1038/nnano.2014.84, concerning particles, methods of making and using them and measurements thereof.

Particles delivery systems within the scope of the present invention may be provided in any form, including but not limited to solid, semi-solid, emulsion, or colloidal particles. As such any of the delivery systems described herein, including but not limited to, e.g., lipid-based systems, liposomes, micelles, microvesicles, exosomes, or gene gun may be provided as particle delivery systems within the scope of the present invention.

## Exosomes

Exosomes are endogenous nano-vesicles that transport RNAs and proteins, and which can deliver RNA to the brain and other target organs. To reduce immunogenicity, Alvarez-Erviti et al. (2011, Nat Biotechnol 29: 341) used self-derived dendritic cells for exosome production. Targeting to the brain was achieved by engineering the dendritic cells to express Lamp2b, an exosomal membrane protein, fused to the neuron-specific RVG peptide. Purified exosomes were loaded with exogenous RNA by electroporation. Intravenously injected RVG-targeted exosomes delivered GAPDH siRNA specifically to neurons, microglia, oligodendrocytes in the brain, resulting in a specific gene knockdown. Pre-exposure to RVG exosomes did not attenuate knockdown, and non-specific uptake in other tissues was not observed. The therapeutic potential of exosome-mediated siRNA delivery was demonstrated by the strong mRNA (60%) and protein (62%) knockdown of BACE1, a therapeutic target in Alzheimer's disease.

To obtain a pool of immunologically inert exosomes, Alvarez-Erviti et al. harvested bone marrow from inbred C57BL/6 mice with a homogenous major histocompatibility complex (MHC) haplotype. As immature dendritic cells produce large quantities of exosomes devoid of T-cell activators such as MHC-II and CD86, Alvarez-Erviti et al. selected for dendritic cells with granulocyte/macrophage-colony stimulating factor (GM-CSF) for 7 d. Exosomes were purified from the culture supernatant the following day using well-established ultracentrifugation protocols. The exosomes produced were physically homogenous, with a size distribution peaking at 80 nm in diameter as determined by nanoparticle tracking analysis (NTA) and electron microscopy. Alvarez-Erviti et al. obtained 6-12 $\mu$g of exosomes (measured based on protein concentration) per $10^6$ cells.

Next, Alvarez-Erviti et al. investigated the possibility of loading modified exosomes with exogenous cargoes using electroporation protocols adapted for nanoscale applications. As electroporation for membrane particles at the nanometer scale is not well-characterized, nonspecific Cy5-labeled RNA was used for the empirical optimization of the electroporation protocol. The amount of encapsulated RNA was assayed after ultracentrifugation and lysis of exosomes. Electroporation at 400 V and 125 $\mu$F resulted in the greatest retention of RNA and was used for all subsequent experiments.

Alvarez-Erviti et al. administered 150 μg of each BACE1 siRNA encapsulated in 150 μg of RVG exosomes to normal C57BL/6 mice and compared the knockdown efficiency to four controls: untreated mice, mice injected with RVG exosomes only, mice injected with BACE1 siRNA complexed to an in vivo cationic liposome reagent and mice injected with BACE1 siRNA complexed to RVG-9R, the RVG peptide conjugated to 9 D-arginines that electrostatically binds to the siRNA. Cortical tissue samples were analyzed 3 d after administration and a significant protein knockdown (45%, P < 0.05, versus 62%, P < 0.01) in both siRNA-RVG-9R-treated and siRNARVG exosome-treated mice was observed, resulting from a significant decrease in BACE1 mRNA levels (66% [+ or -] 15%, P < 0.001 and 61% [+ or -] 13% respectively, P < 0.01). Moreover, Applicants demonstrated a significant decrease (55%, P < 0.05) in the total [beta]-amyloid 1-42 levels, a main component of the amyloid plaques in Alzheimer's pathology, in the RVG-exosome-treated animals. The decrease observed was greater than the β-amyloid 1-40 decrease demonstrated in normal mice after intraventricular injection of BACE1 inhibitors. Alvarez-Erviti et al. carried out 5'-rapid amplification of cDNA ends (RACE) on BACE1 cleavage product, which provided evidence of RNAi-mediated knockdown by the siRNA.

Finally, Alvarez-Erviti et al. investigated whether RNA-RVG exosomes induced immune responses in vivo by assessing IL-6, IP-10, TNFα and IFN-α serum concentrations. Following exosome treatment, nonsignificant changes in all cytokines were registered similar to siRNA-transfection reagent treatment in contrast to siRNA-RVG-9R, which potently stimulated IL-6 secretion, confirming the immunologically inert profile of the exosome treatment. Given that exosomes encapsulate only 20% of siRNA, delivery with RVG-exosome appears to be more efficient than RVG-9R delivery as comparable mRNA knockdown and greater protein knockdown was achieved with fivefold less siRNA without the corresponding level of immune stimulation. This experiment demonstrated the therapeutic potential of RVG-exosome technology, which is potentially suited for long-term silencing of genes related to neurodegenerative diseases. The exosome delivery system of Alvarez-Erviti et al. may be applied to deliver the CRISPR-Cas system of the present invention to therapeutic targets, especially neurodegenerative diseases. A dosage of about 100 to 1000 mg of CRISPR Cas encapsulated in about 100 to 1000 mg of RVG exosomes may be contemplated for the present invention.

El-Andaloussi et al. (Nature Protocols 7,2112-2126(2012)) discloses how exosomes derived from cultured cells can be harnessed for delivery of RNA in vitro and in vivo. This protocol first describes the generation of targeted exosomes through transfection of an expression vector, comprising an exosomal protein fused with a peptide ligand. Next, El-Andaloussi et al. explain how to purify and characterize exosomes from transfected cell supernatant. Next, El-Andaloussi et al. detail crucial steps for loading RNA into exosomes. Finally, El-Andaloussi et al. outline how to use exosomes to efficiently deliver RNA in vitro and in vivo in mouse brain. Examples of anticipated results in which exosome-mediated RNA delivery is evaluated by functional assays and imaging are also provided. The entire protocol takes ~3 weeks. Delivery or administration according to the invention may be performed using exosomes produced from self-derived dendritic cells. From the herein teachings, this can be employed in the practice of the invention

In another embodiment, the plasma exosomes of Wahlgren et al. (Nucleic Acids Research, 2012, Vol. 40, No. 17 e130) are contemplated. Exosomes are nano-sized vesicles (30-90nm in size) produced by many cell types, including dendritic cells (DC), B cells, T cells, mast cells, epithelial cells and tumor cells. These vesicles are formed by inward budding of late endosomes and are then released to the extracellular environment upon fusion with the plasma membrane. Because exosomes naturally carry RNA between cells, this property may be useful in gene therapy, and from this disclosure can be employed in the practice of the instant invention.

Exosomes from plasma can be prepared by centrifugation of buffy coat at 900g for 20 min to isolate the plasma followed by harvesting cell supernatants, centrifuging at 300g for 10 min to eliminate cells and at 16 500g for 30 min followed by filtration through a 0.22 mm filter. Exosomes are pelleted by ultracentrifugation at 120 000g for70 min. Chemical transfection of siRNA into exosomes is carried out according to the manufacturer's instructions in RNAi Human/Mouse Starter Kit (Quiagen, Hilden, Germany). siRNA is added to 100 ml PBS at a final concentration of 2 mmol/ml. After adding HiPerFect transfection reagent, the mixture is incubated for 10 min at RT. In order to remove the excess of micelles, the exosomes are re-isolated using aldehyde/sulfate latex beads. The chemical transfection of CRISPR Cas into exosomes may be conducted similarly to siRNA. The exosomes may be co-cultured with monocytes and lymphocytes isolated from the peripheral blood of healthy donors. Therefore, it may be contemplated that exosomes containing CRISPR Cas may be introduced to monocytes and lymphocytes of and autologously reintroduced into a human. Accordingly, delivery or administration according to the invention may beperformed using plasma exosomes.

## Liposomes

Delivery or administration according to the invention can be performed with liposomes. Liposomes are spherical vesicle structures composed of a uni- or multilamellar lipid bilayer surrounding internal aqueous compartments and a relatively impermeable outer lipophilic phospholipid bilayer. Liposomes have gained considerable attention as drug delivery carriers because they are biocompatible, nontoxic, can deliver both hydrophilic and lipophilic drug molecules, protect their cargo from degradation by plasma enzymes, and transport their load across biological membranes and the blood brain barrier (BBB) (see, e.g., Spuch and Navarro, Journal of Drug Delivery, vol. 2011, Article ID 469679, 12 pages, 2011. doi: 10.1155/2011/469679 for review).

Liposomes can be made from several different types of lipids; however, phospholipids are most commonly used to generate liposomes as drug carriers. Although liposome formation is spontaneous when a lipid film is mixed with an aqueous solution, it can also be expedited by applying force in the form of shaking by using a homogenizer, sonicator, or an extrusion apparatus (see, e.g., Spuch and Navarro, Journal of Drug Delivery, vol. 2011, Article ID 469679, 12 pages, 2011. doi: 10.1155/2011/469679 for review).

Several other additives may be added to liposomes in order to modify their structure and properties. For instance, either cholesterol or sphingomyelin may be added to the liposomal mixture in order to help stabilize the liposomal structure and to prevent the leakage of the liposomal inner cargo. Further, liposomes are prepared from hydrogenated egg phosphatidylcholine or egg phosphatidylcholine, cholesterol, and dicetyl phosphate, and their mean vesicle sizes were adjusted to about 50 and 100 nm. (see, e.g., Spuch and Navarro, Journal of Drug Delivery, vol. 2011, Article ID 469679, 12 pages, 2011. doi: 10.1155/2011/469679 for review).

A liposome formulation may be mainly comprised of natural phospholipids and lipids such as 1,2-distearoryl-sn-glycero-3-phosphatidyl choline (DSPC), sphingomyelin, egg phosphatidylcholines and monosialoganglioside. Since this formulation is made up of phospholipids only, liposomal formulations have encountered many challenges, one of the ones being the instability in plasma. Several attempts to overcome these challenges have been made, specifically in the manipulation of the lipid membrane. One of these attempts focused on the manipulation of cholesterol. Addition of cholesterol to conventional formulations reduces rapid release of the encapsulated bioactive compound into the plasma or 1,2-dioleoyl-sn-glycero-3-phosphoethanolamine (DOPE) increases the stability (see, e.g., Spuch and Navarro, Journal of Drug Delivery, vol. 2011, Article ID 469679, 12 pages, 2011. doi:10.1155/2011/469679 for review).

In a particularly advantageous embodiment, Trojan Horse liposomes (also known as Molecular Trojan Horses) are desirable and protocols may be found at http://cshprotocols.cshlp.org/content/2010/4/pdb.prot5407.long. These particles allow delivery of a transgene to the entire brain after an intravascular injection. Without being bound by limitation, it is believed that neutral lipid particles with specific antibodies conjugated to surface allow crossing of the blood brain barrier via endocytosis. Applicant postulates utilizing Trojan Horse Liposomes to deliver the CRISPR family of nucleases to the brain via an intravascular injection, which would allow whole brain transgenic animals without the need for embryonic manipulation. About 1-5 g of DNA or RNA may be contemplated for in vivo administration in liposomes.

In another embodiment, the CRISPR Cas system may be administered in liposomes, such as a stable nucleic-acid-lipid particle (SNALP) (see, e.g., Morrissey et al., Nature Biotechnology, Vol. 23, No. 8, August 2005). Daily intravenous injections of about 1, 3 or 5 mg/kg/day of a specific CRISPR Cas targeted in a SNALP are contemplated. The daily treatment may be over about three days and then weekly for about five weeks. In another embodiment, a specific CRISPR Cas encapsulated SNALP) administered by intravenous injection to at doses of about 1 or 2.5 mg/kg are also contemplated (see, e.g., Zimmerman et al., Nature Letters, Vol. 441, 4 May 2006). The SNALP formulation may contain the lipids 3-N-[(wmethoxypoly(ethylene glycol) 2000) carbamoyl] -1,2-dimyristyloxy-propylamine (PEG-C-DMA), 1,2-dilinoleyloxy-N,N-dimethyl-3-aminopropane (DLinDMA), 1,2-distearoyl-sn-glycero-3-phosphocholine (DSPC) and cholesterol, in a 2:40:10:48 molar per cent ratio (see, e.g., Zimmerman et al., Nature Letters, Vol. 441, 4 May 2006).

In another embodiment, stable nucleic-acid-lipid particles (SNALPs) have proven to be effective delivery molecules to highly vascularized HepG2-derived liver tumors but not in poorly vascularized HCT-116 derived liver tumors (see, e.g., Li, Gene Therapy (2012) 19, 775-780). The SNALP liposomes may be prepared by formulating D-Lin-DMA and PEG-C-DMA with distearoylphosphatidylcholine (DSPC), Cholesterol and siRNA using a 25:1 lipid/siRNA ratio and a 48/40/10/2 molar ratio of Cholesterol/D-Lin-DMA/DSPC/PEG-C-DMA. The resulted SNALP liposomes are about 80-100 nm in size.

In yet another embodiment, a SNALP may comprise synthetic cholesterol (Sigma-Aldrich, St Louis, MO, USA), dipalmitoylphosphatidylcholine (Avanti Polar Lipids, Alabaster, AL, USA), 3-N-[(w-methoxy poly(ethylene glycol)2000)carbamoyl]-1,2-dimyrestyloxypropylamine, and cationic 1,2-dilinoleyloxy-3-N,Ndimethylaminopropane (see, e.g., Geisbert et al., Lancet 2010; 375: 1896-905). A dosage of about 2 mg/kg total CRISPR Cas per dose administered as, for example, a bolus intravenous infusion may be contemplated.

In yet another embodiment, a SNALP may comprise synthetic cholesterol (Sigma-Aldrich), 1,2-distearoyl-sn-glycero-3-phosphocholine (DSPC; Avanti Polar Lipids Inc.), PEGcDMA, and 1,2-dilinoleyloxy-3-(N;N-dimethyl)aminopropane (DLinDMA) (see, e.g., Judge, J. Clin. Invest. 119:661-673 (2009)). Formulations used for in vivo studies may comprise a final lipid/RNA mass ratio of about 9:1.

The safety profile of RNAi nanomedicines has been reviewed by Barros and Gollob of Alnylam Pharmaceuticals (see, e.g., Advanced Drug Delivery Reviews 64 (2012) 1730-1737). The stable nucleic acid lipid particle (SNALP) is comprised of four different lipids - an ionizable lipid (DLinDMA) that is cationic at low pH, a neutral helper lipid, cholesterol, and a diffusible polyethylene glycol (PEG)-lipid. The particle is approximately 80 nm in diameter and is chargeneutral at physiologic pH. During formulation, the ionizable lipid serves to condense lipid with the anionic RNA during particle formation. When positively charged under increasingly acidic endosomal conditions, the ionizable lipid also mediates the fusion of SNALP with the endosomal membrane enabling release of RNA into the cytoplasm. The PEG-lipid stabilizes the particle and reduces aggregation during formulation, and subsequently provides a neutral hydrophilic exterior that improves pharmacokinetic properties.

To date, two clinical programs have been initiated using SNALP formulations with RNA. Tekmira Pharmaceuticals recently completed a phase I single-dose study of SNALP-ApoB in adult volunteers with elevated LDL cholesterol. ApoB is predominantly expressed in the liver and jejunum and is essential for the assembly and secretion of VLDL and LDL. Seventeen subjects received a single dose of SNALP-ApoB (dose escalation across 7 dose levels). There was no evidence of liver toxicity (anticipated as the potential dose-limiting toxicity based on preclinical studies). One (of two) subjects at the highest dose experienced flu-like symptoms consistent with immune system stimulation, and the decision was made to conclude the trial.

Alnylam Pharmaceuticals has similarly advanced ALN-TTR01, which employs the SNALP technology described above and targets hepatocyte production of both mutant and wildtype TTR to treat TTR amyloidosis (ATTR). Three ATTR syndromes have been described: familial amyloidotic polyneuropathy (FAP) and familial amyloidotic cardiomyopathy (FAC) - both caused by autosomal dominant mutations in TTR; and senile systemic amyloidosis (SSA) cause by wildtype TTR. A placebo-controlled, single dose-escalation phase I trial of ALN-TTR01 was recently completed in patients with ATTR. ALN-TTR01 was administered as a 15-minute IV infusion to 31 patients (23 with study drug and 8 with placebo) within a dose range of 0.01 to 1.0 mg/kg (based on siRNA). Treatment was well tolerated with no significant increases in liver function tests. Infusion-related reactions were noted in 3 of 23 patients at$\geq$0.4 mg/kg; all responded to slowing of the infusion rate and all continued on study. Minimal and transient elevations of serum cytokines IL-6, IP-10 and IL-1ra were noted in two patients at the highest dose of 1 mg/kg (as anticipated from preclinical and NHP studies). Lowering of serum TTR, the expected pharmacodynamics effect of ALN-TTR01, was observed at 1 mg/kg.

In yet another embodiment, a SNALP may be made by solubilizing a cationic lipid, DSPC, cholesterol and PEG-lipid e.g., in ethanol, e.g., at a molar ratio of 40:10:40:10, respectively (see, Semple et al., Nature Niotechnology, Volume 28 Number 2 February 2010, pp. 172-177). The lipid mixture was added to an aqueous buffer (50 mM citrate, pH 4) with mixing to a final ethanol and lipid concentration of 30% (vol/vol) and 6.1 mg/ml, respectively, and allowed to equilibrate at 22 °C for 2 min before extrusion. The hydrated lipids were extruded through two stacked 80 nm pore-sized filters (Nuclepore) at 22 °C using a Lipex Extruder (Northern Lipids) until a vesicle diameter of 70-90 nm, as determined by dynamic light scattering analysis, was obtained. This generally required 1-3 passes. The siRNA (solubilized in a 50 mM citrate, pH 4 aqueous solution containing 30% ethanol) was added to the pre-equilibrated (35 °C) vesicles at a rate of ~5 ml/min with mixing. After a final target siRNA/lipid ratio of 0.06 (wt/wt) was reached, the mixture was incubated for a further 30 min at 35 °C to allow vesicle reorganization and encapsulation of the siRNA. The ethanol was then removed and the external buffer replaced with PBS (155 mM NaCl, 3 mM $Na_2HPO_4$, 1 mM $KH_2PO_4$, pH 7.5) by either dialysis or tangential flow diafiltration. siRNA were encapsulated in SNALP using a controlled step-wise dilution method process. The lipid constituents of KC2-SNALP were DLin-KC2-DMA (cationic lipid), dipalmitoylphosphatidylcholine (DPPC; Avanti Polar Lipids), synthetic cholesterol (Sigma) and PEG-C-DMA used at a molar ratio of 57.1:7.1:34.3:1.4. Upon formation of the loaded particles, SNALP were dialyzed against PBS and filter sterilized through a 0.2 $\mu$m filter before use. Mean particle sizes were 75-85 nm and 90-95% of the siRNA was encapsulated within the lipid particles. The final siRNA/lipid ratio in formulations used for in vivo testing was ~0.15 (wt/wt). LNP-siRNA systems containing Factor VII siRNA were diluted to the appropriate concentrations in sterile PBS immediately before use and the formulations were administered intravenously through the lateral tail vein in a total volume of 10 ml/kg. This method and these delivery systems may be extrapolated to the CRISPR Cas system of the present invention.

## Other Lipids

Other cationic lipids, such as amino lipid 2,2-dilinoleyl-4-dimethylaminoethyl-[1,3]-dioxolane (DLin-KC2-DMA) may be utilized to encapsulate CRISPR Cas or components thereof or nucleic acid molecule(s) coding therefor e.g., similar to SiRNA (see, e.g., Jayaraman, Angew. Chem. Int. Ed. 2012, 51, 8529 -8533), and hence may be employed in the practice of the invention. A preformed vesicle with the following lipid composition may be contemplated: amino lipid, distearoylphosphatidylcholine (DSPC), cholesterol and (R)-2,3-bis(octadecyloxy) propyl-1-(methoxy poly(ethylene glycol)2000)propylcarbamate (PEG-lipid) in the molar ratio 40/10/40/10, respectively, and a FVII siRNA/total lipid ratio of approximately 0.05 (w/w). To ensure a narrow particle size distribution in the range of 70-90 nm and a low polydispersity index of 0.11±0.04 (n=56), the particles may be extruded up to three times through 80 nm membranes prior to adding the CRISPR Cas RNA. Particles containing the highly potent amino lipid 16 may be used, in which the molar ratio of the four lipid components 16, DSPC, cholesterol and PEG-lipid (50/10/38.5/1.5) which may be further optimized to enhance in vivo activity.

Michael S D Kormann et al. ("Expression of therapeutic proteins after delivery of chemically modified mRNA in mice: Nature Biotechnology, Volume:29, Pages: 154-157 (2011)) describes the use of lipid envelopes to deliver RNA. Use of lipid envelopes is also preferred in the present invention.

In another embodiment, lipids may be formulated with the CRISPR Cas system of the present invention to form lipid nanoparticles (LNPs). Lipids include, but are not limited to, DLin-KC2-DMA4, C12-200 and colipids disteroylphosphatidyl choline, cholesterol, and PEG-DMG may be formulated with CRISPR Cas instead of siRNA (see, e.g., Novobrantseva, Molecular Therapy-Nucleic Acids (2012) 1, e4; doi:10.1038/mtna.2011.3) using a spontaneous vesicle formation procedure. The component molar ratio may be about 50/10/38.5/1.5 (DLin-KC2-DMA or C12-200/disteroylphosphatidyl choline/cholesterol/PEG-DMG). The final lipid:siRNA weight ratio may be ~12:1 and 9:1 in the case of DLin-KC2-DMA and C12-200 lipid nanoparticles (LNPs), respectively. The formulations may have mean particle diameters of ~80 nm with >90% entrapment efficiency. A 3 mg/kg dose may be contemplated.

Tekmira has a portfolio of approximately 95 patent families, in the U.S. and abroad, that are directed to various aspects of LNPs and LNP formulations (see, e.g., U.S. Pat. Nos. 7,982,027; 7,799,565; 8,058,069; 8,283,333; 7,901,708; 7,745,651; 7,803,397; 8,101,741; 8,188,263; 7,915,399; 8,236,943 and 7,838,658 and European Pat. Nos 1766035; 1519714; 1781593 and 1664316), all of which may be used and/or adapted to the present invention.

The CRISPR Cas system or components thereof or nucleic acid molecule(s) coding therefor may be delivered encapsulated in PLGA Microspheres such as that further described in US published applications 20130252281 and 20130245107 and 20130244279 (assigned to Moderna Therapeutics) which relate to aspects of formulation of compositions comprising modified nucleic acid molecules which may encode a protein, a protein precursor, or a partially or fully processed form of the protein or a protein precursor. The formulation may have a molar ratio 50:10:38.5:1.5-3.0 (cationic lipid:fusogenic lipid:cholesterol:PEG lipid). The PEG lipid may be selected from, but is not limited to PEG-c-DOMG, PEG-DMG. The fusogenic lipid may be DSPC. See also, Schrum et al., Delivery and Formulation of Engineered Nucleic Acids, US published application 20120251618.

Nanomerics' technology addresses bioavailability challenges for a broad range of therapeutics, including low molecular weight hydrophobic drugs, peptides, and nucleic acid based therapeutics (plasmid, siRNA, miRNA). Specific administration routes for which the technology has demonstrated clear advantages include the oral route, transport across the blood-brain-barrier, delivery to solid tumours, as well as to the eye. See, e.g., Mazza et al., 2013, ACS Nano. 2013 Feb 26;7(2):1016-26; Uchegbu and Siew, 2013, J Pharm Sci. 102(2):305-10 and Lalatsa et al., 2012, J Control Release. 2012 Jul 20; 161(2):523-36.

US Patent Publication No. 20050019923 describes cationic dendrimers for delivering bioactive molecules, such as polynucleotide molecules, peptides and polypeptides and/or pharmaceutical agents, to a mammalian body. The dendrimers are suitable for targeting the delivery of the bioactive molecules to, for example, the liver, spleen, lung, kidney or heart (or even the brain). Dendrimers are synthetic 3-dimensional macromolecules that are prepared in a step-wise fashion from simple branched monomer units, the nature and functionality of which can be easily controlled and varied. Dendrimers are synthesised from the repeated addition of building blocks to a multifunctional core (divergent approach to synthesis), or towards a multifunctional core (convergent approach to synthesis) and each addition of a 3-dimensional shell of building blocks leads to the formation of a higher generation of the dendrimers. Polypropylenimine dendrimers start from a diaminobutane core to which is added twice the number of amino groups by a double Michael addition of acrylonitrile to the primary amines followed by the hydrogenation of the nitriles. This results in a doubling of the amino groups. Polypropylenimine dendrimers contain 100% protonable nitrogens and up to 64 terminal amino groups (generation 5, DAB 64). Protonable groups are usually amine groups which are able to accept protons at neutral pH. The use of dendrimers as gene delivery agents has largely focused on the use of the polyamidoamine. and phosphorous containing compounds with a mixture of amine/amide or N--P($O_2$)S as the conjugating units respectively with no work being reported on the use of the lower generation polypropylenimine dendrimers for gene delivery. Polypropylenimine dendrimers have also been studied as pH sensitive controlled release systems for drug delivery and for their encapsulation of guest molecules when chemically modified by peripheral amino acid groups. The cytotoxicity and interaction of polypropylenimine dendrimers with DNA as well as the transfection efficacy of DAB 64 has also been studied.

US Patent Publication No. 20050019923 is based upon the observation that, contrary to earlier reports, cationic dendrimers, such as polypropylenimine dendrimers, display suitable properties, such as specific targeting and low toxicity, for use in the targeted delivery of bioactive molecules, such as genetic material. In addition, derivatives of the cationic dendrimer also display suitable properties for the targeted delivery of bioactive molecules. See also, Bioactive Polymers, US published application 20080267903, which discloses "Various polymers, including cationic polyamine polymers and dendrimeric polymers, are shown to possess anti-proliferative activity, and may therefore be useful for treatment of disorders characterised by undesirable cellular proliferation such as neoplasms and tumours, inflammatory disorders (including autoimmune disorders), psoriasis and atherosclerosis. The polymers may be used alone as active agents, or as delivery vehicles for other therapeutic agents, such as drug molecules or nucleic acids for gene therapy. In such cases, the polymers' own intrinsic anti-tumour activity may complement the activity of the agent to be delivered." The disclosures of these patent publications may be employed in conjunction with herein teachings for delivery of CRISPR Cas system(s) or component(s) thereof or nucleic acid molecule(s) coding therefor.

## Supercharged proteins

Supercharged proteins are a class of engineered or naturally occurring proteins with unusually high positive or negative net theoretical charge and may be employed in delivery of CRISPR Cas system(s) or component(s) thereof or nucleic acid molecule(s) coding therefor. Both supernegatively and superpositively charged proteins exhibit a remarkable ability to withstand thermally or chemically induced aggregation. Superpositively charged proteins are also able to penetrate mammalian cells. Associating cargo with these proteins, such as plasmid DNA, RNA, or other proteins, can enable the functional delivery of these macromolecules into mammalian cells both in vitro and in vivo. David Liu's lab reported the creation and characterization of supercharged proteins in 2007 (Lawrence et al., 2007, Journal of the American Chemical Society 129, 10110-10112).

The nonviral delivery of RNA and plasmid DNA into mammalian cells are valuable both for research and therapeutic applications (Akinc et al., 2010, Nat. Biotech. 26, 561-569). Purified +36 GFP protein (or other superpositively charged protein) is mixed with RNAs in the appropriate serum-free media and allowed to complex prior addition to cells. Inclusion of serum at this stage inhibits formation of the supercharged protein-RNA complexes and reduces the effectiveness of the treatment. The following protocol has been found to be effective for a variety of cell lines (McNaughton et al., 2009, Proc. Natl. Acad. Sci. USA 106, 6111-6116). However, pilot experiments varying the dose of protein and RNA should be performed to optimize the procedure for specific cell lines.

(1) One day before treatment, plate $1 \times 10^5$ cells per well in a 48-well plate.
(2) On the day of treatment, dilute purified +36 GFP protein in serumfree media to a final concentration 200nM. Add RNA to a final concentration of 50nM. Vortex to mix and incubate at room temperature for 10min.
(3) During incubation, aspirate media from cells and wash once with PBS.
(4) Following incubation of +36 GFP and RNA, add the protein-RNA complexes to cells.
(5) Incubate cells with complexes at 37 °C for 4h.
(6) Following incubation, aspirate the media and wash three times with 20 U/mL heparin PBS. Incubate cells with serum-containing media for a further 48h or longer depending upon the assay for activity.
(7) Analyze cells by immunoblot, qPCR, phenotypic assay, or other appropriate method.

David Liu's lab has further found +36 GFP to be an effective plasmid delivery reagent in a range of cells. As plasmid DNA is a larger cargo than siRNA, proportionately more +36 GFP protein is required to effectively complex plasmids. For effective plasmid delivery Applicants have developed a variant of +36 GFP bearing a C-terminal HA2 peptide tag, a known endosomedisrupting peptide derived from the influenza virus hemagglutinin protein. The following protocol has been effective in a variety of cells, but as above it is advised that plasmid DNA and supercharged protein doses be optimized for specific cell lines and delivery applications.

(1) One day before treatment, plate $1 \times 10^5$ per well in a 48-well plate.
(2) On the day of treatment, dilute purified Þ36 GFP protein in serumfree media to a final concentration 2 mM. Add 1mg of plasmid DNA. Vortex to mix and incubate at room temperature for 10min.
(3) During incubation, aspirate media from cells and wash once with PBS.
(4) Following incubation of Þ36 GFP and plasmid DNA, gently add the protein-DNA complexes to cells.
(5) Incubate cells with complexes at 37 C for 4h.
(6) Following incubation, aspirate the media and wash with PBS. Incubate cells in serum-containing media and incubate for a further 24-48h.
(7) Analyze plasmid delivery (e.g., by plasmid-driven gene expression) as appropriate.

See also, e.g., McNaughton et al., Proc. Natl. Acad. Sci. USA 106, 6111-6116 (2009); Cronican et al., ACS Chemical Biology 5, 747-752 (2010); Cronican et al., Chemistry & Biology 18, 833-838 (2011); Thompson et al., Methods in Enzymology 503, 293-319 (2012); Thompson, D.B., et al., Chemistry & Biology 19 (7), 831-843 (2012). The methods of the super charged proteins may be used and/or adapted for delivery of the CRISPR Cas system of the present invention. These systems of Dr. Lui and documents herein in inconjunction with herein teachints can be employed in the delivery of CRISPR Cas system(s) or component(s) thereof or nucleic acid molecule(s) coding therefor.

## Implantable devices

In another embodiment, implantable devices are also contemplated for delivery of the CRISPR Cas system or component(s) thereof or nucleic acid molecule(s) coding therefor. For example, US Patent Publication 20110195123 discloses an implantable medical device which elutes a drug locally and in prolonged period is provided, including several types of such a device, the treatment modes of implementation and methods of implantation. The device comprising of polymeric substrate, such as a matrix for example, that is used as the device body, and drugs, and in some cases additional scaffolding materials, such as metals or additional polymers, and materials to enhance visibility and imaging. An implantable delivery device can be advantageous in providing release locally and over a prolonged period, where drug is released directly to the extracellular matrix (ECM) of the diseased area such as tumor, inflammation, degeneration or for symptomatic objectives, or to injured smooth muscle cells, or for prevention. One kind of drug is RNA, as disclosed above, and this system may be used/and or adapted to the CRISPR Cas system of the present invention. The modes of implantation in some embodiments are existing implantation procedures that are developed and used today for other treatments, including brachytherapy and needle biopsy. In such cases the dimensions of the new implant described in this invention are similar to the original implant. Typically a few devices are implanted during the same treatment procedure.

As described in US Patent Publication 20110195123, there is provided a drug delivery implantable or insertable system, including systems applicable to a cavity such as the abdominal cavity and/or any other type of administration in which the drug delivery system is not anchored or attached, comprising a biostable and/or degradable and/or bioabsorbable polymeric substrate, which may for example optionally be a matrix. It should be noted that the term "insertion" also includes implantation. The drug delivery system is preferably implemented as a "Loder" as described in US Patent Publication 20110195123.

The polymer or plurality of polymers are biocompatible, incorporating an agent and/or plurality of agents, enabling the release of agent at a controlled rate, wherein the total volume of the polymeric substrate, such as a matrix for example, in some embodiments is optionally and preferably no greater than a maximum volume that permits a therapeutic level of the agent to be reached. As a non-limiting example, such a volume is preferably within the range of $0.1 \text{ m}^3$ to $1000 \text{ mm}^3$, as required by the volume for the agent load. The Loder may optionally be larger, for example when incorporated with a device whose size is determined by functionality, for example and without limitation, a knee joint, an intra-uterine or cervical ring and the like.

The drug delivery system (for delivering the composition) is designed in some embodiments to preferably employ degradable polymers, wherein the main release mechanism is bulk erosion; or in some embodiments, non degradable, or slowly degraded polymers are used, wherein the main release mechanism is diffusion rather than bulk erosion, so that the outer part functions as membrane, and its internal part functions as a drug reservoir, which practically is not affected by the surroundings for an extended period (for example from about a week to about a few months). Combinations of different polymers with different release mechanisms may also optionally be used. The concentration gradient at the surface is preferably maintained effectively constant during a significant period of the total drug releasing period, and therefore the diffusion rate is effectively constant (termed "zero mode" diffusion). By the term "constant" it is meant a diffusion rate that is preferably maintained above the lower threshold of therapeutic effectiveness, but which may still optionally feature an initial burst and/or may fluctuate, for example increasing and decreasing to a certain degree. The diffusion rate is preferably so maintained for a prolonged period, and it can be considered constant to a certain level to optimize the therapeutically effective period, for example the effective silencing period.

The drug delivery system optionally and preferably is designed to shield the nucleotide based therapeutic agent from degradation, whether chemical in nature or due to attack from enzymes and other factors in the body of the subject.

The drug delivery system as described in US Patent Publication 20110195123 is optionally associated with sensing and/or activation appliances that are operated at and/or after implantation of the device, by non and/or minimally invasive methods of activation and/or acceleration/deceleration, for example optionally including but not limited to thermal heating and cooling, laser beams, and ultrasonic, including focused ultrasound and/or RF (radiofrequency) methods or devices.

According to some embodiments of US Patent Publication 20110195123, the site for local delivery may optionally include target sites characterized by high abnormal proliferation of cells, and suppressed apoptosis, including tumors, active and or chronic inflammation and infection including autoimmune diseases states, degenerating tissue including muscle and nervous tissue, chronic pain, degenerative sites, and location of bone fractures and other wound locations for enhancement of regeneration of tissue, and injured cardiac, smooth and striated muscle.

The site for implantation of the composition, or target site, preferably features a radius, area and/or volume that is sufficiently small for targeted local delivery. For example, the target site optionally has a diameter in a range of from about 0.1 mm to about 5 cm.

The location of the target site is preferably selected for maximum therapeutic efficacy. For example, the composition of the drug delivery system (optionally with a device for implantation as described above) is optionally and preferably implanted within or in the proximity of a tumor environment, or the blood supply associated thereof.

For example the composition (optionally with the device) is optionally implanted within or in the proximity to pancreas, prostate, breast, liver, via the nipple, within the vascular system and so forth.

The target location is optionally selected from the group consisting of (as non-limiting examples only, as optionally any site within the body may be suitable for implanting a Loder): 1. brain at degenerative sites like in Parkinson or Alzheimer disease at the basal ganglia, white and gray matter; 2. spine as in the case of amyotrophic lateral sclerosis (ALS); 3. uterine cervix to prevent HPV infection; 4. active and chronic inflammatory joints; 5. dermis as in the case of psoriasis; 6. sympathetic and sensoric nervous sites for analgesic effect; 7. Intra osseous implantation; 8. acute and chronic infection sites; 9. Intra vaginal; 10. Inner ear--auditory system, labyrinth of the inner ear, vestibular system; 11. Intra tracheal; 12. Intra-cardiac; coronary, epicardiac; 13. urinary bladder; 14. biliary system; 15. parenchymal tissue including and not limited to the kidney, liver, spleen; 16. lymph nodes; 17. salivary glands; 18. dental gums; 19. Intra-articular (into joints); 20. Intra-ocular; 21. Brain tissue; 22. Brain ventricles; 23. Cavities, including abdominal cavity (for example but without limitation, for ovary cancer); 24. Intra esophageal and 25. Intra rectal.

Optionally insertion of the system (for example a device containing the composition) is associated with injection of material to the ECM at the target site and the vicinity of that site to affect local pH and/or temperature and/or other biological factors affecting the diffusion of the drug and/or drug kinetics in the ECM, of the target site and the vicinity of such a site.

Optionally, according to some embodiments, the release of said agent could be associated with sensing and/or activation appliances that are operated prior and/or at and/or after insertion, by non and/or minimally invasive and/or else methods of activation and/or acceleration/deceleration, including laser beam, radiation, thermal heating and cooling, and ultrasonic, including focused ultrasound and/or RF (radiofrequency) methods or devices, and chemical activators.

According to other embodiments of US Patent Publication 20110195123, the drug preferably comprises a RNA, for example for localized cancer cases in breast, pancreas, brain, kidney, bladder, lung, and prostate as described below. Although exemplified with RNAi, many drugs are applicable to be encapsulated in Loder, and can be used in association with this invention, as long as such drugs can be encapsulated with the Loder substrate, such as a matrix for example, and this system may be used and/or adapted to deliver the CRISPR Cas system of the present invention.

As another example of a specific application, neuro and muscular degenerative diseases develop due to abnormal gene expression. Local delivery of RNAs may have therapeutic properties for interfering with such abnormal gene expression. Local delivery of anti apoptotic, anti inflammatory and anti degenerative drugs including small drugs and macromolecules may also optionally be therapeutic. In such cases the Loder is applied for prolonged release at constant rate and/or through a dedicated device that is implanted separately. All of this may be used and/or adapted to the CRISPR Cas system of the present invention.

As yet another example of a specific application, psychiatric and cognitive disorders are treated with gene modifiers. Gene knockdown is a treatment option. Loders locally delivering agents to central nervous system sites are therapeutic options for psychiatric and cognitive disorders including but not limited to psychosis, bi-polar diseases, neurotic disorders and behavioral maladies. The Loders could also deliver locally drugs including small drugs and macromolecules upon implantation at specific brain sites. All of this may be used and/or adapted to the CRISPR Cas system of the present invention.

As another example of a specific application, silencing of innate and/or adaptive immune mediators at local sites enables the prevention of organ transplant rejection. Local delivery of RNAs and immunomodulating reagents with the Loder implanted into the transplanted organ and/or the implanted site renders local immune suppression by repelling immune cells such as CD8 activated against the transplanted organ. All of this may be used/and or adapted to the CRISPR Cas system of the present invention.

As another example of a specific application, vascular growth factors including VEGFs and angiogenin and others are essential for neovascularization. Local delivery of the factors, peptides, peptidomimetics, or suppressing their repressors is an important therapeutic modality; silencing the repressors and local delivery of the factors, peptides, macromolecules and small drugs stimulating angiogenesis with the Loder is therapeutic for peripheral, systemic and cardiac vascular disease.

The method of insertion, such as implantation, may optionally already be used for other types of tissue implantation and/or for insertions and/or for sampling tissues, optionally without modifications, or alternatively optionally only with non-major modifications in such methods. Such methods optionally include but are not limited to brachytherapy methods, biopsy, endoscopy with and/or without ultrasound, such as ERCP, stereotactic methods into the brain tissue, Laparoscopy, including implantation with a laparoscope into joints, abdominal organs, the bladder wall and body cavities.

The doses herein are based on an average 70 kg individual. The frequency of administration is within the ambit of the medical or veterinary practitioner (e.g., physician, veterinarian), or scientist skilled in the art. In this regard, one can scale up from experiments involving mice without any undue experimentation, including taking into consideration that an average mouse is 20 g. Doses may be based on or extrapolated to an average 70 kg individual, and can be adjusted for patients, subjects, mammals of different weight and species. Frequency of administration is within the ambit of the medical or veterinary practitioner (e.g., physician, veterinarian), depending on usual factors including the age, sex, general health, other conditions of the patient or subject and the particular condition or symptoms being addressed.

Viral vectors can be injected into the tissue of interest. For cell-type specific genome modification, the expression of mutated Cas9 can be driven by a cell-type specific promoter. For example, liver-specific expression might use the Albumin promoter and neuron-specific expression might use the Synapsin I promoter. Optimal concentrations of mutated CRISPR enzyme mRNA and guide RNA can be determined by testing different concentrations in a cellular or animal model and using deep sequencing the analyze the extent of modification at potential off-target genomic loci. For example, for the guide sequence targeting 5'-GAGTCCGAGCAGAAGAAGAA-3' in the EMX1 gene of the human genome, deep sequencing can be used to assess the level of modification at the following two off-target loci, 1: 5'-GAGTCCTAGCAGGAGAAGAA-3' and 2: 5'-GAGTCTAAGCAGAAGAAGAA-3'. The concentration that gives the highest level of on-target modification while minimizing the level of off-target modification should be chosen for in vivo delivery. To minimize the level of toxicity and off-target effect, mutated CRISPR enzyme nickase mRNA (for example S. pyogenes Cas9 with a N863A mutation) can be delivered with a pair of guide RNAs targeting a site of interest. The two guide RNAs need to be spaced as follows. Guide sequences in red (single underline) and blue (double underline) respectively (these examples are based on the PAM requirement for *Streptococcus pyogenes* Cas9.

| Overhang length (bp) | Guide RNA design (guide sequence and PAM color coded) |
|---|---|
| 14 | 5'-NNNNNNNNNNNNNNNNNNNNNCCNNNNNNNNNNNNNNNNNNNNNNNGGNNNNNNNNNNNNNNNN-3' |
| 13 | 3'-NNNNNNNNNNNNNNNNNNNNNNNGGNNNNNNNNNNNNNNNNNNNNNNNCCNNNNNNNNNNNNNNNN-5' |
| | 5'-NNNNNNNNNNNNNNNNNNNNNNNCCNNNNNNNNNNNNNNNNNNNNNNNGGNNNNNNNNNNNNNNNN-3' |
| 12 | 3'-NNNNNNNNNNNNNNNNNNNNNNNNGGNNNNNNNNNNNNNNNNNNNNNNNCCNNNNNNNNNNNNNNNN-5' |
| 11 | 5'-NNNNNNNNNNNNNNNNNNNNNNNNCNNNNNNNNNNNNNNNNNNNNNNNNGGNNNNNNNNNNNNNNNN-3' |
| 10 | 3'-NNNNNNNNNNNNNNNNNNNNNNNNGGNNNNNNNNNNNNNNNNNNNNNNNNCCNNNNNNNNNNNNNNNN-5' |
| 9 | 5'-NNNNNNNNNNNNNNNNNNNNNNNNNCCNNNNNNNNNNNNNNNNNNNNNNNGGNNNNNNNNNNNNNNNN-3' |
| 8 | 3'-NNNNNNNNNNNNNNNNNNNNNNNNGGNNNNNNNNNNNNNNNNNNNNNNCCNNNNNNNNNNNNNNNN-5' |
| | 5'-NNNNNNNNNNNNNNNNNNNNNNNCCNNNNNNNNNNNNNNNNNNNNNNGGNNNNNNNNNNNNNNNN-3' |
| 7 | 3'-NNNNNNNNNNNNNNNNNNNNNNGGNNNNNNNNNNNNNNNNNNNNNNCCNNNNNNNNNNNNNNNN-5' |
| 6 | 5'-NNNNNNNNNNNNNNNNNNNNNNNCCNNNNNNNNNNNNNNNNNNNNNGGNNNNNNNNNNNNNNNN-3' |
| 5 | 3'-NNNNNNNNNNNNNNNNNNNNNNGGNNNNNNNNNNNNNNNNNNNNCCNNNNNNNNNNNNNNNNNN-5' |
| 4 | 5'-NNNNNNNNNNNNNNNNNNNNNNNNNCCNNNNNNNNNNNNNNNNNNNNGGNNNNNNNNNNNNNNNN-3' |
| 3 | 3'-NNNNNNNNNNNNNNNNNNNNNNNGGNNNNNNNNNNNNNNNNNNNCCNNNNNNNNNNNNNNNNNNNN-5' |
| | 5'-NNNNNNNNNNNNNNNNNNNNNNNNNCCNNNNNNNNNNNNNNNNNNNGGNNNNNNNNNNNNNNNNNN-3' |
| 2 | 3'-NNNNNNNNNNNNNNNNNNNNNNNNGGNNNNNNNNNNNNNNNNCCNNNNNNNNNNNNNNNNNNNNNN-5' |
| 1 | 5'-NNNNNNNNNNNNNNNNNNNNNNNNNCCNNNNNNNNNNNNNNNNNNGGNNNNNNNNNNNNNNNNNNNN-3' |

(continued)

| Overhang length (bp) | Guide RNA design (guide sequence and PAM color coded) |
|---|---|
| blunt | 3'-NNNNNNNNNNNNNNNNNNNNGGNNNNNNNNNNNNNNCCNNNNNNNNNNNNNNNNNNNNNNN-5' |
| 1 | 5'-NNNNNNNNNNNNNNNNNNNNNNCCNNNNNNNNNNNNNGGNNNNNNNNNNNNNNNNNNNNNN-3' |
| 2 | 3'-NNNNNNNNNNNNNNNNNNNNNGGNNNNNNNNNNNNNCCNNNNNNNNNNNNNNNNNNNNNNN-5' |
|  | 5'-NNNNNNNNNNNNNNNNNNNNNNCCNNNNNNNNNNNNNNGGNNNNNNNNNNNNNNNNNNNNNN-3' |
| 3 | 3'-NNNNNNNNNNNNNNNNNNNNNNGGNNNNNNNNNNNNNCCNNNNNNNNNNNNNNNNNNNNNNN-5' |
| 4 | 5'-NNNNNNNNNNNNNNNNNNNNNCCNNNNNNNNNNNNNNNGGNNNNNNNNNNNNNNNNNNNNNN-3' |
| 5 | 3'-NNNNNNNNNNNNNNNNNNNNNNGGNNNNNNNNNNNNCCNNNNNNNNNNNNNNNNNNNNNNN-5' |
| 6 | 5'-NNNNNNNNNNNNNNNNNNNNNCCNNNNNNNNNNNNNNGGNNNNNNNNNNNNNNNNNNNNNNN-3' |
| 7 | 3'-NNNNNNNNNNNNNNNNNNNNNNGGNNNNNNNNNNNCCNNNNNNNNNNNNNNNNNNNNNNN-5' |
|  | 5'-NNNNNNNNNNNNNNNNNNNNCCNNNNNNNNNNNGGNNNNNNNNNNNNNNNNNNNNNNNNNN-3' |
| 8 | 3'-NNNNNNNNNNNNNNNNNNNNNNGGNNNNNNNNNNCCNNNNNNNNNNNNNNNNNNNNNNN-5' |
| 12 | 5'-NNNNNNNNNNNNNNNNNNNNNNCCNNNNNNNNNGGNNNNNNNNNNNNNNNNNNNNNNNNN-3' |
| 13 | 3'-NNNNNNNNNNNNNNNNNNNNNNGGNNNNNNNNCCNNNNNNNNNNNNNNNNNNNNNNNN-5' |
| 14 | 5'-NNNNNNNNNNNNNNNNNNNNNCCNNNNNNNNGGNNNNNNNNNNNNNNNNNNNNNNNNNN-3' |
| 15 | 3'-NNNNNNNNNNNNNNNNNNNNNNGGNNNNNNNCCNNNNNNNNNNNNNNNNNNNNNNNN-5' |
| 16 | 5'-NNNNNNNNNNNNNNNNNNNNCCNNNNNNNGGNNNNNNNNNNNNNNNNNNNNNNNNNNNN-3' |

(continued)

| Overhang length (bp) | Guide RNA design (guide sequence and PAM color coded) |
|---|---|
| 17 | 3'-NNNNNNNNNNNNNNNNNNNGGNNNNNNCCNNNNNNNNNNNNNNNNNNNNNNNNNNNNNNN-5'<br><br>5'-NNNNNNNNNNNNNNNNNNNNNNNCCNNNNNNGGNNNNNNNNNNNNNNNNNNNNNNNNNNNN-3'<br><br>3'-NNNNNNNNNNNNNNNNNNNNGGNNNNNCCNNNNNNNNNNNNNNNNNNNNNNNNNNNNNNNN-5'<br><br>5'-NNNNNNNNNNNNNNNNNNNNNNNCCNNNNNGGNNNNNNNNNNNNNNNNNNNNNNNNNNNNN-3'<br><br>3'-NNNNNNNNNNNNNNNNNNNNNGGNNNNCCNNNNNNNNNNNNNNNNNNNNNNNNNNNNNNNN-5'<br><br>5'-NNNNNNNNNNNNNNNNNNNNNNNCCNNNNGGNNNNNNNNNNNNNNNNNNNNNNNNNNNNNN-3'<br><br>3'-NNNNNNNNNNNNNNNNNNNNNNGGNNNCCNNNNNNNNNNNNNNNNNNNNNNNNNNNNNNNN-5'<br><br>5'-NNNNNNNNNNNNNNNNNNNNNNNCCNNGGNNNNNNNNNNNNNNNNNNNNNNNNNNNNNNNN-3'<br><br>3'-NNNNNNNNNNNNNNNNNNNNNNNGGNNCCNNNNNNNNNNNNNNNNNNNNNNNNNNNNNNNN-5'<br><br>5'-NNNNNNNNNNNNNNNNNNNNNNNNCCNGGNNNNNNNNNNNNNNNNNNNNNNNNNNNNNNNN-3'<br><br>3'-NNNNNNNNNNNNNNNNNNNNNNNNGGNCCNNNNNNNNNNNNNNNNNNNNNNNNNNNNNNNN-5' |

(continued)

| Overhang length (bp) | Guide RNA design (guide sequence and PAM color coded) |
|---|---|
| | 5'-NNNNNNNNNNNNNNNNNNNNNNNCCGGNNNNNNNNNNNNNNNNNNNNNNNNNNNNNNNNNN-3'<br><br>3'-NNNNNNNNNNNNNNNNNNNNNNNGGCCNNNNNNNNNNNNNNNNNNNNNNNNNNNNNNNNNN-5' |
| | 5'-NNNNNNNNNNNNNNNNNNNNNNNNNNGGNNNNNNNNNNNNNNNNNNNNNNNNNNNNNNNNNN-3'<br><br>3'-NNNNNNNNNNNNNNNNNNNNNNNNNCCGGNNNNNNNNNNNNNNNNNNNNNNNNNNNNNNNNNN-5' |
| | 5'-NNNNNNNNNNNNNNNNNNNNNNNNNCGGNNNNNNNNNNNNNNNNNNNNNNNNNNNNNNNNNN-3'<br><br>3'-NNNNNNNNNNNNNNNNNNNNNNNNNCCNGGNNNNNNNNNNNNNNNNNNNNNNNNNNNNNNNNNN-5' |
| | 5'-NNNNNNNNNNNNNNNNNNNNNNNNNCGGNNNNNNNNNNNNNNNNNNNNNNNNNNNNNNNNNN-3'<br><br>3'-NNNNNNNNNNNNNNNNNNNNNNNNNCCNNGGNNNNNNNNNNNNNNNNNNNNNNNNNNNNNNNNNN-5' |
| | 5'-NNNNNNNNNNNNNNNNNNNNNNNNNCGGNNNNNNNNNNNNNNNNNNNNNNNNNNNNNNNNNN-3'<br><br>3'-NNNNNNNNNNNNNNNNNNNNNNNNNCCNNNGGNNNNNNNNNNNNNNNNNNNNNNNNNNNNNNNNNN-5' |
| | 5'-NNNNNNNNNNNNNNNNNNNNNNNNNCGGNNNNNNNNNNNNNNNNNNNNNNNNNNNNNNNNNN-3'<br><br>3'-NNNNNNNNNNNNNNNNNNNNNNNNNCCNNNNGGNNNNNNNNNNNNNNNNNNNNNNNNNNNNNNNNN-5' |
| | 5'-NNNNNNNNNNNNNNNNNNNNNNNNNCGGNNNNNNNNNNNNNNNNNNNNNNNNNNNNNNNNNN-3'<br><br>3'-NNNNNNNNNNNNNNNNNNNNNNNNNCCNNNNNGGNNNNNNNNNNNNNNNNNNNNNNNNNNNNNNNN-5' |

Functional domains associated with the present CRISPR enzyme or with modified guides for use with the present CRISPR enzyme: In some embodiments, one or more functional domains are associated with the CRISPR enzyme, preferably the Type II Cas9 Sp or Sa Cas9 enzyme or mutated ortholog thereof. In some embodiments, one or more functional domains are associated with an adaptor protein, for example as used with the modified guides of Konermann et al. (Nature 517, 583-588, 29 January 2015). Reference to a functional domain could be a functional domain associated with the CRISPR enzyme or a functional domain associated with the adaptor protein. In some embodiments, the one or more functional domains is an NLS (Nuclear Localization Sequence) or an NES (Nuclear Export Signal). In some embodiments, the one or more functional domains is a transcriptional activation domain comprises VP64, p65, MyoD1, HSF1, RTA, SET7/9 and a histone acetyltransferase. Other references herein to activation (or activator) domains in respect of those associated with the CRISPR enzyme include any known transcriptional activation domain and specifically VP64, p65, MyoD1, HSF1, RTA, SET7/9 or a histone acetyltransferase. In some embodiments, the one or more functional domains is a transcriptional repressor domain. In some embodiments, the transcriptional repressor domain is a KRAB domain. In some embodiments, the transcriptional repressor domain is a NuE domain, NcoR domain, SID domain or a SID4X domain. In some embodiments, the one or more functional domains have one or more activities comprising methylase activity, demethylase activity, transcription activation activity, transcription repression activity, transcription release factor activity, histone modification activity, RNA cleavage activity, DNA cleavage activity, DNA integration activity or nucleic acid binding activity. Histone modifying domains are also preferred in some embodiments. Exemplary histone modifying domains are discussed below. Transposase domains, HR (Homologous Recombination) machinery domains, recombinase domains, and/or integrase domains are also preferred as the present functional domains. In some embodiments, DNA integration activity includes HR machinery domains, integrase domains, recombinase domains and/or transposase domains. Histone acetyltransferases are preferred in some embodiments. In some embodiments, the DNA cleavage activity is due to a nuclease. In some embodiments, the nuclease comprises a Fok1 nuclease. In some embodiments, the one or more functional domains is attached to the CRISPR enzyme so that upon binding to the sgRNA and target the functional domain is in a spatial orientation allowing for the functional domain to function in its attributed function. In some embodiments, the one or more functional domains is attached to the adaptor protein so that upon binding of the CRISPR enzyme to the sgRNA and target, the functional domain is in a spatial orientation allowing for the functional domain to function in its attributed function. In an aspect the disclosure provides a composition as herein discussed wherein the one or more functional domains is attached to the CRISPR enzyme or adaptor protein via a linker, optionally a GlySer linker, as discussed herein. Endogenous transcriptional repression is often mediated by chromatin modifying enzymes such as histone methyltransferases (HMTs) and deacetylases (HDACs). Repressive histone effector domains are known and an exemplary list is provided below. In the exemplary table, preference was given to proteins and functional truncations of small size to facilitate efficient viral packaging (for instance via AAV). In general, however, the domains may include HDACs, histone methyltransferases (HMTs), and histone acetyltransferase (HAT) inhibitors, as well as HDAC and HMT recruiting proteins. The functional domain may be or include, in some embodiments, HDAC Effector Domains, HDAC Recruiter Effector Domains, Histone Methyltransferase (HMT) Effector Domains, Histone Methyltransferase (HMT) Recruiter Effector Domains, or Histone Acetyltransferase Inhibitor Effector Domains.

HDAC Effector Domains

| Subtype/ Complex | Name | Substrate (if known) | Modification (if known) | Organism | Full size (aa) | Selected truncation (aa) | Final size (aa) | Catalytic domain |
|---|---|---|---|---|---|---|---|---|
| HDAC I | **HDAC8** | - | - | *X. laevis* | 325 | 1-325 | 325 | 1-272: HDAC |
| HDAC I | **RPD3** | - | - | *S. cerevisiae* | 433 | 19-340 | 322 (Vannier) | 19-331: HDAC |
| HDAC IV | **MesoLo4** | - | - | *M. loti* | 300 | 1-300 (Gregoretti) | 300 | - |
| HDAC IV | **HDAC11** | - | - | *H. sapiens* | 347 | 1-347 (Gao) | 347 | 14-326: HDAC |
| HD2 | **HDT1** | - | - | *A. thaliana* | 245 | 1-211 (Wu) | 211 | - |
| SIRT I | **SIRT3** | H3K9Ac H4K16Ac H3K56Ac | - | *H. sapiens* | 399 | 143-399 (Scher) | 257 | 126-382: SIRT |
| SIRT I | **HST2** | - | - | *C. albicans* | 331 | 1-331 (Hnisz) | 331 | - |
| SIRT I | **CobB** | - | - | *E. coli (K12)* | 242 | 1-242 (Landry) | 242 | - |
| SIRT I | **HST2** | - | - | *S. cerevisiae* | 357 | 8-298 (Wilson) | 291 | - |
| SIRT III | **SIRT5** | H4K8Ac H4K16Ac | - | *H. sapiens* | 310 | 37-310 (Gertz) | 274 | 41-309: SIRT |
| SIRT III | **Sir2A** | - | - | *P. falciparum* | 273 | 1-273 (Zhu) | 273 | 19-273: SIRT |
| SIRT IV | **SIRT6** | H3K9Ac H3K56Ac | - | *H. sapiens* | 355 | 1-289 (Tennen) | 289 | 35-274: SIRT |

Accordingly, the repressor domains of the present invention may be selected from histone methyltransferases (HMTs), histone deacetylases (HDACs), histone acetyltransferase (HAT) inhibitors, as well as HDAC and HMT recruiting proteins.

The HDAC domain may be any of those in the table above, namely: HDAC8, RPD3, MesoLo4, HDAC11, HDT1, SIRT3, HST2, CobB, HST2, SIRT5, Sir2A, or SIRT6.

In some embodiment, the functional domain may be a HDAC Recruiter Effector Domain. Preferred examples include those in the Table below, namely MeCP2, MBD2b, Sin3a, NcoR, SALL1, RCOR1. NcoR is exemplified in the present Examples and, although preferred, it is envisaged that others in the class will also be useful.

Table of HDAC Recruiter Effector Domains

| Subtype/ Complex | Name | Substrate (if known) | Modification (if known) | Organism | Full size (aa) | Selected truncation (aa) | Final size (aa) | Catalytic domain |
|---|---|---|---|---|---|---|---|---|
| Sin3a | **MeCP2** | - | - | *R. norvegicus* | 492 | 207-492 (Nan) | 286 | - |
| Sin3a | **MBD2b** | - | - | *H. sapiens* | 262 | 45-262 (Boeke) | 218 | - |
| Sin3a | **Sin3a** | - | - | *H. sapiens* | 1273 | 524-851 (Laherty) | 328 | 627-829: HDAC1 interaction |
| NcoR | **NcoR** | - | - | *H. sapiens* | 2440 | 420-488 (Zhang) | 69 | - |
| NuRD | **SALL1** | - | - | *M. musculus* | 1322 | 1-93 (Lauberth) | 93 | - |
| CoREST | **RCOR1** | - | - | *H. sapiens* | 482 | 81-300 (Gu, Ouyang) | 220 | - |

In some embodiment, the functional domain may be a Methyltransferase (HMT) Effector Domain. Preferred examples include those in the Table below, namely NUE, vSET, EHMT2/G9A, SUV39H1, dim-5, KYP, SUVR4, SET4, SET1, SETD8, and TgSET8. NUE is exemplified in the present Examples and, although preferred, it is envisaged that others in the class will also be useful.

Table of Histone Methyltransferase (HMT) Effector Domains

| Subtype/ Complex | Name | Substrat e (if known) | Modification (if known) | Organism | Full size (aa) | Selected truncation (aa) | Final size (aa) | Catalyti c domain |
|---|---|---|---|---|---|---|---|---|
| SET | **NUE** | H2B, H3, H4 | - | C. trachomat is | 219 | 1-219 (Pennini) | 219 | - |
| SET | **vSET** | - | H3K27me3 | P. bursaria chlorella virus | 119 | 1-119 (Mujtaba) | 119 | 4-112: SET2 |
| SUV39 family | **EHMT2/G9 A** | H1.4K2, H3K9, H3K27 | H3K9mel/2, H1K25me1 | M. musculus | 1263 | 969-1263 (Tachiban a) | 295 | 1025-1233: preSET , SET, postSE T |
| SUV39 | **SUV39H1** | - | H3K9me2/3 | H. sapiens | 412 | 79-412 (Snowden ) | 334 | 172-412: preSET , SET, postSE T |
| Suvar3-9 | **dim-5** | - | H3K9me3 | N. crassa | 331 | 1-331 (Rathert) | 331 | 77-331: preSET , SET, postSE T |
| Suvar3-9 (SUVH subfamil y) | **KYP** | - | H3K9mel/2 | A. thaliana | 624 | 335-601 | 267 (Jackson) | - |
| Suvar3-9 (SUVR subfamil y) | **SUVR4** | H3K9me 1 | H3K9me2/3 | A. thaliana | 492 | 180-492 | 313 (Thorstense n) | 192-462: preSET , SET, postSE T |
| Suvar4-20 | **SET4** | - | H4K20me3 | C. elegans | 288 | 1-288 (Vielle) | 288 | - |
| SET8 | **SET1** | - | H4K20mel | C. elegans | 242 | 1-242 (Vielle) | 242 | - |
| SET8 | **SETD8** | - | H4K20mel | H. sapiens | 393 | 185-393 | 209 (Couture) | 256-382: SET |
| SET8 | **TgSET8** | - | H4K20mel/2 /3 | T. gondii | 1893 | 1590-1893 (Sautel) | 304 | 1749-1884: SET |

In some embodiment, the functional domain may be a Histone Methyltransferase (HMT) Recruiter Effector Domain. Preferred examples include those in the Table below, namely Hp1a, PHF19, and NIPP1.

Table of Histone Methyltransferase (HMT) Recruiter Effector Domains

| Subtype/ Complex | Name | Substrate (if known) | Modification (if known) | Organism | Full size (aa) | Selected truncation (aa) | Final size (aa) | Catalytic domain |
|---|---|---|---|---|---|---|---|---|
| - | **Hpla** | - | H3K9me3 | *M. musculus* | 191 | 73-191 | 119 (Hathaway) | 121-179: chromoshadow |
| - | **PHF19** | - | H3K27me3 | *H. sapiens* | 580 | (1-250) + GGSG | 335 (Ballaré) | 163-250: PHD2 |
| | | | | | | linker + (500-580) | | |
| - | **NIPP1** | - | H3K27me3 | *H. sapiens* | 351 | 1-329 (Jin) | 329 | 310-329: EED |

In some embodiment, the functional domain may be Histone Acetyltransferase Inhibitor Effector Domain. Preferred examples include SET/TAF-1β listed in the Table below.

Table of Histone Acetyltransferase Inhibitor Effector Domains

| Subtype/ Complex | Name | Substrate (if known) | Modification (if known) | Organism | Full size (aa) | Selected truncation (aa) | Final size (aa) | Catalytic domain |
|---|---|---|---|---|---|---|---|---|
| - | **SET/TAF-1β** | - | - | *M. musculus* | 289 | 1-289 (Cervoni) | 289 | - |

It is also preferred to target endogenous (regulatory) control elements (such as enhancers and silencers) in addition to a promoter or promoter-proximal elements. Thus, the invention can also be used to target endogenous control elements (including enhancers and silencers) in addition to targeting of the promoter. These control elements can be located upstream and downstream of the transcriptional start site (TSS), starting from 200bp from the TSS to 100kb away. Targeting of known control elements can be used to activate or repress the gene of interest. In some cases, a single control element can influence the transcription of multiple target genes. Targeting of a single control element could therefore be used to control the transcription of multiple genes simultaneously.

Targeting of putative control elements on the other hand (e.g. by tiling the region of the putative control element as well as 200bp up to 100kB around the element) can be used as a means to verify such elements (by measuring the transcription of the gene of interest) or to detect novel control elements (e.g. by tiling 100kb upstream and downstream of the TSS of the gene of interest). In addition, targeting of putative control elements can be useful in the context of understanding genetic causes of disease. Many mutations and common SNP variants associated with disease phenotypes are located outside coding regions. Targeting of such regions with either the activation or repression systems described herein can be followed by readout of transcription of either a) a set of putative targets (e.g. a set of genes located in closest proximity to the control element) or b) whole-transcriptome readout by e.g. RNAseq or microarray. This would allow for the identification of likely candidate genes involved in the disease phenotype. Such candidate genes could be useful as novel drug targets.

Histone acetyltransferase (HAT) inhibitors are mentioned herein. However, an alternative in some embodiments is for the one or more functional domains to comprise an acetyltransferase, preferably a histone acetyltransferase. These are useful in the field of epigenomics, for example in methods of interrogating the epigenome. Methods of interrogating the epigenome may include, for example, targeting epigenomic sequences. Targeting epigenomic sequences may include the guide being directed to an epigenomic target sequence. Epigenomic target sequence may include, in some embodiments, include a promoter, silencer or an enhancer sequence.

Use of a functional domain linked to a CRISPR-Cas enzyme as described herein, preferably a nickase Cas, or a Cas exhibiting little or not or not more than 5 or 4 or 3 or 2 or 1% nuclease activity (as compared to non-muted Cas), e.g., a dead-Cas, to target epigenomic sequences can be used to activate or repress promoters, silencer or enhancers. In the instant invention it is preferred that the Cas be a nickase, including as the invention can involve dual nickases.

Examples of acetyltransferases are known but may include, in some embodiments, histone acetyltransferases. In some embodiments, the histone acetyltransferase may comprise the catalytic core of the human acetyltransferase p300 (Gerbasch & Reddy, Nature Biotech 6th April 2015).

In some preferred embodiments, the functional domain is linked to the Cas9 enzyme to target and activate epigenomic sequences such as promoters or enhancers. One or more guides directed to such promoters or enhancers may also be provided to direct the binding of the CRISPR enzyme to such promoters or enhancers.

The term "associated with" is used here in relation to the association of the functional domain to the CRISPR enzyme or the adaptor protein. It is used in respect of how one molecule 'associates' with respect to another, for example between an adaptor protein and a functional domain, or between the CRISPR enzyme and a functional domain. In the case of such proteinprotein interactions, this association may be viewed in terms of recognition in the way an antibody recognizes an epitope. Alternatively, one protein may be associated with another protein via a fusion of the two, for instance one subunit being fused to another subunit. Fusion typically occurs by addition of the amino acid sequence of one to that of the other, for instance via splicing together of the nucleotide sequences that encode each protein or subunit. Alternatively, this may essentially be viewed as binding between two molecules or direct linkage, such as a fusion protein. In any event, the fusion protein may include a linker between the two subunits of interest (i.e. between the enzyme and the functional domain or between the adaptor protein and the functional domain). Thus, in some embodiments, the CRISPR enzyme or adaptor protein is associated with a functional domain by binding thereto. In other embodiments, the CRISPR enzyme or adaptor protein is associated with a functional domain because the two are fused together, optionally via an intermediate linker.

Attachment can be via a linker, e.g., a flexible glycine-serine (GlyGlyGlySer) or (GGGS)$_3$ or a rigid alpha-helical linker such as (Ala(GluAlaAlaAlaLys)Ala). Linkers such as (GGGGS)$_3$ are preferably used herein to separate protein or peptide domains. (GGGGS)$_3$ is preferable because it is a relatively long linker (15 amino acids). The glycine residues are the most flexible and the serine residues enhance the chance that the linker is on the outside of the protein. (GGGGS)$_6$ (GGGGS)$_9$ or (GGGGS)$_{12}$ may preferably be used as alternatives. Other preferred alternatives are (GGGGS)$_1$, (GGGGS)$_2$, (GGGGS)$_4$, (GGGGS)$_5$, (GGGGS)$_7$, (GGGGS)$_8$, (GGGGS)$_{10}$, or (GGGGS)$_{11}$. Alternative linkers are available, but highly flexible linkers are thought to work best to allow for maximum opportunity for the 2 parts of the Cas9 to come together and thus reconstitute Cas9 activity. One alternative is that the NLS of nucleoplasmin can be used as a linker. For example, a linker can also be used between the Cas9 and any functional domain. Again, a (GGGGS)$_3$ linker may be used here (or the 6, 9, or 12 repeat versions therefore) or the NLS of nucleoplasmin can be used as a linker between Cas9 and the functional domain.

Aspects of the invention relate to the expression of the gene product being decreased or a template polynucleotide being further introduced into the DNA molecule encoding the gene product or an intervening sequence being excised by allowing the two 3' overhangs to reanneal and ligate or the activity or function of the gene product being altered or the expression of the gene product being increased. In an embodiment of the invention, the gene product is a protein. The excision of the intervening sequence can have precision through the use of the 3' overhangs. Thus, the invention envisions an intervening sequence being precisely excised by allowing the two 3' overhangs to reanneal and ligate or the activity or function of the gene product being altered or the expression of the gene product being increased.

Delivery options advantageous for delivery to the brain include encapsulation of mutated CRISPR enzyme and guide RNA in the form of either DNA or RNA into liposomes and conjugating to molecular Trojan horses for trans-blood brain barrier (BBB) delivery. Molecular Trojan horses have been shown to be effective for delivery of B-gal expression vectors into the brain of non-human primates. The same approach can be used to delivery vectors containing CRISPR enzyme and guide RNA. For instance, Xia CF and Boado RJ, Pardridge WM ("Antibody-mediated targeting of siRNA via the human insulin receptor using avidin-biotin technology." Mol Pharm. 2009 May-Jun;6(3):747-51. doi: 10.1021/mp800194) describes how delivery of short interfering RNA (siRNA) to cells in culture, and in vivo, is possible with combined use of a receptor-specific monoclonal antibody (mAb) and avidin-biotin technology. The authors also report that because the bond between the targeting mAb and the siRNA is stable with avidin-biotin technology, and RNAi effects at distant sites such as brain are observed in vivo following an intravenous administration of the targeted siRNA.

Zhang Y, Schlachetzki F, Pardridge WM. ("Global non-viral gene transfer to the primate brain following intravenous administration." Mol Ther. 2003 Jan;7(1):11-8.) describe how expression plasmids encoding reporters such as luciferase were encapsulated in the interior of an "artificial virus" comprised of an 85 nm pegylated immunoliposome, which was targeted to the rhesus monkey brain in vivo with a monoclonal antibody (MAb) to the human insulin receptor (HIR). The HIRMAb enables the liposome carrying the exogenous gene to undergo transcytosis across the blood-brain barrier and endocytosis across the neuronal plasma membrane following intravenous injection. The level of luciferase gene expression in the brain was 50-fold higher in the rhesus monkey as compared to the rat. Widespread neuronal expression of the beta-galactosidase gene in primate brain was demonstrated by both histochemistry and confocal microscopy. The authors indicate that this approach makes feasible reversible adult transgenics in 24 hours. Accordingly, the use of immunoliposome is preferred. These may be used in conjunction with antibodies to target specific tissues or cell surface proteins. Other means of delivery or for RNA delivery are also preferred, such as via nanoparticles (Cho, S., Goldberg, M., Son, S., Xu, Q., Yang, F., Mei, Y., Bogatyrev, S., Langer, R. and Anderson, D., Lipid-like nanoparticles for small interfering RNA delivery to endothelial cells, Advanced Functional Materials, 19: 3112-3118, 2010) or exosomes (Schroeder, A., Levins, C., Cortez, C., Langer, R., and Anderson, D., Lipid-based nanotherapeutics for siRNA delivery, Journal of Internal Medicine, 267: 9-21, 2010, PMID: 20059641). Indeed, exozomes have been shown to be particularly useful in delivery siRNA, a system with some parallels to the CRISPR system. For instance, El-Andaloussi S, et al. ("Exosome-mediated delivery of siRNA in vitro and in vivo." Nat Protoc. 2012 Dec;7(12):2112-26. doi: 10.1038/nprot.2012.131. Epub 2012 Nov 15.) describe how exosomes are promising tools for drug delivery across different biological barriers and can be harnessed for delivery of siRNA in vitro and in vivo. Their approach is to generate targeted exosomes through transfection of an expression vector, comprising an exosomal protein fused with a peptide ligand. The exosomes are then purify and characterized from transfected cell supernatant, then siRNA is loaded into the exosomes.

Targeted deletion of genes is preferred. Preferred are, therefore, genes involved in cholesterol biosynthesis, fatty acid biosynthesis, and other metabolic disorders, genes encoding mis-folded proteins involved in amyloid and other diseases, oncogenes leading to cellular transformation, latent viral genes, and genes leading to dominant-negative disorders, amongst other disorders. As exemplified here, Applicants prefer gene delivery of a CRISPR-Cas system to the liver, brain, ocular, epithelial, hematopoetic, or another tissue of a subject or a patient in need thereof, suffering from metabolic disorders, amyloidosis and protein-aggregation related diseases, cellular transformation arising from genetic mutations and translocations, dominant negative effects of gene mutations, latent viral infections, and other related symptoms, using either viral or nanoparticle delivery system.

Therapeutic applications of the CRISPR-Cas system include Glaucoma, Amyloidosis, and Huntington's disease.

As an example, chronic infection by HIV-1 may be treated or prevented. In order to accomplish this, one may generate CRISPR-Cas guide RNAs that target the vast majority of the HIV-1 genome while taking into account HIV-1 strain variants for maximal coverage and effectiveness. One may accomplish delivery of the CRISPR-Cas system by conventional adenoviral or lentiviral-mediated infection of the host immune system. Depending on approach, host immune cells could be a) isolated, transduced with CRISPR-Cas, selected, and re-introduced in to the host or b) transduced in vivo by systemic delivery of the CRISPR-Cas system. The first approach allows for generation of a resistant immune population whereas the second is more likely to target latent viral reservoirs within the host.

It is also envisaged that the present disclosure generates a gene knockout cell library. Each cell may have a single gene knocked out.

One may make a library of ES cells where each cell has a single gene knocked out, and the entire library of ES cells will have every single gene knocked out. This library is useful for the screening of gene function in cellular processes as well as diseases. To make this cell library, one may integrate mutated Cas9 driven by an inducible promoter (e.g. doxycycline inducible promoter) into the ES cell. In addition, one may integrate a single guide RNA targeting a specific gene in the ES cell. To make the ES cell library, one may simply mix ES cells with a library of genes encoding guide RNAs targeting each gene in the human genome. One may first introduce a single BxB1 attB site into the AAVS1 locus of the human ES cell. Then one may use the BxB1 integrase to facilitate the integration of individual guide RNA genes into the BxB1 attB site in AAVS1 locus. To facilitate integration, each guide RNA gene may be contained on a plasmid that carries of a single attP site. This way BxB1 will recombine the attB site in the genome with the attP site on the guide RNA containing plasmid. To generate the cell library, one may take the library of cells that have single guide RNAs integrated and induce mutated Cas9 expression. After induction, paired mutated Cas9 mediates paired nicking at sites specified by the guide RNA.

Chronic administration of protein therapeutics may elicit unacceptable immune responses to the specific protein. The immunogenicity of protein drugs can be ascribed to a few immunodominant helper T lymphocyte (HTL) epitopes. Reducing the MHC binding affinity of these HTL epitopes contained within these proteins can generate drugs with lower immunogenicity (Tangri S, et al. ("Rationally engineered therapeutic proteins with reduced immunogenicity" J Immunol. 2005 Mar 15;174(6):3187-96.) In the present disclosure, the immunogenicity of the CRISPR enzyme in particular may be reduced following the approach first set out in Tangri et al with respect to erythropoietin and subsequently developed. Accordingly, directed evolution or rational design may be used to reduce the immunogenicity of the mutated CRISPR enzyme (for instance a mutated Cas9) in the host species (human or other species).

Trinucleotide repeat disorders are preferred conditions to be treated.

According to another aspect, a method of gene therapy for the treatment of a subject having a mutation in the CFTR gene is provided and comprises administering a therapeutically effective amount of a CRISPR-Cas gene therapy particle, optionally via a biocompatible pharmaceutical carrier, to the cells of a subject. Preferably, the target DNA comprises the mutation deltaF508. In general, it is of preferred that the mutation is repaired to the wildtype. In this case, the mutation is a deletion of the three nucleotides that comprise the codon for phenylalanine (F) at position 508. Accordingly, repair in this instance requires reintroduction of the missing codon into the mutant.

To implement this Gene Repair Strategy, it is preferred that an adenovirus/AAV vector system is introduced into the host cell, cells or patient. Preferably, the system comprises a mutated Cas9 (or mutated Cas9 nickase) and the guide RNA along with a adenovirus/AAV vector system comprising the homology repair template containing the F508 residue. This may be introduced into the subject via one of the methods of delivery discussed earlier. The CRISPR-Cas system may be guided by the CFTRdelta 508 chimeric guide RNA. It targets a specific site of the CFTR genomic locus to be nicked or cleaved. After cleavage, the repair template is inserted into the cleavage site via homologous recombination correcting the deletion that results in cystic fibrosis or causes cystic fibrosis related symptoms. This strategy to direct delivery and provide systemic introduction of CRISPR systems with appropriate guide RNAs can be employed to target genetic mutations to edit or otherwise manipulate genes that cause metabolic, liver, kidney and protein diseases and disorders such as those in Table B.

The treated subjects in this instance receive pharmaceutically effective amount of aerosolized AAV vector system per lung endobronchially delivered while spontaneously breathing. As such, aerosolized delivery is preferred for AAV delivery in general. An adenovirus or an AAV particle may be used for delivery. Suitable gene constructs, each operably linked to one or more regulatory sequences, may be cloned into the delivery vector. In this instance, the following constructs are provided as examples: Cbh or EF1a promoter for Cas9, U6 or H1 promoter for chimeric guide RNA),: A preferred arrangement is to use a CFTRdelta508 targeting chimeric guide, a repair template for deltaF508 mutation and a codon optimized mutated Cas9 nickase with optionally one or more nuclear localization signal or sequence(s) (NLS(s)), e.g., two (2) or two (2) or more NLSs. Constructs without NLS are also envisaged. However, advantageous embodiments can involve two or two or more NLSs.

In order to identify the Cas9 target site, Applicants analyzed the human CFTR genomic locus and identified the Cas9 target site. Preferably, in general and in this CF case, the PAM may contain a NGG or a NNAGAAW motif.

Alternatives to CF include any genetic disorder and examples of these are well known. Another preferred method or use of the invention is for correcting defects in the EMP2A and EMP2B genes that have been identified to be associated with Lafora disease.

In some embodiments, a "guide sequence" may be distinct from "guide RNA". A guide sequence may refer to an approx. 20bp sequence, within the guide RNA, that specifies the target site.

It will be readily apparent that a host of other diseases can be treated in a similar fashion. Some examples of genetic diseases caused by mutations are provided herein, but many more are known. The above strategy can be applied to these diseases.

The invention uses nucleic acids to bind target DNA sequences. This is advantageous as nucleic acids are much easier and cheaper to produce and the specificity can be varied according to the length of the stretch where homology is sought. Complex 3-D positioning of multiple fingers, for example is not required.

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. The term also encompasses nucleic-acid-like structures with synthetic backbones, see, e.g., Eckstein, 1991; Baserga et al., 1992; Milligan, 1993; WO 97/03211; WO 96/39154; Mata, 1997; Strauss-Soukup, 1997; and Samstag, 1996. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

As used herein the term "wild type" is a term of the art understood by skilled persons and means the typical form of an organism, strain, gene or characteristic as it occurs in nature as distinguished from mutant or variant forms.

As used herein the term "variant" should be taken to mean the exhibition of qualities that have a pattern that deviates from what occurs in nature.

The terms "non-naturally occurring" or "engineered" are used interchangeably and indicate the involvement of the hand of man. The terms, when referring to nucleic acid molecules or polypeptides mean that the nucleic acid molecule or the polypeptide is at least substantially free from at least one other component with which they are naturally associated in nature and as found in nature.

"Complementarity" refers to the ability of a nucleic acid to form hydrogen bond(s) with another nucleic acid sequence by either traditional Watson-Crick base pairing or other nontraditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology-Hybridization With Nucleic Acid Probes Part I, Second Chapter "Overview of principles of hybridization and the strategy of nucleic acid probe assay", Elsevier, N.Y. Where reference is made to a polynucleotide sequence, then complementary or partially complementary sequences are also envisaged. These are preferably capable of hybridising to the reference sequence under highly stringent conditions. Generally, in order to maximize the hybridization rate, relatively low-stringency hybridization conditions are selected: about 20 to 25° C. lower than the thermal melting point ($T_m$). The $T_m$ is the temperature at which 50% of specific target sequence hybridizes to a perfectly complementary probe in solution at a defined ionic strength and pH. Generally, in order to require at least about 85% nucleotide complementarity of hybridized sequences, highly stringent washing conditions are selected to be about 5 to 15° C. lower than the $T_m$. In order to require at least about 70% nucleotide complementarity of hybridized sequences, moderately-stringent washing conditions are selected to be about 15 to 30° C. lower than the $T_m$. Highly permissive (very low stringency) washing conditions may be as low as 50° C. below the T

$_m$, allowing a high level of mis-matching between hybridized sequences. Those skilled in the art will recognize that other physical and chemical parameters in the hybridization and wash stages can also be altered to affect the outcome of a detectable hybridization signal from a specific level of homology between target and probe sequences. Preferred highly stringent conditions comprise incubation in 50% formamide, 5×SSC, and 1% SDS at 42° C., or incubation in 5×SSC and 1% SDS at 65° C., with wash in 0.2×SSC and 0.1% SDS at 65° C.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PCR, or the cleavage of a polynucleotide by an enzyme. A sequence capable of hybridizing with a given sequence is referred to as the "complement" of the given sequence.

As used herein, the term "genomic locus" or "locus" (plural loci) is the specific location of a gene or DNA sequence on a chromosome. A "gene" refers to stretches of DNA or RNA that encode a polypeptide or an RNA chain that has functional role to play in an organism and hence is the molecular unit of heredity in living organisms. For the purpose of this invention it may be considered that genes include regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

As used herein, "expression of a genomic locus" or "gene expression" is the process by which information from a gene is used in the synthesis of a functional gene product. The products of gene expression are often proteins, but in non-protein coding genes such as rRNA genes or tRNA genes, the product is functional RNA. The process of gene expression is used by all known life - eukaryotes (including multicellular organisms), prokaryotes (bacteria and archaea) and viruses to generate functional products to survive. As used herein "expression" of a gene or nucleic acid encompasses not only cellular gene expression, but also the transcription and translation of nucleic acid(s) in cloning systems and in any other context. As used herein, "expression" also refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The terms "polypeptide", "peptide" and "protein" are used interchangeably herein to refer to polymers of amino acids of any length. The polymer may be linear or branched, it may comprise modified amino acids, and it may be interrupted by non amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" includes natural and/or unnatural or synthetic amino acids, including glycine and both the D or L optical isomers, and amino acid analogs and peptidomimetics.

As used herein, the term "domain" or "protein domain" refers to a part of a protein sequence that may exist and function independently of the rest of the protein chain.

As described in aspects of the invention, sequence identity is related to sequence homology. Homology comparisons may be conducted by eye, or more usually, with the aid of readily available sequence comparison programs. These commercially available computer programs may calculate percent (%) homology between two or more sequences and may also calculate the sequence identity shared by two or more amino acid or nucleic acid sequences. In some preferred embodiments, the capping region of the dTALEs described herein have sequences that are at least 95% identical or share identity to the capping region amino acid sequences provided herein.

Sequence homologies may be generated by any of a number of computer programs known in the art, for example BLAST or FASTA, etc. A suitable computer program for carrying out such an alignment is the GCG Wisconsin Bestfit package (University of Wisconsin, U.S.A; Devereux et al., 1984, Nucleic Acids Research 12:387). Examples of other software than may perform sequence comparisons include, but are not limited to, the BLAST package (see Ausubel et al., 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). However it is preferred to use the GCG Bestfit program.

Percentage (%) sequence homology may be calculated over contiguous sequences, i.e., one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion may cause the following amino acid residues to be put out of alignment, thus potentially resulting in a large reduction in % homology when a global alignment is performed. Consequently, most sequence comparison methods are designed to produce optimal alignments that take into consideration possible insertions and deletions without unduly penalizing the overall homology or identity score. This is achieved by inserting "gaps" in the sequence alignment to try to maximize local homology or identity.

However, these more complex methods assign "gap penalties" to each gap that occurs in the alignment so that, for the same number of identical amino acids, a sequence alignment with as few gaps as possible - reflecting higher relatedness between the two compared sequences - may achieve a higher score than one with many gaps. "Affinity gap costs" are typically used that charge a relatively high cost for the existence of a gap and a smaller penalty for each subsequent residue in the gap. This is the most commonly used gap scoring system. High gap penalties may, of course, produce optimized alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum % homology therefore first requires the production of an optimal alignment, taking into consideration gap penalties. A suitable computer program for carrying out such an alignment is the GCG Wisconsin Bestfit package (Devereux et al., 1984 Nuc. Acids Research 12 p387). Examples of other software than may perform sequence comparisons include, but are not limited to, the BLAST package (see Ausubel et al., 1999 Short Protocols in Molecular Biology, 4th Ed. - Chapter 18), FASTA (Altschul et al., 1990 J. Mol. Biol. 403-410) and the GENEWORKS suite of comparison tools. Both BLAST and FASTA are available for offline and online searching (see Ausubel et al., 1999, Short Protocols in Molecular Biology, pages 7-58 to 7-60). However, for some applications, it is preferred to use the GCG Bestfit program. A new tool, called BLAST 2 Sequences is also available for comparing protein and nucleotide sequences (see FEMS Microbiol Lett. 1999 174(2): 247-50; FEMS Microbiol Lett. 1999 177(1): 187-8 and the website of the National Center for Biotechnology information at the website of the National Institutes for Health).

Although the final % homology may be measured in terms of identity, the alignment process itself is typically not based on an all-or-nothing pair comparison. Instead, a scaled similarity score matrix is generally used that assigns scores to each pair-wise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table, if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244). Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in amino acid properties (such as polarity, charge, solubility, hydrophobicity, hydrophilicity, and/or the amphipathic nature of the residues) and it is therefore useful to group amino acids together in functional groups. Amino acids may be grouped together based on the properties of their side chains alone. However, it is more useful to include mutation data as well. The sets of amino acids thus derived are likely to be conserved for structural reasons. These sets may be described in the form of a Venn diagram (Livingstone C.D. and Barton G.J. (1993) "Protein sequence alignments: a strategy for the hierarchical analysis of residue conservation" Comput. Appl. Biosci. 9: 745-756) (Taylor W.R. (1986) "The classification of amino acid conservation" J. Theor. Biol. 119; 205-218). Conservative substitutions may be made, for example according to the table below which describes a generally accepted Venn diagram grouping of amino acids.

| Set | | Sub-set | |
|---|---|---|---|
| Hydrophobic | FWYHKMILVAGC | Aromatic | FWYH |
| | | Aliphatic | I L V |
| Polar | WYHKREDCSTNQ | Charged | HKRED |
| | | Positively charged | HKR |
| | | Negatively charged | ED |
| Small | VCAGSPTND | Tiny | AGS |

Embodiments of the invention include sequences (both polynucleotide or polypeptide) which may comprise homologous substitution (substitution and replacement are both used herein to mean the interchange of an existing amino acid residue or nucleotide, with an alternative residue or nucleotide) that may occur i.e., like-for-like substitution in the case of amino acids such as basic for basic, acidic for acidic, polar for polar, etc. Non-homologous substitution may also occur i.e., from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, which involves the presence of one or more amino acid residues in peptoid form, may be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The practice of the present invention employs, unless otherwise indicated, conventional techniques of immunology, biochemistry, chemistry, molecular biology, microbiology, cell biology, genomics and recombinant DNA, which are within the skill of the art. See Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney, ed. (1987)).

In one aspect, the disclosure provides for vectors that are used in the engineering and optimization of CRISPR-Cas systems.

A used herein, a "vector" is a tool that allows or facilitates the transfer of an entity from one environment to another. It is a replicon, such as a plasmid, phage, or cosmid, into which another DNA segment may be inserted so as to bring about the replication of the inserted segment. Generally, a vector is capable of replication when associated with the proper control elements. In general, the term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g. circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g. retroviruses, replication defective retroviruses, adenoviruses, replication defective adenoviruses, and adeno-associated viruses (AAVs)). Viral vectors also include polynucleotides carried by a virus for transfection into a host cell. Certain vectors are capable of autonomous replication in a host cell into which they are introduced (e.g. bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Common expression vectors of utility in recombinant DNA techniques are often in the form of plasmids.

Recombinant expression vectors can comprise a nucleic acid of the invention in a form suitable for expression of the nucleic acid in a host cell, which means that the recombinant expression vectors include one or more regulatory elements, which may be selected on the basis of the host cells to be used for expression, that is operatively-linked to the nucleic acid sequence to be expressed. Within a recombinant expression vector, "operably linked" is intended to mean that the nucleotide sequence of interest is linked to the regulatory element(s) in a manner that allows for expression of the nucleotide sequence (e.g. in an in vitro transcription/translation system or in a host cell when the vector is introduced into the host cell). With regards to recombination and cloning methods, mention is made of U.S. patent application 10/815,730, published September 2, 2004 as US 2004-0171156 A1.

Aspects of the disclosure relate to bicistronic vectors for chimeric RNA and mutated Cas9. Bicistronic expression vectors for chimeric RNA and mutated Cas9 are preferred. In general and particularly in this embodiment mutated Cas9 is preferably driven by the CBh promoter. The chimeric RNA may preferably be driven by a U6 promoter. Ideally the two are combined. The chimeric guide RNA typically consists of a 20bp guide sequence (Ns) and this may be joined to the tracr sequence (running from the first "U" of the lower strand to the end of the transcript). The tracr sequence may be truncated at various positions as indicated. The guide and tracr sequences are separated by the tracr-mate sequence, which may be GUUUUAGAGCUA. This may be followed by the loop sequence GAAA as shown. Both of these are preferred examples. Applicants have demonstrated mutated Cas9-mediated indels at the human *EMX1* and *PVALB* loci by SURVEYOR assays. ChiRNAs are indicated by their "+n" designation, and crRNA refers to a hybrid RNA where guide and tracr sequences are expressed as separate transcripts. Throughout this application, chimeric RNA may also be called single guide, or synthetic guide RNA (sgRNA). The loop is preferably GAAA, but it is not limited to this sequence or indeed to being only 4bp in length. Indeed, preferred loop forming sequences for use in hairpin structures are four nucleotides in length, and most preferably have the sequence GAAA. However, longer or shorter loop sequences may be used, as may alternative sequences. The sequences preferably include a nucleotide triplet (for example, AAA), and an additional nucleotide (for example C or G). Examples of loop forming sequences include CAAA and AAAG.

The term "regulatory element" is intended to include promoters, enhancers, internal ribosomal entry sites (IRES), and other expression control elements (e.g. transcription termination signals, such as polyadenylation signals and poly-U sequences). Such regulatory elements are described, for example, in Goeddel, GENE EXPRESSION TECHNOLOGY: METHODS IN ENZYMOLOGY 185, Academic Press, San Diego, Calif. (1990). Regulatory elements include those that direct constitutive expression of a nucleotide sequence in many types of host cell and those that direct expression of the nucleotide sequence only in certain host cells (e.g., tissue-specific regulatory sequences). A tissue-specific promoter may direct expression primarily in a desired tissue of interest, such as muscle, neuron, bone, skin, blood, specific organs (e.g. liver, pancreas), or particular cell types (e.g. lymphocytes). Regulatory elements may also direct expression in a temporal-dependent manner, such as in a cell-cycle dependent or developmental stage-dependent manner, which may or may not also be tissue or cell-type specific. In some embodiments, a vector comprises one or more pol III promoter (e.g. 1, 2, 3, 4, 5, or more pol I promoters), one or more pol II promoters (e.g. 1, 2, 3, 4, 5, or more pol II promoters), one or more pol I promoters (e.g. 1, 2, 3, 4, 5, or more pol I promoters), or combinations thereof. Examples of pol III promoters include, but are not limited to, U6 and H1 promoters. Examples of pol II promoters include, but are not limited to, the retroviral Rous sarcoma virus (RSV) LTR promoter (optionally with the RSV enhancer), the cytomegalovirus (CMV) promoter (optionally with the CMV enhancer) [see, e.g., Boshart et al, Cell, 41:521-530 (1985)], the SV40 promoter, the dihydrofolate reductase promoter, the β-actin promoter, the phosphoglycerol kinase (PGK) promoter, and the EF1α promoter. Also encompassed by the term "regulatory element" are enhancer elements, such as WPRE; CMV enhancers; the R-U5' segment in LTR of HTLV-I (Mol. Cell. Biol., Vol. 8(1), p. 466-472, 1988); SV40 enhancer; and the intron sequence between exons 2 and 3 of rabbit β-globin (Proc. Natl. Acad. Sci. USA., Vol. 78(3), p. 1527-31, 1981). It will be appreciated by those skilled in the art that the design of the expression vector can depend on such factors as the choice of the host cell to be transformed, the level of expression desired, etc. A vector can be introduced into host cells to thereby produce transcripts, proteins, or peptides, including fusion proteins or peptides, encoded by nucleic acids as described herein (e.g., clustered regularly interspersed short palindromic repeats (CRISPR) transcripts, proteins, enzymes, mutant forms thereof, fusion proteins thereof, etc.). With regards to regulatory sequences, mention is made of U.S. patent application 10/491,026. With regards to promoters, mention is made of PCT publication WO 2011/028929 and U.S. application 12/511,940.

Vectors can be designed for expression of CRISPR transcripts (e.g. nucleic acid transcripts, proteins, or enzymes) in prokaryotic or eukaryotic cells. For example, CRISPR transcripts can be expressed in bacterial cells such as Escherichia coli, insect cells (using baculovirus expression vectors), yeast cells, or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated in vitro, for example using T7 promoter regulatory sequences and T7 polymerase.

Vectors may be introduced and propagated in a prokaryote or prokaryotic cell. In some embodiments, a prokaryote is used to amplify copies of a vector to be introduced into a eukaryotic cell or as an intermediate vector in the production of a vector to be introduced into a eukaryotic cell (e.g. amplifying a plasmid as part of a viral vector packaging system). In some embodiments, a prokaryote is used to amplify copies of a vector and express one or more nucleic acids, such as to provide a source of one or more proteins for delivery to a host cell or host organism. Expression of proteins in prokaryotes is most often carried out in Escherichia coli with vectors containing constitutive or inducible promoters directing the expression of either fusion or non-fusion proteins. Fusion vectors add a number of amino acids to a protein encoded therein, such as to the amino terminus of the recombinant protein. Such fusion vectors may serve one or more purposes, such as: (i) to increase expression of recombinant protein; (ii) to increase the solubility of the recombinant protein; and (iii) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Example fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion E. coli expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calif. (1990) 60-89).

In some embodiments, a vector is a yeast expression vector. Examples of vectors for expression in yeast Saccharomyces cerivisae include pYepSec1 (Baldari, et al., 1987. EMBO J. 6: 229-234), pMFa (Kuijan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.).

In some embodiments, a vector drives protein expression in insect cells using baculovirus expression vectors. Baculovirus vectors available for expression of proteins in cultured insect cells (e.g., SF9 cells) include the pAc series (Smith, et al., 1983. Mol. Cell. Biol. 3: 2156-2165) and the pVL series (Lucklow and Summers, 1989. Virology 170: 31-39).

In some embodiments, a vector is capable of driving expression of one or more sequences in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are typically provided by one or more regulatory elements. For example, commonly used promoters are derived from polyoma, adenovirus 2, cytomegalovirus, simian virus 40, and others disclosed herein and known in the art. For other suitable expression systems for both prokaryotic and eukaryotic cells see, e.g., Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

In some embodiments, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Baneiji, et al., 1983. Cell 33: 729-740; Queen and Baltimore, 1983. Cell 33: 741-748), neuron-specific promoters (e.g., the neurofilament promoter; Byrne and Ruddle, 1989. Proc. Natl. Acad. Sci. USA 86: 5473-5477), pancreas-specific promoters (Edlund, et al., 1985. Science 230: 912-916), and mammary gland-specific promoters (e.g., milk whey promoter; U.S. Pat. No. 4,873,316 and European Application Publication No. 264,166). Developmentally-regulated promoters are also encompassed, e.g., the murine hox promoters (Kessel and Gruss, 1990. Science 249: 374-379) and the $\alpha$-fetoprotein promoter (Campes and Tilghman, 1989. Genes Dev. 3: 537-546). With regards to these prokaryotic and eukaryotic vectors, mention is made of U.S. Patent 6,750,059. Other embodiments of the invention may relate to the use of viral vectors, with regards to which mention is made of U.S. Patent application 13/092,085. Tissue-specific regulatory elements are known in the art and in this regard, mention is made of U.S. Patent 7,776,321.

In some embodiments, a regulatory element is operably linked to one or more elements of a CRISPR system so as to drive expression of the one or more elements of the CRISPR system. In general, CRISPRs (Clustered Regularly Interspaced Short Palindromic Repeats), also known as SPIDRs (SPacer Interspersed Direct Repeats), constitute a family of DNA loci that are usually specific to a particular bacterial species. The CRISPR locus comprises a distinct class of interspersed short sequence repeats (SSRs) that were recognized in E. coli (Ishino et al., J. Bacteriol., 169:5429-5433 [1987]; and Nakata et al., J. Bacteriol., 171:3553-3556 [1989]), and associated genes. Similar interspersed SSRs have been identified in Haloferax mediterranei, Streptococcus pyogenes, Anabaena, and Mycobacterium tuberculosis (See, Groenen et al., Mol. Microbiol., 10:1057-1065 [1993]; Hoe et al., Emerg. Infect. Dis., 5:254-263 [1999]; Masepohl et al., Biochim. Biophys. Acta 1307:26-30 [1996]; and Mojica et al., Mol. Microbiol., 17:85-93 [1995]). The CRISPR loci typically differ from other SSRs by the structure of the repeats, which have been termed short regularly spaced repeats (SRSRs) (Janssen et al., OMICS J. Integ. Biol., 6:23-33 [2002]; and Mojica et al., Mol. Microbiol., 36:244-246 [2000]). In general, the repeats are short elements that occur in clusters that are regularly spaced by unique intervening sequences with a substantially constant length (Mojica et al., [2000], supra). Although the repeat sequences are highly conserved between strains, the number of interspersed repeats and the sequences of the spacer regions typically differ from strain to strain (van Embden et al., J. Bacteriol., 182:2393-2401 [2000]). CRISPR loci have been identified in more than 40 prokaryotes (See e.g., Jansen et al., Mol. Microbiol., 43:1565-1575 [2002]; and Mojica et al., [2005]) including, but not limited to Aeropyrum, Pyrobaculum, Sulfolobus, Archaeoglobus, Halocarcula, Methanobacterium, Methanococcus, Methanosarcina, Methanopyrus, Pyrococcus, Picrophilus, Thermoplasma, Corynebacterium, Mycobacterium, Streptomyces, Aquifex, Porphyromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myxococcus, Campylobacter, Wolinella, Acinetobacter, Erwinia, Escherichia, Legionella, Methylococcus, Pasteurella, Photobacterium, Salmonella, Xanthomonas, Yersinia, Treponema, and Thermotoga.

In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. In embodiments of the invention the terms guide sequence and guide RNA are used interchangeably. In some embodiments, one or more elements of a CRISPR system is derived from a type I, type II, or type III CRISPR system. In some embodiments, one or more elements of a CRISPR system is derived from a particular organism comprising an endogenous CRISPR system, such as Streptococcus pyogenes. In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise any polynucleotide, such as DNA or RNA polynucleotides. In some embodiments, a target sequence is located in the nucleus or cytoplasm of a cell.

In some embodiments, direct repeats may be identified *in silico* by searching for repetitive motifs that fulfill any or all of the following criteria:

1. found in a 2Kb window of genomic sequence flanking the type II CRISPR locus;
2. span from 20 to 50 bp; and
3. interspaced by 20 to 50 bp.

In some embodiments, 2 of these criteria may be used, for instance 1 and 2, 2 and 3, or 1 and 3. In some embodiments, all 3 criteria may be used.

In some embodiments, candidate tracrRNA may be subsequently predicted by sequences that fulfill any or all of the following criteria:

1. sequence homology to direct repeats (motif search in Geneious with up to 18-bp mismatches);
2. presence of a predicted Rho-independent transcriptional terminator in direction of transcription; and
3. stable hairpin secondary structure between tracrRNA and direct repeat.

In some embodiments, 2 of these criteria may be used, for instance 1 and 2, 2 and 3, or 1 and 3. In some embodiments, all 3 criteria may be used.

In some embodiments, chimeric synthetic guide RNAs (sgRNAs) designs may incorporate at least 12 bp of duplex structure between the direct repeat and tracrRNA.

In the invention, the CRISPR system is a type II CRISPR system and the Cas enzyme is mutated Cas9 (N863A), which catalyzes DNA cleavage. Enzymatic action by mutated Cas9 derived from Streptococcus pyogenes or any closely related Cas9 generates double stranded breaks at target site sequences which hybridize to 20 nucleotides of the guide sequence and that have a protospacer-adjacent motif (PAM) sequence (examples include NGG/NRG or a PAM that can be determined as described herein) following the 20 nucleotides of the target sequence. CRISPR activity through mutated Cas9 for site-specific DNA recognition and cleavage is defined by the guide sequence, the tracr sequence that hybridizes in part to the guide sequence and the PAM sequence. More aspects of the CRISPR system are described in Karginov and Hannon, The CRISPR system: small RNA-guided defence in bacteria and archaea, Mole Cell 2010, January 15; 37(1): 7.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. In some embodiments, a vector comprises an insertion site upstream of a tracr mate sequence, and optionally downstream of a regulatory element operably linked to the tracr mate sequence, such that following insertion of a guide sequence into the insertion site and upon expression the guide sequence directs sequence-specific binding of a CRISPR complex to a target sequence in a eukaryotic cell. In some embodiments, a vector comprises two or more insertion sites, each insertion site being located between two tracr mate sequences so as to allow insertion of a guide sequence at each site. In such an arrangement, the two or more guide sequences may comprise two or more copies of a single guide sequence, two or more different guide sequences, or combinations of these. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell..

In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human mammal or primate. In some embodiments, processes for modifying the germ line genetic identity of human beings and/or processes for modifying the genetic identity of animals which are likely to cause them suffering without any substantial medical benefit to man or animal, and also animals resulting from such processes, may be excluded.

In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at www.kazusa.orjp/codon/ (visited Jul. 9, 2002), and these tables can be adapted in a number of ways. See Nakamura, Y., et al. "Codon usage tabulated from the international DNA sequence databases: status for the year 2000"Nucl. Acids Res. 28:292 (2000). Computer algorithms for codon optimizing a particular sequence for expression in a particular host cell are also available, such as Gene Forge (Aptagen; Jacobus, PA), are also available. In some embodiments, one or more codons (e.g. 1, 2, 3, 4, 5, 10, 15, 20, 25, 50, or more, or all codons) in a sequence encoding a CRISPR enzyme correspond to the most frequently used codon for a particular amino acid.

In some embodiments, a vector encodes a mutated CRISPR enzyme comprising one or two or more nuclear localization sequences (NLSs), such as about or more than about 1, 2, 3, 4, 5, 6, 7, 8, 9, 10, or more NLSs. In some embodiments, the mutated CRISPR enzyme comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. one or more NLS at the amino-terminus and one or more NLS at the carboxy terminus). When more than one NLS is present, each may be selected independently of the others, such that a single NLS may be present in more than one copy and/or in combination with one or more other NLSs present in one or more copies. In a preferred embodiment of the invention, the mutated CRISPR enzyme comprises at most 6 NLSs. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C-terminus. Non-limiting examples of NLSs include an NLS sequence derived from: the NLS of the SV40 virus large T-antigen, having the amino acid sequence PKKKRKV; the NLS from nucleoplasmin (e.g. the nucleoplasmin bipartite NLS with the sequence KRPAATKKAGQAKKKK); the c-myc NLS having the amino acid sequence PAAKRVKLD or RQRRNELKRSP; the hRNPA1 M9 NLS having the sequence NQSSNFGPMKGGNFGGRSSGPYGGGGQYFAKPRNQGGY; the sequence RMRIZFKNKGKDTAELRRRRVEVSVELRKAKKDEQILKRRNV of the IBB domain from importin-alpha; the sequences VSRKRPRP and PPKKARED of the myoma T protein; the sequence POPKKKPL of human p53; the sequence SALIKKKKKMAP of mouse c-abl IV; the sequences DRLRR and PKQKKRK of the influenza virus NS1; the sequence RKLKKKIKKL of the Hepatitis virus delta antigen; the sequence REKKKFLKRR of the mouse Mx1 protein; the sequence KRKGDEVDGVDEVAKKKSKK of the human poly(ADP-ribose) polymerase; and the sequence RKCLQAGMNLEARKTKK of the steroid hormone receptors (human) glucocorticoid.

In general, the one or more NLSs are of sufficient strength to drive accumulation of the mutated CRISPR enzyme in a detectable amount in the nucleus of a eukaryotic cell. In general, strength of nuclear localization activity may derive from the number of NLSs in the CRISPR enzyme, the particular NLS(s) used, or a combination of these factors. Detection of accumulation in the nucleus may be performed by any suitable technique. For example, a detectable marker may be fused to the mutated CRISPR enzyme, such that location within a cell may be visualized, such as in combination with a means for detecting the location of the nucleus (e.g. a stain specific for the nucleus such as DAPI). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of CRISPR complex formation (e.g. assay for DNA cleavage or mutation at the target sequence, or assay for altered gene expression activity affected by CRISPR complex formation and/or CRISPR enzyme activity), as compared to a control no exposed to the mutated CRISPR enzyme or complex, or exposed to a mutated CRISPR enzyme lacking the one or more NLSs.

It is preferred, and this can apply to any of the aspects or embodiments of the invention, that the methods, systems and compositions described herein do not include an NLS. In other words, although generally useful, use of an NLS can be optional. For example, if mitochondrial DNA is to be targeted, then an NLS is not required.

In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

As mentioned above, optimal guide length for Sa may be, in some embodiments, 20 or more preferably, 21, 22, 23 or 24 nucleotides.

A guide sequence may be selected to target any target sequence. In some embodiments, the target sequence is a sequence within a genome of a cell. Exemplary target sequences include those that are unique in the target genome. For example, for the *S. pyogenes* Cas9, a unique target sequence in a genome may include a Cas9 target site of the form MMMMMMMMNNNNNNNNNNNNXGG where NNNNNNNNNNNNXGG (N is A, G, T, or C; and X can be anything) has a single occurrence in the genome. A unique target sequence in a genome may include an *S. pyogenes* Cas9 target site of the form MMMMMMMMMNNNNNNNNNNNXGG where NNNNNNNNNNNXGG (N is A, G, T, or C; and X can be anything) has a single occurrence in the genome. For the *S. thermophilus* CRISPR1 Cas9, a unique target sequence in a genome may include a Cas9 target site of the form MMMMMMMMNNNNNNNNNNNNXXAGAAW where NNNNNNNNNNNNXXAGAAW (N is A, G, T, or C; X can be anything; and W is A or T) has a single occurrence in the genome. A unique target sequence in a genome may include an *S. thermophilus* CRISPR1 Cas9 target site of the form MMMMMMMMMNNNNNNNNNNNXXAGAAW where NNNNNNNNNNNXXAGAAW (N is A, G, T, or C; X can be anything; and W is A or T) has a single occurrence in the genome. For the *S. pyogenes* Cas9, a unique target sequence in a genome may include a Cas9 target site of the form MMMMMMMMNNNNNNNNNNNNXGGXG where NNNNNNNNNNNNXGGXG (N is A, G, T, or C; and X can be anything) has a single occurrence in the genome. A unique target sequence in a genome may include an *S. pyogenes* Cas9 target site of the form MMMMMMMMMNNNNNNNNNNNXGGXG where NNNNNNNNNNNXGGXG (N is A, G, T, or C; and X can be anything) has a single occurrence in the genome. In each of these sequences "M" may be A, G, T, or C, and need not be considered in identifying a sequence as unique.

In some embodiments, a guide sequence is selected to reduce the degree secondary structure within the guide sequence. In some embodiments, about or less than about 75%, 50%, 40%, 30%, 25%, 20%, 15%, 10%, 5%, 1%, or fewer of the nucleotides of the guide sequence participate in self-complementary base pairing when optimally folded. Optimal folding may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

In general, a tracr mate sequence includes any sequence that has sufficient complementarity with a tracr sequence to promote one or more of: (1) excision of a guide sequence flanked by tracr mate sequences in a cell containing the corresponding tracr sequence; and (2) formation of a CRISPR complex at a target sequence, wherein the CRISPR complex comprises the tracr mate sequence hybridized to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for secondary structures, such as selfcomplementarity within either the tracr sequence or tracr mate sequence. In some embodiments, the degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and tracr mate sequence are contained within a single transcript, such that hybridization between the two produces a transcript having a secondary structure, such as a hairpin. In an embodiment of the invention, the transcript or transcribed polynucleotide sequence has at least two or more hairpins. In preferred embodiments, the transcript has two, three, four or five hairpins. In a further embodiment of the invention, the transcript has at most five hairpins. In a hairpin structure the portion of the sequence 5' of the final "N" and upstream of the loop corresponds to the tracr mate sequence, and the portion of the sequence 3' of the loop corresponds to the tracr sequence Further non-limiting examples of single polynucleotides comprising a guide sequence, a tracr mate sequence, and a tracr sequence are as follows (listed 5' to 3'), where "N" represents a base of a guide sequence, the first block of lower case letters represent the tracr mate sequence, and the second block of lower case letters represent the tracr sequence, and the final poly-T sequence represents the transcription terminator:

(1)

NNNNNNNNNNNNNNNNNNNNgttttttgtactctcaagatttaGAAAtaaatcttgcagaagctacaaagataa
ggcttcatgccgaaatcaacaccctgtcattttatggcagggtgttttcgttatttaaTTTTTT;

(2)

NNNNNNNNNNNNNNNNNNNNgttttttgtactctcaGAAAtgcagaagctacaaagataaggcttcatgccg
aaatcaacaccctgtcattttatggcagggtgttttcgttatttaaTTTTTT;

(3)

NNNNNNNNNNNNNNNNNNNNgttttttgtactctcaGAAAtgcagaagctacaaagataaggcttcatgccg
aaatcaacaccctgtcattttatggcagggtgtTTTTTT;

(4)

NNNNNNNNNNNNNNNNNNNNgttttagagctaGAAAtagcaagttaaaataaggctagtccgttatcaactt
gaaaaagtggcaccgagtcggtgcTTTTTT;

(5)

NNNNNNNNNNNNNNNNNNNNgttttagagctaGAAATAGcaagttaaaataaggctagtccgttatcaac
ttgaaaaagtgTTTTTTT;

and
(6)

NNNNNNNNNNNNNNNNNNNNgttttagagctagAAATAGcaagttaaaataaggctagtccgttatcaTT
TTTTT.

In some embodiments, sequences (1) to (3) are used in combination with Cas9 from *S. thermophilus* CRISPR1. In some embodiments, sequences (4) to (6) are used in combination with mutated Cas9 from *S. pyogenes.* In some embodiments, the tracr sequence is a separate transcript from a transcript comprising the tracr mate sequence.
In some embodiments, a recombination template is also provided. A recombination template may be a component of another vector as described herein, contained in a separate vector, or provided as a separate polynucleotide. In some embodiments, a recombination template is designed to serve as a template in homologous recombination, such as within or near a target sequence nicked by a mutated CRISPR enzyme as a part of a CRISPR complex. A template polynucleotide may be of any suitable length, such as about or more than about 10, 15, 20, 25, 50, 75, 100, 150, 200, 500, 1000, or more nucleotides in length. In some embodiments, the template polynucleotide is complementary to a portion of a polynucleotide comprising the target sequence. When optimally aligned, a template polynucleotide might overlap with one or more nucleotides of a target sequences (e.g. about or more than about 1, 5, 10, 15, 20, or more nucleotides). In some embodiments, when a template sequence and a polynucleotide comprising a target sequence are optimally aligned, the nearest nucleotide of the template polynucleotide is within about 1, 5, 10, 15, 20, 25, 50, 75, 100, 200, 300, 400, 500, 1000, 5000, 10000, or more nucleotides from the target sequence.

In some embodiments, the mutated CRISPR enzyme is part of a fusion protein comprising one or more heterologous protein domains (e.g. about or more than about 1, 2, 3, 4, 5, 6, 7, 8, 9, 10, or more domains in addition to the mutated CRISPR enzyme). A mutated CRISPR enzyme fusion protein may comprise any additional protein sequence, and optionally a linker sequence between any two domains. Examples of protein domains that may be fused to a mutated CRISPR enzyme include, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). A mutated CRISPR enzyme may be fused to a gene sequence encoding a protein or a fragment of a protein that bind DNA molecules or bind other cellular molecules, including but not limited to maltose binding protein (MBP), S-tag, Lex A DNA binding domain (DBD) fusions, GAL4 DNA binding domain fusions, and herpes simplex virus (HSV) BP16 protein fusions. Additional domains that may form part of a fusion protein comprising a mutated CRISPR enzyme are described in US20110059502. In some embodiments, a tagged mutated CRISPR enzyme is used to identify the location of a target sequence.

In some embodiments, a mutated CRISPR enzyme may form a component of an inducible system. The inducible nature of the system would allow for spatiotemporal control of gene editing or gene expression using a form of energy. The form of energy may include but is not limited to electromagnetic radiation, sound energy, chemical energy and thermal energy. Examples of inducible system include tetracycline inducible promoters (Tet-On or Tet-Off), small molecule two-hybrid transcription activations systems (FKBP, ABA, etc), or light inducible systems (Phytochrome, LOV domains, or cryptochrome).In one embodiment, the mutated CRISPR enzyme may be a part of a Light Inducible Transcriptional Effector (LITE) to direct changes in transcriptional activity in a sequence-specific manner. The components of a light may include a CRISPR enzyme, a light-responsive cytochrome heterodimer (e.g. from Arabidopsis thaliana), and a transcriptional activation/repression domain. Further examples of inducible DNA binding proteins and methods for their use are provided in patents and patent application cited herein, such as US8889418, US8895308, US20140186919, US20140242700, US20140273234, US20140335620, WO2014093635A1, WO2014093635A9, and US 61/736465 and US 61/721,283.

As herein discussed, in some aspects, the invention provides methods comprising delivering one or more polynucleotides, such as or one or more vectors as described herein, one or more transcripts thereof, and/or one or proteins transcribed therefrom, to a host cell. In some aspects, the disclosure further provides cells produced by such methods, and animals comprising or produced from such cells. In some embodiments, a mutated CRISPR enzyme in combination with (and optionally complexed with) a guide sequence is delivered to a cell. Conventional viral and non-viral based gene transfer methods can be used to introduce nucleic acids in mammalian cells or target tissues. Such methods can be used to administer nucleic acids encoding components of a CRISPR system to cells in culture, or in a host organism. Non-viral vector delivery systems include DNA plasmids, RNA (e.g. a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell. For a review of gene therapy procedures, see Anderson, Science 256:808-813 (1992); Nabel & Feigner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Böhm (eds) (1995); and Yu et al., Gene Therapy 1:13-26 (1994). As herein discussed, methods of non-viral delivery of nucleic acids include electroporation, lipofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam™ and Lipofectin™). Cationic and neutral lipids that are suitable for efficient receptor-recognition lipofection of polynucleotides include those of Feigner, WO 91/17424; WO 91/16024. Delivery can be to cells (e.g. in vitro or ex vivo administration) or target tissues (e.g. in vivo administration). The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787). As herein discussed the invention can involve RNA or DNA viral based delivery systems. The use of RNA or DNA viral based systems for the delivery of nucleic acids take advantage of highly evolved processes for targeting a virus to specific cells in the body and trafficking the viral payload to the nucleus. Viral vectors can be administered directly to patients (in vivo) or they can be used to treat cells in vitro, and the modified cells may optionally be administered to patients (ex vivo). Conventional viral based systems could include retroviral, lentivirus, adenoviral, adeno-associated and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues. The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system would therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., J. Virol. 66:2731-2739 (1992); Johann et al., J. Virol. 66:1635-1640 (1992); Sommnerfelt et al., Virol. 176:58-59 (1990); Wilson et al., J. Virol. 63:2374-2378 (1989); Miller et al., J. Virol. 65:2220-2224 (1991); PCT/US94/05700). In applications where transient expression is preferred, adenoviral based systems may be used. Adenoviral based vectors are capable of very high transduction efficiency in many cell types and do not require cell division. With such vectors, high titer and levels of expression have been obtained. This vector can be produced in large quantities in a relatively simple system. Adeno-associated virus ("AAV") vectors may also be used to transduce cells with target nucleic acids, e.g., in the in vitro production of nucleic acids and peptides, and for in vivo and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989). Packaging cells are typically used to form virus particles that are capable of infecting a host cell. Such cells include 293 cells, which package adenovirus, and ψ2 cells or PA317 cells, which package retrovirus. Viral vectors used in gene therapy are usually generated by producer a cell line that packages a nucleic acid vector into a viral particle. The vectors typically contain the minimal viral sequences required for packaging and subsequent integration into a host, other viral sequences being replaced by an expression cassette for the polynucleotide(s) to be expressed. The missing viral functions are typically supplied in trans by the packaging cell line. For example, AAV vectors used in gene therapy typically only possess ITR sequences from the AAV genome which are required for packaging and integration into the host genome. Viral DNA is packaged in a cell line, which contains a helper plasmid encoding the other AAV genes, namely rep and cap, but lacking ITR sequences. The cell line may also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV. Accordingly, AAV is considered an advantageous candidate for use as a transducing vector. Such AAV transducing vectors can comprise sufficient cis-acting functions to replicate in the presence of adenovirus or herpesvirus or poxvirus (e.g., vaccinia virus) helper functions provided in trans. Recombinant AAV (rAAV) can be used to carry exogenous genes into cells of a variety of lineages. In these vectors, the AAV cap and/or rep genes are deleted from the viral genome and replaced with a DNA segment of choice. Current AAV vectors may accommodate up to 4300 bases of inserted DNA. There are a number of ways to produce rAAV, and the invention provides rAAV and methods for preparing rAAV. For example, plasmid(s) containing or consisting essentially of the desired viral construct are transfected into AAV-infected cells. In addition, a second or additional helper plasmid is cotransfected into these cells to provide the AAV rep and/or cap genes which are obligatory for replication and packaging of the recombinant viral construct. Under these conditions, the rep and/or cap proteins of AAV act in trans to stimulate replication and packaging of the rAAV construct. Two to Three days after transfection, rAAV is harvested. Traditionally rAAV is harvested from the cells along with adenovirus. The contaminating adenovirus is then inactivated by heat treatment. In the instant disclosure, rAAV is advantageously harvested not from the cells themselves, but from cell supernatant. Accordingly, in an initial aspect the disclosure provides for preparing rAAV, and in addition to the foregoing, rAAV can be prepared by a method that comprises or consists essentially of: infecting susceptible cells with a rAAV containing exogenous DNA including DNA for expression, and helper virus (e.g., adenovirus, herpesvirus, poxvirus such as vaccinia virus) wherein the rAAV lacks functioning cap and/or rep (and the helper virus (e.g., adenovirus, herpesvirus, poxvirus such as vaccinia virus) provides the cap and/or rev function that the rAAV lacks); or infecting susceptible cells with a rAAV containing exogenous DNA including DNA for expression, wherein the recombinant lacks functioning cap and/or rep, and transfecting said cells with a plasmid supplying cap and/or rep function that the rAAV lacks; or infecting susceptible cells with a rAAV containing exogenous DNA including DNA for expression, wherein the recombinant lacks functioning cap and/or rep, wherein said cells supply cap and/or rep function that the recombinant lacks; or transfecting the susceptible cells with an AAV lacking functioning cap and/or rep and plasmids for inserting exogenous DNA into the recombinant so that the exogenous DNA is expressed by the recombinant and for supplying rep and/or cap functions whereby transfection results in an rAAV containing the exogenous DNA including DNA for expression that lacks functioning cap and/or rep. The rAAV can be from an AAV as herein described, and advantageously can be an rAAV1, rAAV2, AAV5 or rAAV having hybrid or capsid which may comprise AAV1, AAV2, AAV5 or any combination thereof. One can select the AAV of the rAAV with regard to the cells to be targeted by the rAAV; e.g., one can select AAV serotypes 1, 2, 5 or a hybrid or capsid AAV1, AAV2, AAV5 or any combination thereof for targeting brain or neuronal cells; and one can select AAV4 for targeting cardiac tissue. The disclosure provides rAAV that contains or consists essentially of an exogenous nucleic acid molecule encoding a CRISPR (Clustered Regularly Interspaced Short Palindromic Repeats) system, e.g., a plurality of cassettes comprising or consisting a first cassette comprising or consisting essentially of a promoter, a nucleic acid molecule encoding a mutated CRISPR-associated (Cas) protein (putative nuclease or helicase proteins), e.g., mutated Cas9 (N863A) and a terminator, and a two, or more, advantageously up to the packaging size limit of the vector, e.g., in total (including the first cassette) five, cassettes comprising or consisting essentially of a promoter, nucleic acid molecule encoding guide RNA (gRNA) and a terminator (e.g., each cassette schematically represented as Promoter-gRNAl-terminator, Promoter-gRNA2-terminator ... Promoter-gRNA(N)-terminator (where N is a number that can be inserted that is at an upper limit of the packaging size limit of the vector), or two or more individual rAAVs, each containing one or more than one cassette of a CRISPR system, e.g., a first rAAV containing the first cassette comprising or consisting essentially of a promoter, a nucleic acid molecule encoding Cas, e.g., Cas9 and a terminator, and a second rAAV containing a plurality, four, cassettes comprising or consisting essentially of a promoter, nucleic acid molecule encoding guide RNA (gRNA) and a terminator (e.g., each cassette schematically represented as Promoter-gRNAl-terminator, Promoter-gRNA2-terminator ... Promoter-gRNA(N)-terminator (where N is a number that can be inserted that is at an upper limit of the packaging size limit of the vector). As rAAV is a DNA virus, the nucleic acid molecules in the herein discussion concerning AAV or rAAV are advantageously DNA. The promoter is in some embodiments advantageously human Synapsin I promoter (hSyn). Additional methods for the delivery of nucleic acids to cells are known to those skilled in the art. See, for example, US20030087817.

In some embodiments, a host cell is transiently or non-transiently transfected with one or more vectors described herein. In some embodiments, a cell is transfected as it naturally occurs in a subject. In some embodiments, a cell that is transfected is taken from a subject. In some embodiments, the cell is derived from cells taken from a subject, such as a cell line. A wide variety of cell lines for tissue culture are known in the art. Examples of cell lines include, but are not limited to, C8161, CCRF-CEM, MOLT, mIMCD-3, NHDF, HeLa-S3, Huh1, Huh4, Huh7, HUVEC, HASMC, HEKn, HEKa, MiaPaCell, Panc1, PC-3, TF1, CTLL-2, C1R, Rat6, CV1, RPTE, A10, T24, J82, A375, ARH-77, Calu1, SW480, SW620, SKOV3, SK-UT, CaCo2, P388D1, SEM-K2, WEHI-231, HB56, TIB55, Jurkat, J45.01, LRMB, Bcl-1, BC-3, IC21, DLD2, Raw264.7, NRK, NRK-52E, MRC5, MEF, Hep G2, HeLa B, HeLa T4, COS, COS-1, COS-6, COS-M6A, BS-C-1 monkey kidney epithelial, BALB/ 3T3 mouse embryo fibroblast, 3T3 Swiss, 3T3-L1, 132-d5 human fetal fibroblasts; 10.1 mouse fibroblasts, 293-T, 3T3, 721, 9L, A2780, A2780ADR, A2780cis, A172, A20, A253, A431, A-549, ALC, B16, B35, BCP-1 cells, BEAS-2B, bEnd.3, BHK-21, BR 293, BxPC3, C3H-10T1/2, C6/36, Cal-27, CHO, CHO-7, CHO-IR, CHO-K1, CHO-K2, CHO-T, CHO Dhfr -/-, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepalclc7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MDCK II, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN / OPCT cell lines, Peer, PNT-1A / PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR, and transgenic varieties thereof. Cell lines are available from a variety of sources known to those with skill in the art (see, e.g., the American Type Culture Collection (ATCC) (Manassus, Va.)). In some embodiments, a cell transfected with one or more vectors described herein is used to establish a new cell line comprising one or more vector-derived sequences. In some embodiments, a cell transiently transfected with the components of a CRISPR system as described herein (such as by transient transfection of one or more vectors, or transfection with RNA), and modified through the activity of a CRISPR complex, is used to establish a new cell line comprising cells containing the modification but lacking any other exogenous sequence. In some embodiments, cells transiently or non-transiently transfected with one or more vectors described herein, or cell lines derived from such cells are used in assessing one or more test compounds.

In some embodiments, one or more vectors described herein are used to produce a non-human transgenic animal or transgenic plant, for example a model organism. In some embodiments, the transgenic animal is a mammal, such as a mouse, rat, or rabbit. Methods for producing transgenic plants and animals are known in the art, and generally begin with a method of cell transfection, such as described herein.

With respect to use of the CRISPR-Cas system generally, mention is made of the documents, including patent applications, patents, and patent publications cited throughout this disclosure as embodiments of the invention can be used as in those documents. CRISPR-Cas system(s) (e.g., single or multiplexed) can be used in conjunction with recent advances in crop genomics. Such CRISPR-Cas system(s) can be used to perform efficient and cost effective plant gene or genome interrogation or editing or manipulation-for instance, for rapid investigation and/or selection and/or interrogations and/or comparison and/or manipulations and/or transformation of plant genes or genomes; e.g., to create, identify, develop, optimize, or confer trait(s) or characteristic(s) to plant(s) or to transform a plant genome. There can accordingly be improved production of plants, new plants with new combinations of traits or characteristics or new plants with enhanced traits. Such CRISPR-Cas system(s) can be used with regard to plants in Site-Directed Integration (SDI) or Gene Editing (GE) or any Near Reverse Breeding (NRB) or Reverse Breeding (RB) techniques. With respect to use of the CRISPR-Cas system in plants, mention is made of the University of Arizona website "CRISPR-PLANT" (http://www.genome.arizona.edu/crispr/) (supported by Penn State and AGI). Emodiments of the invention can be used in genome editing in plants or where RNAi or similar genome editing techniques have been used previously; see, e.g., Nekrasov, "Plant genome editing made easy: targeted mutagenesis in model and crop plants using the CRISPR/Cas system," Plant Methods 2013, 9:39 (doi:10.1186/1746-4811-9-39); Brooks, "Efficient gene editing in tomato in the first generation using the CRISPR/Cas9 system," Plant Physiology September 2014 pp 114.247577; Shan, "Targeted genome modification of crop plants using a CRISPR-Cas system," Nature Biotechnology 31, 686-688 (2013); Feng, "Efficient genome editing in plants using a CRISPR/Cas system," Cell Research (2013) 23:1229-1232. doi:10.1038/cr.2013.114; published online 20 August 2013; Xie, "RNA-guided genome editing in plants using a CRISPR-Cas system," Mol Plant. 2013 Nov;6(6):1975-83. doi: 10.1093/mp/sst119. Epub 2013 Aug 17; Xu, "Gene targeting using the Agrobacterium tumefaciens-mediated CRISPR-Cas system in rice," Rice 2014, 7:5 (2014), Zhou et al., "Exploiting SNPs for biallelic CRISPR mutations in the outcrossing woody perennial Populus reveals 4-coumarate: CoA ligase specificity and Redundancy," New Phytologist (2015) (Forum) 1-4 (available online only at www.newphytologist.com); Caliando et al, "Targeted DNA degradation using a CRISPR device stably carried in the host genome, NATURE COMMUNICATIONS 6:6989, DOI: 10.1038/ncomms7989, www.nature.com/naturecommunications DOI: 10.1038/ncomms7989; US Patent No. 6,603,061 - Agrobacterium-Mediated Plant Transformation Method; US Patent No. 7,868,149 - Plant Genome Sequences and Uses Thereof and US 2009/0100536 - Transgenic Plants with Enhanced Agronomic Traits. In the practice of the invention, the contents and disclosure of Morrell et al "Crop genomics: advances and applications," Nat Rev Genet. 2011 Dec 29;13(2):85-96; Accordingly, reference herein to animal cells may also apply, mutatis mutandis, to plant cells unless otherwise apparent.

In one aspect, the invention provides for methods of modifying a target polynucleotide in a eukaryotic cell, which may be *ex vivo*. In some embodiments, the method comprises sampling a cell or population of cells from a human or non-human animal or plant (including micro-algae), and modifying the cell or cells. Culturing may occur at any stage *ex vivo*. The cell or cells may even be re-introduced into the non-human animal or plant (including micro-algae). For re-introduced cells it is particularly preferred that the cells are stem cells.

In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence.

In one aspect, the invention provides a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a mutated CRISPR enzyme complexed with a guide sequence hybridized to a target sequence within said polynucleotide, wherein said guide sequence is linked to a tracr mate sequence which in turn hybridizes to a tracr sequence. Similar considerations and conditions apply as above for methods of modifying a target polynucleotide. In fact, these sampling, culturing and re-introduction options apply across the aspects of the present invention.

Indeed, in any aspect of the invention, the CRISPR complex may comprise a mutated CRISPR enzyme complexed with a guide sequence hybridized to a target sequence, wherein said guide sequence may be linked to a tracr mate sequence which in turn may hybridize to a tracr sequence. Similar considerations and conditions apply as above for methods of modifying a target polynucleotide.

In one aspect, the disclosure provides kits containing any one or more of the elements disclosed in the above methods and compositions. Elements may be provided individually or in combinations, and may be provided in any suitable container, such as a vial, a bottle, or a tube. In some embodiments, the kit includes instructions in one or more languages, for example in more than one language.

In some embodiments, a kit comprises one or more reagents for use in a process utilizing one or more of the elements described herein. Reagents may be provided in any suitable container. For example, a kit may provide one or more reaction or storage buffers. Reagents may be provided in a form that is usable in a particular assay, or in a form that requires addition of one or more other components before use (e.g. in concentrate or lyophilized form). A buffer can be any buffer, , including but not limited to a sodium carbonate buffer, a sodium bicarbonate buffer, a borate buffer, a Tris buffer, a MOPS buffer, a HEPES buffer, and combinations thereof. In some embodiments, the buffer is alkaline. In some embodiments, the buffer has a pH from about 7 to about 10. In some embodiments, the kit comprises one or more oligonucleotides corresponding to a guide sequence for insertion into a vector so as to operably link the guide sequence and a regulatory element. In some embodiments, the kit comprises a homologous recombination template polynucleotide.

In one aspect, the invention provides methods for using one or more elements of a CRISPR system. The CRISPR complex of the invention provides an effective means for modifying a target polynucleotide. The CRISPR complex of the invention has a wide variety of utility including modifying (e.g., deleting, inserting, translocating, inactivating, activating) a target polynucleotide in a multiplicity of cell types. As such the CRISPR complex of the invention has a broad spectrum of applications in, e.g., gene therapy, drug screening, disease diagnosis, and prognosis. An exemplary CRISPR complex comprises a mutated CRISPR enzyme complexed with a guide sequence hybridized to a target sequence within the target polynucleotide. The guide sequence is linked to a tracr mate sequence, which in turn hybridizes to a tracr sequence.

The break created by the CRISPR complex can be repaired by a repair processes such as the error prone non-homologous end joining (NHEJ) pathway or the high fidelity homologydirected repair (HDR). During these repair process, an exogenous polynucleotide template can be introduced into the genome sequence. In some methods, the HDR process is used modify genome sequence. For example, an exogenous polynucleotide template comprising a sequence to be integrated flanked by an upstream sequence and a downstream sequence is introduced into a cell. The upstream and downstream sequences share sequence similarity with either side of the site of integration in the chromosome.

Where desired, a donor polynucleotide can be DNA, e.g., a DNA plasmid, a bacterial artificial chromosome (BAC), a yeast artificial chromosome (YAC), a viral vector, a linear piece of DNA, a PCR fragment, a naked nucleic acid, or a nucleic acid complexed with a delivery vehicle such as a liposome or poloxamer.

The exogenous polynucleotide template comprises a sequence to be integrated (e.g., a mutated gene). The sequence for integration may be a sequence endogenous or exogenous to the cell. Examples of a sequence to be integrated include polynucleotides encoding a protein or a non-coding RNA (e.g., a microRNA). Thus, the sequence for integration may be operably linked to an appropriate control sequence or sequences. Alternatively, the sequence to be integrated may provide a regulatory function.

The upstream and downstream sequences in the exogenous polynucleotide template are selected to promote recombination between the chromosomal sequence of interest and the donor polynucleotide. The upstream sequence is a nucleic acid sequence that shares sequence similarity with the genome sequence upstream of the targeted site for integration. Similarly, the downstream sequence is a nucleic acid sequence that shares sequence similarity with the chromosomal sequence downstream of the targeted site of integration. The upstream and downstream sequences in the exogenous polynucleotide template can have 75%, 80%, 85%, 90%, 95%, or 100% sequence identity with the targeted genome sequence. Preferably, the upstream and downstream sequences in the exogenous polynucleotide template have about 95%, 96%, 97%, 98%, 99%, or 100% sequence identity with the targeted genome sequence. In some methods, the upstream and downstream sequences in the exogenous polynucleotide template have about 99% or 100% sequence identity with the targeted genome sequence.

An upstream or downstream sequence may comprise from about 20 bp to about 2500 bp, for example, about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. In some methods, the exemplary upstream or downstream sequence have about 200 bp to about 2000 bp, about 600 bp to about 1000 bp, or more particularly about 700 bp to about 1000 bp.

In some methods, the exogenous polynucleotide template may further comprise a marker. Such a marker may make it easy to screen for targeted integrations. Examples of suitable markers include restriction sites, fluorescent proteins, or selectable markers. The exogenous polynucleotide template of the invention can be constructed using recombinant techniques (see, for example, Sambrook et al., 2001 and Ausubel et al., 1996).

In other embodiments, this invention provides a method of modifying expression of a polynucleotide in a eukaryotic cell. The method comprises increasing or decreasing expression of a target polynucleotide by using a CRISPR complex that binds to the polynucleotide.

In some methods, a target polynucleotide can be inactivated to effect the modification of the expression in a cell. For example, upon the binding of a CRISPR complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein is not produced.

In some methods, a control sequence can be inactivated such that it no longer functions as a control sequence. As used herein, "control sequence" refers to any nucleic acid sequence that effects the transcription, translation, or accessibility of a nucleic acid sequence. Examples of a control sequence include, a promoter, a transcription terminator, and an enhancer are control sequences.

The inactivated target sequence may include a deletion mutation (i.e., deletion of one or more nucleotides), an insertion mutation (i.e., insertion of one or more nucleotides), or a nonsense mutation (i.e., substitution of a single nucleotide for another nucleotide such that a stop codon is introduced). In some methods, the inactivation of a target sequence results in "knockout" of the target sequence.

A method of the invention may be used to create a plant, an animal or cell that may be used as a disease model. As used herein, "disease" refers to a disease, disorder, or indication in a subject. For example, a method of the invention may be used to create an animal or cell that comprises a modification in one or more nucleic acid sequences associated with a disease, or a plant, animal or cell in which the expression of one or more nucleic acid sequences associated with a disease are altered. Such a nucleic acid sequence may encode a disease associated protein sequence or may be a disease associated control sequence. Accordingly, it is understood that in embodiments of the invention, a plant, subject, patient, organism or cell can be a non-human subject, patient, organism or cell. Thus, the invention provides a plant, animal or cell, produced by the present methods, or a progeny thereof. The progeny may be a clone of the produced plant or animal, or may result from sexual reproduction by crossing with other individuals of the same species to introgress further desirable traits into their offspring. The cell may be *in vivo* or *ex vivo* in the cases of multicellular organisms, particularly animals or plants. In the instance where the cell is in cultured, a cell line may be established if appropriate culturing conditions are met and preferably if the cell is suitably adapted for this purpose (for instance a stem cell). Bacterial cell lines produced by the disclosure are also envisaged. Hence, cell lines are also envisaged.

In some methods, the disease model can be used to study the effects of mutations on the animal or cell and development and/or progression of the disease using measures commonly used in the study of the disease. Alternatively, such a disease model is useful for studying the effect of a pharmaceutically active compound on the disease.

In some methods, the disease model can be used to assess the efficacy of a potential gene therapy strategy. That is, a disease-associated gene or polynucleotide can be modified such that the disease development and/or progression is inhibited or reduced. In particular, the method comprises modifying a disease-associated gene or polynucleotide such that an altered protein is produced and, as a result, the animal or cell has an altered response. Accordingly, in some methods, a genetically modified animal may be compared with an animal predisposed to development of the disease such that the effect of the gene therapy event may be assessed.

In another embodiment, this disclosure provides a method of developing a biologically active agent that modulates a cell signaling event associated with a disease gene. The method comprises contacting a test compound with a cell comprising one or more vectors that drive expression of one or more of a mutated CRISPR enzyme, a guide sequence linked to a tracr mate sequence, and a tracr sequence; and detecting a change in a readout that is indicative of a reduction or an augmentation of a cell signaling event associated with, e.g., a mutation in a disease gene contained in the cell.

A cell model or animal model can be constructed in combination with the method of the disclosure for screening a cellular function change. Such a model may be used to study the effects of a genome sequence modified by the CRISPR complex of the invention on a cellular function of interest. For example, a cellular function model may be used to study the effect of a modified genome sequence on intracellular signaling or extracellular signaling. Alternatively, a cellular function model may be used to study the effects of a modified genome sequence on sensory perception. In some such models, one or more genome sequences associated with a signaling biochemical pathway in the model are modified.

Several disease models have been specifically investigated. These include *de novo* autism risk genes CHD8, KATNAL2, and SCN2A; and the syndromic autism (Angelman Syndrome) gene UBE3A. These genes and resulting autism models are of course preferred, but serve to show the broad applicability of the invention across genes and corresponding models.

An altered expression of one or more genome sequences associated with a signaling biochemical pathway can be determined by assaying for a difference in the mRNA levels of the corresponding genes between the test model cell and a control cell, when they are contacted with a candidate agent. Alternatively, the differential expression of the sequences associated with a signaling biochemical pathway is determined by detecting a difference in the level of the encoded polypeptide or gene product.

To assay for an agent-induced alteration in the level of mRNA transcripts or corresponding polynucleotides, nucleic acid contained in a sample is first extracted according to standard methods in the art. For instance, mRNA can be isolated using various lytic enzymes or chemical solutions according to the procedures set forth in Sambrook et al. (1989), or extracted by nucleic-acid-binding resins following the accompanying instructions provided by the manufacturers. The mRNA contained in the extracted nucleic acid sample is then detected by amplification procedures or conventional hybridization assays (e.g. Northern blot analysis) according to methods widely known in the art or based on the methods exemplified herein.

For purpose of this invention, amplification means any method employing a primer and a polymerase capable of replicating a target sequence with reasonable fidelity. Amplification may be carried out by natural or recombinant DNA polymerases such as TaqGold™, T7 DNA polymerase, Klenow fragment of E.coli DNA polymerase, and reverse transcriptase. A preferred amplification method is PCR. In particular, the isolated RNA can be subjected to a reverse transcription assay that is coupled with a quantitative polymerase chain reaction (RT-PCR) in order to quantify the expression level of a sequence associated with a signaling biochemical pathway.

Detection of the gene expression level can be conducted in real time in an amplification assay. In one aspect, the amplified products can be directly visualized with fluorescent DNA-binding agents including but not limited to DNA intercalators and DNA groove binders. Because the amount of the intercalators incorporated into the double-stranded DNA molecules is typically proportional to the amount of the amplified DNA products, one can conveniently determine the amount of the amplified products by quantifying the fluorescence of the intercalated dye using conventional optical systems in the art. DNA-binding dye suitable for this application include SYBR green, SYBR blue, DAPI, propidium iodine, Hoeste, SYBR gold, ethidium bromide, acridines, proflavine, acridine orange, acriflavine, fluorcoumanin, ellipticine, daunomycin, chloroquine, distamycin D, chromomycin, homidium, mithramycin, ruthenium polypyridyls, anthramycin, and the like.

In another aspect, other fluorescent labels such as sequence specific probes can be employed in the amplification reaction to facilitate the detection and quantification of the amplified products. Probe-based quantitative amplification relies on the sequence-specific detection of a desired amplified product. It utilizes fluorescent, target-specific probes (e.g., TaqMan® probes) resulting in increased specificity and sensitivity. Methods for performing probe-based quantitative amplification are well established in the art and are taught in U.S. Patent No. 5,210,015.

In yet another aspect, conventional hybridization assays using hybridization probes that share sequence homology with sequences associated with a signaling biochemical pathway can be performed. Typically, probes are allowed to form stable complexes with the sequences associated with a signaling biochemical pathway contained within the biological sample derived from the test subject in a hybridization reaction. It will be appreciated by one of skill in the art that where antisense is used as the probe nucleic acid, the target polynucleotides provided in the sample are chosen to be complementary to sequences of the antisense nucleic acids. Conversely, where the nucleotide probe is a sense nucleic acid, the target polynucleotide is selected to be complementary to sequences of the sense nucleic acid.

Hybridization can be performed under conditions of various stringency. Suitable hybridization conditions for the practice of the present invention are such that the recognition interaction between the probe and sequences associated with a signaling biochemical pathway is both sufficiently specific and sufficiently stable. Conditions that increase the stringency of a hybridization reaction are widely known and published in the art. See, for example, (Sambrook, et al., (1989); Nonradioactive In Situ Hybridization Application Manual, Boehringer Mannheim, second edition). The hybridization assay can be formed using probes immobilized on any solid support, including but are not limited to nitrocellulose, glass, silicon, and a variety of gene arrays. A preferred hybridization assay is conducted on high-density gene chips as described in U.S. Patent No. 5,445,934.

For a convenient detection of the probe-target complexes formed during the hybridization assay, the nucleotide probes are conjugated to a detectable label. Detectable labels suitable for use in the present invention include any composition detectable by photochemical, biochemical, spectroscopic, immunochemical, electrical, optical or chemical means. A wide variety of appropriate detectable labels are known in the art, which include fluorescent or chemiluminescent labels, radioactive isotope labels, enzymatic or other ligands. In preferred embodiments, one will likely desire to employ a fluorescent label or an enzyme tag, such as digoxigenin, β-galactosidase, urease, alkaline phosphatase or peroxidase, avidin/biotin complex.

The detection methods used to detect or quantify the hybridization intensity will typically depend upon the label selected above. For example, radiolabels may be detected using photographic film or a phosphoimager. Fluorescent markers may be detected and quantified using a photodetector to detect emitted light. Enzymatic labels are typically detected by providing the enzyme with a substrate and measuring the reaction product produced by the action of the enzyme on the substrate; and finally colorimetric labels are detected by simply visualizing the colored label.

An agent-induced change in expression of sequences associated with a signaling biochemical pathway can also be determined by examining the corresponding gene products. Determining the protein level typically involves a) contacting the protein contained in a biological sample with an agent that specifically bind to a protein associated with a signaling biochemical pathway; and (b) identifying any agent:protein complex so formed. In one aspect of this embodiment, the agent that specifically binds a protein associated with a signaling biochemical pathway is an antibody, preferably a monoclonal antibody.

The reaction is performed by contacting the agent with a sample of the proteins associated with a signaling biochemical pathway derived from the test samples under conditions that will allow a complex to form between the agent and the proteins associated with a signaling biochemical pathway. The formation of the complex can be detected directly or indirectly according to standard procedures in the art. In the direct detection method, the agents are supplied with a detectable label and unreacted agents may be removed from the complex; the amount of remaining label thereby indicating the amount of complex formed. For such method, it is preferable to select labels that remain attached to the agents even during stringent washing conditions. It is preferable that the label does not interfere with the binding reaction.

In the alternative, an indirect detection procedure may use an agent that contains a label introduced either chemically or enzymatically. A desirable label generally does not interfere with binding or the stability of the resulting agent:polypeptide complex. However, the label is typically designed to be accessible to an antibody for an effective binding and hence generating a detectable signal.

A wide variety of labels suitable for detecting protein levels are known in the art. Non-limiting examples include radioisotopes, enzymes, colloidal metals, fluorescent compounds, bioluminescent compounds, and chemiluminescent compounds.

The amount of agent:polypeptide complexes formed during the binding reaction can be quantified by standard quantitative assays. As illustrated above, the formation of agent:polypeptide complex can be measured directly by the amount of label remained at the site of binding. In an alternative, the protein associated with a signaling biochemical pathway is tested for its ability to compete with a labeled analog for binding sites on the specific agent. In this competitive assay, the amount of label captured is inversely proportional to the amount of protein sequences associated with a signaling biochemical pathway present in a test sample.

A number of techniques for protein analysis based on the general principles outlined above are available in the art. They include but are not limited to radioimmunoassays, ELISA (enzyme linked immunoradiometric assays), "sandwich" immunoassays, immunoradiometric assays, in situ immunoassays (using e.g., colloidal gold, enzyme or radioisotope labels), western blot analysis, immunoprecipitation assays, immunofluorescent assays, and SDS-PAGE.

Antibodies that specifically recognize or bind to proteins associated with a signaling biochemical pathway are preferable for conducting the aforementioned protein analyses. Where desired, antibodies that recognize a specific type of post-translational modifications (e.g., signaling biochemical pathway inducible modifications) can be used. Post-translational modifications include but are not limited to glycosylation, lipidation, acetylation, and phosphorylation. These antibodies may be purchased from commercial vendors. For example, anti-phosphotyrosine antibodies that specifically recognize tyrosine-phosphorylated proteins are available from a number of vendors including Invitrogen and Perkin Elmer. Anti-phosphotyrosine antibodies are particularly useful in detecting proteins that are differentially phosphorylated on their tyrosine residues in response to an ER stress. Such proteins include but are not limited to eukaryotic translation initiation factor 2 alpha (eIF-2$\alpha$). Alternatively, these antibodies can be generated using conventional polyclonal or monoclonal antibody technologies by immunizing a host animal or an antibody-producing cell with a target protein that exhibits the desired post-translational modification.

In practicing the subject method, it may be desirable to discern the expression pattern of an protein associated with a signaling biochemical pathway in different bodily tissue, in different cell types, and/or in different subcellular structures. These studies can be performed with the use of tissue-specific, cell-specific or subcellular structure specific antibodies capable of binding to protein markers that are preferentially expressed in certain tissues, cell types, or subcellular structures.

An altered expression of a gene associated with a signaling biochemical pathway can also be determined by examining a change in activity of the gene product relative to a control cell. The assay for an agent-induced change in the activity of a protein associated with a signaling biochemical pathway will dependent on the biological activity and/or the signal transduction pathway that is under investigation. For example, where the protein is a kinase, a change in its ability to phosphorylate the downstream substrate(s) can be determined by a variety of assays known in the art. Representative assays include but are not limited to immunoblotting and immunoprecipitation with antibodies such as anti-phosphotyrosine antibodies that recognize phosphorylated proteins. In addition, kinase activity can be detected by high throughput chemiluminescent assays such as AlphaScreen™ (available from Perkin Elmer) and eTag™ assay (Chan-Hui, et al. (2003) Clinical Immunology 111: 162-174).

Where the protein associated with a signaling biochemical pathway is part of a signaling cascade leading to a fluctuation of intracellular pH condition, pH sensitive molecules such as fluorescent pH dyes can be used as the reporter molecules. In another example where the protein associated with a signaling biochemical pathway is an ion channel, fluctuations in membrane potential and/or intracellular ion concentration can be monitored. A number of commercial kits and high-throughput devices are particularly suited for a rapid and robust screening for modulators of ion channels. Representative instruments include FLIPRTM (Molecular Devices, Inc.) and VIPR (Aurora Biosciences). These instruments are capable of detecting reactions in over 1000 sample wells of a microplate simultaneously, and providing realtime measurement and functional data within a second or even a minisecond.

In practicing any of the methods disclosed herein, a suitable vector can be introduced to a cell or an embryo via one or more methods known in the art, including without limitation, microinjection, electroporation, sonoporation, biolistics, calcium phosphate-mediated transfection, cationic transfection, liposome transfection, dendrimer transfection, heat shock transfection, nucleofection transfection, magnetofection, lipofection, impalefection, optical transfection, proprietary agent-enhanced uptake of nucleic acids, and delivery via liposomes, immunoliposomes, virosomes, or artificial virions. In some methods, the vector is introduced into an embryo by microinjection. The vector or vectors may be microinjected into the nucleus or the cytoplasm of the embryo. In some methods, the vector or vectors may be introduced into a cell by nucleofection.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA).

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA).

The target polynucleotide of a CRISPR complex may include a number of disease-associated genes and polynucleotides as well as signaling biochemical pathway-associated genes and polynucleotides as listed in the US patents mentioned herein including US Patents Nos. 8,999,641, 8,993,233, 8,945,839, 8,932,814, 8,906,616, 8,895,308, 8,889,418, 8,889,356, 8,871,445, 8,865,406, 8,795,965, 8,771,945 and 8,697,359 and US provisional patent applications 61/736,527 and 61/748,427, both entitled SYSTEMS METHODS AND COMPOSITIONS FOR SEQUENCE MANIPULATION filed on December 12, 2012 and January 2, 2013, respectively.

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

Examples of disease-associated genes and polynucleotides are listed in Tables A and B. Disease specific information is available from McKusick-Nathans Institute of Genetic Medicine, Johns Hopkins University (Baltimore, Md.) and National Center for Biotechnology Information, National Library of Medicine (Bethesda, Md.), available on the World Wide Web. Examples of signaling biochemical pathway-associated genes and polynucleotides are listed in Table C.

Mutations in these genes and pathways can result in production of improper proteins or proteins in improper amounts which affect function. Further examples of genes, diseases and proteins are hereby cited by reference from US Patents Nos. 8,999,641, 8,993,233, 8,945,839, 8,932,814, 8,906,616, 8,895,308, 8,889,418, 8,889,356, 8,871,445, 8,865,406, 8,795,965, 8,771,945 and 8,697,359 and US Provisional applications 61/736,527 filed on December 12, 2012 and 61/748,427 filed on January 2, 2013. Such genes, proteins and pathways may be the target polynucleotide of a CRISPR complex.

**Table A**

| DISEASE/DISORDER S | GENE(S) |
| --- | --- |
| Neoplasia | PTEN; ATM; ATR; EGFR; ERBB2; ERBB3; ERBB4; |
| | Notch1; Notch2; Notch3; Notch4; AKT; AKT2; AKT3; HIF; |
| | HIF1a; HIF3a; Met; HRG; Bcl2; PPAR alpha; PPAR |
| | gamma; WT1 (Wilms Tumor); FGF Receptor Family |
| | members (5 members: 1, 2, 3, 4, 5); CDKN2a; APC; RB |
| | (retinoblastoma); MEN1; VHL; BRCA1; BRCA2; AR |
| | (Androgen Receptor); TSG101; IGF; IGF Receptor; Igf1 (4 |

(continued)

| DISEASE/DISORDER S | GENE(S) |
|---|---|
| | variants); Igf2 (3 variants); Igf 1 Receptor; Igf 2 Receptor; |
| | Bax; Bcl2; caspases family (9 members: |
| | 1, 2, 3, 4, 6, 7, 8, 9, 12); Kras; Apc |
| Age-related Macular | Abcr; Ccl2; Cc2; cp (ceruloplasmin); Timp3; cathepsinD; |
| Degeneration | Vldlr; Ccr2 |
| Schizophrenia | Neuregulin1 (Nrg1); Erb4 (receptor for Neuregulin); |
| | Complexin1 (Cplx1); Tph1 Tryptophan hydroxylase; Tph2 |
| | Tryptophan hydroxylase 2; Neurexin 1; GSK3; GSK3a; |
| | GSK3b |
| Disorders | 5-HTT (Slc6a4); COMT; DRD (Drd1a); SLC6A3; DAOA; |
| | DTNBP1; Dao (Dao1) |
| Trinucleotide Repeat | HTT (Huntington's Dx); SBMA/SMAX1/AR (Kennedy's |
| Disorders | Dx); FXN/X25 (Friedrich's Ataxia); ATX3 (Machado- |
| | Joseph's Dx); ATXN1 and ATXN2 (spinocerebellar |
| | ataxias); DMPK (myotonic dystrophy); Atrophin-1 and Atn1 |
| | (DRPLA Dx); CBP (Creb-BP - global instability); VLDLR |
| | (Alzheimer's); Atxn7; Atxn10 |
| Fragile X Syndrome | FMR2; FXR1; FXR2; mGLUR5 |
| Secretase Related | APH-1 (alpha and beta); Presenilin (Psen1); nicastrin |
| Disorders | (Ncstn); PEN-2 |
| Others | Nos1; Parp1; Nat1; Nat2 |
| Prion - related disorders | Prp |
| ALS | SOD1; ALS2; STEX; FUS; TARDBP; VEGF (VEGF-a; |
| | VEGF-b; VEGF-c) |
| Drug addiction | Prkce (alcohol); Drd2; Drd4; ABAT (alcohol); GRIA2; |
| | Grm5; Grin1; Htrlb; Grin2a; Drd3; Pdyn; Gria1 (alcohol) |
| Autism | Mecp2; BZRAP1; MDGA2; Sema5A; Neurexin 1; Fragile X |
| | (FMR2 (AFF2); FXR1; FXR2; Mglur5) |
| Alzheimer's Disease | E1; CHIP; UCH; UBB; Tau; LRP; PICALM; Clusterin; PS1; |
| | SORL1; CR1; Vldlr; Uba1; Uba3; CHIP28 (Aqp1, |
| | Aquaporin 1); Uchl1; Uchl3; APP |
| Inflammation | IL-10; IL-1 (IL-1a; IL-1b); IL-13; IL-17 (IL-17a (CTLA8); IL- |
| | 17b; IL-17c; IL-17d; IL-17f); 11-23; Cx3cr1; ptpn22; TNFa; |
| | NOD2/CARD15 for IBD; IL-6; IL-12 (IL-12a; IL-12b); |
| | CTLA4; Cx3cl1 |
| Parkinson's Disease | x-Synuclein; DJ-1; LRRK2; Parkin; PINK1 |

**Table B:**

| Blood and coagulation diseases and disorders | Anemia (CDAN1, CDA1, RPS19, DBA, PKLR, PK1, NT5C3, UMPH1, PSN1, RHAG, RH50A, NRAMP2, SPTB, ALAS2, ANH1, ASB, ABCB7, ABC7, ASAT); Bare lymphocyte syndrome (TAPBP, TPSN, TAP2, ABCB3, PSF2, RING11, MHC2TA, C2TA, RFX5, RFXAP, RFX5), Bleeding disorders (TBXA2R, P2RX1, P2X1); Factor H and factor H-like 1 (HF1, CFH, HUS); Factor V and factor VIII (MCFD2); Factor VII deficiency (F7); Factor X deficiency (F10); Factor XI deficiency (F11); Factor XII deficiency (F12, HAF); Factor XIIIA deficiency (F13A1, F13A); Factor XIIIB deficiency (F13B); Fanconi anemia (FANCA, FACA, FA1, FA, FAA, FAAP95, FAAP90, FLJ34064, FANCB, FANCC, FACC, BRCA2, FANCD1, FANCD2, FANCD, FACD, FAD, FANCE, FACE, FANCF, XRCC9, FANCG, BRIP1, BACH1, FANCJ, PHF9, FANCL, FANCM, KIAA1596); Hemophagocytic lymphohistiocytosis disorders (PRF1, HPLH2, UNC13D, MUNC13-4, HPLH3, HLH3, FHL3); Hemophilia A (F8, F8C, HEMA); Hemophilia B (F9, HEMB), Hemorrhagic disorders (PI, ATT, F5); Leukocyde deficiencies and disorders (ITGB2, CD18, LCAMB, LAD, EIF2B1, EIF2BA, EIF2B2, EIF2B3, EIF2B5, LVWM, CACH, CLE, EIF2B4); Sickle cell anemia (HBB); Thalassemia (HBA2, HBB, HBD, LCRB, HBA1). |
|---|---|
| | |
| Cell dysregulation and oncology diseases and disorders | B-cell non-Hodgkin lymphoma (BCL7A, BCL7); Leukemia (TAL1, TCL5, SCL, TAL2, FLT3, NBS1, NBS, ZNFN1A1, IK1, LYF1, HOXD4, HOX4B, BCR, CML, PHL, ALL, ARNT, KRAS2, RASK2, GMPS, AF10, ARHGEF12, LARG, KIAA0382, CALM, CLTH, CEBPA, CEBP, CHIC2, BTL, FLT3, KIT, PBT, LPP, NPM1, NUP214, D9S46E, CAN, CAIN, RUNX1, CBFA2, AML1, WHSC1L1, NSD3, FLT3, AF1Q, NPM1, NUMA1, ZNF145, PLZF, PML, MYL, STAT5B, AF10, CALM, CLTH, ARL11, ARLTS1, P2RX7, P2X7, BCR, CML, PHL, ALL, GRAF, NF1, VRNF, WSS, NFNS, PTPN11, PTP2C, SHP2, NS1, BCL2, CCND1, PRAD1, BCL1, TCRA, GATA1, GF1, ERYF1, NFE1, ABL1, NQO1, DIA4, NMOR1, NUP214, D9S46E, CAN, CAIN). |
| | |
| Inflammation and immune related diseases and disorders | AIDS (KIR3DL1, NKAT3, NKB1, AMB11, KIR3DS1, IFNG, CXCL12, SDF1); Autoimmune lymphoproliferative syndrome (TNFRSF6, APT1, FAS, CD95, ALPS1A); Combined immunodeficiency, (IL2RG, SCIDX1, SCIDX, IMD4); HIV-1 (CCL5, SCYA5, D17S136E, TCP228), HIV susceptibility or infection (IL10, CSIF, CMKBR2, CCR2, CMKBR5, CCCKR5 (CCR5)); Immunodeficiencies (CD3E, CD3G, AICDA, AID, HIGM2, TNFRSF5, CD40, UNG, DGU, HIGM4, TNFSF5, CD40LG, HIGM1, IGM, FOXP3, IPEX, AIID, XPID, PIDX, TNFRSF14B, TACI); Inflammation (IL-10, IL-1 (IL-1a, IL-1b), IL-13, IL-17 (IL-17a (CTLA8), IL-17b, IL-17c, IL-17d, IL-17f), 11-23, Cx3cr1, |
| | ptpn22, TNFa, NOD2/CARD15 for IBD, IL-6, IL-12 (IL-12a, IL-12b), CTLA4, Cx3cl1); Severe combined immunodeficiencies (SCIDs)(JAK3, JAKL, DCLRE1C, ARTEMIS, SCIDA, RAG1, RAG2, ADA, PTPRC, CD45, LCA, IL7R, CD3D, T3D, IL2RG, SCIDX1, SCIDX, IMD4). |
| | |
| Metabolic, liver, kidney and protein diseases and disorders | Amyloid neuropathy (TTR, PALB); Amyloidosis (APOA1, APP, AAA, CVAP, AD1, GSN, FGA, LYZ, TTR, PALB); Cirrhosis (KRT18, KRT8, CIRH1A, NAIC, TEX292, KIAA1988); Cystic fibrosis (CFTR, ABCC7, CF, MRP7); Glycogen storage diseases (SLC2A2, GLUT2, G6PC, G6PT, G6PT1, GAA, LAMP2, LAMPB, AGL, GDE, GBE1, GYS2, PYGL, PFKM); Hepatic adenoma, 142330 (TCF1, HNF1A, MODY3), Hepatic failure, early onset, and neurologic disorder (SCOD1, SCO1), Hepatic lipase deficiency (LIPC), Hepatoblastoma, cancer and carcinomas (CTNNB1, PDGFRL, PDGRL, PRLTS, AXIN1, AXIN, CTNNB1, TP53, P53, LFS1, IGF2R, MPRI, MET, CASP8, MCH5; Medullary cystic kidney disease (UMOD, HNFJ, FJHN, MCKD2, ADMCKD2); Phenylketonuria (PAH, PKU1, QDPR, DHPR, PTS); Polycystic kidney and hepatic disease (FCYT, PKHD1, ARPKD, PKD1, PKD2, PKD4, PKDTS, PRKCSH, G19P1, PCLD, SEC63). |
| | |

(continued)

| Muscular / Skeletal diseases and disorders | Becker muscular dystrophy (DMD, BMD, MYF6), Duchenne Muscular Dystrophy (DMD, BMD); Emery-Dreifuss muscular dystrophy (LMNA, LMN1, EMD2, FPLD, CMD1A, HGPS, LGMD1B, LMNA, LMN1, EMD2, FPLD, CMD1A); Facioscapulohumeral muscular dystrophy (FSHMD1A, FSHD1A); Muscular dystrophy (FKRP, MDC1C, LGMD2I, LAMA2, LAMM, LARGE, KIAA0609, MDC1D, FCMD, TTID, MYOT, CAPN3, CANP3, DYSF, LGMD2B, SGCG, LGMD2C, DMDA1, SCG3, SGCA, ADL, DAG2, LGMD2D, DMDA2, SGCB, LGMD2E, SGCD, SGD, LGMD2F, CMD1L, TCAP, LGMD2G, CMD1N, TRIM32, HT2A, LGMD2H, FKRP, MDC1C, LGMD2I, TTN, CMD1G, TMD, LGMD2J, POMT1, CAV3, LGMD1C, SEPN1, SELN, RSMD1, PLEC1, PLTN, EBS1); Osteopetrosis (LRP5, BMND1, LRP7, LR3, OPPG, VBCH2, CLCN7, CLC7, OPTA2, OSTM1, GL, TCIRG1, TIRC7, OC116, OPTB1); Muscular atrophy (VAPB, VAPC, ALS8, SMN1, SMA1, SMA2, SMA3, SMA4, BSCL2, SPG17, GARS, SMAD1, CMT2D, HEXB, IGHMBP2, SMUBP2, CATF1, SMARD1). |
|---|---|
| Neurological and neuronal diseases and disorders | ALS (SOD1, ALS2, STEX, FUS, TARDBP, VEGF (VEGF-a, VEGF-b, VEGF-c); Alzheimer disease (APP, AAA, CVAP, AD1, APOE, AD2, PSEN2, AD4, STM2, APBB2, FE65L1, NOS3, PLAU, URK, ACE, DCP1, ACE1, MPO, PACIP1, PAXIP1L, PTIP, A2M, BLMH, BMH, PSEN1, AD3); Autism (Mecp2, BZRAP1, MDGA2, Sema5A, Neurexin 1, GLO1, MECP2, RTT, PPMX, MRX16, MRX79, NLGN3, NLGN4, KIAA1260, AUTSX2); Fragile X Syndrome (FMR2, FXR1, FXR2, mGLUR5); Huntington's disease and disease like disorders (HD, IT15, PRNP, PRIP, JPH3, JP3, HDL2, TBP, SCA17); Parkinson disease |
| | (NR4A2, NURR1, NOT, TINUR, SNCAIP, TBP, SCA17, SNCA, NACP, PARK1, PARK4, DJ1, PARK7, LRRK2, PARK8, PINK1, PARK6, UCHL1, PARK5, SNCA, NACP, PARK1, PARK4, PRKN, PARK2, PDJ, DBH, NDUFV2); Rett syndrome (MECP2, RTT, PPMX, MRX16, MRX79, CDKL5, STK9, MECP2, RTT, PPMX, MRX16, MRX79, x-Synuclein, DJ-1); Schizophrenia (Neuregulin1 (Nrg1), Erb4 (receptor for Neuregulin), Complexin1 (Cplx1), Tph1 Tryptophan hydroxylase, Tph2, Tryptophan hydroxylase 2, Neurexin 1, GSK3, GSK3a, GSK3b, 5-HTT (Slc6a4), COMT, DRD (Drd1a), SLC6A3, DAOA, DTNBP1, Dao (Dao1)); Secretase Related Disorders (APH-1 (alpha and beta), Presenilin (Psen1), nicastrin, (Ncstn), PEN-2, Nos1, Parp1, Natl, Nat2); Trinucleotide Repeat Disorders (HTT (Huntington's Dx), SBMA/SMAX1/AR (Kennedy's Dx), FXN/X25 (Friedrich's Ataxia), ATX3 (Machado- Joseph's Dx), ATXN1 and ATXN2 (spinocerebellar ataxias), DMPK (myotonic dystrophy), Atrophin-1 and Atn1 (DRPLA Dx), CBP (Creb-BP - global instability), VLDLR (Alzheimer's), Atxn7, Atxn10). |
| Occular diseases and disorders | Age-related macular degeneration (Abcr, Ccl2, Cc2, cp (ceruloplasmin), Timp3, cathepsinD, Vldlr, Ccr2); Cataract (CRYAA, CRYA1, CRYBB2, CRYB2, PITX3, BFSP2, CP49, CP47, CRYAA, CRYA1, PAX6, AN2, MGDA, CRYBA1, CRYB1, CRYGC, CRYG3, CCL, LIM2, MP19, CRYGD, CRYG4, BFSP2, CP49, CP47, HSF4, CTM, HSF4, CTM, MIP, AQP0, CRYAB, CRYA2, CTPP2, CRYBB1, CRYGD, CRYG4, CRYBB2, CRYB2, CRYGC, CRYG3, CCL, CRYAA, CRYA1, GJA8, CX50, CAE1, GJA3, CX46, CZP3, CAE3, CCM1, CAM, KRIT1); Corneal clouding and dystrophy (APOA1, TGFBI, CSD2, CDGG1, CSD, BIGH3, CDG2, TACSTD2, TROP2, M1S1, VSX1, RINX, PPCD, PPD, KTCN, COL8A2, FECD, PPCD2, PIP5K3, CFD); Cornea plana congenital (KERA, CNA2); Glaucoma (MYOC, TIGR, GLC1A, JOAG, GPOA, OPTN, GLC1E, FIP2, HYPL, NRP, CYP1B1, GLC3A, OPA1, NTG, NPG, CYP1B1, GLC3A); Leber congenital amaurosis (CRB1, RP12, CRX, CORD2, CRD, RPGRIP1, LCA6, CORD9, RPE65, RP20, AIPL1, LCA4, GUCY2D, GUC2D, LCA1, CORD6, RDH12, LCA3); Macular dystrophy (ELOVL4, ADMD, STGD2, STGD3, RDS, RP7, PRPH2, PRPH, AVMD, AOFMD, VMD2). |

**Table C:**

| CELLULAR FUNCTION | GENES |
|---|---|
| PI3K/AKT Signaling | PRKCE; ITGAM; ITGA5; IRAKI; PRKAA2; EIF2AK2; |

(continued)

| CELLULAR FUNCTION | GENES |
|---|---|
| | PTEN; EIF4E; PRKCZ; GRK6; MAPK1; TSC1; PLK1; |
| | AKT2; IKBKB; PIK3CA; CDK8; CDKN1B; NFKB2; BCL2; |
| | PIK3CB; PPP2R1A; MAPK8; BCL2L1; MAPK3; TSC2; |
| | ITGA1; KRAS; EIF4EBP1; RELA; PRKCD; NOS3; |
| | PRKAA1; MAPK9; CDK2; PPP2CA; PIM1; ITGB7; |
| | YWHAZ; ILK; TP53; RAF1; IKBKG; RELB; DYRK1A; |
| | CDKN1A; ITGB1; MAP2K2; JAK1; AKT1; JAK2; PIK3R1; |
| | CHUK; PDPK1; PPP2R5C; CTNNB1; MAP2K1; NFKB1; |
| | PAK3; ITGB3; CCND1; GSK3A; FRAP1; SFN; ITGA2; |
| | TTK; CSNK1A1; BRAF; GSK3B; AKT3; FOXO1; SGK; |
| | HSP90AA1; RPS6KB1 |
| ERK/MAPK Signaling | PRKCE; ITGAM; ITGA5; HSPB1; IRAK1; PRKAA2; |
| | EIF2AK2; RAC1; RAP1A; TLN1; EIF4E; ELK1; GRK6; |
| | MAPK1; RAC2; PLK1; AKT2; PIK3CA; CDK8; CREB1; |
| | PRKCI; PTK2; FOS; RPS6KA4; PIK3CB; PPP2R1A; |
| | PIK3C3; MAPK8; MAPK3; ITGA1; ETS1; KRAS; MYCN; |
| | EIF4EBP1; PPARG; PRKCD; PRKAA1; MAPK9; SRC; |
| | CDK2; PPP2CA; PIM1; PIK3C2A; ITGB7; YWHAZ; |
| | PPP1CC; KSR1; PXN; RAF1; FYN; DYRK1A; ITGB1; |
| | MAP2K2; PAK4; PIK3R1; STAT3; PPP2R5C; MAP2K1; |
| | PAK3; ITGB3; ESR1; ITGA2; MYC; TTK; CSNK1A1; |
| | CRKL; BRAF; ATF4; PRKCA; SRF; STAT1; SGK |
| Glucocorticoid Receptor Signaling | RAC1; TAF4B; EP300; SMAD2; TRAF6; PCAF; ELK1; |
| | MAPK1; SMAD3; AKT2; IKBKB; NCOR2; UBE2I; |
| | PIK3CA; CREB1; FOS; HSPA5; NFKB2; BCL2; |
| | MAP3K14; STAT5B; PIK3CB; PIK3C3; MAPK8; BCL2L1; |
| | MAPK3; TSC22D3; MAPK10; NRIP1; KRAS; MAPK13; |
| | RELA; STAT5A; MAPK9; NOS2A; PBX1; NR3C1; |
| | PIK3C2A; CDKN1C; TRAF2; SERPINE1; NCOA3; |
| | MAPK14; TNF; RAF1; IKBKG; MAP3K7; CREBBP; |
| | CDKN1A; MAP2K2; JAK1; IL8; NCOA2; AKT1; JAK2; |
| | PIK3R1; CHUK; STAT3; MAP2K1; NFKB1; TGFBR1; |
| | ESR1; SMAD4; CEBPB; JUN; AR; AKT3; CCL2; MMP1; |
| | STAT1; IL6; HSP90AA1 |
| Axonal Guidance Signaling | PRKCE; ITGAM; ROCK1; ITGA5; CXCR4; ADAM12; |
| | IGF1; RAC1; RAP1A; EIF4E; PRKCZ; NRP1; NTRK2; |
| | ARHGEF7; SMO; ROCK2; MAPK1; PGF; RAC2; |
| | PTPN11; GNAS; AKT2; PIK3CA; ERBB2; PRKCI; PTK2; |

(continued)

| CELLULAR FUNCTION | GENES |
|---|---|
|  | CFL1; GNAQ; PIK3CB; CXCL12; PIK3C3; WNT11; |
|  | PRKD1; GNB2L1; ABL1; MAPK3; ITGA1; KRAS; RHOA; |
|  | PRKCD; PIK3C2A; ITGB7; GLI2; PXN; VASP; RAF1; |
|  | FYN; ITGB1; MAP2K2; PAK4; ADAM17; AKT1; PIK3R1; |
|  | GLI1; WNT5A; ADAM10; MAP2K1; PAK3; ITGB3; |
|  | CDC42; VEGFA; ITGA2; EPHA8; CRKL; RND1; GSK3B; |
|  | AKT3; PRKCA |
| Ephrin Receptor Signaling | PRKCE; ITGAM; ROCK1; ITGA5; CXCR4; IRAKI; |
|  | PRKAA2; EIF2AK2; RAC1; RAP1A; GRK6; ROCK2; |
|  | MAPK1; PGF; RAC2; PTPN11; GNAS; PLK1; AKT2; |
|  | DOK1; CDK8; CREB1; PTK2; CFL1; GNAQ; MAP3K14; |
|  | CXCL12; MAPK8; GNB2L1; ABL1; MAPK3; ITGA1; |
|  | KRAS; RHOA; PRKCD; PRKAA1; MAPK9; SRC; CDK2; |
|  | PIM1; ITGB7; PXN; RAF1; FYN; DYRK1A; ITGB1; |
|  | MAP2K2; PAK4; AKT1; JAK2; STAT3; ADAM10; |
|  | MAP2K1; PAK3; ITGB3; CDC42; VEGFA; ITGA2; |
|  | EPHA8; TTK; CSNK1A1; CRKL; BRAF; PTPN13; ATF4; |
|  | AKT3; SGK |
| Actin Cytoskeleton | ACTN4; PRKCE; ITGAM; ROCK1; ITGA5; IRAKI; |
| Signaling | PRKAA2; EIF2AK2; RAC1; INS; ARHGEF7; GRK6; |
|  | ROCK2; MAPK1; RAC2; PLK1; AKT2; PIK3CA; CDK8; |
|  | PTK2; CFL1; PIK3CB; MYH9; DIAPH1; PIK3C3; MAPK8; |
|  | F2R; MAPK3; SLC9A1; ITGA1; KRAS; RHOA; PRKCD; |
|  | PRKAA1; MAPK9; CDK2; PIM1; PIK3C2A; ITGB7; |
|  | PPP1CC; PXN; VIL2; RAF1; GSN; DYRK1A; ITGB1; |
|  | MAP2K2; PAK4; PIP5K1A; PIK3R1; MAP2K1; PAK3; |
|  | ITGB3; CDC42; APC; ITGA2; TTK; CSNK1A1; CRKL; |
|  | BRAF; VAV3; SGK |
| Huntington's Disease | PRKCE; IGF1; EP300; RCOR1; PRKCZ; HDAC4; TGM2; |
| Signaling | MAPK1; CAPNS1; AKT2; EGFR; NCOR2; SP1; CAPN2; |
|  | PIK3CA; HDAC5; CREB1; PRKCI; HSPA5; REST; |
|  | GNAQ; PIK3CB; PIK3C3; MAPK8; IGF1R; PRKD1; |
|  | GNB2L1; BCL2L1; CAPN1; MAPK3; CASP8; HDAC2; |
|  | HDAC7A; PRKCD; HDAC11; MAPK9; HDAC9; PIK3C2A; |
|  | HDAC3; TP53; CASP9; CREBBP; AKT1; PIK3R1; |
|  | PDPK1; CASP1; APAF1; FRAP1; CASP2; JUN; BAX; |
|  | ATF4; AKT3; PRKCA; CLTC; SGK; HDAC6; CASP3 |
| Apoptosis Signaling | PRKCE; ROCK1; BID; IRAK1; PRKAA2; EIF2AK2; BAK1; |

(continued)

| CELLULAR FUNCTION | GENES |
|---|---|
| | BIRC4; GRK6; MAPK1; CAPNS1; PLK1; AKT2; IKBKB; |
| | CAPN2; CDK8; FAS; NFKB2; BCL2; MAP3K14; MAPK8; |
| | BCL2L1; CAPN1; MAPK3; CASP8; KRAS; RELA; |
| | PRKCD; PRKAA1; MAPK9; CDK2; PIM1; TP53; TNF; |
| | RAF1; IKBKG; RELB; CASP9; DYRK1A; MAP2K2; |
| | CHUK; APAF1; MAP2K1; NFKB1; PAK3; LMNA; CASP2; |
| | BIRC2; TTK; CSNK1A1; BRAF; BAX; PRKCA; SGK; |
| | CASP3; BIRC3; PARP1 |
| B Cell Receptor Signaling | RAC1; PTEN; LYN; ELK1; MAPK1; RAC2; PTPN11; |
| | AKT2; IKBKB; PIK3CA; CREB1; SYK; NFKB2; CAMK2A; |
| | MAP3K14; PIK3CB; PIK3C3; MAPK8; BCL2L1; ABL1; |
| | MAPK3; ETS1; KRAS; MAPK13; RELA; PTPN6; MAPK9; |
| | EGR1; PIK3C2A; BTK; MAPK14; RAF1; IKBKG; RELB; |
| | MAP3K7; MAP2K2; AKT1; PIK3R1; CHUK; MAP2K1; |
| | NFKB1; CDC42; GSK3A; FRAP1; BCL6; BCL10; JUN; |
| | GSK3B; ATF4; AKT3; VAV3; RPS6KB1 |
| Leukocyte Extravasation | ACTN4; CD44; PRKCE; ITGAM; ROCK1; CXCR4; CYBA; |
| Signaling | RAC1; RAP1A; PRKCZ; ROCK2; RAC2; PTPN11; |
| | MMP14; PIK3CA; PRKCI; PTK2; PIK3CB; CXCL12; |
| | PIK3C3; MAPK8; PRKD1; ABL1; MAPK10; CYBB; |
| | MAPK13; RHOA; PRKCD; MAPK9; SRC; PIK3C2A; BTK; |
| | MAPK14; NOX1; PXN; VIL2; VASP; ITGB1; MAP2K2; |
| | CTNND1; PIK3R1; CTNNB1; CLDN1; CDC42; F11R; ITK; |
| | CRKL; VAV3; CTTN; PRKCA; MMP1; MMP9 |
| Integrin Signaling | ACTN4; ITGAM; ROCK1; ITGA5; RAC1; PTEN; RAP1A; |
| | TLN1; ARHGEF7; MAPK1; RAC2; CAPNS1; AKT2; |
| | CAPN2; PIK3CA; PTK2; PIK3CB; PIK3C3; MAPK8; |
| | CAV1; CAPN1; ABL1; MAPK3; ITGA1; KRAS; RHOA; |
| | SRC; PIK3C2A; ITGB7; PPP1CC; ILK; PXN; VASP; |
| | RAF1; FYN; ITGB1; MAP2K2; PAK4; AKT1; PIK3R1; |
| | TNK2; MAP2K1; PAK3; ITGB3; CDC42; RND3; ITGA2; |
| | CRKL; BRAF; GSK3B; AKT3 |
| Acute Phase Response | IRAK1; SOD2; MYD88; TRAF6; ELK1; MAPK1; PTPN11; |
| Signaling | AKT2; IKBKB; PIK3CA; FOS; NFKB2; MAP3K14; |
| | PIK3CB; MAPK8; RIPK1; MAPK3; IL6ST; KRAS; |
| | MAPK13; IL6R; RELA; SOCS1; MAPK9; FTL; NR3C1; |
| | TRAF2; SERPINE1; MAPK14; TNF; RAF1; PDK1; |
| | IKBKG; RELB; MAP3K7; MAP2K2; AKT1; JAK2; PIK3R1; |

(continued)

| CELLULAR FUNCTION | GENES |
|---|---|
| | CHUK; STAT3; MAP2K1; NFKB1; FRAP1; CEBPB; JUN; |
| | AKT3; IL1R1; IL6 |
| PTEN Signaling | ITGAM; ITGA5; RAC1; PTEN; PRKCZ; BCL2L11; |
| | MAPK1; RAC2; AKT2; EGFR; IKBKB; CBL; PIK3CA; |
| | CDKN1B; PTK2; NFKB2; BCL2; PIK3CB; BCL2L1; |
| | MAPK3; ITGA1; KRAS; ITGB7; ILK; PDGFRB; INSR; |
| | RAF1; IKBKG; CASP9; CDKN1A; ITGB1; MAP2K2; |
| | AKT1; PIK3R1; CHUK; PDGFRA; PDPK1; MAP2K1; |
| | NFKB1; ITGB3; CDC42; CCND1; GSK3A; ITGA2; |
| | GSK3B; AKT3; FOXO1; CASP3; RPS6KB1 |
| p53 Signaling | PTEN; EP300; BBC3; PCAF; FASN; BRCA1; GADD45A; |
| | BIRC5; AKT2; PIK3CA; BCL2; |
| | PIK3CB; PIK3C3; MAPK8; THBS1; ATR; BCL2L1; E2F1; |
| | PMAIP1; CHEK2; TNFRSF10B; TP73; RB1; HDAC9; |
| | CDK2; PIK3C2A; MAPK14; TP53; LRDD; CDKN1A; |
| | HIPK2; AKT1; PIK3R1; RRM2B; APAF1; CTNNB1; |
| | SIRT1; CCND1; PRKDC; ATM; SFN; CDKN2A; JUN; |
| | SNAI2; GSK3B; BAX; AKT3 |
| Aryl Hydrocarbon Receptor | HSPB1; EP300; FASN; TGM2; RXRA; MAPK1; NQO1; |
| Signaling | NCOR2; SP1; ARNT; CDKN1B; FOS; CHEK1; |
| | SMARCA4; NFKB2; MAPK8; ALDH1A1; ATR; E2F1; |
| | MAPK3; NRIP1; CHEK2; RELA; TP73; GSTP1; RB1; |
| | SRC; CDK2; AHR; NFE2L2; NCOA3; TP53; TNF; |
| | CDKN1A; NCOA2; APAF1; NFKB1; CCND1; ATM; ESR1; |
| | CDKN2A; MYC; JUN; ESR2; BAX; IL6; CYP1B1; |
| | HSP90AA1 |
| Xenobiotic Metabolism | PRKCE; EP300; PRKCZ; RXRA; MAPK1; NQO1; |
| Signaling | NCOR2; PIK3CA; ARNT; PRKCI; NFKB2; CAMK2A; |
| | PIK3CB; PPP2R1A; PIK3C3; MAPK8; PRKD1; |
| | ALDH1A1; MAPK3; NRIP1; KRAS; MAPK13; PRKCD; |
| | GSTP1; MAPK9; NOS2A; ABCB1; AHR; PPP2CA; FTL; |
| | NFE2L2; PIK3C2A; PPARGC1A; MAPK14; TNF; RAF1; |
| | CREBBP; MAP2K2; PIK3R1; PPP2R5C; MAP2K1; |
| | NFKB1; KEAP1; PRKCA; EIF2AK3; IL6; CYP1B1; |
| | HSP90AA1 |
| SAPK/JNK Signaling | PRKCE; IRAKI; PRKAA2; EIF2AK2; RAC1; ELK1; |
| | GRK6; MAPK1; GADD45A; RAC2; PLK1; AKT2; PIK3CA; |
| | FADD; CDK8; PIK3CB; PIK3C3; MAPK8; RIPK1; |

(continued)

| CELLULAR FUNCTION | GENES |
|---|---|
| | GNB2L1; IRS1; MAPK3; MAPK10; DAXX; KRAS; |
| | PRKCD; PRKAA1; MAPK9; CDK2; PIM1; PIK3C2A; |
| | TRAF2; TP53; LCK; MAP3K7; DYRK1A; MAP2K2; |
| | PIK3R1; MAP2K1; PAK3; CDC42; JUN; TTK; CSNK1A1; |
| | CRKL; BRAF; SGK |
| PPAr/RXR Signaling | PRKAA2; EP300; INS; SMAD2; TRAF6; PPARA; FASN; |
| | RXRA; MAPK1; SMAD3; GNAS; IKBKB; NCOR2; |
| | ABCA1; GNAQ; NFKB2; MAP3K14; STAT5B; MAPK8; |
| | IRS1; MAPK3; KRAS; RELA; PRKAA1; PPARGC1A; |
| | NCOA3; MAPK14; INSR; RAF1; IKBKG; RELB; MAP3K7; |
| | CREBBP; MAP2K2; JAK2; CHUK; MAP2K1; NFKB1; |
| | TGFBR1; SMAD4; JUN; IL1R1; PRKCA; IL6; HSP90AA1; |
| | ADIPOQ |
| NF-KB Signaling | IRAK1; EIF2AK2; EP300; INS; MYD88; PRKCZ; TRAF6; |
| | TBK1; AKT2; EGFR; IKBKB; PIK3CA; BTRC; NFKB2; |
| | MAP3K14; PIK3CB; PIK3C3; MAPK8; RIPK1; HDAC2; |
| | KRAS; RELA; PIK3C2A; TRAF2; TLR4; PDGFRB; TNF; |
| | INSR; LCK; IKBKG; RELB; MAP3K7; CREBBP; AKT1; |
| | PIK3R1; CHUK; PDGFRA; NFKB1; TLR2; BCL10; |
| | GSK3B; AKT3; TNFAIP3; IL1R1 |
| Neuregulin Signaling | ERBB4; PRKCE; ITGAM; ITGA5; PTEN; PRKCZ; ELK1; |
| | MAPK1; PTPN11; AKT2; EGFR; ERBB2; PRKCI; |
| | CDKN1B; STAT5B; PRKD1; MAPK3; ITGA1; KRAS; |
| | PRKCD; STAT5A; SRC; ITGB7; RAF1; ITGB1; MAP2K2; |
| | ADAM17; AKT1; PIK3R1; PDPK1; MAP2K1; ITGB3; |
| | EREG; FRAP1; PSEN1; ITGA2; MYC; NRG1; CRKL; |
| | AKT3; PRKCA; HSP90AA1; RPS6KB1 |
| Wnt & Beta catenin | CD44; EP300; LRP6; DVL3; CSNK1E; GJA1; SMO; |
| Signaling | AKT2; PIN1; CDH1; BTRC; GNAQ; MARK2; PPP2R1A; |
| | WNT11; SRC; DKK1; PPP2CA; SOX6; SFRP2; ILK; |
| | LEF1; SOX9; TP53; MAP3K7; CREBBP; TCF7L2; AKT1; |
| | PPP2R5C; WNT5A; LRP5; CTNNB1; TGFBR1; CCND1; |
| | GSK3A; DVL1; APC; CDKN2A; MYC; CSNK1A1; GSK3B; |
| | AKT3; SOX2 |
| Insulin Receptor Signaling | PTEN; INS; EIF4E; PTPN1; PRKCZ; MAPK1; TSC1; |
| | PTPN11; AKT2; CBL; PIK3CA; PRKCI; PIK3CB; PIK3C3; |
| | MAPK8; IRS1; MAPK3; TSC2; KRAS; EIF4EBP1; |
| | SLC2A4; PIK3C2A; PPP1CC; INSR; RAF1; FYN; |

(continued)

| CELLULAR FUNCTION | GENES |
|---|---|
| | MAP2K2; JAK1; AKT1; JAK2; PIK3R1; PDPK1; MAP2K1; |
| | GSK3A; FRAP1; CRKL; GSK3B; AKT3; FOXO1; SGK; |
| | RPS6KB1 |
| IL-6 Signaling | HSPB1; TRAF6; MAPKAPK2; ELK1; MAPK1; PTPN11; |
| | IKBKB; FOS; NFKB2; MAP3K14; MAPK8; MAPK3; |
| | MAPK10; IL6ST; KRAS; MAPK13; IL6R; RELA; SOCS1; |
| | MAPK9; ABCB1; TRAF2; MAPK14; TNF; RAF1; IKBKG; |
| | RELB; MAP3K7; MAP2K2; IL8; JAK2; CHUK; STAT3; |
| | MAP2K1; NFKB1; CEBPB; JUN; IL1R1; SRF; IL6 |
| Hepatic Cholestasis | PRKCE; IRAKI; INS; MYD88; PRKCZ; TRAF6; PPARA; |
| | RXRA; IKBKB; PRKCI; NFKB2; MAP3K14; MAPK8; |
| | PRKD1; MAPK10; RELA; PRKCD; MAPK9; ABCB1; |
| | TRAF2; TLR4; TNF; INSR; IKBKG; RELB; MAP3K7; IL8; |
| | CHUK; NR1H2; TJP2; NFKB1; ESR1; SREBF1; FGFR4; |
| | JUN; IL1R1; PRKCA; IL6 |
| IGF-1 Signaling | IGF1; PRKCZ; ELK1; MAPK1; PTPN11; NEDD4; AKT2; |
| | PIK3CA; PRKCI; PTK2; FOS; PIK3CB; PIK3C3; MAPK8; |
| | IGF1R; IRS1; MAPK3; IGFBP7; KRAS; PIK3C2A; |
| | YWHAZ; PXN; RAF1; CASP9; MAP2K2; AKT1; PIK3R1; |
| | PDPK1; MAP2K1; IGFBP2; SFN; JUN; CYR61; AKT3; |
| | FOXO1; SRF; CTGF; RPS6KB1 |
| NRF2-mediated Oxidative | PRKCE; EP300; SOD2; PRKCZ; MAPK1; SQSTM1; |
| Stress Response | NQO1; PIK3CA; PRKCI; FOS; PIK3CB; PIK3C3; MAPK8; |
| | PRKD1; MAPK3; KRAS; PRKCD; GSTP1; MAPK9; FTL; |
| | NFE2L2; PIK3C2A; MAPK14; RAF1; MAP3K7; CREBBP; |
| | MAP2K2; AKT1; PIK3R1; MAP2K1; PPIB; JUN; KEAP1; |
| | GSK3B; ATF4; PRKCA; EIF2AK3; HSP90AA1 |
| Hepatic Fibrosis/Hepatic | EDN1; IGF1; KDR; FLT1; SMAD2; FGFR1; MET; PGF; |
| Stellate Cell Activation | SMAD3; EGFR; FAS; CSF1; NFKB2; BCL2; MYH9; |
| | IGF1R; IL6R; RELA; TLR4; PDGFRB; TNF; RELB; IL8; |
| | PDGFRA; NFKB1; TGFBR1; SMAD4; VEGFA; BAX; |
| | IL1R1; CCL2; HGF; MMP1; STAT1; IL6; CTGF; MMP9 |
| PPAR Signaling | EP300; INS; TRAF6; PPARA; RXRA; MAPK1; IKBKB; |
| | NCOR2; FOS; NFKB2; MAP3K14; STAT5B; MAPK3; |
| | NRIP1; KRAS; PPARG; RELA; STAT5A; TRAF2; |
| | PPARGC1A; PDGFRB; TNF; INSR; RAF1; IKBKG; |
| | RELB; MAP3K7; CREBBP; MAP2K2; CHUK; PDGFRA; |
| | MAP2K1; NFKB1; JUN; IL1R1; HSP90AA1 |

(continued)

| CELLULAR FUNCTION | GENES |
|---|---|
| Fc Epsilon RI Signaling | PRKCE; RAC1; PRKCZ; LYN; MAPK1; RAC2; PTPN11; |
| | AKT2; PIK3CA; SYK; PRKCI; PIK3CB; PIK3C3; MAPK8; |
| | PRKD1; MAPK3; MAPK10; KRAS; MAPK13; PRKCD; |
| | MAPK9; PIK3C2A; BTK; MAPK14; TNF; RAF1; FYN; |
| | MAP2K2; AKT1; PIK3R1; PDPK1; MAP2K1; AKT3; |
| | VAV3; PRKCA |
| G-Protein Coupled | PRKCE; RAP1A; RGS16; MAPK1; GNAS; AKT2; IKBKB; |
| Receptor Signaling | PIK3CA; CREB1; GNAQ; NFKB2; CAMK2A; PIK3CB; |
| | PIK3C3; MAPK3; KRAS; RELA; SRC; PIK3C2A; RAF1; |
| | IKBKG; RELB; FYN; MAP2K2; AKT1; PIK3R1; CHUK; |
| | PDPK1; STAT3; MAP2K1; NFKB1; BRAF; ATF4; AKT3; |
| | PRKCA |
| Inositol Phosphate | PRKCE; IRAKI; PRKAA2; EIF2AK2; PTEN; GRK6; |
| Metabolism | MAPK1; PLK1; AKT2; PIK3CA; CDK8; PIK3CB; PIK3C3; |
| | MAPK8; MAPK3; PRKCD; PRKAA1; MAPK9; CDK2; |
| | PIM1; PIK3C2A; DYRK1A; MAP2K2; PIP5K1A; PIK3R1; |
| | MAP2K1; PAK3; ATM; TTK; CSNK1A1; BRAF; SGK |
| PDGF Signaling | EIF2AK2; ELK1; ABL2; MAPK1; PIK3CA; FOS; PIK3CB; |
| | PIK3C3; MAPK8; CAV1; ABL1; MAPK3; KRAS; SRC; |
| | PIK3C2A; PDGFRB; RAF1; MAP2K2; JAK1; JAK2; |
| | PIK3R1; PDGFRA; STAT3; SPHK1; MAP2K1; MYC; |
| | JUN; CRKL; PRKCA; SRF; STAT1; SPHK2 |
| VEGF Signaling | ACTN4; ROCK1; KDR; FLT1; ROCK2; MAPK1; PGF; |
| | AKT2; PIK3CA; ARNT; PTK2; BCL2; PIK3CB; PIK3C3; |
| | BCL2L1; MAPK3; KRAS; HIF1A; NOS3; PIK3C2A; PXN; |
| | RAF1; MAP2K2; ELAVL1; AKT1; PIK3R1; MAP2K1; SFN; |
| | VEGFA; AKT3; FOXO1; PRKCA |
| Natural Killer Cell Signaling | PRKCE; RAC1; PRKCZ; MAPK1; RAC2; PTPN11; |
| | KIR2DL3; AKT2; PIK3CA; SYK; PRKCI; PIK3CB; |
| | PIK3C3; PRKD1; MAPK3; KRAS; PRKCD; PTPN6; |
| | PIK3C2A; LCK; RAF1; FYN; MAP2K2; PAK4; AKT1; |
| | PIK3R1; MAP2K1; PAK3; AKT3; VAV3; PRKCA |
| Cell Cycle: G1/S | HDAC4; SMAD3; SUV39H1; HDAC5; CDKN1B; BTRC; |
| Checkpoint Regulation | ATR; ABL1; E2F1; HDAC2; HDAC7A; RB1; HDAC11; |
| | HDAC9; CDK2; E2F2; HDAC3; TP53; CDKN1A; CCND1; |
| | E2F4; ATM; RBL2; SMAD4; CDKN2A; MYC; NRG1; |
| | GSK3B; RBL1; HDAC6 |
| T Cell Receptor Signaling | RAC1; ELK1; MAPK1; IKBKB; CBL; PIK3CA; FOS; |

(continued)

| CELLULAR FUNCTION | GENES |
|---|---|
| | NFKB2; PIK3CB; PIK3C3; MAPK8; MAPK3; KRAS; |
| | RELA; PIK3C2A; BTK; LCK; RAF1; IKBKG; RELB; FYN; |
| | MAP2K2; PIK3R1; CHUK; MAP2K1; NFKB1; ITK; BCL10; |
| | JUN; VAV3 |
| Death Receptor Signaling | CRADD; HSPB1; BID; BIRC4; TBK1; IKBKB; FADD; |
| | FAS; NFKB2; BCL2; MAP3K14; MAPK8; RIPK1; CASP8; |
| | DAXX; RELA; IKBKG; RELB; |
| | CASP9; CHUK; APAF1; NFKB1; CASP2; BIRC2; CASP3; |
| | BIRC3 |
| FGF Signaling | RAC1; FGFR1; MET; MAPKAPK2; MAPK1; PTPN11; |
| | AKT2; PIK3CA; CREB1; PIK3CB; PIK3C3; MAPK8; |
| | MAPK3; MAPK13; PTPN6; PIK3C2A; MAPK14; RAF1; |
| | AKT1; PIK3R1; STAT3; MAP2K1; FGFR4; CRKL; ATF4; |
| | AKT3; PRKCA; HGF |
| GM-CSF Signaling | LYN; ELK1; MAPK1; PTPN11; AKT2; PIK3CA; CAMK2A; |
| | STAT5B; PIK3CB; PIK3C3; GNB2L1; BCL2L1; MAPK3; |
| | ETS1; KRAS; RUNX1; PIM1; PIK3C2A; RAF1; MAP2K2; |
| | AKT1; JAK2; PIK3R1; STAT3; MAP2K1; CCND1; AKT3; |
| | STAT1 |
| Amyotrophic Lateral | BID; IGF1; RAC1; BIRC4; PGF; CAPNS1; CAPN2; |
| Sclerosis Signaling | PIK3CA; BCL2; PIK3CB; PIK3C3; BCL2L1; CAPN1; |
| | PIK3C2A; TP53; CASP9; PIK3R1; RAB5A; CASP1; |
| | APAF1; VEGFA; BIRC2; BAX; AKT3; CASP3; BIRC3 |
| JAK/Stat Signaling | PTPN1; MAPK1; PTPN11; AKT2; PIK3CA; STAT5B; |
| | PIK3CB; PIK3C3; MAPK3; KRAS; SOCS1; STAT5A; |
| | PTPN6; PIK3C2A; RAF1; CDKN1A; MAP2K2; JAK1; |
| | AKT1; JAK2; PIK3R1; STAT3; MAP2K1; FRAP1; AKT3; |
| | STAT1 |
| Nicotinate and Nicotinamide | PRKCE; IRAKI; PRKAA2; EIF2AK2; GRK6; MAPK1; |
| Metabolism | PLK1; AKT2; CDK8; MAPK8; MAPK3; PRKCD; PRKAA1; |
| | PBEF1; MAPK9; CDK2; PIM1; DYRK1A; MAP2K2; |
| | MAP2K1; PAK3; NT5E; TTK; CSNK1A1; BRAF; SGK |
| Chemokine Signaling | CXCR4; ROCK2; MAPK1; PTK2; FOS; CFL1; GNAQ; |
| | CAMK2A; CXCL12; MAPK8; MAPK3; KRAS; MAPK13; |
| | RHOA; CCR3; SRC; PPP1CC; MAPK14; NOX1; RAF1; |
| | MAP2K2; MAP2K1; JUN; CCL2; PRKCA |
| IL-2 Signaling | ELK1; MAPK1; PTPN11; AKT2; PIK3CA; SYK; FOS; |
| | STAT5B; PIK3CB; PIK3C3; MAPK8; MAPK3; KRAS; |

(continued)

| CELLULAR FUNCTION | GENES |
|---|---|
| | SOCS1; STAT5A; PIK3C2A; LCK; RAF1; MAP2K2; |
| | JAK1; AKT1; PIK3R1; MAP2K1; JUN; AKT3 |
| Synaptic Long Term | PRKCE; IGF1; PRKCZ; PRDX6; LYN; MAPK1; GNAS; |
| Depression | PRKCI; GNAQ; PPP2R1A; IGF1R; PRKD1; MAPK3; |
| | KRAS; GRN; PRKCD; NOS3; NOS2A; PPP2CA; |
| | YWHAZ; RAF1; MAP2K2; PPP2R5C; MAP2K1; PRKCA |
| Estrogen Receptor | TAF4B; EP300; CARM1; PCAF; MAPK1; NCOR2; |
| Signaling | SMARCA4; MAPK3; NRIP1; KRAS; SRC; NR3C1; |
| | HDAC3; PPARGC1A; RBM9; NCOA3; RAF1; CREBBP; |
| | MAP2K2; NCOA2; MAP2K1; PRKDC; ESR1; ESR2 |
| Protein Ubiquitination | TRAF6; SMURF1; BIRC4; BRCA1; UCHL1; NEDD4; |
| Pathway | CBL; UBE2I; BTRC; HSPA5; USP7; USP10; FBXW7; |
| | USP9X; STUB 1; USP22; B2M; BIRC2; PARK2; USP8; |
| | USP1; VHL; HSP90AA1; BIRC3 |
| IL-10 Signaling | TRAF6; CCR1; ELK1; IKBKB; SP1; FOS; NFKB2; |
| | MAP3K14; MAPK8; MAPK13; RELA; MAPK14; TNF; |
| | IKBKG; RELB; MAP3K7; JAK1; CHUK; STAT3; NFKB1; |
| | JUN; IL1R1; IL6 |
| VDR/RXR Activation | PRKCE; EP300; PRKCZ; RXRA; GADD45A; HES1; |
| | NCOR2; SP1; PRKCI; CDKN1B; PRKD1; PRKCD; |
| | RUNX2; KLF4; YY1; NCOA3; CDKN1A; NCOA2; SPP1; |
| | LRP5; CEBPB; FOXO1; PRKCA |
| TGF-beta Signaling | EP300; SMAD2; SMURF1; MAPK1; SMAD3; SMAD1; |
| | FOS; MAPK8; MAPK3; KRAS; MAPK9; RUNX2; |
| | SERPINE1; RAF1; MAP3K7; CREBBP; MAP2K2; |
| | MAP2K1; TGFBR1; SMAD4; JUN; SMAD5 |
| Toll-like Receptor Signaling | IRAK1; EIF2AK2; MYD88; TRAF6; PPARA; ELK1; |
| | IKBKB; FOS; NFKB2; MAP3K14; MAPK8; MAPK13; |
| | RELA; TLR4; MAPK14; IKBKG; RELB; MAP3K7; CHUK; |
| | NFKB1; TLR2; JUN |
| p38 MAPK Signaling | HSPB1; IRAK1; TRAF6; MAPKAPK2; ELK1; FADD; FAS; |
| | CREB1; DDIT3; RPS6KA4; DAXX; MAPK13; TRAF2; |
| | MAPK14; TNF; MAP3K7; TGFBR1; MYC; ATF4; IL1R1; |
| | SRF; STAT1 |
| Neurotrophin/TRK Signaling | NTRK2; MAPK1; PTPN11; PIK3CA; CREB1; FOS; |
| | PIK3CB; PIK3C3; MAPK8; MAPK3; KRAS; PIK3C2A; |
| | RAF1; MAP2K2; AKT1; PIK3R1; PDPK1; MAP2K1; |
| | CDC42; JUN; ATF4 |

(continued)

| CELLULAR FUNCTION | GENES |
|---|---|
| FXR/RXR Activation | INS; PPARA; FASN; RXRA; AKT2; SDC1; MAPK8; |
| | APOB; MAPK10; PPARG; MTTP; MAPK9; PPARGC1A; |
| | TNF; CREBBP; AKT1; SREBF1; FGFR4; AKT3; FOXO1 |
| Synaptic Long Term | PRKCE; RAP1A; EP300; PRKCZ; MAPK1; CREB1; |
| Potentiation | PRKCI; GNAQ; CAMK2A; PRKD1; MAPK3; KRAS; |
| | PRKCD; PPP1CC; RAF1; CREBBP; MAP2K2; MAP2K1; |
| | ATF4; PRKCA |
| Calcium Signaling | RAP1A; EP300; HDAC4; MAPK1; HDAC5; CREB1; |
| | CAMK2A; MYH9; MAPK3; HDAC2; HDAC7A; HDAC11; |
| | HDAC9; HDAC3; CREBBP; CALR; CAMKK2; ATF4; |
| | HDAC6 |
| EGF Signaling | ELK1; MAPK1; EGFR; PIK3CA; FOS; PIK3CB; PIK3C3; |
| | MAPK8; MAPK3; PIK3C2A; RAF1; JAK1; PIK3R1; |
| | STAT3; MAP2K1; JUN; PRKCA; SRF; STAT1 |
| Hypoxia Signaling in the | EDN1; PTEN; EP300; NQO1; UBE2I; CREB1; ARNT; |
| Cardiovascular System | HIF1A; SLC2A4; NOS3; TP53; LDHA; AKT1; ATM; |
| | VEGFA; JUN; ATF4; VHL; HSP90AA1 |
| LPS/IL-1 Mediated Inhibition | IRAK1; MYD88; TRAF6; PPARA; RXRA; ABCA1; |
| of RXR Function | MAPK8; ALDH1A1; GSTP1; MAPK9; ABCB1; TRAF2; |
| | TLR4; TNF; MAP3K7; NR1H2; SREBF1; JUN; IL1R1 |
| LXR/RXR Activation | FASN; RXRA; NCOR2; ABCA1; NFKB2; IRF3; RELA; |
| | NOS2A; TLR4; TNF; RELB; LDLR; NR1H2; NFKB1; |
| | SREBF1; IL1R1; CCL2; IL6; MMP9 |
| Amyloid Processing | PRKCE; CSNK1E; MAPK1; CAPNS1; AKT2; CAPN2; |
| | CAPN1; MAPK3; MAPK13; MAPT; MAPK14; AKT1; |
| | PSEN1; CSNK1A1; GSK3B; AKT3; APP |
| IL-4 Signaling | AKT2; PIK3CA; PIK3CB; PIK3C3; IRS1; KRAS; SOCS1; |
| | PTPN6; NR3C1; PIK3C2A; JAK1; AKT1; JAK2; PIK3R1; |
| | FRAP1; AKT3; RPS6KB1 |
| Cell Cycle: G2/M DNA | EP300; PCAF; BRCA1; GADD45A; PLK1; BTRC; |
| Damage Checkpoint | CHEK1; ATR; CHEK2; YWHAZ; TP53; CDKN1A; |
| Regulation | PRKDC; ATM; SFN; CDKN2A |
| Nitric Oxide Signaling in the | KDR; FLT1; PGF; AKT2; PIK3CA; PIK3CB; PIK3C3; |
| Cardiovascular System | CAV1; PRKCD; NOS3; PIK3C2A; AKT1; PIK3R1; |
| | VEGFA; AKT3; HSP90AA1 |
| Purine Metabolism | NME2; SMARCA4; MYH9; RRM2; ADAR; EIF2AK4; |
| | PKM2; ENTPD1; RAD51; RRM2B; TJP2; RAD51C; |
| | NT5E; POLD1; NME1 |

(continued)

| CELLULAR FUNCTION | GENES |
|---|---|
| cAMP-mediated Signaling | RAP1A; MAPK1; GNAS; CREB1; CAMK2A; MAPK3; |
| | SRC; RAF1; MAP2K2; STAT3; MAP2K1; BRAF; ATF4 |
| Mitochondrial Dysfunction | SOD2; MAPK8; CASP8; MAPK10; MAPK9; CASP9; |
| | PARK7; PSEN1; PARK2; APP; CASP3 |
| Notch Signaling | HES1; JAG1; NUMB; NOTCH4; ADAM17; NOTCH2; |
| | PSEN1; NOTCH3; NOTCH1; DLL4 |
| Endoplasmic Reticulum | HSPA5; MAPK8; XBP1; TRAF2; ATF6; CASP9; ATF4; |
| Stress Pathway | EIF2AK3; CASP3 |
| Pyrimidine Metabolism | NME2; AICDA; RRM2; EIF2AK4; ENTPD1; RRM2B; |
| | NT5E; POLD1; NME1 |
| Parkinson's Signaling | UCHL1; MAPK8; MAPK13; MAPK14; CASP9; PARK7; |
| | PARK2; CASP3 |
| Cardiac & Beta Adrenergic | GNAS; GNAQ; PPP2R1A; GNB2L1; PPP2CA; PPP1CC; |
| Signaling | PPP2R5C |
| Glycolysis/Gluconeogene sis | HK2; GCK; GPI; ALDH1A1; PKM2; LDHA; HK1 |
| Interferon Signaling | IRF1; SOCS1; JAK1; JAK2; IFITM1; STAT1; IFIT3 |
| Sonic Hedgehog Signaling | ARRB2; SMO; GLI2; DYRK1A; GLI1; GSK3B; DYRK1B |
| Glycerophospholipid | PLD1; GRN; GPAM; YWHAZ; SPHK1; SPHK2 |
| Metabolism | |
| Phospholipid Degradation | PRDX6; PLD1; GRN; YWHAZ; SPHK1; SPHK2 |
| Tryptophan Metabolism | SIAH2; PRMT5; NEDD4; ALDH1A1; CYP1B1; SIAH1 |
| Lysine Degradation | SUV39H1; EHMT2; NSD1; SETD7; PPP2R5C |
| Nucleotide Excision Repair | ERCC5; ERCC4; XPA; XPC; ERCC1 |
| Pathway | |
| Starch and Sucrose | UCHL1; HK2; GCK; GPI; HK1 |
| Metabolism | |
| Aminosugars Metabolism | NQO1; HK2; GCK; HK1 |
| Arachidonic Acid | PRDX6; GRN; YWHAZ; CYP1B1 |
| Metabolism | |
| Circadian Rhythm Signaling | CSNK1E; CREB1; ATF4; NR1D1 |
| Coagulation System | BDKRB1; F2R; SERPINE1; F3 |
| Dopamine Receptor | PPP2R1A; PPP2CA; PPP1CC; PPP2R5C |
| Signaling | |
| Glutathione Metabolism | IDH2; GSTP1; ANPEP; IDH1 |
| Glycerolipid Metabolism | ALDH1A1; GPAM; SPHK1; SPHK2 |
| Linoleic Acid Metabolism | PRDX6; GRN; YWHAZ; CYP1B1 |
| Methionine Metabolism | DNMT1; DNMT3B; AHCY; DNMT3A |
| Pyruvate Metabolism | GLO1; ALDH1A1; PKM2; LDHA |

(continued)

| CELLULAR FUNCTION | GENES |
|---|---|
| Arginine and Proline Metabolism | ALDH1A1; NOS3; NOS2A |
| Eicosanoid Signaling | PRDX6; GRN; YWHAZ |
| Fructose and Mannose Metabolism | HK2; GCK; HK1 |
| Galactose Metabolism | HK2; GCK; HK1 |
| Stilbene, Coumarine and Lignin Biosynthesis | PRDX6; PRDX1; TYR |
| Antigen Presentation Pathway | CALR; B2M |
| Biosynthesis of Steroids | NQO1; DHCR7 |
| Butanoate Metabolism | ALDH1A1; NLGN1 |
| Citrate Cycle | IDH2; IDH1 |
| Fatty Acid Metabolism | ALDH1A1; CYP1B1 |
| Glycerophospholipid Metabolism | PRDX6; CHKA |
| Histidine Metabolism | PRMT5; ALDH1A1 |
| Inositol Metabolism | ERO1L; APEX1 |
| Metabolism of Xenobiotics by Cytochrome p450 | GSTP1; CYP1B1 |
| Methane Metabolism | PRDX6; PRDX1 |
| Phenylalanine Metabolism | PRDX6; PRDX1 |
| Propanoate Metabolism | ALDH1A1; LDHA |
| Selenoamino Acid Metabolism | PRMT5; AHCY |
| Sphingolipid Metabolism | SPHK1; SPHK2 |
| Aminophosphonate Metabolism | PRMT5 |
| Androgen and Estrogen Metabolism | PRMT5 |
| Ascorbate and Aldarate Metabolism | ALDH1A1 |
| Bile Acid Biosynthesis | ALDH1A1 |
| Cysteine Metabolism | LDHA |
| Fatty Acid Biosynthesis | FASN |
| Glutamate Receptor Signaling | GNB2L1 |
| NRF2-mediated Oxidative | PRDX1 |

(continued)

| CELLULAR FUNCTION | GENES |
|---|---|
| Stress Response | |
| Pentose Phosphate | GPI |
| Pathway | |
| Pentose and Glucuronate | UCHL1 |
| Interconversions | |
| Retinol Metabolism | ALDH1A1 |
| Riboflavin Metabolism | TYR |
| Tyrosine Metabolism | PRMT5, TYR |
| Ubiquinone Biosynthesis | PRMT5 |
| Valine, Leucine and | ALDH1A1 |
| Isoleucine Degradation | |
| Glycine, Serine and | CHKA |
| Threonine Metabolism | |
| Lysine Degradation | ALDH1A1 |
| Pain/Taste | TRPM5; TRPA1 |
| Pain | TRPM7; TRPC5; TRPC6; TRPC1; Cnr1; cnr2; Grk2; |
| | Trpa1; Pomc; Cgrp; Crf; Pka; Era; Nr2b; TRPM5; Prkaca; |
| | Prkacb; Prkar1a; Prkar2a |
| Mitochondrial Function | AIF; CytC; SMAC (Diablo); Aifm-1; Aifm-2 |
| Developmental Neurology | BMP-4; Chordin (Chrd); Noggin (Nog); WNT (Wnt2; |
| | Wnt2b; Wnt3a; Wnt4; Wnt5a; Wnt6; Wnt7b; Wnt8b; |
| | Wnt9a; Wnt9b; Wnt10a; Wnt10b; Wnt16); beta-catenin; |
| | Dkk-1; Frizzled related proteins; Otx-2; Gbx2; FGF-8; |
| | Reelin; Dab1; unc-86 (Pou4f1 or Brn3a); Numb; Rein |

Embodiments of the invention also relate to methods and compositions related to knocking out genes, amplifying genes and repairing particular mutations associated with DNA repeat instability and neurological disorders (Robert D. Wells, Tetsuo Ashizawa, Genetic Instabilities and Neurological Diseases, Second Edition, Academic Press, Oct 13, 2011 - Medical). Specific aspects of tandem repeat sequences have been found to be responsible for more than twenty human diseases (New insights into repeat instability: role of RNA•DNA hybrids. McIvor EI, Polak U, Napierala M. RNA Biol. 2010 Sep-Oct;7(5):551-8). The CRISPR-Cas system may be harnessed to correct these defects of genomic instability.

A further aspect of the invention relates to utilizing the CRISPR-Cas system for correcting defects in the EMP2A and EMP2B genes that have been identified to be associated with Lafora disease. Lafora disease is an autosomal recessive condition which is characterized by progressive myoclonus epilepsy which may start as epileptic seizures in adolescence. A few cases of the disease may be caused by mutations in genes yet to be identified. The disease causes seizures, muscle spasms, difficulty walking, dementia, and eventually death. There is currently no therapy that has proven effective against disease progression. Other genetic abnormalities associated with epilepsy may also be targeted by the CRISPR-Cas system and the underlying genetics is further described in Genetics of Epilepsy and Genetic Epilepsies, edited by Giuliano Avanzini, Jeffrey L. Noebels, Mariani Foundation Paediatric Neurology:20; 2009).

In yet another aspect of the invention, the CRISPR-Cas system may be used to correct ocular defects that arise from several genetic mutations further described in Genetic Diseases of the Eye, Second Edition, edited by Elias I. Traboulsi, Oxford University Press, 2012.

Several further aspects of the invention relate to correcting defects associated with a wide range of genetic diseases which are further described on the website of the National Institutes of Health under the topic subsection Genetic Disorders (website at health.nih.gov/topic/GeneticDisorders). The genetic brain diseases may include but are not limited to Adrenoleukodystrophy, Agenesis of the Corpus Callosum, Aicardi Syndrome, Alpers' Disease, Alzheimer's Disease, Barth Syndrome, Batten Disease, CADASIL, Cerebellar Degeneration, Fabry's Disease, Gerstmann-Straussler-Scheinker Disease, Huntington's Disease and other Triplet Repeat Disorders, Leigh's Disease, Lesch-Nyhan Syndrome, Menkes Disease, Mitochondrial Myopathies and NINDS Colpocephaly. These diseases are further described on the website of the National Institutes of Health under the subsection Genetic Brain Disorders.

In some embodiments, the condition may be neoplasia. In some embodiments, where the condition is neoplasia, the genes to be targeted are any of those listed in Table A (in this case PTEN and so forth). In some embodiments, the condition may be Age-related Macular Degeneration. In some embodiments, the condition may be a Schizophrenic Disorder. In some embodiments, the condition may be a Trinucleotide Repeat Disorder. In some embodiments, the condition may be Fragile X Syndrome. In some embodiments, the condition may be a Secretase Related Disorder. In some embodiments, the condition may be a Prion - related disorder. In some embodiments, the condition may be ALS. In some embodiments, the condition may be a drug addiction. In some embodiments, the condition may be Autism. In some embodiments, the condition may be Alzheimer's Disease. In some embodiments, the condition may be inflammation. In some embodiments, the condition may be Parkinson's Disease.

Examples of proteins associated with Parkinson's disease include but are not limited to α-synuclein, DJ-1, LRRK2, PINK1, Parkin, UCHL1, Synphilin-1, and NURR1.

Examples of addiction-related proteins may include ABAT for example.

Examples of inflammation-related proteins may include the monocyte chemoattractant protein-1 (MCP1) encoded by the Ccr2 gene, the C-C chemokine receptor type 5 (CCR5) encoded by the Ccr5 gene, the IgG receptor IIB (FCGR2b, also termed CD32) encoded by the Fcgr2b gene, or the Fc epsilon R1g (FCER1g) protein encoded by the Fcerlg gene, for example.

Examples of cardiovascular diseases associated proteins may include IL1B (interleukin 1, beta), XDH (xanthine dehydrogenase), TP53 (tumor protein p53), PTGIS (prostaglandin I2 (prostacyclin) synthase), MB (myoglobin), IL4 (interleukin 4), ANGPT1 (angiopoietin 1), ABCG8 (ATP-binding cassette, sub-family G (WHITE), member 8), or CTSK (cathepsin K), for example.

Examples of Alzheimer's disease associated proteins may include the very low density lipoprotein receptor protein (VLDLR) encoded by the VLDLR gene, the ubiquitin-like modifier activating enzyme 1 (UBA1) encoded by the UBA1 gene, or the NEDD8-activating enzyme E1 catalytic subunit protein (UBE1C) encoded by the UBA3 gene, for example.

Examples of proteins associated Autism Spectrum Disorder may include the benzodiazapine receptor (peripheral) associated protein 1 (BZRAP1) encoded by the BZRAP1 gene, the AF4/FMR2 family member 2 protein (AFF2) encoded by the AFF2 gene (also termed MFR2), the fragile X mental retardation autosomal homolog 1 protein (FXR1) encoded by the FXR1 gene, or the fragile X mental retardation autosomal homolog 2 protein (FXR2) encoded by the FXR2 gene, for example.

Examples of proteins associated Macular Degeneration may include the ATP-binding cassette, sub-family A (ABC1) member 4 protein (ABCA4) encoded by the ABCR gene, the apolipoprotein E protein (APOE) encoded by the APOE gene, or the chemokine (C-C motif) Ligand 2 protein (CCL2) encoded by the CCL2 gene, for example.

Examples of proteins associated Schizophrenia may include NRG1, ErbB4, CPLX1, TPH1, TPH2, NRXN1, GSK3A, BDNF, DISC1, GSK3B, and combinations thereof.

Examples of proteins involved in tumor suppression may include ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, or Notch 4, for example.

Examples of proteins associated with a secretase disorder may include PSENEN (presenilin enhancer 2 homolog (C. elegans)), CTSB (cathepsin B), PSEN1 (presenilin 1), APP (amyloid beta (A4) precursor protein), APH1B (anterior pharynx defective 1 homolog B (C. elegans)), PSEN2 (presenilin 2 (Alzheimer disease 4)), or BACE1 (beta-site APP-cleaving enzyme 1), for example.

Examples of proteins associated with Amyotrophic Lateral Sclerosis may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins associated with prion diseases may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins related to neurodegenerative conditions in prion disorders may include A2M (Alpha-2-Macroglobulin), AATF (Apoptosis antagonizing transcription factor), ACPP (Acid phosphatase prostate), ACTA2 (Actin alpha 2 smooth muscle aorta), ADAM22 (ADAM metallopeptidase domain), ADORA3 (Adenosine A3 receptor), or ADRA1D (Alpha-ID adrenergic receptor for Alpha-1D adrenoreceptor), for example.

Examples of proteins associated with Immunodeficiency may include A2M [alpha-2-macroglobulin]; AANAT [arylalkylamine N-acetyltransferase]; ABCA1 [ATP-binding cassette, sub-family A (ABC1), member 1]; ABCA2 [ATP-binding cassette, sub-family A (ABC1), member 2]; or ABCA3 [ATP-binding cassette, sub-family A (ABC1), member 3]; for example.

Examples of proteins associated with Trinucleotide Repeat Disorders include AR (androgen receptor), FMR1 (fragile X mental retardation 1), HTT (huntingtin), or DMPK (dystrophia myotonica-protein kinase), FXN (frataxin), ATXN2 (ataxin 2), for example.

Examples of proteins associated with Neurotransmission Disorders include SST (somatostatin), NOS1 (nitric oxide synthase 1 (neuronal)), ADRA2A (adrenergic, alpha-2A-, receptor), ADRA2C (adrenergic, alpha-2C-, receptor), TACR1 (tachykinin receptor 1), or HTR2c (5-hydroxytryptamine (serotonin) receptor 2C), for example.

Examples of neurodevelopmental-associated sequences include A2BP1 [ataxin 2-binding protein 1], AADAT [aminoadipate aminotransferase], AANAT [arylalkylamine N-acetyltransferase], ABAT [4-aminobutyrate aminotransferase], ABCA1 [ATP-binding cassette, sub-family A (ABC1), member 1], or ABCA13 [ATP-binding cassette, sub-family A (ABC1), member 13], for example.

Further examples of preferred conditions treatable with the present system include may be selected from: Aicardi-Goutières Syndrome; Alexander Disease; Allan-Herndon-Dudley Syndrome; POLG-Related Disorders; Alpha-Mannosidosis (Type II and III); Alström Syndrome; Angelman; Syndrome; Ataxia-Telangiectasia; Neuronal Ceroid-Lipofuscinoses; Beta-Thalassemia; Bilateral Optic Atrophy and (Infantile) Optic Atrophy Type 1; Retinoblastoma (bilateral); Canavan Disease; Cerebrooculofacioskeletal Syndrome 1 [COFS1]; Cerebrotendinous Xanthomatosis; Cornelia de Lange Syndrome; MAPT-Related Disorders; Genetic Prion Diseases; Dravet Syndrome; Early-Onset Familial Alzheimer Disease; Friedreich Ataxia [FRDA]; Fryns Syndrome; Fucosidosis; Fukuyama Congenital Muscular Dystrophy; Galactosialidosis; Gaucher Disease; Organic Acidemias; Hemophagocytic Lymphohistiocytosis; Hutchinson-Gilford Progeria Syndrome; Mucolipidosis II; Infantile Free Sialic Acid Storage Disease; PLA2G6-Associated Neurodegeneration; Jervell and Lange-Nielsen Syndrome; Junctional Epidermolysis Bullosa; Huntington Disease; Krabbe Disease (Infantile); Mitochondrial DNA-Associated Leigh Syndrome and NARP; Lesch-Nyhan Syndrome; LIS1-Associated Lissencephaly; Lowe Syndrome; Maple Syrup Urine Disease; MECP2 Duplication Syndrome; ATP7A-Related Copper Transport Disorders; LAMA2-Related Muscular Dystrophy; Arylsulfatase A Deficiency; Mucopolysaccharidosis Types I, II or III; Peroxisome Biogenesis Disorders, Zellweger Syndrome Spectrum; Neurodegeneration with Brain Iron Accumulation Disorders; Acid Sphingomyelinase Deficiency; Niemann-Pick Disease Type C; Glycine Encephalopathy; ARX-Related Disorders; Urea Cycle Disorders; COL1A1/2-Related Osteogenesis Imperfecta; Mitochondrial DNA Deletion Syndromes; PLP1-Related Disorders; Perry Syndrome; Phelan-McDermid Syndrome; Glycogen Storage Disease Type II (Pompe Disease) (Infantile); MAPT-Related Disorders; MECP2-Related Disorders; Rhizomelic Chondrodysplasia Punctata Type 1; Roberts Syndrome; Sandhoff Disease; Schindler Disease - Type 1; Adenosine Deaminase Deficiency; Smith-Lemli-Opitz Syndrome; Spinal Muscular Atrophy; Infantile-Onset Spinocerebellar Ataxia; Hexosaminidase A Deficiency; Thanatophoric Dysplasia Type 1; Collagen Type VI-Related Disorders; Usher Syndrome Type I; Congenital Muscular Dystrophy; Wolf-Hirschhorn Syndrome; Lysosomal Acid Lipase Deficiency; and Xeroderma Pigmentosum.

As will be apparent, it is envisaged that the present system can be used to target any polynucleotide sequence of interest. Some examples of conditions or diseases that might be usefully treated using the present system are included in the Tables above and examples of genes currently associated with those conditions are also provided there. However, the genes exemplified are not exhaustive.

For example, "wild type StCas9" refers to wild type Cas9 from S thermophilus, the protein sequence of which is given in the SwissProt database under accession number G3ECR1. Similarly, S pyogenes Cas9 is included in SwissProt under accession number Q99ZW2. SaCas9 is also well-characterized.

The ability to use CRISPR-Cas systems to perform efficient and cost effective gene editing and manipulation will allow the rapid selection and comparison of single and multiplexed genetic manipulations to transform such genomes for improved production and enhanced traits. In this regard reference is made to US patents and publications: US Patent No. 6,603,061 - Agrobacterium-Mediated Plant Transformation Method; US Patent No. 7,868,149 - Plant Genome Sequences and Uses Thereof and US 2009/0100536 - Transgenic Plants with Enhanced Agronomic Traits. In the practice of the invention, the contents and disclosure of Morrell et al "Crop genomics:advances and applications" Nat Rev Genet. 2011 Dec 29;13(2):85-96.

The current invention is based on several technical effects, which are, inter alia, generally defined by optimized double nicking, generation of 3' overhangs, inhibition of NHEJ, and improved HDR efficiency.

The current invention is based the technical effect of improved HDR efficiency using SpCas9N863A mutant or an ortholog thereof having a mutation corresponding to SpCas9N863A. Specifically, the improved HDR efficiency is the result of inhibition of NHEJ events and thus a bias (i.e. increase) in HDR events. Such an ortholog can be a mutated S.

*aureus* Cas9.

The current invention is also based on the technical effect of optimized double nicking due to optimal target sequence selection so that the 5' PAM sequences face away from one another.

Further, the current invention is based on the technical effect that nuclease activity of the SpCas9N863A mutant or an ortholog having a mutation corresponding to SpCas9N863A always results in strand cleavage in the non-complementary strand. Under these conditions, 3' overhangs are generated if the target sequences (as defined by the sgRNA) on the individual strands are arranged such that the corresponding 5' PAM sequences (located immediately 3' to the target sequences) face away from one another. Such an ortholog can be a mutated *S. aureus* Cas9.

Further, the current invention is based on a technical effect that 3' overhangs result in inhibition of NHEJ. The technical effect of directed generation of 3' overhangs is improved HDR efficiency as such overhangs results in inhibition of NHEJ events and thus a bias (i.e. increase) in HDR events (i.e. improved HDR efficiency and/or reduced indel formation).

An improved HDR efficiency is considered a higher frequency of HDR events (and/or reduced indel formation) as a result of double nickase activity resulting from either the use of SpCas9N863A mutant or an ortholog having a mutation corresponding to SpCas9N863A as compared to double nickase activity resulting from a SpCas9 which does not comprise the N863A mutation or an ortholog not comprising a corresponding mutation to SpCas9N863A. Such an ortholog can be a mutated *S. aureus* Cas9.

By performing the methods of the invention of modifying an organism or a genomic locus of interest, the skilled person will inevitably arrive at minimized off-target modifications.

## EXAMPLES

The following examples are given for the purpose of illustrating various embodiments of the invention and are not meant to limit the present invention in any fashion. The present examples, along with the methods described herein are presently representative of preferred embodiments, are exemplary, and are not intended as limitations on the scope of the invention. Changes therein and other uses which are encompassed within the scope of the claims will occur to those skilled in the art.

### Example 1: Target Selection

SpCas9 targets are any 20-bp DNA sequence followed at the 3' end by 5'-NGG-3'. The CRISPR DESIGN website of the Zhang Lab, MIT, provides an online tool (http://tools.genome-engineering.org) accepts a region of interest as input and provides as a output a list of all potential sgRNA target sites within that region. Each sgRNA target site is then associated with a list of predicted genomic off-targets.

The tool also generates double-nicking sgRNA pairs automatically. The most important consideration for double-nicking sgRNA design is the spacing between the two targets (Ran et al., 2013). If the "offset" between two guides is defined as the distance between the PAM-distal (5') ends of an sgRNA pair, an offset of -4 to 20 bp is ideal, though offsets as large as 100 bp can induce DSB-mediated indels. sgRNA pairs for double nicking target opposite DNA strands.

### Example 2: Plasmid sgRNA construction

sgRNA expression vectors are constructed by cloning 20-bp target sequences into a plasmid backbone encoding a human U6 promoter-driven sgRNA expression cassette and a CBh-driven Cas9-D10A (pSpCas9n(BB), Addgene #48873). The N863A nickase is exchanged with D10A in all cases. It is recommended to prepare this plasmid as an endotoxin-free maxiprep. The generalized oligos used to clone a new target into pSpCas9n(BB) are described in Table 1 and are purchased from Integrated DNA Technologies (IDT). Note that the PAM sequence required for target recognition by Cas9 is not present as part of the sgRNA itself.

**Table 1:**

| Primer | Sequence (5' to 3') | Description |
|---|---|---|
| sgRNA-fwd | CACCGNNNNNNNNNNNNNNNNNNN | Sticky overhang plus specific 20-bp genomic target to be cloned into sgRNA backbones |
| sgRNA-rev | AAACNNNNNNNNNNNNNNNNNNNNC | Complimentary annealing oligo for cloning new target into sgRNA backbones. |

1. A target sequence is cloned into an sgRNA backbone vector. The sgRNA-fwd and sgRNA-rev oligos are resuspended to 100 uM. Note that these oligos include an appended guanine (lowercase) not present in the target site in order to increase transcription from the U6 promoter.

2. 1 μL of each oligo is combined with 1 μL T4 ligation buffer, 10x (New England Biolabs (NEB) B0202S), 0.5 μL T4 PNK (NEB M0201S) and 6.5 μL ddH20 for a 10 μL reaction total. Solution is treated with polynucleotide kinase to add 5' phosphate and the oligos are annealed in a thermocycler with the following protocol: 37 °C for 30 min, 95 °C for 5 mins, ramp down to 25 °C at 5 °C/min.

3. The annealed oligos are diluted (10 μL reaction) by adding 90 μL ddH$_2$0.

4. A Golden Gate digestion/ligation is performed with pSpCas9n(BB) and the annealed oligos as a cloning insert. The resulting plasmid contains twin BsmBI restriction sites in place of the sgRNA target sequence such that digestion leaves overhangs complimentary to the annealed oligo overhangs. In a 25 μL reaction, 25 ng pSpCas9n(BB), 1 μL diluted annealed oligos from step (3), 12.5 μL Rapid Ligation Buffer, 2x (Enzymatics L6020L), 1 μL FastDigest BsmBI (ThermoScientific FD1014), 2.5 μL 10x BSA (NEB B9001), 0.125 μL T7 Ligase (Enzymatics L6020L), and 7 μL ddH$_2$0 are combined.

5. A negative control is performed using the same conditions as above and substituting the insert oligos with ddH$_2$0.

6. The ligation is incubated in a thermocycler for 6 cycles of 37 °C for 5 min, 20 °C for 5 min. The ligation is stable for storage at -20 °C.

7. 2 μL of the ligation reaction is transformed into a competent *E. coli* strain using the appropriate protocol - the Stbl3 strain is preferred - plate onto ampicillin selection plates (100 μg/mL ampicillin), and incubate overnight at 37 °C. Typically transformation occurs at high efficiency; no colonies form on the negative control plate, and hundreds form when the sgRNA oligos have been successfully cloned into the backbone.

8. Two or more colonies are picked 14 hours later from the transformation with a sterile pipette tip and the bacteria are used to inoculate 3 mL LB or TB broth with 100 μg/mL ampicillin. The culture is shook at 37 °C for 14 hours.

9. The plasmid DNA is isolated from the cultures using the Qiagen Spin Miniprep Kit (27104) and the DNA concentration is determined by spectrophotometry. These constructs are Sanger sequence-verified through the sgRNA scaffold to confirm correct insertion of the target sequence. For optimal transfection conditions downstream, endotoxin-free plasmid are prepared.

**Example 3: Validation of sgRNAs in Cell Lines**

This example describes the functional validation of sgRNAs in HEK293FT cells; culture and transfection conditions may vary for other cell types.

1. HEK293FT cells (Life Technologies R700-07) are maintained in sterile D10 media (DMEM, high glucose (Life Technologies 10313-039) supplemented with 10% vol/vol fetal bovine serum (Seradigm 1500-500) and 10 mM HEPES (Life Technologies 15630-080)). For optimal health, cells are passaged every day at a ratio of 1:2-2.5 and kept under 80% confluence.

2. Cells are plated for transfection. 120,000 cells are seeded per well of a 24-well tissue-culture treated plate in a total volume of 500 μL. Cultures and transfections are proportionally scaled up or down for different formats based on growth surface area. For many adherent cell types, poly-D-lysine coated plastic may improve adherence and viability.

3. After 18 hours the plates are checked to determine the confluence of the cells - generally 90% is ideal. Lipofectamine 2000 (Life Technologies 11668109) reagent is used to transfect DNA according to the manufacturer's protocol. For a 24-well plate transfection is no more than 500 ng DNA total.

4. For delivery of one nicking pSpCas9n(sgRNA) plasmid, 500 ng is transfected; for multiple nicking constructs, e.g., delivering two sgRNAs for double nicking, different constructs up to 500 ng at equimolar ratios are mixed before transfection.

5. Transfection controls are added (untransfected wells and GFP plasmid), as well as experimental controls, (Cas9n without guides or guides alone), in these experiments. Transfecting in technical triplicates facilitate analysis.

6. Within 6 hours of transfection, the media is changed to 2 mL of fresh, pre-warmed D10 media per well. At 24 hours, transfection efficiency is estimated by examining GFP-transfected wells. >80% of cells is GFP-positive.

7. The cells are harvested for genomic DNA extraction and/or downstream analysis at 48-72 hours. For harvesting at 72 hour time point, the media is changed again at 48 hours to maintain optimal cell health.

When working with different cell types, alternative transfection reagents should be compared for efficiency and toxicity. It may also be informative to titrate pSpCas9n(sgRNA) in order to find the optimal transfection concentration with highest efficacy.

**Example 4: Cell harvest and DNA extraction**

1. Cells are harvested in 24-well plate format by aspirating the medium completely and adding 100 μL of TrypLE Express reagent (Life Technologies 12604013) to facilitate dissociation.
2. The cell suspension is collected in a 1.5 mL Eppendorf tube and spun for 5 min at 1500 g, the supernatant is completely aspirated, and the cell pellet is resuspended in 200 μL DPBS (Life Technologies 14190-250) for washing.
3. The cell suspension is again spun for 5 min at 1500 g and resuspended in 50 μL QuickExtract (Epicentre QE09050).
4. The QuickExtract suspension is transferred to a 0.2 mL PCR tube and genomic DNA is extracted according to the following thermocycler protocol adapted from the manufacturer's instructions: 65 °C for 15 min, 98 °C for 10 min.
5. The reaction product is centrifuged to pellet cell debris and cleared supernatant is transferred into a fresh tube for further analysis.
6. The DNA concentration of the extraction is determined by spectrophotometry and normalized to 100-200 ng/μL with ddH$_2$0.

**Example 5: SURVEYOR indel analysis**

The SURVEYOR assay (Transgenomic 706025) is a method for detecting polymorphisms and small indels. DNA samples are PCR-amplified, and the products are heated to denature and cooled slowly to form heteroduplexes. Mismatched duplexes are then cleaved by the SURVEYOR nuclease, and cleavage products are analyzed by gel electrophoresis.

1. PCR is performed on genomic DNA. The primers for SURVEYOR PCR produce a clean approximate 500 bp amplicon in untransfected cell samples. Genomic PCR primers are designed using software such as Primer3. A 50 μL reaction is set up containing 1 μL of each 10 μM SURVEYOR primer, 10 μL Herculase II Reaction Buffer 5x (Agilent 600675), 0.5 μL 100 mM dNTP, 0.5 μL Herculase II Fusion Polymerase, 2 μL of 25 mM MgCl$_2$ and 36 μL ddH$_2$0. Solution is denatured for 20 s at 95 °C, annealed for 20 s at 60 °C, and extended for 20 s at 72 °C.
2. A high-fidelity polymerase is used to avoid false positives.
3. 2 μL of the PCR product is run on a 1% agarose gel to ensure that a single product of expected size has formed.
4. The PCR product is purified using the QIAquick PCR Purification Kit (28104) according to the instructions provided. The DNA concentration of the eluate is measured by spectrophotometry and normalized to 20 ng/μL using ddH20.
5. 18 μL of normalized PCR product is mixed with 2 μL Taq PCR buffer, 10x, for a 20 μL reaction total. The products are melted and re-hybridized gradually in a thermocycler: melt at 95 °C for 10 min, the temperature is ramped down to 85 °C at a rate of -0.3 °C/s. Held at 85 °C 1 min, then ramped to 75 °C at 0.3 °C/s. Held at 75 °C 1 min, then ramped to 65 °C... and so on, until the temperature reaches 25 °C. From 25 °C, ramped down to 4 °C at 0.3 °C/s and held.
6. 2.5 μL 0.15 M MgCl$_2$, 0.5 μL ddH$_2$0, 1 μL SURVEYOR nuclease S, and 1 μL SURVEYOR enhancer S are mixed with all of the annealed product from step (5) for a 25 μL total reaction volume. The digestion is performed by incubating the reaction at 42 °C for 30 minutes. Samples that have mutations within the rehybridized PCR amplicons are be cleaved by SURVEYOR.
7. The digestion products are mixed with an appropriate loading dye and visualized by electrophoresis on a 4-20% polyacrylamide TBE gel (see for example, **Figure 2B**).
8. Genome modification rates are estimated first by calculating the relative intensities of digestion products *a* and *b*, and the undigested band c. The frequency of cutting $f_{cut}$ is then given by (a + b)/(a + b + c). The following formula, based on the binomial probability distribution of duplex formation, estimates the percentage of indels in the sample.

$$\%indel = \left(1 - \sqrt{(1 - f_{cut})}\right)100$$

**Example 6: HDR and non-HDR insertion using Cas9n**

1. ssODN homology arms are designed to be as long as possible, with at least 40 nucleotides of homology on either side of the sequence to be introduced. The Ultramer service provided by IDT allows the synthesis of oligos up to 200 bp in length. Homology templates are diluted to 10 μM and stored at -20 °C (see design example, **Figure 3**).
2. Delivery by Nucleofection is optimal for ssODNs. The 4D Nucleofector X Kit S (Lonza V4XC-2032) is used for HEK293FT cells seeded in 6-well tissue culture-treated plates. The manufacturer provides an optimal protocol for nucleofection of these and other cell types.

3. 500 ng total pSpCas9n(sgRNA) plasmids is mixed with 1 μL 10 μM ssODN for nucleofection.

HDR in mammalian cells proceeds via the generation of 3' overhangs followed by strand invasion of a homologous locus by the 3' end. The technical effect is that the generation of 3' overhang products by N863A-mediated double nicking increase HDR efficiency.

**Example 7: Analysis of HDR and insertion events**

HDR outcomes can be assessed and utilized in a variety of ways. Here, the FACS isolation of clonal pSpCas9n(sgRNA)-GFP 293FT cells is described. It is important to note that FACS procedures can vary between cell types.

1. FACS media is prepared (D10 without phenol red to facilitate fluorescence sorting): DMEM, high glucose, no phenol red (Life Technologies 31053-028) supplemented with 10% vol/vol fetal bovine serum and 10 mM HEPES supplemented with 1% penicillin-streptomycin (Life Technologies 15140122).

2. 96-well plates are prepared for clone sorting by adding 100 μL standard D10 media to each well.

3. 24 hours after the transfection in section (7), the medium is aspirated completely and the cells dissociated using sufficient TrypLE Express to cover the growth surface minimally.

4. Trypsinization is stopped by adding D10 medium, the cells transferred to a fresh 15 mL tube, and triturating continued gently 20 times. It is critical that the cells are in a single-cell suspension before proceeding.

5. The cells are spun for 5 min at 200 g, the supernatant aspirated completely, and the pellet resuspended thoroughly and carefully in 200 μL FACS medium.

6. The cells are filtered through a cell strainer (BD Falcon 352235) to filter out cell aggregates and the cells placed on ice.

7. Single cells are sorted in the plates prepared in (2). The FACS machine can be gated on GFP+ cells in order to enrich for transfected cells. Wells can be visually inspected to check for the presence of one cell.

8. The cells are incubated and expanded for 2-3 weeks, media changed to fresh D10 as necessary.

9. When cells exceed 60% confluence, clonal populations are passaged into replica plates containing fresh D10 media. Cells dissociated, 20% of the cells are passaged into replica plates, and 80% conserved for DNA extraction as described in section (5).

10. Genotyping is performed by PCR amplification of the locus of interest, PCR purification, and Sanger sequencing of the products.

**Example 7: SaCas9 nickases D10A and N580A**

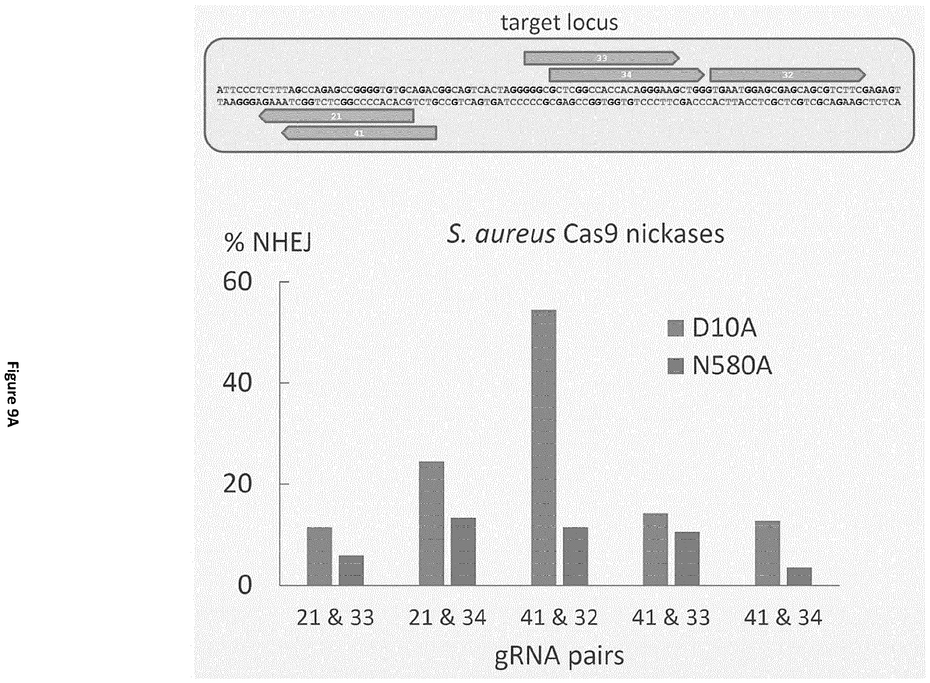

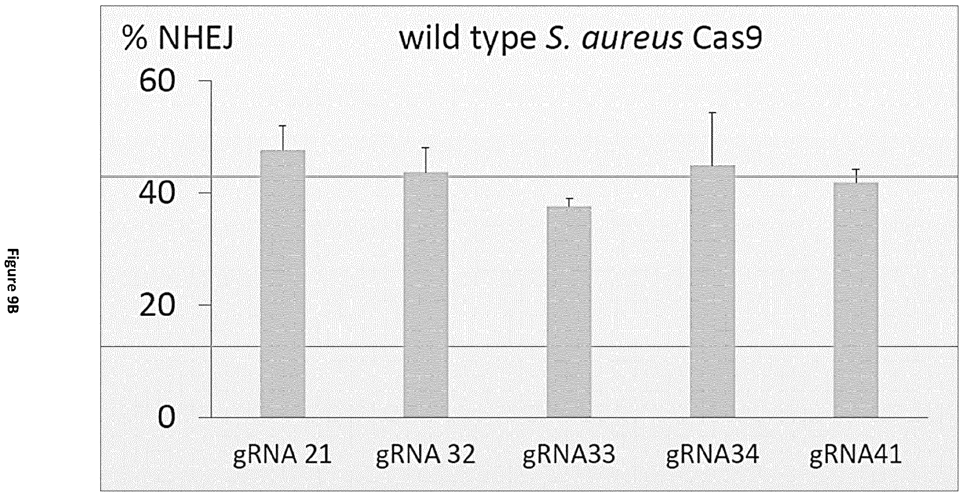

Editas Medicine reported data demonstrating nickase activity of D10A and N580A mutants of SaCas9. (Figure 9).

**References**

Ding, Q. et al. A TALEN genome-editing system for generating human stem cell-based disease models. Cell Stem Cell 12, 238-251 (2013).

Soldner, F. et al. Generation of isogenic pluripotent stem cells differing exclusively at two early onset Parkinson point mutations. Cell 146, 318-331 (2011).

Carlson, D.F. et al. Efficient TALEN-mediated gene knockout in livestock. Proc Natl Acad Sci U S A 109, 17382-17387 (2012).

Geurts, A.M. et al. Knockout Rats via Embryo Microinjection of Zinc-Finger Nucleases. Science 325, 433-433 (2009).

Takasu, Y. et al. Targeted mutagenesis in the silkworm Bombyx mori using zinc finger nuclease mRNA injection. Insect Biochem Molec 40, 759-765 (2010).

Watanabe, T. et al. Non-transgenic genome modifications in a hemimetabolous insect using zinc-finger and TAL effector nucleases. Nat Commun 3 (2012).

Porteus, M.H. & Baltimore, D. Chimeric nucleases stimulate gene targeting in human cells. Science 300, 763 (2003).

Miller, J.C. et al. An improved zinc-finger nuclease architecture for highly specific genome editing. Nat Biotechnol 25, 778-785 (2007).

Sander, J.D. et al. Selection-free zinc-finger-nuclease engineering by context-dependent assembly (CoDA). Nat Methods 8, 67-69 (2011).

Wood, A.J. et al. Targeted genome editing across species using ZFNs and TALENs. Science 333, 307 (2011).

Christian, M. et al. Targeting DNA double-strand breaks with TAL effector nucleases. Genetics 186, 757-761 (2010).

Zhang, F. et al. Efficient construction of sequence-specific TAL effectors for modulating mammalian transcription. Nat Biotechnol 29, 149-153 (2011).

Miller, J.C. et al. A TALE nuclease architecture for efficient genome editing. Nat Biotechnol 29, 143-148 (2011).

Reyon, D. et al. FLASH assembly of TALENs for high-throughput genome editing. Nat Biotechnol 30, 460-465 (2012).

Boch, J. et al. Breaking the code of DNA binding specificity of TAL-type III effectors. Science 326, 1509-1512 (2009).

Moscou, M.J. & Bogdanove, A.J. A simple cipher governs DNA recognition by TAL effectors. Science 326, 1501 (2009).

Sanjana, N.E. et al. A transcription activator-like effector toolbox for genome engineering. Nat Protoc 7, 171-192 (2012).

Deveau, H., Garneau, J.E. & Moineau, S. CRISPR-Cas system and its role in phage-bacteria interactions. Annu Rev Microbiol 64, 475-493 (2010).

Horvath, P. & Barrangou, R. CRISPR-Cas, the immune system of bacteria and archaea. Science 327, 167-170 (2010).

Makarova, K.S. et al. Evolution and classification of the CRISPR-Cas systems. Nat Rev Microbiol 9, 467-477 (2011).

Bhaya, D., Davison, M. & Barrangou, R. CRISPR-Cas systems in bacteria and archaea: versatile small RNAs for adaptive defense and regulation. Annu Rev Genet 45, 273-297 (2011).

Cong, L. et al. Multiplex genome engineering using CRISPR-Cas systems. Science 339, 819-823 (2013).

Mali, P. et al. RNA-guided human genome engineering via Cas9. Science 339, 823-826 (2013b).

Jinek, M. et al. RNA-programmed genome editing in human cells. eLife 2, e00471 (2013).

Cho, S.W., Kim, S., Kim, J.M. & Kim, J.S. Targeted genome engineering in human cells with the Cas9 RNA-guided endonuclease. Nat Biotechnol 31, 230-232 (2013).

Garneau, J.E. et al. The CRISPR-Cas bacterial immune system cleaves bacteriophage and plasmid DNA. Nature 468, 67-71 (2010).

Jinek, M. et al. A programmable dual-RNA-guided DNA endonuclease in adaptive bacterial immunity. Science 337, 816-821 (2012).

Gasiunas, G., Barrangou, R., Horvath, P. & Siksnys, V. Cas9-crRNA ribonucleoprotein complex mediates specific DNA cleavage for adaptive immunity in bacteria. Proc Natl Acad Sci U S A 109, E2579-2586 (2012).

Urnov, F.D., Rebar, E.J., Holmes, M.C., Zhang, H.S. & Gregory, P.D. Genome editing with engineered zinc finger nucleases. Nat Rev Genet 11, 636-646 (2010).

Hsu, P.D. & Zhang, F. Dissecting neural function using targeted genome engineering technologies. ACS Chem Neurosci 3, 603-610 (2012).

Perez, E.E. et al. Establishment of HIV-1 resistance in CD4(+) T cells by genome editing using zinc-finger nucleases. Nat Biotechnol 26, 808-816 (2008).

Cong, L. et al. Multiplex Genome Engineering Using CRISPR-Cas Systems. Science 339, 819-823 (2013).

Chen, F.Q. et al. High-frequency genome editing using ssDNA oligonucleotides with zinc-finger nucleases. Nat Methods 8, 753-U796 (2011).

Bedell, V.M. et al. In vivo genome editing using a high-efficiency TALEN system. Nature 491, 114-U133 (2012).

Saleh-Gohari, N. & Helleday, T. Conservative homologous recombination preferentially repairs DNA double-strand breaks in the S phase of the cell cycle in human cells. Nucleic Acids Res 32, 3683-3688 (2004).

Deltcheva, E. et al. CRISPR RNA maturation by trans-encoded small RNA and host factor RNase III. Nature 471, 602-607 (2011).

Sapranauskas, R. et al. The Streptococcus thermophilus CRISPR-Cas system provides immunity in Escherichia coli. Nucleic Acids Res 39, 9275-9282 (2011).

Hwang, W.Y. et al. Efficient genome editing in zebrafish using a CRISPR-Cas system. Nat Biotechnol 31, 227-229 (2013).

Wang, H. et al. One-Step Generation of Mice Carrying Mutations in Multiple Genes by CRISPR-Cas-Mediated Genome Engineering. Cell 153, 910-918 (2013).

Shen, B. et al. Generation of gene-modified mice via Cas9/RNA-mediated gene targeting. Cell Res 23, 720-723 (2013).

Qi, L.S. et al. Repurposing CRISPR as an RNA-guided platform for sequence-specific control of gene expression. Cell 152, 1173-1183 (2013).

Tuschl, T. Expanding small RNA interference. Nat Biotechnol 20, 446-448 (2002).

Smithies, O., Gregg, R.G., Boggs, S.S., Koralewski, M.A. & Kucherlapati, R.S. Insertion of DNA sequences into the human chromosomal beta-globin locus by homologous recombination. Nature 317, 230-234 (1985).

Thomas, K.R., Folger, K.R. & Capecchi, M.R. High frequency targeting of genes to specific sites in the mammalian genome. Cell 44, 419-428 (1986).

Hasty, P., Rivera-Perez, J. & Bradley, A. The length of homology required for gene targeting in embryonic stem cells. Mol Cell Biol 11, 5586-5591 (1991).

Wu, S., Ying, G.X., Wu, Q. & Capecchi, M.R. A protocol for constructing gene targeting vectors: generating knockout mice for the cadherin family and beyond. Nat Protoc 3, 1056-1076 (2008).

Guschin, D.Y. et al. A rapid and general assay for monitoring endogenous gene modification. Methods Mol Biol 649, 247-256 (2010).

Oliveira, T.Y. et al. Translocation capture sequencing: a method for high throughput mapping of chromosomal rearrangements. J Immunol Methods 375, 176-181 (2012).

Deltcheva, E. et al. CRISPR RNA maturation by trans-encoded small RNA and host factor RNase III. Nature 471, 602-607 (2011).

Bogenhagen, D.F. & Brown, D.D. Nucleotide sequences in Xenopus 5S DNA required for transcription termination. Cell 24, 261-270 (1981).

Bultmann, S. et al. Targeted transcriptional activation of silent oct4 pluripotency gene by combining designer TALEs and inhibition of epigenetic modifiers. Nucleic Acids Res 40, 5368-5377 (2012).

Valton, J. et al. Overcoming transcription activator-like effector (TALE) DNA binding domain sensitivity to cytosine methylation. J Biol Chem 287, 38427-38432 (2012).

Christian, M. et al. Targeting DNA double-strand breaks with TAL effector nucleases. Genetics 186, 757-761 (2010).

Mussolino, C. et al. A novel TALE nuclease scaffold enables high genome editing activity in combination with low toxicity. Nucleic acids research 39, 9283-9293 (2011).

Bobis-Wozowicz, S., Osiak, A., Rahman, S.H. & Cathomen, T. Targeted genome editing in pluripotent stem cells using zinc-finger nucleases. Methods 53, 339-346 (2011).

Jiang, W., Bikard, D., Cox, D., Zhang, F. & Marraffini, L.A. RNA-guided editing of bacterial genomes using CRISPR-Cas systems. Nat Biotechnol 31, 233-239 (2013).

Michaelis, L.M., Maud "Die kinetik der invertinwirkung.". Biochem. z (1913).

Mahfouz, M.M. et al. De novo-engineered transcription activator-like effector (TALE) hybrid nuclease with novel DNA binding specificity creates double-strand breaks. Proc Natl Acad Sci U S A 108, 2623-2628 (2011).

Wilson, E.B. Probable inference, the law of succession, and statistical inference. J Am Stat Assoc 22, 209-212 (1927).

Tangri S, et al., Rationally engineered therapeutic proteins with reduced immunogenicity, J Immunol. 2005 Mar 15; 174(6):3187-96.

REMINGTON'S PHARMACEUTICAL SCIENCES (Mack Pub. Co., N.J. 1991)

Lopes, V.S., etc al., Retinal gene therapy with a large MYO7A cDNA using adeno-assocaited virus. Gene Ther, 2013 Jan 24. doi: 10.1038/gt 2013.3.[Epub ahead of print]

Kaplitt, M.G., et al., Safety and tolerability of gene therapy with an adeno-associated virus (AAV) borne GAD gene for Parkinson's disease: an open label, phase I trial. Lancet. 2007 Jun 23;369(9579):2097-105.

Nathwani, A.C., et al., Adenovirus-associated virus vector-mediated gene transfer in hemophilia B. N Engl J Med. 2011 Dec 22;365(25):2357-65. doi: 10.1056/NEJMoa1108046. Epub 2011 Dec 10.

Gray SJ, Foti SB, Schwartz JW, Bachaboina L, Taylor-Blake B, Coleman J, Ehlers MD, Zylka MJ, McCown TJ, Samulski RJ. Optimizing promoters for recombinant adeno-associated virus-mediated gene expression in the peripheral and central nervous system using self-complementary vectors. Hum Gene Ther. 2011 Sep;22(9):1143-53. doi: 10.1089/hum.2010.245.

Liu D, Fischer I. Two alternative promoters direct neuron-specific expression of the rat microtubule-associated protein 1B gene. J Neurosci. 1996 Aug 15;16(16):5026-36.

Levitt N. Briggs D. Gil A. Proudfoot N.J. Definition of an efficient synthetic poly(A) site. Genes Dev. 1989;3:1019-1025.

McClure C, Cole KL, Wulff P, Klugmann M, Murray AJ. Production and titering of recombinant adeno-associated viral vectors. J Vis Exp. 2011 Nov 27;(57):e3348. doi: 10.3791/3348.

Banker G, Goslin K. Developments in neuronal cell culture. Nature. 1988 Nov 10;336(6195):185-6.

Chang, N., Sun, C., Gao, L., Zhu, D., Xu, X., Zhu, X., Xiong, J.W., and Xi, J.J. (2013). Genome editing with RNA-guided Cas9 nuclease in zebrafish embryos. Cell research 23, 465-472.

Dianov, G.L., and Hubscher, U. (2013). Mammalian base excision repair: the forgotten archangel. Nucleic acids research 41, 3483-3490.

Friedland, A.E., Tzur, Y.B., Esvelt, K.M., Colaiacovo, M.P., Church, G.M., and Calarco, J.A. (2013). Heritable genome editing in C. elegans via a CRISPR-Cas9 system. Nature methods 10, 741-743.

Fu, Y., Foden, J.A., Khayter, C., Maeder, M.L., Reyon, D., Joung, J.K., and Sander, J.D. (2013). High-frequency off-target mutagenesis induced by CRISPR-Cas nucleases in human cells. Nature biotechnology.

Gratz, S.J., Cummings, A.M., Nguyen, J.N., Hamm, D.C., Donohue, L.K., Harrison, M.M., Wildonger, J., and O'Connor-Giles, K.M. (2013). Genome Engineering of Drosophila with the CRISPR RNA-Guided Cas9 Nuclease. Genetics 194, 1029-1035.

Hsu, P.D., Scott, D.A., Weinstein, J.A., Ran, F.A., Konermann, S., Agarwala, V., Li, Y., Fine, E.J., Wu, X., Shalem, O., et al. (2013). DNA targeting specificity of RNA-guided Cas9 nucleases. Nature biotechnology.

Mali, P., Aach, J., Stranges, P.B., Esvelt, K.M., Moosburner, M., Kosuri, S., Yang, L., and Church, G.M. (2013a). CAS9 transcriptional activators for target specificity screening and paired nickases for cooperative genome engineering. Nature biotechnology.

Maresca, M., Lin, V.G., Guo, N., and Yang, Y. (2013). Obligate ligation-gated recombination (ObLiGaRe): custom-designed nuclease-mediated targeted integration through nonhomologous end joining. Genome research 23, 539-546.

Pattanayak, V., Lin, S., Guilinger, J.P., Ma, E., Doudna, J.A., and Liu, D.R. (2013). High-throughput profiling of off-target DNA cleavage reveals RNA-programmed Cas9 nuclease specificity. Nature biotechnology.

Barrangou, R., Fremaux, C., Deveau, H., Richards, M., Boyaval, P., Moineau, S., ... Horvath, P. (2007). CRISPR provides acquired resistance against viruses in prokaryotes. Science, 315(5819), 1709-1712. doi: 10.1126/science.1138140.

Barrangou, R., & Marraffini, L. A. (2014). CRISPR-Cas systems: Prokaryotes upgrade to adaptive immunity. Mol Cell, 54(2), 234-244. doi: 10.1016/j.molcel.2014.03.011.

Boch, J., & Bonas, U. (2010). Xanthomonas AvrBs3 family-type III effectors: discovery and function. Annu Rev Phytopathol, 48, 419-436. doi: 10.1146/annurev-phyto-080508-081936.

Chylinski, K., Makarova, K. S., Charpentier, E., & Koonin, E. V. (2014). Classification and evolution of type II CRISPR-Cas systems. Nucleic Acids Res, 42(10), 6091-6105. doi: 10.1093/nar/gku241.

Cong, L., Ran, F. A., Cox, D., Lin, S., Barretto, R., Habib, N., ... Zhang, F. (2013). Multiplex genome engineering using CRISPR/Cas systems. Science, 339(6121), 819-823. doi: 10.1126/science.1231143.

Deltcheva, E., Chylinski, K., Sharma, C. M., Gonzales, K., Chao, Y., Pirzada, Z. A., . . . Charpentier, E. (2011). CRISPR RNA maturation by trans-encoded small RNA and host factor RNase III. Nature, 471(7340), 602-607. doi: 10.1038/nature09886.

Dianov, G. L., & Hubscher, U. (2013). Mammalian base excision repair: the forgotten archangel. Nucleic Acids Res, 41(6), 3483-3490. doi: 10.1093/nar/gkt076.

Fu, Y., Foden, J. A., Khayter, C., Maeder, M. L., Reyon, D., Joung, J. K., & Sander, J. D. (2013). High-frequency off-target mutagenesis induced by CRISPR-Cas nucleases in human cells. Nat Biotechnol, 31(9), 822-826. doi: 10.1038/nbt.2623.

Garneau, J. E., Dupuis, M. E., Villion, M., Romero, D. A., Barrangou, R., Boyaval, P., ... Moineau, S. (2010). The CRISPR/Cas bacterial immune system cleaves bacteriophage and plasmid DNA. Nature, 468(7320), 67-71. doi: 10.1038/nature09523.

Gasiunas, G., Sinkunas, T., & Siksnys, V. (2014). Molecular mechanisms of CRISPR-mediated microbial immunity. Cell Mol Life Sci, 71(3), 449-465. doi: 10.1007/s00018-013-1438-6.

Hsu, P. D., Lander, E. S., & Zhang, F. (2014). Development and Applications of CRISPR-Cas9 for Genome Engineering. Cell, 157(6), 1262-1278. doi: 10.1016/j.cell.2014.05.010.

Hsu, P. D., Scott, D. A., Weinstein, J. A., Ran, F. A., Konermann, S., Agarwala, V., ... Zhang, F. (2013). DNA targeting specificity of RNA-guided Cas9 nucleases. Nat Biotechnol, 31(9), 827-832. doi: 10.1038/nbt.2647.

Jinek, M., Chylinski, K., Fonfara, I., Hauer, M., Doudna, J. A., & Charpentier, E. (2012). A programmable dual-RNA-guided DNA endonuclease in adaptive bacterial immunity. Science, 337(6096), 816-821. doi: 10.1126/science.1225829.

Klug, A. (2010). The discovery of zinc fingers and their applications in gene regulation and genome manipulation. Annu Rev Biochem, 79, 213-231. doi: 10.1146/annurev-biochem-010909-095056.

Kuscu, C., Arslan, S., Singh, R., Thorpe, J., & Adli, M. (2014). Genome-wide analysis reveals characteristics of off-target sites bound by the Cas9 endonuclease. Nat Biotechnol. doi: 10.1038/nbt.2916.

Mali, P., Aach, J., Stranges, P. B., Esvelt, K. M., Moosburner, M., Kosuri, S., ... Church, G. M. (2013). CAS9 transcriptional activators for target specificity screening and paired nickases for cooperative genome engineering. Nat Biotechnol, 31(9), 833-838. doi: 10.1038/nbt.2675.

Mojica, F. J., Diez-Villasenor, C., Garcia-Martinez, J., & Almendros, C. (2009). Short motif sequences determine the targets of the prokaryotic CRISPR defence system. Microbiology, 155(Pt 3), 733-740. doi: 10.1099/mic.0.023960-0.

Nishimasu, H., Ran, F. A., Hsu, P. D., Konermann, S., Shehata, S. I., Dohmae, N., ... Nureki, O. (2014). Crystal structure of cas9 in complex with guide RNA and target DNA. Cell, 156(5), 935-949. doi: 10.1016/j.cell.2014.02.001.

Pattanayak, V., Lin, S., Guilinger, J. P., Ma, E., Doudna, J. A., & Liu, D. R. (2013). High-throughput profiling of off-target DNA cleavage reveals RNA-programmed Cas9 nuclease specificity. Nat Biotechnol, 31(9), 839-843. doi: 10.1038/nbt.2673.

Ran, F. A., Hsu, P. D., Lin, C. Y., Gootenberg, J. S., Konermann, S., Trevino, A. E., ... Zhang, F. (2013). Double nicking by RNA-guided CRISPR Cas9 for enhanced genome editing specificity. Cell, 154(6), 1380-1389. doi: 10.1016/j.cell.2013.08.021.

Wang, H., Yang, H., Shivalila, C. S., Dawlaty, M. M., Cheng, A. W., Zhang, F., & Jaenisch, R. (2013). One-step generation of mice carrying mutations in multiple genes by CRISPR/Cas-mediated genome engineering. Cell, 153(4), 910-918. doi: 10.1016/j.cell.2013.04.025.

Wiedenheft, B., Sternberg, S. H., & Doudna, J. A. (2012). RNA-guided genetic silencing systems in bacteria and archaea. Nature, 482(7385), 331-338. doi: 10.1038/nature10886.

Wu, X., Scott, D. A., Kriz, A. J., Chiu, A. C., Hsu, P. D., Dadon, D. B., ... Sharp, P. A. (2014). Genome-wide binding of the CRISPR endonuclease Cas9 in mammalian cells. Nat Biotechnol. doi: 10.1038/nbt.2889.

Ran et al., In vivo genome editing using Staphylococcus aureus Cas9, Ran et al, Nature, 1st April 2015 doi:10.1038/nature 14299 (Ran 2015),

**Claims**

1. A non-naturally occurring or engineered composition and a repair template for use in therapy, by manipulation of first and second target sequence on opposite strands of a DNA duplex in a genomic locus of interest in a cell by promoting homology directed repair, wherein the composition comprises:

I. a first CRISPR-Cas system chimeric RNA (chiRNA) polynucleotide sequence, wherein the first polynucleotide sequence comprises:

> (a) a first guide sequence capable of hybridizing to the first target sequence,
> (b) a first tracr mate sequence, and
> (c) a first tracr sequence,

II. a second CRISPR-Cas system chiRNA polynucleotide sequence, wherein the second polynucleotide sequence comprises:

> (a) a second guide sequence capable of hybridizing to the second target sequence,
> (b) a second tracr mate sequence, and
> (c) a second tracr sequence, and

III. a Type II Cas9 enzyme or a polynucleotide sequence encoding it, wherein the Cas9 enzyme is a SpCas9 protein comprising the mutation N863A, or an ortholog thereof, having a mutation corresponding to SpCas9N863A, and comprising zero, or at least one or two or more nuclear localization sequences,

> wherein (a), (b) and (c) are arranged in a 5' to 3' orientation;
> wherein the polynucleotide sequence encoding said Cas9 enzyme is DNA or RNA
> wherein the first guide sequence directs cleavage of one strand of the DNA duplex near the first target sequence and the second guide sequence directs cleavage of the other strand near the second target sequence result in 3' overhangs;
> and wherein the composition further comprises the repair template or wherein the repair template is an exogenous template.

2.  An *ex vivo* method of modifying a cell of an organism by manipulation of a first and a second target sequence on opposite strands of a DNA duplex in a genomic locus of interest in a cell by promoting homology directed repair, comprising

    delivering a non-naturally occurring or engineered composition to the cell comprising:

    I. a first CRISPR-Cas system chimeric RNA (chiRNA) polynucleotide sequence, wherein the first polynucleotide sequence comprises:

    > (a) a first guide sequence capable of hybridizing to the first target sequence,
    > (b) a first tracr mate sequence, and
    > (c) a first tracr sequence,

    II. a second CRISPR-Cas system chiRNA polynucleotide sequence, wherein the second polynucleotide sequence comprises:

    > (a) a second guide sequence capable of hybridizing to the second target sequence,
    > (b) a second tracr mate sequence, and
    > (c) a second tracr sequence, and

    III. a Type II Cas9 enzyme or a polynucleotide sequence encoding it, wherein the Cas9 enzyme is a SpCas9 protein comprising the mutation N863A, or an ortholog thereof, having a mutation corresponding to SpCas9N863A, and comprising zero, at least one or two or more nuclear localization sequences,

    > wherein (a), (b) and (c) are arranged in a 5' to 3' orientation,
    > wherein the polynucleotide sequence encoding said Cas9 enzyme is DNA or RNA,
    > delivering a repair template wherein the repair template is contemporaneously or separately delivered from any or all the Cas9 enzyme, guide, tracr mate or tracr sequences;
    > and wherein the first guide sequence directs cleavage of one strand of the DNA duplex near the first target sequence and the second guide sequence directs cleavage of the other strand near the second target sequence inducing a double strand break, thereby modifying said cell of an organism, and wherein the first guide sequence directs cleavage of one strand of the DNA duplex near the first target sequence and the second guide sequence directs cleavage of the other strand near the second target sequence result in 3' overhangs.

**3.** The *ex vivo* method according to claim 2, wherein the composition further comprises a repair template.

**4.** The *ex vivo* method according to claim 3, wherein the repair template further comprises a restriction endonuclease restriction site.

**5.** The composition for use or the *ex vivo* method according to any one of claims 1 to 4, wherein any or all of the polynucleotide sequence encoding the Cas9 enzyme, the first and the second guide sequence, the first and the second tracr mate sequence or the first and the second tracr sequence, is/are RNA.

**6.** The composition for use or the *ex vivo* method according to any one of claims 1 to 5, wherein the polynucleotides comprising the sequence encoding the Cas9 enzyme, the first and the second guide sequence, the first and the second tracr mate sequence or the first and the second tracr sequence, is/are RNA and are suitable for or are delivered via nanoparticles, exosomes, microvesicles, or a gene-gun.

**7.** The composition for use or the *ex vivo* method according to any one of claims 1 to 6,
wherein any or all of the polynucleotide sequence encoding the Cas9 enzyme, the first and the second guide sequence, the first and the second tracr mate sequence, the first and the second tracr sequence, are suitable for or are delivered via a vector system, wherein the vector system comprises one or more vectors

**8.** The composition for use or the *ex vivo* method according to claim 7, wherein the one or more vectors comprises one or more viral vectors.

**9.** The composition for use or the *ex vivo* method according to claim 8, wherein the viral vector is an AAV viral vector.

**10.** The composition for use or the *ex vivo* method according to any one of claims 1 to 9, wherein the first and second tracr mate sequence share 100% identity and/or wherein the first and second tracr sequence share 100% identity.

**11.** The composition for use or the *ex vivo* method according to any one of claims 1 to 10, wherein the 3' overhang is 1-100 base pairs.

**12.** The composition for use or the *ex vivo* method according to any one of claims 1 to 11, wherein the Cas9 is a mutated *S. aureus* Cas9 comprising the mutation N580A.

**13.** The composition for use according to any one of claims 1, and 5-12 wherein the therapy is a therapy of a genetic disease.

**14.** The composition for use according to claim 13, wherein the genetic disease is an ocular disorder, optionally wherein the ocular disorder is Leber Congenital Amaurosis (LCA).

**Patentansprüche**

**1.** Nicht natürlich vorkommende oder technisch veränderte Zusammensetzung und Reparatur-Template zur Verwendung bei der Therapie, durch Manipulieren der ersten und zweiten Targetsequenz an entgegengesetzten Strängen eines DNA-Doppelstrangs in einem genomischen Locus, der in einer Zelle von Interesse ist, durch Unterstützen homologiegelenkter Reparatur, wobei die Zusammensetzung Folgendes umfasst:

I. eine erste Polynukleotidsequenz einer chimären CRISPR-Cas-System-RNA (chiRNA),
wobei die erste Polynukleotidsequenz Folgendes umfasst:

(a) eine erste Führungssequenz, die in der Lage ist, an die erste Targetsequenz zu hybridisieren,
(b) eine erste tracr-Mate-Sequenz und
(c) eine erste tracr-Sequenz,

II. eine zweite Polynukleotidsequenz einer CRISPR-Cas-System-chiRNA,
wobei die zweite Poynukleotidsequenz Folgendes umfasst:

(a) eine zweite Führungssequenz, die in der Lage ist, an die zweite Targetsequenz zu hybridisieren,
(b) eine zweite tracr-Mate-Sequenz und
(c) eine zweite tracr-Sequenz und

III. ein Typ II-Cas9-Enzym oder eine für dieses kodierende Polynukleotidsequenz, wobei das Cas9-Enzym ein SpCas9-Protein ist, das die Mutation N863A oder ein Orthologon davon umfasst, das eine Mutation aufweist, die SpCas9N863A entspricht und null oder mindestens eine oder zwei oder mehr Kernlokalisierungssequenzen umfasst,

wobei (a), (b) und (c) in einer 5'- nach 3'-Orientierung angeordnet sind,
wobei die Polynukleotidsequenz, die für das Cas9-Enzym kodiert, DNA oder RNA ist,
wobei die erste Führungssequenz die Spaltung eines Strangs des DNA-Doppelstrangs in der Nähe der der ersten Targetsequenz lenkt und die zweite Führungssequenz die Spaltung des anderen Strangs in der Nähe der zweiten Targetsequenz lenkt und in 3'-Überhängen resultieren;
und wobei die Zusammensetzung ferner das Reparatur-Template umfasst oder wobei das Reparatur-Template ein exogenes Template ist.

2. Ex-vivo-Verfahrenen zum Modifizieren einer Zelle eines Organismus durch Manipulieren einer ersten und einer zweiten Targetsequenz an entgegengesetzten Strängen eines DNA-Doppelstrangs in einem genomischen Locus, der in einer Zelle von Interesse ist, durch Unterstützen der homologiegelenkten Reparatur, umfassend das Abgeben einer nicht natürlich vorkommenden oder technisch veränderten Zusammensetzung an die Zelle, umfassend

I. eine erste Polynukleotidsequenz einer chimären CRISPR-Cas-System-RNA (chiRNA),
wobei die erste Polynukleotidsequenz Folgendes umfasst:

(a) eine erste Führungssequenz, die in der Lage ist, an die erste Targetsequenz zu hybridisieren,
(b) eine erste tracr-Mate-Sequenz und
(c) eine erste tracr-Sequenz,

II. eine zweite Polynukleotidsequenz einer CRISPR-Cas-System-chiRNA, wobei die zweite Poynukleotidsequenz Folgendes umfasst:

(a) eine zweite Führungssequenz, die in der Lage ist, an die zweite Targetsequenz zu hybridisieren,
(b) eine zweite tracr-Mate-Sequenz und
(c) eine zweite tracr-Sequenz und

III. ein Typ II-Cas9-Enzym oder eine für dieses kodierende Polynukleotidsequenz, wobei das Cas9-Enzym ein SpCas9-Protein ist, das die Mutation N863A oder ein Orthologon davon umfasst, das eine Mutation aufweist, die SpCass9N863A entspricht und null, mindestens eine oder zwei oder mehr Kernlokalisierungssequenzen umfasst,

wobei (a), (b) und (c ) in einer 5'- nach 3'-Orientierung angeordnet sind,
wobei die Polynukleotidsequenz, die für das Cas9-Enzym kodiert, DNA oder RNA ist,
Abgeben eines Reparatur-Templates, wobei das Reparatur-Template gleichzeitig oder separat von irgendeinem oder allen von dem Cas9-Enzym, der Führungs-, tracr-Mate oder tracr-Sequenzen abgegeben wird;
und wobei die erste Führungssequenz die Spaltung eines Strangs des DNA-Doppelstrangs in der Nähe der der ersten Targetsequenz lenkt und die zweite Führungssequenz die Spaltung des anderen Strangs in der Nähe der zweiten Targetsequenz lenkt, wodurch ein Doppelstrangbruch ausgelöst wird, wodurch die Zelle eines Organismus modifiziert wird,
und wobei die erste Führungssequenz die Spaltung eines Strangs des DNA-Doppelstrangs in der Nähe der ersten Targetsequenz lenkt und die zweite Führungssequenz die Spaltung des anderen Strangs in der Nähe der zweiten Targetsequenz lenkt und in 3'-Überhängen resultieren.

3. Ex vivo-Verfahren nach Anspruch 2, wobei die Zusammensetzung ferner ein Reparatur-Template umfasst.

4. Ex-vivo-Verfahren nach Anspruch 3, wobei das Reparatur-Template ferner eine Restriktionsendonuklease-Restriktionsstelle umfasst.

5. Zusammensetzung zur Verwendung oder Ex-vivo-Verfahren nach einem der Ansprüche 1 bis 4, wobei irgendeine oder alle von der Polynukleotidsequenz, die für das Cas9-Enzym kodiert, der ersten und der zweiten Führungssequenz, der ersten und der zweiten tracr-Mate-Sequenz oder der ersten und der zweiten tracr-Sequenz RNA ist/sind.

6. Zusammensetzung zur Verwendung oder Ex-vivo-Verfahren nach einem der Ansprüche 1 bis 5, wobei die Polynukleotide, die die Sequenz, die für das Cas9-Enzym kodiert, die erste und die zweite Führungssequenz, die erste und die zweite tracr-Mate-Sequenz oder die erste und die zweite tracr-Sequenz umfassen, RNA sind und geeignet sind für oder abgegeben werden über Nanopartikel, Exonome, Mikrovesikel oder eine Genkanone.

7. Zusammensetzung zur Verwendung oder Ex-vivo-Verfahren nach einem der Ansprüche 1 bis 6, wobei irgendeine oder alle von der Polynukleotidsequenz, die für das Cas9-Enzym kodiert, der ersten und der zweiten Führungssequenz, der ersten und der zweiten tracr-Mate-Sequenz, der ersten und der zweiten tracr-Sequenz geeignet sind für oder abgegeben werden über ein Vektorsystem, wobei das Vektorsystem einen oder mehrere Vektoren umfasst.

8. Zusammensetzung zur Verwendung oder Ex-vivo-Verfahren nach Anspruch 7, wobei der eine oder die mehreren Vektoren einen oder mehrere Virusvektoren umfasst/umfassen.

9. Zusammensetzung zur Verwendung oder Ex-vivo-Verfahren nach Anspruch 8, wobei der Virusvektor ein AAV-Virusvektor ist.

10. Zusammensetzung zur Verwendung oder Ex-vivo-Verfahren nach einem der Ansprüche 1 bis 9, wobei die erste und zweite tracr-Mate-Sequenz 100 % Identität gemeinsam haben und/oder wobei die erste und zweite tracr-Sequenz % Identität gemeinsam haben.

11. Zusammensetzung zur Verwendung oder Ex-vivo-Verfahren nach einem der Ansprüche 1 bis 10, wobei der 3'-Überhang 1-100 Basenpaare ist.

12. Zusammensetzung zur Verwendung oder Ex-vivo-Verfahren nach einem der Ansprüche 1 bis 11, wobei das Cas9 ein mutiertes *S. Aureus*-Cas9 ist, das die Mutation N580A umfasst.

13. Zusammensetzung zur Verwendung nach einem der Ansprüche 1 und 5-12, wobei die Therapie eine Therapie für eine genetische Krankheit ist.

14. Zusammensetzung zur Verwendung nach Anspruch 13, wobei die genetische Krankheit ein Augenleiden ist, wobei wahlweise das Augenleiden Leber'sehe kongenitale Amaurose (LCA) ist.

**Revendications**

1. Composition non naturelle ou modifiée et matrice de réparation pour leur utilisation en thérapie, par manipulation d'une première et d'une seconde séquence cible sur des brins opposés d'un ADN duplex dans un locus génomique d'intérêt dans une cellule par promotion de la réparation dirigée par homologie, dans lesquelles la composition comprend :

I. une première séquence polynucléotidique d'ARN chimérique (ARNchi) de système CRISPR-Cas, dans lesquelles la première séquence polynucléotidique comprend :

(a) une première séquence guide capable de s'hybrider à la première séquence cible,
(b) une première séquence d'appariement à tracr, et
(c) une première séquence tracr,

II. une seconde séquence polynucléotidique d'ARNchi de système CRISPR-Cas, dans lesquelles la seconde séquence polynucléotidique comprend :

(a) une seconde séquence guide capable de s'hybrider à la seconde séquence cible,
(b) une seconde séquence d'appariement à tracr, et
(c) une seconde séquence tracr, et

III. une enzyme Cas9 de type II ou une séquence polynucléotidique codant pour celle-ci, dans lesquelles l'enzyme Cas9 est une protéine SpCas9 comprenant la mutation N863A, ou un orthologue de celle-ci, ayant une mutation correspondant à SpCas9N863A, et comprenant zéro, au moins une ou deux ou plus séquences de localisation nucléaire,

dans lesquelles (a), (b) et (c) sont agencées dans une orientation 5' à 3' ;
dans lesquelles la séquence polynucléotidique codant pour ladite enzyme Cas9 est un ADN ou un ARN,
dans lesquelles la première séquence guide dirige le clivage d'un brin de l'ADN duplex à proximité de la première séquence cible et la seconde séquence guide dirige le clivage de l'autre brin à proximité de la seconde séquence cible résultant en des extrémités débordantes en 3' ;
et dans lesquelles la composition comprend en outre la matrice de réparation ou dans lesquelles la matrice de réparation est une matrice exogène.

2. Procédé *ex vivo* de modification d'une cellule d'un organisme par manipulation d'une première et d'une seconde séquence cible sur des brins opposés d'un ADN duplex dans un locus génomique d'intérêt dans une cellule par promotion d'une réparation dirigée par homologie, comprenant
la délivrance d'une composition non naturelle ou modifiée à la cellule comprenant :

I. une première séquence polynucléotidique d'ARN chimérique (ARNchi) de système CRISPR-Cas, dans lequel la première séquence polynucléotidique comprend :

(a) une première séquence guide capable de s'hybrider à la première séquence cible,
(b) une première séquence d'appariement à tracr, et
(c) une première séquence tracr,

II. une seconde séquence polynucléotidique d'ARNchi de système CRISPR-Cas, dans lequel la seconde séquence polynucléotidique comprend :

(a) une seconde séquence guide capable de s'hybrider à la seconde séquence cible,
(b) une seconde séquence d'appariement à tracr, et
(c) une seconde séquence tracr, et

III. une enzyme Cas9 de type II ou une séquence polynucléotidique codant pour celle-ci, dans lequel l'enzyme Cas9 est une protéine SpCas9 comprenant la mutation N863A, ou un orthologue de celle-ci, ayant une mutation correspondant à SpCas9N863A, et comprenant zéro, au moins une ou deux ou plus séquences de localisation nucléaire,

dans lequel (a), (b) et (c) sont agencées dans une orientation 5' à 3' ;
dans lequel la séquence polynucléotidique codant pour ladite enzyme Cas9 est un ADN ou un ARN,
la délivrance d'une matrice de réparation, dans lequel la matrice de réparation est délivrée en même temps ou séparément de l'une quelconque ou la totalité des séquences de l'enzyme Cas9, guide, d'appariement à tracr ou tracr ;
et dans lequel la première séquence guide dirige le clivage d'un brin de l'ADN duplex à proximité de la première séquence cible et la seconde séquence guide dirige le clivage de l'autre brin à proximité de la seconde séquence cible induisant une cassure double-brin, modifiant ainsi ladite cellule d'un organisme, et dans lequel la première séquence guide dirige le clivage d'un brin de l'ADN duplex à proximité de la première séquence cible et la seconde séquence guide dirige le clivage de l'autre brin à proximité de la seconde séquence cible résultant en des extrémités débordantes en 3'.

3. Procédé *ex vivo* selon la revendication 2, dans lequel la composition comprend en outre une matrice de réparation.

4. Procédé *ex vivo* selon la revendication 3, dans lequel la matrice de réparation comprend en outre un site de restriction d'endonucléase de restriction.

5. Composition pour son utilisation ou procédé *ex vivo* selon l'une quelconque des revendications 1 à 4, dans lesquels l'une quelconque ou la totalité de la séquence polynucléotidique codant pour l'enzyme Cas9, de la première et la seconde séquence guide, de la première et la seconde séquence d'appariement à tracr ou de la première et la seconde séquence tracr est/sont de l'ARN.

**6.** Composition pour son utilisation ou procédé *ex vivo* selon l'une quelconque des revendications 1 à 5, dans lesquels les polynucléotides composant la séquence codant pour l'enzyme Cas9, la première et la seconde séquence guide, la première et la seconde séquence d'appariement à tracr ou la première et la seconde séquence tracr, sont de l'ARN et sont aptes à être délivrés via des nanoparticules, des exosomes, des microvésicules, ou un canon à gènes.

**7.** Composition pour son utilisation ou procédé *ex vivo* selon l'une quelconque des revendications 1 à 6, dans lesquels l'une quelconque ou la totalité de la séquence polynucléotidique codant pour l'enzyme Cas9, de la première et la seconde séquence guide, de la première et la seconde séquence d'appariement à tracr, de la première et la seconde séquence tracr, sont aptes à être délivrées via un système de vecteur, dans lesquels le système de vecteur comprend un ou plusieurs vecteurs.

**8.** Composition pour son utilisation ou procédé *ex vivo* selon la revendication 7, dans lesquels les un ou plusieurs vecteurs comprennent un ou plusieurs vecteurs viraux.

**9.** Composition pour son utilisation ou procédé *ex vivo* selon la revendication 8, dans lesquels le vecteur viral est un vecteur viral AAV.

**10.** Composition pour son utilisation ou procédé *ex vivo* selon l'une quelconque des revendications 1 à 9, dans lesquels la première et la seconde séquence d'appariement à tracr partagent une identité de 100 % et/ou dans lesquels la première et la seconde séquence tracr partagent une identité de 100 %.

**11.** Composition pour son utilisation ou procédé *ex vivo* selon l'une quelconque des revendications 1 à 10, dans lesquels l'extrémité débordante en 3' est constituée de 1 à 100 paires de bases.

**12.** Composition pour son utilisation selon l'une quelconque des revendications 1 à 11, dans laquelle la Cas9 est une Cas9 de *S. aureus* mutée comprenant la mutation N580A.

**13.** Composition pour son utilisation selon l'une quelconque des revendications 1 et 5 à 12 dans laquelle la thérapie est une thérapie d'une maladie génétique.

**14.** Composition pour son utilisation selon la revendication 13, dans laquelle la maladie génétique est une maladie oculaire, facultativement dans laquelle la maladie oculaire est l'amaurose congénitale de Leber (ACL).

Figure 1

**A**

human EXM1 locus ├ ------ 23 bp offset ------ ┤

sgRNA 1      PAM

5' . . ACCGGAGGACAAAGTACAAACGGCAGAAGCTGGAGGAGGAAGGGCCT*GAGTCCGAGCAGAAGAAGAA*GGGC .

    | | | |     | | | |

3' . . TGGC*CTCCTGTTTCATGTTTGCCG*TCTTCGACCTCCTCCTTCCCGGACTCAGGCTCGTCTTCTTCTTCCCG .

    PAM      sgRNA 2

known off-target sites for sgRNA 1

off-target (OT)-1 GAG**G**CCGAGCAGAAGAA**A**GACGG

off-target (OT)-2 **A**AGTC**T**GAGCA**C**AAGAAGAATGG

off-target (OT)-3 GAGTCC**T**AGCAG**G**AGAAGAAG**A**G

off-target (OT)-4 GAGTC**TA**AGCAGAAGAAGAAG**A**G

off-target (OT)-5 GAGT**TA**GAGCAGAAGAAGAAAGG

**B**

EMX1 on-target cleavage

| Cas9 WT | – | – | – | + | + | – |
|---|---|---|---|---|---|---|
| Cas9n | + | + | + | – | – | – |
| sgRNA 2 | + | + | – | + | – | – |
| sgRNA 1 | + | – | + | – | + | – |

Indel (%)    24.1        25.0   32.1

**C**

EMX1 off-target cleavage

☐ Cas9 WT / sgRNA 1
▨ Cas9n / sgRNA 1 + 2

Indel frequency (%)

OT-1   OT-2   OT-3   OT-4   OT-5

Known sgRNA1 off-target sites

Figure 2

Figure 3

116

EP 3 686 279 B1

Figure 4A

117

Figure 4B

Figure 4C

Figure 4D

S. pyogenes ▼

S. thermophilus
CRISPR1

S. thermophilus
CRISPR3

Figure 4E

gi|152978060|ref|YP_001343689.1| , CRISPR-associated endonuclease Csn1 family protein [Actinobacillus succinogenes 130Z]

gi|307256472|ref|ZP_07538254.1| , CRISPR-associated protein, Csn1 family [Actinobacillus pleuropneumoniae serovar 10 str. D130...

gi|345430422|ref|YP_004823543.1| , hypothetical protein PARA_18570 [Haemophilus parainfluenzae T3T1]

gi|240949037|ref|ZP_04753391.1| , CRISPR-associated endonuclease Csn1 family protein [Actinobacillus minor NM305]

gi|15602992|ref|NP_246064.1| , hypothetical protein PM1127 [Pasteurella multocida subsp. multocida str. Pm70]

gi|333374624|ref|ZP_08466464.1| , CRISPR-associated endonuclease Csn1 family protein [Kingella kingae ATCC 23330]

gi|329117879|ref|ZP_08246593.1| , hypothetical protein HMPREF9123_0020 [Neisseria bacilliformis ATCC BAA-1200]

gi|218767588|ref|YP_002342100.1| , hypothetical protein NMA0631 [Neisseria meningitidis Z2491]

gi|254804356|ref|YP_003082577.1| , putative CRISPR-associated protein [Neisseria meningitidis alpha14]

gi|161869390|ref|YP_001598557.1| , hypothetical protein NMCC_0397 [Neisseria meningitidis 053442]

gi|261378287|ref|ZP_05982860.1| , CRISPR-associated protein, Csn1 family [Neisseria cinerea ATCC 14685]

gi|313669044|ref|YP_004049328.1| , hypothetical protein NLA_17660 [Neisseria lactamica 020-06]

gi|241759613|ref|ZP_04757714.1| , CRISPR-associated protein, Csn1 family [Neisseria flavescens SK114]

gi|404379108|ref|ZP_10984177.1| , CRISPR-associated protein cas9/csn1, subtype II/nmemi [Simonsiella muelleri ATCC 29453]

gi|222109285|ref|YP_002551549.1| , crispr-associated protein, csn1 family [Acidovorax ebreus TPSY]

gi|312879015|ref|ZP_07738815.1| , CRISPR-associated protein, Csn1 family [Aminomonas paucivorans DSM 12260]

gi|90425961|ref|YP_534331.1| , hypothetical protein RPC_4489 [Rhodopseudomonas palustris BisB18]

gi|91975509|ref|YP_568168.1| , CRISPR-associated Cas5e family protein [Rhodopseudomonas palustris BisB5]

gi|296446027|ref|ZP_06887976.1| , CRISPR-associated protein, Csn1 family [Methylosinus trichosporium OB3b]

gi|323139312|ref|ZP_08074365.1| , CRISPR-associated protein, Csn1 family [Methylocystis sp. ATCC 49242]

gi|162147907|ref|YP_001602368.1| , hypothetical protein GDI_2123 [Gluconacetobacter diazotrophicus PAl 5]

gi|209542524|ref|YP_002274753.1| , Csn1 family CRISPR-associated protein [Gluconacetobacter diazotrophicus PAl 5]

gi|182624245|ref|ZP_02952031.1| , crispr-associated protein, Csn1 family [Clostridium perfringens D str. JGS1721]

gi|229113166|ref|ZP_04242662.1| , Crispr-associated protein, Csn1 [Bacillus cereus Rock1-15]

gi|225377804|ref|ZP_03755025.1| , hypothetical protein ROSEINA2194_03455 [Roseburia inulinivorans DSM 16841]

gi|257413184|ref|ZP_04742247.2| , CRISPR-associated protein, Csn1 family [Roseburia intestinalis L1-82]

gi|323142435|ref|ZP_08077256.1| , CRISPR-associated protein, Csn1 family [Phascolarctobacterium succinatutens YIT 12067]

gi|310780384|ref|YP_003968716.1| , CRISPR-associated protein, Csn1 family [Ilyobacter polytropus DSM 2926]

gi|336393381|ref|ZP_08574780.1| , CRISPR-associated protein, Csn1 family [Lactobacillus coryniformis subsp. torquens KCTC 3535...

gi|189485225|ref|YP_001956166.1| , CRISPR-associated protein Csn1 [uncultured Termite group 1 bacterium phylotype Rs-D17]

gi|220930482|ref|YP_002507391.1| , CRISPR-associated protein, Csn1 family [Clostridium cellulolyticum H10]

gi|157415744|ref|YP_001483000.1| , hypothetical protein C8J_1425 [Campylobacter jejuni subsp. jejuni 81116]

gi|218563121|ref|YP_002344900.1| , CRISPR-associated protein [Campylobacter jejuni subsp. jejuni NCTC 11168 = ATCC 700819]

gi|153952471|ref|YP_001398821.1| , CRISPR-associated Cas5e family protein [Campylobacter jejuni subsp. doylei 269.97]

gi|313144862|ref|ZP_07807055.1| , crispr-protein [Helicobacter cinaedi CCUG 18818]

gi|253828136|ref|ZP_04871021.1| , crispr-associated protein, Csn1 family [Helicobacter canadensis MIT 98-5491]

gi|291276265|ref|YP_003516037.1| , CRISPR associated protein [Helicobacter mustelae 12198]

gi|34557790|ref|NP_907605.1| , hypothetical protein WS1445 [Wolinella succinogenes DSM 1740]

EP 3 686 279 B1

Figure 5A

Figure 5B

gi|148255343|ref|YP_001239928.1| , hypothetical protein BBta_3952 [Bradyrhizobium sp. BTAi1]

gi|288957741|ref|YP_003448082.1| , CRISPR-associated protein, Csn1 family [Azospirillum sp. B510]

gi|159042956|ref|YP_001531750.1| , CRISPR-associated protein [Dinoroseobacter shibae DFL 12]

gi|332188827|ref|ZP_08390536.1| , hypothetical protein SUS17_3938 [Sphingomonas sp. S17]

gi|319760940|ref|YP_004124877.1| , crispr-associated protein, csn1 family [Alicycliphilus denitrificans BC]

gi|330822845|ref|YP_004386148.1| , CRISPR-associated protein, Csn1 family [Alicycliphilus denitrificans K601]

gi|326315085|ref|YP_004232757.1| , CRISPR-associated protein, Csn1 family [Acidovorax avenae subsp. avenae ATCC 19860]

gi|121608211|ref|YP_996018.1| , CRISPR-associated endonuclease Csn1 family protein [Verminephrobacter eiseniae EF01-2]

gi|325983496|ref|YP_004295898.1| , CRISPR-associated protein, Csn1 family [Nitrosomonas sp. AL212]

gi|304313029|ref|YP_003812627.1| , hypothetical protein HDN1F_34120 [gamma proteobacterium HdN1]

gi|344171927|emb|CCA84553.1| , conserved hypothetical protein [Ralstonia syzygii R24]

gi|294086111|ref|YP_003552871.1| , CRISPR-associated protein, Csn1 family [Candidatus Puniceispirillum marinum IMCC1322]

gi|187250660|ref|YP_001875142.1| , CRISPR-associated endonuclease Csn1 family protein [Elusimicrobium minutum Pei191]

gi|189485059|ref|YP_001956000.1| , Csn1-like CRISPR-associated protein [uncultured Termite group 1 bacterium phylotype Rs-D17]

gi|325972003|ref|YP_004248194.1| , CRISPR-associated protein, Csn1 family [Sphaerochaeta globus str. Buddy]

gi|282878504|ref|ZP_06287286.1| , CRISPR-associated protein, Csn1 family [Prevotella buccalis ATCC 35310]

gi|282880052|ref|ZP_06288774.1| , CRISPR-associated protein, Csn1 family [Prevotella timonensis CRIS 5C-B1]

gi|258648111|ref|ZP_05735580.1| , CRISPR-associated protein, Csn1 family [Prevotella tannerae ATCC 51259]

gi|294674019|ref|YP_003574635.1| , Csn1 family CRISPR-associated protein [Prevotella ruminicola 23]

gi|347536497|ref|YP_004843922.1| , putative CRISPR-associated (Cas) protein [Flavobacterium branchiophilum FL-15]

gi|365959402|ref|YP_004940969.1| , putative CRISPR-associated (Cas) protein [Flavobacterium columnare ATCC 49512]

gi|218252638|ref|ZP_03474966.1| , hypothetical protein PRABACTJOHN_00621 [Parabacteroides johnsonii DSM 18315]

gi|256840409|ref|ZP_05545917.1| , CRISPR-associated protein [Parabacteroides sp. D13]

gi|224535832|ref|ZP_03676371.1| , hypothetical protein BACCELL_00696 [Bacteroides cellulosilyticus DSM 14838]

gi|60683389|ref|YP_213533.1| , hypothetical protein BF3954 [Bacteroides fragilis NCTC 9343]

gi|323344874|ref|ZP_08085098.1| , csn1 family CRISPR-associated protein [Prevotella oralis ATCC 33269]

gi|213962376|ref|ZP_03390639.1| , crispr-associated protein, Csn1 family [Capnocytophaga sputigena Capno]

gi|256819408|ref|YP_003140687.1| , CRISPR-associated protein, Csn1 family [Capnocytophaga ochracea DSM 7271]

gi|298373376|ref|ZP_06983365.1| , CRISPR-associated protein, Csn1 family [Bacteroidetes oral taxon 274 str. F0058]

gi|340622236|ref|YP_004740688.1| , hypothetical protein Ccan_14650 [Capnocytophaga canimorsus Cc5]

gi|163754820|ref|ZP_02161941.1| , hypothetical protein KAOT1_02362 [Kordia algicida OT-1]

gi|295136244|ref|YP_003586920.1| , hypothetical protein ZPR_4422 [Zunongwangia profunda SM-A87]

gi|150025575|ref|YP_001296401.1| , CRISPR-associated endonuclease Csn1 family protein [Flavobacterium psychrophilum JIP02/86]

gi|212694363|ref|ZP_03302491.1| , hypothetical protein BACDOR_03889 [Bacteroides dorei DSM 17855]

gi|301311869|ref|ZP_07217791.1| , conserved hypothetical protein [Bacteroides sp. 20_3]

gi|224026357|ref|ZP_03644723.1| , hypothetical protein BACCOPRO_03113 [Bacteroides coprophilus DSM 18228]

gi|261414553|ref|YP_003248236.1| , CRISPR-associated protein, Csn1 family [Fibrobacter succinogenes subsp. succinogenes S85]

gi|260592128|ref|ZP_05857586.1| , CRISPR-associated protein, Csn1 family [Prevotella veroralis F0319]

gi|288802595|ref|ZP_06408034.1| , conserved hypothetical protein [Prevotella melaninogenica D18]

gi|315607525|ref|ZP_07882520.1| , csn1 family CRISPR-associated protein [Prevotella buccae ATCC 33574]

gi|282858617|ref|ZP_06267779.1| , CRISPR-associated protein, Csn1 family [Prevotella bivia JCVIHMP010]

# Figure 5C

Figure 5D

Figure 5E

gi|22537057|ref|NP_687908.1|, hypothetical protein SAG0894 [Streptococcus agalactiae 2603V/R]

gi|76788458|ref|YP_329639.1|, hypothetical protein SAK_1017 [Streptococcus agalactiae A909]

gi|25010965|ref|NP_735360.1|, hypothetical protein gbs0911 [Streptococcus agalactiae NEM316]

gi|24379809|ref|NP_721764.1|, hypothetical protein SMU_1405c [Streptococcus mutans UA159]

gi|290580220|ref|YP_003484612.1|, hypothetical protein SmuNN2025_0694 [Streptococcus mutans NN2025]

gi|306833855|ref|ZP_07466980.1|, csn1 family CRISPR-associated protein [Streptococcus bovis ATCC 700338]

gi|325978669|ref|YP_004288385.1|, CRISPR-associated protein [Streptococcus gallolyticus subsp. gallolyticus ATCC BAA-2069]

gi|288905639|ref|YP_003430861.1|, CRISPR-associated protein [Streptococcus gallolyticus UCN34]

gi|320547102|ref|ZP_08041398.1|, csn1 family CRISPR-associated protein [Streptococcus equinus ATCC 9812]

gi|315223162|ref|ZP_07865023.1|, CRISPR-associated protein, Csn1 family [Streptococcus anginosus F0211]

gi|357636406|ref|ZP_09134281.1|, CRISPR-associated protein Cas9/Csn1, subtype II/NMEMI [Streptococcus macacae NCTC 11558]

gi|313890160|ref|ZP_07823795.1|, CRISPR-associated protein, Csn1 family [Streptococcus pseudoporcinus SPIN 20026]

gi|195978435|ref|YP_002123679.1| CRISPR-Associated Protein Csn1 [Streptococcus equi subsp. zooepidemicus MGCS10565]

gi|94994317|ref|YP_602415.1|, hypothetical protein MGAS10750_Spy0921 [Streptococcus pyogenes MGAS10750]

gi|251782637|ref|YP_002996940.1|, hypothetical protein SDEG_1231 [Streptococcus dysgalactiae subsp. equisimilis GGS_124]

gi|21910213|ref|NP_664481.1|, hypothetical protein SpyM3_0677 [Streptococcus pyogenes MGAS315]

gi|28896088|ref|NP_802438.1|, hypothetical protein SPs1176 [Streptococcus pyogenes SSI-1]

gi|209559356|ref|YP_002285828.1|, hypothetical protein Spy49_0823 [Streptococcus pyogenes NZ131]

gi|15675041|ref|NP_269215.1|, hypothetical protein SPy_1046 [Streptococcus pyogenes M1 GAS]

gi|71910582|ref|YP_282132.1|, hypothetical protein M5005_Spy_0769 [Streptococcus pyogenes MGAS5005]

gi|94990395|ref|YP_598495.1|, putative cytoplasmic protein [Streptococcus pyogenes MGAS10270]

gi|94988516|ref|YP_596617.1|, cytoplasmic protein [Streptococcus pyogenes MGAS9429]

gi|94992340|ref|YP_600439.1|, putative cytoplasmic protein [Streptococcus pyogenes MGAS2096]

gi|71903413|ref|YP_280216.1|, cytoplasmic protein [Streptococcus pyogenes MGAS6180]

Figure 5F

Figure 6

SpCas9 mutation positions

**hSpCas9**

5' ATGGACAAGAAGTACAGCATCGGCCTGGACATCGGCACCAACTCTGTGGGCTGGGCCGTG 60

hSpCas9

D10

RevCl

| M | D | K | K | Y | S | I | G | L | D | I | G | T | N | S | V | G | W | A | V |
|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 |

5' ATCACCGACGAGTACAAGGTGCCCAGCAAGAAATTCAAGGTGCTGGGCAACACCGACCGG 120

hSpCas9

R...l

| I | T | D | E | Y | K | V | P | S | K | K | F | K | V | L | G | N | T | D | R |
|---|---|---|---|---|---|---|---|---|---|---|---|---|---|---|---|---|---|---|---|
| 21 | 22 | 23 | 24 | 25 | 26 | 27 | 28 | 29 | 30 | 31 | 32 | 33 | 34 | 35 | 36 | 37 | 38 | 39 | 40 |

5' CACAGCATCAAGAAGAACCTGATCGGAGCCCTGCTGTTCGACAGCGGCGAAACAGCCGAG 180

hSpCas9

| H | S | I | K | K | N | L | I | G | A | L | L | F | D | S | G | E | T | A | E |
|---|---|---|---|---|---|---|---|---|---|---|---|---|---|---|---|---|---|---|---|
| 41 | 42 | 43 | 44 | 45 | 46 | 47 | 48 | 49 | 50 | 51 | 52 | 53 | 54 | 55 | 56 | 57 | 58 | 59 | 60 |

5' GCCACCCGGCTGAAGAGAACCGCCAGAAGAAGATACACCAGACGGAAGAACCGGATCTGC 240

hSpCas9

| A | T | R | L | K | R | T | A | R | R | R | Y | T | R | R | K | N | R | I | C |
|---|---|---|---|---|---|---|---|---|---|---|---|---|---|---|---|---|---|---|---|
| 61 | 62 | 63 | 64 | 65 | 66 | 67 | 68 | 69 | 70 | 71 | 72 | 73 | 74 | 75 | 76 | 77 | 78 | 79 | 80 |

5' TATCTGCAAGAGATCTTCAGCAACGAGATGGCCAAGGTGGACGACAGCTTCTTCCACAGA 300

hSpCas9

| Y | L | Q | E | I | F | S | N | E | M | A | K | V | D | D | S | F | F | H | R |
|---|---|---|---|---|---|---|---|---|---|---|---|---|---|---|---|---|---|---|---|
| 81 | 82 | 83 | 84 | 85 | 86 | 87 | 88 | 89 | 90 | 91 | 92 | 93 | 94 | 95 | 96 | 97 | 98 | 99 | 100 |

# Figure 7A

hSpCas9

5'  CTGGAAGAGTCCTTCCTGGTGGAAGAGGATAAGAAGCACGAGCGGCACCCCATCTTCGGC
                                                                    360
    hSpCas9
    L   E   E   S   F   L   V   E   E   D   K   K   H   E   R   H   P   I   F   G
    101 102 103 104 105 106 107 108 109 110 111 112 113 114 115 116 117 118 119 120

5'  AACATCGTGGACGAGGTGGCCTACCACGAGAAGTACCCCACCATCTACCACCTGAGAAAG
                                                                    420
    hSpCas9
    N   I   V   D   E   V   A   Y   H   E   K   Y   P   T   I   Y   H   L   R   K
    121 122 123 124 125 126 127 128 129 130 131 132 133 134 135 136 137 138 139 140

5'  AAACTGGTGGACAGCACCGACAAGGCCGACCTGCGGCTGATCTATCTGGCCCTGGCCCAC
                                                                    480
    hSpCas9
    K   L   V   D   S   T   D   K   A   D   L   R   L   I   Y   L   A   L   A   H
    141 142 143 144 145 146 147 148 149 150 151 152 153 154 155 156 157 158 159 160

5'  ATGATCAAGTTCCGGGGCCACTTCCTGATCGAGGGCGACCTGAACCCCGACAACAGCGAC
                                                                    540
    hSpCas9
    M   I   K   F   R   G   H   F   L   I   E   G   D   L   N   P   D   N   S   D
    161 162 163 164 165 166 167 168 169 170 171 172 173 174 175 176 177 178 179 180

5'  GTGGACAAGCTGTTCATCCAGCTGGTGCAGACCTACAACCAGCTGTTCGAGGAAAACCCC
                                                                    600
    hSpCas9
    V   D   K   L   F   I   Q   L   V   Q   T   Y   N   Q   L   F   E   E   N   P
    181 182 183 184 185 186 187 188 189 190 191 192 193 194 195 196 197 198 199 200

5'  ATCAACGCCAGCGGCGTGGACGCCAAGGCCATCCTGTCTGCCAGACTGAGCAAGAGCAGA
                                                                    660
    hSpCas9
    I   N   A   S   G   V   D   A   K   A   I   L   S   A   R   L   S   K   S   R
    201 202 203 204 205 206 207 208 209 210 211 212 213 214 215 216 217 218 219 220

# Figure 7B

**hSpCas9**

```
5'   CGGCTGGAAAATCTGATCGCCCAGCTGCCCGGCGAGAAGAAGAATGGCCTGTTCGGCAAC
     +++-|-+++-|-+++-|-+++-|-+++-|-+++-|-+++-|-+++-|-+++-|-+++-|-+++-|   720
     [hSpCas9]
     R   L   E   N   L   I   A   Q   L   P   G   E   K   K   N   G   L   F   G   N
    221 222 223 224 225 226 227 228 229 230 231 232 233 234 235 236 237 238 239 240
```

```
5'   CTGATTGCCCTGAGCCTGGGCCTGACCCCCAACTTCAAGAGCAACTTCGACCTGGCCGAG
     +++-|-+++-|-+++-|-+++-|-+++-|-+++-|-+++-|-+++-|-+++-|-+++-|-+++-|   780
     [hSpCas9]
     L   I   A   L   S   L   G   L   T   P   N   F   K   S   N   F   D   L   A   E
    241 242 243 244 245 246 247 248 249 250 251 252 253 254 255 256 257 258 259 260
```

```
5'   GATGCCAAACTGCAGCTGAGCAAGGACACCTACGACGACGACCTGGACAACCTGCTGGCC
     +++-|-+++-|-+++-|-+++-|-+++-|-+++-|-+++-|-+++-|-+++-|-+++-|-+++-|   840
     [hSpCas9]
     D   A   K   L   Q   L   S   K   D   T   Y   D   D   D   L   D   N   L   L   A
    261 262 263 264 265 266 267 268 269 270 271 272 273 274 275 276 277 278 279 280
```

```
5'   CAGATCGGCGACCAGTACGCCGACCTGTTTCTGGCCGCCAAGAACCTGTCCGACGCCATC
     +++-|-+++-|-+++-|-+++-|-+++-|-+++-|-+++-|-+++-|-+++-|-+++-|-+++-|   900
     [hSpCas9]
     Q   I   G   D   Q   Y   A   D   L   F   L   A   A   K   N   L   S   D   A   I
    281 282 283 284 285 286 287 288 289 290 291 292 293 294 295 296 297 298 299 300
```

```
5'   CTGCTGAGCGACATCCTGAGAGTGAACACCGAGATCACCAAGGCCCCCCCTGAGCGCCTCT
     +++-|-+++-|-+++-|-+++-|-+++-|-+++-|-+++-|-+++-|-+++-|-+++-|-+++-|   960
     [hSpCas9]
     L   L   S   D   I   L   R   V   N   T   E   I   T   K   A   P   L   S   A   S
    301 302 303 304 305 306 307 308 309 310 311 312 313 314 315 316 317 318 319 320
```

```
5'   ATGATCAAGAGATACGACGAGCACCACCAGGACCTGACCCTGCTGAAAGCTCTCGTGCGG
     +++-|-+++-|-+++-|-+++-|-+++-|-+++-|-+++-|-+++-|-+++-|-+++-|-+++-|   1020
     [hSpCas9]
     M   I   K   R   Y   D   E   H   H   Q   D   L   T   L   L   K   A   L   V   R
    321 322 323 324 325 326 327 328 329 330 331 332 333 334 335 336 337 338 339 340
```

# Figure 7C

hSpCas9

5' CAGCAGCTGCCTGAGAAGTACAAAGAGATTTTCTTCGACCAGAGCAAGAACGGCTACGCC

1080

hSpCas9

| Q | Q | L | P | E | K | Y | K | E | I | F | F | D | Q | S | K | N | G | Y | A |
|---|---|---|---|---|---|---|---|---|---|---|---|---|---|---|---|---|---|---|---|
| 341 | 342 | 343 | 344 | 345 | 346 | 347 | 348 | 349 | 350 | 351 | 352 | 353 | 354 | 355 | 356 | 357 | 358 | 359 | 360 |

5' GGCTACATTGACGGCGGAGCCAGCCAGGAAGAGTTCTACAAGTTCATCAAGCCCATCCTG

1140

hSpCas9

| G | Y | I | D | G | G | A | S | Q | E | E | F | Y | K | F | I | K | P | I | L |
|---|---|---|---|---|---|---|---|---|---|---|---|---|---|---|---|---|---|---|---|
| 361 | 362 | 363 | 364 | 365 | 366 | 367 | 368 | 369 | 370 | 371 | 372 | 373 | 374 | 375 | 376 | 377 | 378 | 379 | 380 |

5' GAAAAGATGGACGGCACCGAGGAACTGCTCGTGAAGCTGAACAGAGAGGACCTGCTGCGG

1200

hSpCas9

| E | K | M | D | G | T | E | E | L | L | V | K | L | N | R | E | D | L | L | R |
|---|---|---|---|---|---|---|---|---|---|---|---|---|---|---|---|---|---|---|---|
| 381 | 382 | 383 | 384 | 385 | 386 | 387 | 388 | 389 | 390 | 391 | 392 | 393 | 394 | 395 | 396 | 397 | 398 | 399 | 400 |

5' AAGCAGCGGACCTTCGACAACGGCAGCATCCCCCACCAGATCCACCTGGGAGAGCTGCAC

1260

hSpCas9

| K | Q | R | T | F | D | N | G | S | I | P | H | Q | I | H | L | G | E | L | H |
|---|---|---|---|---|---|---|---|---|---|---|---|---|---|---|---|---|---|---|---|
| 401 | 402 | 403 | 404 | 405 | 406 | 407 | 408 | 409 | 410 | 411 | 412 | 413 | 414 | 415 | 416 | 417 | 418 | 419 | 420 |

5' GCCATTCTGCGGCGGCAGGAAGATTTTTACCCATTCCTGAAGGACAACCGGGAAAAGATC

1320

hSpCas9

| A | I | L | R | R | Q | E | D | F | Y | P | F | L | K | D | N | R | E | K | I |
|---|---|---|---|---|---|---|---|---|---|---|---|---|---|---|---|---|---|---|---|
| 421 | 422 | 423 | 424 | 425 | 426 | 427 | 428 | 429 | 430 | 431 | 432 | 433 | 434 | 435 | 436 | 437 | 438 | 439 | 440 |

5' GAGAAGATCCTGACCTTCCGCATCCCCTACTACGTGGGCCCTCTGGCCAGGGGAAACAGC

1380

hSpCas9

| E | K | I | L | T | F | R | I | P | Y | Y | V | G | P | L | A | R | G | N | S |
|---|---|---|---|---|---|---|---|---|---|---|---|---|---|---|---|---|---|---|---|
| 441 | 442 | 443 | 444 | 445 | 446 | 447 | 448 | 449 | 450 | 451 | 452 | 453 | 454 | 455 | 456 | 457 | 458 | 459 | 460 |

# Figure 7D

**hSpCas9**

```
5'   AGATTCGCCTGGATGACCAGAAAGAGCGAGGAAACCATCACCCCCTGGAACTTCGAGGAA
     ++++|++++|++++|++++|++++|++++|++++|++++|++++|++++|++++|++++|   1440
                                 hSpCas9
      R   F   A   W   M   T   R   K   S   E   E   I   T   P   W   N   F   E   E
     461 462 463 464 465 466 467 468 469 470 471 472 473 474 475 476 477 478 479 480
```

```
5'   GTGGTGGACAAGGGCGCTTCCGCCCAGAGCTTCATCGAGCGGATGACCAACTTCGATAAG
     ++++|++++|++++|++++|++++|++++|++++|++++|++++|++++|++++|++++|   1500
                                 hSpCas9
      V   V   D   K   G   A   S   A   Q   S   F   I   E   R   M   T   N   F   D   K
     481 482 483 484 485 486 487 488 489 490 491 492 493 494 495 496 497 498 499 500
```

```
5'   AACCTGCCCAACGAGAAGGTGCTGCCCAAGCACAGCCTGCTGTACGAGTACTTCACCGTG
     ++++|++++|++++|++++|++++|++++|++++|++++|++++|++++|++++|++++|   1560
                                 hSpCas9
      N   L   P   N   E   K   V   L   P   K   H   S   L   L   Y   E   Y   F   T   V
     501 502 503 504 505 506 507 508 509 510 511 512 513 514 515 516 517 518 519 520
```

```
5'   TATAACGAGCTGACCAAAGTGAAATACGTGACCGAGGGAATGAGAAAGCCCGCCTTCCTG
     ++++|++++|++++|++++|++++|++++|++++|++++|++++|++++|++++|++++|   1620
                                 hSpCas9
      Y   N   E   L   T   K   V   K   Y   V   T   E   G   M   R   K   P   A   F   L
     521 522 523 524 525 526 527 528 529 530 531 532 533 534 535 536 537 538 539 540
```

```
5'   AGCGGCGAGCAGAAAAAGGCCATCGTGGACCTGCTGTTCAAGACCAACCGGAAAGTGACC
     ++++|++++|++++|++++|++++|++++|++++|++++|++++|++++|++++|++++|   1680
                                 hSpCas9
      S   G   E   Q   K   K   A   I   V   D   L   L   F   K   T   N   R   K   V   T
     541 542 543 544 545 546 547 548 549 550 551 552 553 554 555 556 557 558 559 560
```

```
5'   GTGAAGCAGCTGAAAGAGGACTACTTCAAGAAAATCGAGTGCTTCGACTCCGTGGAAATC
     ++++|++++|++++|++++|++++|++++|++++|++++|++++|++++|++++|++++|   1740
                                 hSpCas9
      V   K   Q   L   K   E   D   Y   F   K   K   I   E   C   F   D   S   V   E   I
     561 562 563 564 565 566 567 568 569 570 571 572 573 574 575 576 577 578 579 580
```

# Figure 7E

hSpCas9

5' TCCGGCGTGGAAGATCGGTTCAACGCCTCCCTGGGCACATACCACGATCTGCTGAAAATT
1800
hSpCas9
S   G   V   E   D   R   F   N   A   S   L   G   T   Y   H   D   L   L   K   I
581 582 583 584 585 586 587 588 589 590 591 592 593 594 595 596 597 598 599 600

5' ATCAAGGACAAGGACTTCCTGGACAATGAGGAAAACGAGGACATTCTGGAAGATATCGTG
1860
hSpCas9
I   K   D   K   D   F   L   D   N   E   E   N   E   D   I   L   E   D   I   V
601 602 603 604 605 606 607 608 609 610 611 612 613 614 615 616 617 618 619 620

5' CTGACCCTGACACTGTTTGAGGACAGAGAGATGATCGAGGAACGGCTGAAAACCTATGCC
1920
hSpCas9
L   T   L   T   L   F   E   D   R   E   M   I   E   E   R   L   K   T   Y   A
621 622 623 624 625 626 627 628 629 630 631 632 633 634 635 636 637 638 639 640

5' CACCTGTTCGACGACAAAGTGATGAAGCAGCTGAAGCGGCGGAGATACACCGGCTGGGGC
1980
hSpCas9
H   L   F   D   D   K   V   M   K   Q   L   K   R   R   R   Y   T   G   W   G
641 642 643 644 645 646 647 648 649 650 651 652 653 654 655 656 657 658 659 660

5' AGGCTGAGCCGGAAGCTGATCAACGGCATCCGGGACAAGCAGTCCGGCAAGACAATCCTG
2040
hSpCas9
R   L   S   R   K   L   I   N   G   I   R   D   K   Q   S   G   K   T   I   L
661 662 663 664 665 666 667 668 669 670 671 672 673 674 675 676 677 678 679 680

5' GATTTCCTGAAGTCCGACGGCTTCGCCAACAGAAACTTCATGCAGCTGATCCACGACGAC
2100
hSpCas9
D   F   L   K   S   D   G   F   A   N   R   N   F   M   Q   L   I   H   D   D
681 682 683 684 685 686 687 688 689 690 691 692 693 694 695 696 697 698 699 700

# Figure 7F

**hSpCas9**

5' AGCCTGACCTTTAAAGAGGACATCCAGAAAGCCCAGGTGTCCGGCCAGGGCGATAGCCTG  
2160

hSpCas9

| S | L | T | F | K | E | D | I | Q | K | A | Q | V | S | G | Q | G | D | S | L |
|---|---|---|---|---|---|---|---|---|---|---|---|---|---|---|---|---|---|---|---|
| 701 | 702 | 703 | 704 | 705 | 706 | 707 | 708 | 709 | 710 | 711 | 712 | 713 | 714 | 715 | 716 | 717 | 718 | 719 | 720 |

5' CACGAGCACATTGCCAATCTGGCCGGCAGCCCCGCCATTAAGAAGGGCATCCTGCAGACA  
2220

hSpCas9

| H | E | H | I | A | N | L | A | G | S | P | A | I | K | K | G | I | L | Q | T |
|---|---|---|---|---|---|---|---|---|---|---|---|---|---|---|---|---|---|---|---|
| 721 | 722 | 723 | 724 | 725 | 726 | 727 | 728 | 729 | 730 | 731 | 732 | 733 | 734 | 735 | 736 | 737 | 738 | 739 | 740 |

5' GTGAAGGTGGTGGACGAGCTCGTGAAAGTGATGGGCCGGCACAAGCCCGAGAACATCGTG  
2280

hSpCas9

RuvC II

| V | K | V | V | D | E | L | V | K | V | M | G | R | H | K | P | E | N | I | V |
|---|---|---|---|---|---|---|---|---|---|---|---|---|---|---|---|---|---|---|---|
| 741 | 742 | 743 | 744 | 745 | 746 | 747 | 748 | 749 | 750 | 751 | 752 | 753 | 754 | 755 | 756 | 757 | 758 | 759 | 760 |

5' ATCGCCATGGCCAGAGAGAACCAGACCACCCAGAAGGGACAGAAGAACAGCCGCGAGAGA  
2340

hSpCas9

RuvC II

E...

| I | A | M | A | R | E | N | Q | T | T | Q | K | G | Q | K | N | S | R | E | R |
|---|---|---|---|---|---|---|---|---|---|---|---|---|---|---|---|---|---|---|---|
| 761 | 762 | 763 | 764 | 765 | 766 | 767 | 768 | 769 | 770 | 771 | 772 | 773 | 774 | 775 | 776 | 777 | 778 | 779 | 780 |

5' ATGAAGCGGATCGAAGAGGGCATCAAAGAGCTGGGCAGCCAGATCCTGAAAGAACACCCC  
2400

hSpCas9

| M | K | R | I | E | E | G | I | K | E | L | G | S | Q | I | L | K | E | H | P |
|---|---|---|---|---|---|---|---|---|---|---|---|---|---|---|---|---|---|---|---|
| 781 | 782 | 783 | 784 | 785 | 786 | 787 | 788 | 789 | 790 | 791 | 792 | 793 | 794 | 795 | 796 | 797 | 798 | 799 | 800 |

# Figure 7G

hSpCas9

5' GTGGAAAACACCCAGCTGCAGAACGAGAAGCTGTACCTGTACTACCTGCAGAATGGGCGG

○ 2460

hSpCas9

| V | E | N | T | Q | L | Q | N | E | K | L | Y | L | Y | Y | L | Q | N | G | R |
| 801 | 802 | 803 | 804 | 805 | 806 | 807 | 808 | 809 | 810 | 811 | 812 | 813 | 814 | 815 | 816 | 817 | 818 | 819 | 820 |

5' GATATGTACGTGGACCAGGAACTGGACATCAACCGGCTGTCCGACTACGATGTGGACGCC

○ 2520

hSpCas9

HNH

H...

| D | M | Y | V | D | Q | E | L | D | I | N | R | L | S | D | Y | D | V | D | A |
| 821 | 822 | 823 | 824 | 825 | 826 | 827 | 828 | 829 | 830 | 831 | 832 | 833 | 834 | 835 | 836 | 837 | 838 | 839 | 840 |

5' ATCGTGCCTCAGAGCTTTCTGAAGGACGACTCCATCGACGCCAAGGTGCTGACCAGAAGC

○ 2580

hSpCas9

HNH

N...

| I | V | P | Q | S | F | L | K | D | D | S | I | D | A | K | V | L | T | R | S |
| 841 | 842 | 843 | 844 | 845 | 846 | 847 | 848 | 849 | 850 | 851 | 852 | 853 | 854 | 855 | 856 | 857 | 858 | 859 | 860 |

5' GACAAGGCCCGGGGCAAGAGCGACAACGTGCCCTCCGAAGAGGTCGTGAAGAAGATGAAG

○ 2640

hSpCas9

HNH

N...

| D | K | A | R | G | K | S | D | N | V | P | S | E | E | V | V | K | K | M | K |
| 861 | 862 | 863 | 864 | 865 | 866 | 867 | 868 | 869 | 870 | 871 | 872 | 873 | 874 | 875 | 876 | 877 | 878 | 879 | 880 |

5' AACTACTGGCGGCAGCTGCTGAACGCCAAGCTGATTACCCAGAGAAAGTTCGACAATCTG

○ 2700

hSpCas9

| N | Y | W | R | Q | L | L | N | A | K | L | I | T | Q | R | K | F | D | N | L |
| 881 | 882 | 883 | 884 | 885 | 886 | 887 | 888 | 889 | 890 | 891 | 892 | 893 | 894 | 895 | 896 | 897 | 898 | 899 | 900 |

# Figure 7H

hSpCas9

5' ACCAAGGCCGAGAGAGGCGGCCTGAGCGAACTGGATAAGGCCGGCTTCATCAAGAGACAG

2760

hSpCas9

T K A E R G G L S E L D K A G F I K R Q
901 902 903 904 905 906 907 908 909 910 911 912 913 914 915 916 917 918 919 920

5' CTGGTGGAAACCCGGCAGATCACAAAGCACGTGGCACAGATCCTGGACTCCCGGATGAAC

2820

hSpCas9

L V E T R Q I T K H V A Q I L D S R M N
921 922 923 924 925 926 927 928 929 930 931 932 933 934 935 936 937 938 939 940

5' ACTAAGTACGACGAGAATGACAAGCTGATCCGGGAAGTGAAAGTGATCACCCTGAAGTCC

2880

hSpCas9

T K Y D E N D K L I R E V K V I T L K S
941 942 943 944 945 946 947 948 949 950 951 952 953 954 955 956 957 958 959 960

5' AAGCTGGTGTCCGATTTCCGGAAGGATTTCCAGTTTTACAAAGTGCGCGAGATCAACAAC

2940

hSpCas9

K L V S D F R K D F Q F Y K V R E I N N
961 962 963 964 965 966 967 968 969 970 971 972 973 974 975 976 977 978 979 980

5' TACCACCACGCCCACGCCGCCTACCTGAACGCCGTCGTGGGAACCGCCCTGATCAAAAAG

3000

hSpCas9

RuvC III

D...

Y H H A H A A Y L N A V V G T A L I K K
981 982 983 984 985 986 987 988 989 990 991 992 993 994 995 996 997 998 999 1000

# Figure 7I

hSpCas9

5' TACCCTAAGCTGGAAAGCGAGTTCGTGTACGGCGACTACAAGGTGTACGACGTGCGGAAG

3060

hSpCas9

Y P K L E S E F V Y G D Y K V Y D V R K

5' ATGATCGCCAAGAGCGAGCAGGAAATCGGCAAGGCTACCGCCAAGTACTTCTTCTACAGC

3120

hSpCas9

M I A K S E Q E I G K A T A K Y F F Y S

5' AACATCATGAACTTTTTCAAGACCGAGATTACCCTGGCCAACGGCGAGATCCGGAAGCGG

3180

hSpCas9

N I M N F F K T E I T L A N G E I R K R

5' CCTCTGATCGAGACAAACGGCGAAACCGGGGAGATCGTGTGGGATAAGGGCCGGGATTTT

3240

hSpCas9

P L I E T N G E T G E I V W D K G R D F

5' GCCACCGTGCGGAAAGTGCTGAGCATGCCCCAAGTGAATATCGTGAAAAAGACCGAGGTG

3300

hSpCas9

A T V R K V L S M P Q V N I V K K T E V

5' CAGACAGGCGGCTTCAGCAAAGAGTCTATCCTGCCCAAGAGGAACAGCGATAAGCTGATC

3360

hSpCas9

Q T G G F S K E S I L P K R N S D K L I

Figure 7J

**hSpCas9**

```
5'  GCCAGAAAGAAGGACTGGGACCCTAAGAAGTACGGCGGCTTCGACAGCCCCACCGTGGCC
o   +++++|+++++|+++++|+++++|+++++|+++++|+++++|+++++|+++++|+++++|+++++|    3420
o   ████████████████████████hSpCas9████████████████████████████████
    A   R   K   K   D   W   D   P   K   K   Y   G   G   F   D   S   P   T   V   A
    1121 1122 1123 1124 1125 1126 1127 1128 1129 1130 1131 1132 1133 1134 1135 1136 1137 1138 1139 1140
```

```
5'  TATTCTGTGCTGGTGGTGGCCAAAGTGGAAAAGGGCAAGTCCAAGAAACTGAAGAGTGTG
o   +++++|+++++|+++++|+++++|+++++|+++++|+++++|+++++|+++++|+++++|+++++|    3480
o   ████████████████████████hSpCas9████████████████████████████████
    Y   S   V   L   V   V   A   K   V   E   K   G   K   S   K   K   L   K   S   V
    1141 1142 1143 1144 1145 1146 1147 1148 1149 1150 1151 1152 1153 1154 1155 1156 1157 1158 1159 1160
```

```
5'  AAAGAGCTGCTGGGGATCACCATCATGGAAAGAAGCAGCTTCGAGAAGAATCCCATCGAC
o   +++++|+++++|+++++|+++++|+++++|+++++|+++++|+++++|+++++|+++++|+++++|    3540
o   ████████████████████████hSpCas9████████████████████████████████
    K   E   L   L   G   I   T   I   M   E   R   S   S   F   E   K   N   P   I   D
    1161 1162 1163 1164 1165 1166 1167 1168 1169 1170 1171 1172 1173 1174 1175 1176 1177 1178 1179 1180
```

```
5'  TTTCTGGAAGCCAAGGGCTACAAAGAAGTGAAAAAGGACCTGATCATCAAGCTGCCTAAG
o   +++++|+++++|+++++|+++++|+++++|+++++|+++++|+++++|+++++|+++++|+++++|    3600
o   ████████████████████████hSpCas9████████████████████████████████
    F   L   E   A   K   G   Y   K   E   V   K   K   D   L   I   I   K   L   P   K
    1181 1182 1183 1184 1185 1186 1187 1188 1189 1190 1191 1192 1193 1194 1195 1196 1197 1198 1199 1200
```

```
5'  TACTCCCTGTTCGAGCTGGAAAACGGCCGGAAGAGAATGCTGGCCTCTGCCGGCGAACTG
o   +++++|+++++|+++++|+++++|+++++|+++++|+++++|+++++|+++++|+++++|+++++|    3660
o   ████████████████████████hSpCas9████████████████████████████████
    Y   S   L   F   E   L   E   N   G   R   K   R   M   L   A   S   A   G   E   L
    1201 1202 1203 1204 1205 1206 1207 1208 1209 1210 1211 1212 1213 1214 1215 1216 1217 1218 1219 1220
```

```
5'  CAGAAGGGAAACGAACTGGCCCTGCCCTCCAAATATGTGAACTTCCTGTACCTGGCCAGC
o   +++++|+++++|+++++|+++++|+++++|+++++|+++++|+++++|+++++|+++++|+++++|    3720
o   ████████████████████████hSpCas9████████████████████████████████
    Q   K   G   N   E   L   A   L   P   S   K   Y   V   N   F   L   Y   L   A   S
    1221 1222 1223 1224 1225 1226 1227 1228 1229 1230 1231 1232 1233 1234 1235 1236 1237 1238 1239 1240
```

# Figure 7K

hSpCas9

5' CACTATGAGAAGCTGAAGGGCTCCCCCGAGGATAATGAGCAGAAACAGCTGTTTGTGGAA

3780

hSpCas9

H Y E K L K G S P E D N E Q K Q L F V E

1241 1242 1243 1244 1245 1246 1247 1248 1249 1250 1251 1252 1253 1254 1255 1256 1257 1258 1259 1260

5' CAGCACAAGCACTACCTGGACGAGATCATCGAGCAGATCAGCGAGTTCTCCAAGAGAGTG

3840

hSpCas9

Q H K H Y L D E I I E Q I S E F S K R V

1261 1262 1263 1264 1265 1266 1267 1268 1269 1270 1271 1272 1273 1274 1275 1276 1277 1278 1279 1280

5' ATCCTGGCCGACGCTAATCTGGACAAAGTGCTGTCCGCCTACAACAAGCACCGGGATAAG

3900

hSpCas9

I L A D A N L D K V L S A Y N K H R D K

1281 1282 1283 1284 1285 1286 1287 1288 1289 1290 1291 1292 1293 1294 1295 1296 1297 1298 1299 1300

5' CCCATCAGAGAGCAGGCCGAGAATATCATCCACCTGTTTACCCTGACCAATCTGGGAGCC

3960

hSpCas9

P I R E Q A E N I I H L F T L T N L G A

1301 1302 1303 1304 1305 1306 1307 1308 1309 1310 1311 1312 1313 1314 1315 1316 1317 1318 1319 1320

5' CCTGCCGCCTTCAAGTACTTTGACACCACCATCGACCGGAAGAGGTACACCAGCACCAAA

4020

hSpCas9

P A A F K Y F D T T I D R K R Y T S T K

1321 1322 1323 1324 1325 1326 1327 1328 1329 1330 1331 1332 1333 1334 1335 1336 1337 1338 1339 1340

5' GAGGTGCTGGACGCCACCCTGATCCACCAGAGCATCACCGGCCTGTACGAGACACGGATC

4080

hSpCas9

E V L D A T L I H Q S I T G L Y E T R I

1341 1342 1343 1344 1345 1346 1347 1348 1349 1350 1351 1352 1353 1354 1355 1356 1357 1358 1359 1360

Figure 7L

**hSpCas9**

5' GACCTGTCTCAGCTGGGAGGCGAC

4104

hSpCas9

D L S Q L G G D

1361 1362 1363 1364 1365 1366 1367 1368

# Figure 7M

**Figure 8**

target locus

*S. aureus* Cas9 nickases

D10A
N580A

% NHEJ

60

40

20

0

21 & 33    21 & 34    41 & 32    41 & 33    41 & 34

gRNA pairs

**Figure 9A**

EP 3 686 279 B1

Figure 9B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62038358
- US 62180699
- US 8999641 B
- US 8993233 B
- US 8945839 B
- US 8932814 B
- US 8906616 B
- US 8895308 B
- US 8889418 B
- US 8889356 B
- US 8871445 B
- US 8865406 B
- US 8795965 B
- US 8771945 B
- US 8697359 B
- US 20140310830 A
- US 105031
- US 20140287938 A1
- US 213991
- US 20140273234 A1
- US 293674
- US 20140273232 A1
- US 290575
- US 20140273231 A
- US 259420
- US 20140256046 A1
- US 226274
- US 20140248702 A1
- US 258458
- US 20140242700 A1
- US 222930
- US 20140242699 A1
- US 183512
- US 20140242664 A1
- US 104990
- US 20140234972 A1
- US 183471
- US 20140227787 A1
- US 256912
- US 20140189896 A1
- US 105035
- US 20140186958 A
- US 105017
- US 20140186919 A1
- US 104977
- US 20140186843 A1
- US 104900
- US 20140179770 A1
- US 104837

- US 20140179006 A1
- US 183486
- US 20140170753 A
- US 183429
- EP 2784162 B1
- EP 2771468 B1
- EP 2771468 A
- EP 13818570
- EP 2764103 A
- EP 13824232
- EP 2784162 A
- EP 14170383
- WO 2014093661 A
- US 2013074743 W
- WO 2014093694 A
- US 2013074790 W
- WO 2014093595 A
- US 2013074611 W
- WO 2014093718 A
- US 2013074825 W
- WO 2014093709 A
- US 2013074812 W
- WO 2014093622 A
- US 2013074667 W
- WO 2014093635 A
- US 2013074691 W
- WO 2014093655 A
- US 2013074736 W
- WO 2014093712 A
- US 2013074819 W
- WO 2014093701 A
- US 2013074800 W
- WO 2014018423 A
- US 2013051418 W
- WO 2014204723 A
- US 2014041790 W
- WO 2014204724 A
- US 2014041800 W
- WO 2014204725 A
- US 2014041803 W
- WO 2014204726 A
- US 2014041804 W
- WO 2014204727 A
- US 2014041806 W
- WO 2014204728 A
- US 2014041808 W
- WO 2014204729 A
- US 2014041809 W
- US 61758468

- US 61802174 B
- US 61806375 B
- US 61814263 B
- US 61819803 B
- US 61828130 B
- US 61836123
- US 61835931
- US 61835936 B
- US 61836127 B
- US 61836101 B
- US 61836080 B
- US 61835973 B
- US 61862468
- US 61862355 B
- US 61871301 B
- US 61960777 B
- US 61961980 B
- US 201462558 W
- US 61915150
- US 61915301 B
- US 61915267 B
- US 61915260 B
- US 61757972 B
- US 61768959 B
- US 61835931 B
- US 62010888 B
- US 62010879 B
- US 62010329 B
- US 62010441 B
- US 61939228 B
- US 61939242 B
- US 61980012 B
- US 62038358 B
- US 62054490 B
- US 62055484 B
- US 62055460 B
- US 62055487 B
- US 62069243 B
- US 62055484
- US 61980012
- US 61939242
- US 1441806 W
- US 61930214
- US 61915251
- US 62091455 B
- US 62096708 B
- US 62091462 B
- US 62096324 B
- US 62091456 B
- US 62091461 B
- US 62094903 B
- US 62096761 B
- US 62098059 B
- US 62096656 B
- US 62096697 B
- US 62098158 B
- US 62151052 B
- US 62087537 B

- US 62054651 B
- US 62067886 B
- US 62054675 B
- US 62054528 B
- US 62055454 B
- US 62087475 B
- US 62087546 B
- US 62098285 B
- US 1470068 W
- US 61915267
- US 8454972 B2
- US 8404658 B2, Hajjar
- US 5593972 A
- US 5589466 A
- US 5580859 A
- US 8454972 B
- US 8404658 B
- US 5846946 A
- US 20120295960 A
- US 7303910 B
- US 7351585 B
- US 20060281180 A
- US 20090007284 A
- US 20110117189 A
- US 20090017543 A
- US 20070054961 A
- US 20100317109 A
- US 20110293571 A
- US 20040013648 A
- US 20070025970 A
- US 20090111106 A
- US 7259015 B
- US 20110293703 A
- US 20130302401 A
- US 8709843 B
- US 6007845 A
- US 5855913 A
- US 5985309 A
- US 5543158 A
- WO 2012135025 A
- US 20120251560 A
- US 7982027 B
- US 7799565 B
- US 8058069 B
- US 8283333 B
- US 7901708 B
- US 7745651 B
- US 7803397 B
- US 8101741 B
- US 8188263 B
- US 7915399 B
- US 8236943 B
- US 7838658 B
- EP 1766035 A
- EP 1519714 A
- EP 1781593 A
- EP 1664316 A
- US 20130252281 A

- US 20130245107 A
- US 20130244279 A
- US 20120251618 A
- US 20050019923 A
- US 20080267903 A
- US 20110195123 A
- WO 9703211 A
- WO 9639154 A
- US 81573004
- US 20040171156 A1
- US 491026
- WO 2011028929 A
- US 511940
- US 4873316 A
- EP 264166 A
- US 6750059 B
- US 092085
- US 7776321 B
- US 20110059502 A
- US 20140186919 A
- US 20140242700 A
- US 20140273234 A
- US 20140335620 A
- WO 2014093635 A1
- WO 2014093635 A9
- US 61736465 B

- US 61721283 B
- US 5049386 A
- US 4946787 A
- US 4897355 A
- WO 9117424 A
- WO 9116024 A
- US 4186183 A
- US 4217344 A
- US 4235871 A
- US 4261975 A
- US 4485054 A
- US 4501728 A
- US 4774085 A
- US 4837028 A
- US 9405700 W
- US 4797368 A
- WO 9324641 A
- US 5173414 A
- US 20030087817 A
- US 6603061 B
- US 7868149 B
- US 20090100536 A
- US 5210015 A
- US 5445934 A
- US 61736527
- US 61748427 B

**Non-patent literature cited in the description**

- **CONG, L. ; RAN, F.A. ; COX, D ; LIN, S. ; BARRETTO, R ; HABIB, N. ; HSU, P.D. ; WU, X. ; JIANG, W. ; MARRAFFINI, L.A.** Multiplex genome engineering using CRISPR/Cas systems. *Science,* 15 February 2013, vol. 339 (6121), 819-23
- **JIANG W ; BIKARD D. ; COX D. ; ZHANG F ; MARRAFFINI LA.** RNA-guided editing of bacterial genomes using CRISPR-Cas systems. *Nat Biotechnol,* 2013, vol. 31 (3), 233-9
- **WANG H. ; YANG H. ; SHIVALILA CS. ; DAWLATY MM. ; CHENG AW. ; ZHANG F. ; JAENISCH R.** One-Step Generation of Mice Carrying Mutations in Multiple Genes by CRISPR/Cas-Mediated Genome Engineering. *Cell,* 2013, vol. 153 (4), 910-8
- **KONERMANN S ; BRIGHAM MD ; TREVINO AE ; HSU PD ; HEIDENREICH M ; CONG L ; PLATT RJ ; SCOTT DA ; CHURCH GM ; ZHANG F.** Optical control of mammalian endogenous transcription and epigenetic states. *Nature,* 22 August 2013, vol. 500 (7463), 472-6
- *Epub,* 23 August 2013
- **RAN, FA. ; HSU, PD. ; LIN, CY. ; GOOTENBERG, JS. ; KONERMANN, S. ; TREVINO, AE. ; SCOTT, DA. ; INOUE, A ; MATOBA, S. ; ZHANG, Y.** Double Nicking by RNA-Guided CRISPR Cas9 for Enhanced Genome Editing Specificity. *Cell*

- **HSU, P. ; SCOTT, D. ; WEINSTEIN, J. ; RAN, FA. ; KONERMANN, S. ; AGARWALA, V. ; LI, Y. ; FINE, E. ; WU, X. ; SHALEM, O.** DNA targeting specificity of RNA-guided Cas9 nucleases. *Nat Biotechnol,* 2013
- **RAN, FA. ; HSU, PD. ; WRIGHT, J. ; AGARWALA, V. ; SCOTT, DA. ; ZHANG, F.** Genome engineering using the CRISPR-Cas9 system. *Nature Protocols,* November 2013, vol. 8 (11), 2281-308
- **SHALEM, O ; SANJANA, NE ; HARTENIAN, E. ; SHI, X. ; SCOTT, DA. ; MIKKELSON, T. ; HECKL, D. ; EBERT, BL. ; ROOT, DE. ; DOENCH, JG.** Genome-Scale CRISPR-Cas9 Knockout Screening in Human Cells. *Science,* 20131212
- **NISHIMASU, H. ; RAN, FA. ; HSU, PD. ; KONERMANN, S. ; SHEHATA, SI. ; DOHMAE, N. ; ISHITANI, R. ; ZHANG, F. ; NUREKI, O.** Crystal structure of cas9 in complex with guide RNA and target DNA. *Cell,* 27 February 2014, vol. 156 (5), 935-49
- **WU X. ; SCOTT DA. ; KRIZ AJ. ; CHIU AC. ; HSU PD. ; DADON DB. ; CHENG AW. ; TREVINO AE. ; KONERMANN S. ; CHEN S.** Genome-wide binding of the CRISPR endonuclease Cas9 in mammalian cells. *Nat Biotechnol.,* 20 April 2014
- **PLATT et al.** CRISPR-Cas9 Knockin Mice for Genome Editing and Cancer Modeling. *Cell,* 2014, vol. 159 (2), 440-455

- **HSU et al.** Development and Applications of CRISPR-Cas9 for Genome Engineering. *Cell,* 05 June 2014, vol. 157, 1262-1278
- **WANG et al.** Genetic screens in human cells using the CRISPR/Cas9 system. *Science,* 03 January 2014, vol. 343 (6166), 80-84
- **DOENCH et al.** Rational design of highly active sgRNAs for CRISPR-Cas9-mediated gene inactivation. *Nature Biotechnology published online,* 03 September 2014
- **SWIECH et al.** In vivo interrogation of gene function in the mammalian brain using CRISPR-Cas9. *Nature Biotechnology published online,* 19 October 2014
- **KONERMANN et al.** *Genome-scale transcription activation by an engineered CRISPR-Cas9 complex*
- **ZETSCHE et al.** A split-Cas9 architecture for inducible genome editing and transcription modulation. *Nature Biotechnology,* 02 February 2015, vol. 33, 139-142
- **SIDI CHEN et al.** Genome-wide CRISPR Screen in a Mouse Model of Tumor Growth and Metastasis. *Cell,* 12 March 2015, vol. 160, 1246-1260
- **RAN et al.** In vivo genome editing using Staphylococcus aureus Cas9. *Nature,* 01 April 2015, vol. 520, 186-191
- **TSAI et al.** Dimeric CRISPR RNA-guided FokI nucleases for highly specific genome editing. *Nature Biotechnology,* 2014, vol. 32 (6), 569-77
- **ZHANG et al.** *Nature,* 2012, vol. 490 (7421), 556-60
- **CHEN et al.** *PLoS Comput Biol,* 2015, vol. 11 (5), e1004248
- **DEY et al.** *Prot Sci;,* 2013, vol. 22, 359-66
- **DUMITRACHE et al.** *Genetics,* August 2011, vol. 188 (4), 787-797
- REMINGTON'S PHARMACEUTICAL SCIENCES. Mack Pub. Co, 1991
- **SHEN et al.** *FEBS Let.,* 2003, vol. 539, 111-114
- **XIA et al.** *Nat. Biotech.,* 2002, vol. 20, 1006-1010
- **REICH et al.** *Mol. Vision.,* 2003, vol. 9, 210-216
- **SORENSEN et al.** *J. Mol. Biol.,* 2003, vol. 327, 761-766
- **LEWIS et al.** *Nat. Gen.,* 2002, vol. 32, 107-108
- **SIMEONI et al.** *NAR,* 2003, vol. 31 (11), 2717-2724
- **TOLENTINO et al.** *Retina,* vol. 24 (4), 660
- **CHO, S. ; GOLDBERG, M. ; SON, S. ; XU, Q. ; YANG, F. ; MEI, Y. ; BOGATYREV, S. ; LANGER, R. ; ANDERSON, D.** Lipid-like nanoparticles for small interfering RNA delivery to endothelial cells. *Advanced Functional Materials,* 2010, vol. 19, 3112-3118
- **SCHROEDER, A. ; LEVINS, C ; CORTEZ, C. ; LANGER, R. ; ANDERSON, D.** Lipid-based nanotherapeutics for siRNA delivery. *Journal of Internal Medicine,* 2010, vol. 267, 9-21
- **EL-ANDALOUSSI S et al.** Exosome-mediated delivery of siRNA in vitro and in vivo. *Nat Protoc.,* December 2012, vol. 7 (12), 2112-26
- **UNO et al.** *HUMAN GENE THERAPY,* June 2011, vol. 22, 711-719
- **ZOU et al.** *HUMAN GENE THERAPY,* vol. 22, 465-475
- **GRIMM, D. et al.** *J. Virol.,* 2008, vol. 82, 5887-5911
- **BALAGAAN.** *J Gene Med,* 2006, vol. 8, 275-285
- **BINLEY et al.** *HUMAN GENE THERAPY,* September 2012, vol. 23, 980-991
- **SU X ; FRICKE J ; KAVANAGH DG ; IRVINE DJ.** In vitro and in vivo mRNA delivery using lipid-enveloped pH-responsive polymer nanoparticles. *Mol Pharm.,* 06 June 2011, vol. 8 (3), 774-87
- **MAZZA, M. et al.** *ACSNano,* 2013, vol. 7 (2), 1016-1026
- **SIEW, A. et al.** *Mol Pharm,* 2012, vol. 9 (1), 14-28
- **LALATSA, A. et al.** *J Contr Rel,* 2012, vol. 161 (2), 523-36
- **LALATSA, A. et al.** *Mol Pharm,* 2012, vol. 9 (6), 1665-80
- **LALATSA, A. et al.** *Mol Pharm,* 2012, vol. 9 (6), 1764-74
- **GARRETT, N.L. et al.** *J Biophotonics,* 2012, vol. 5 (5-6), 458-68
- **GARRETT, N.L. et al.** *J Raman Spect,* 2012, vol. 43 (5), 681-688
- **AHMAD, S. et al.** *J Royal Soc Interface,* 2010, vol. 7, 423-33
- **UCHEGBU, I.F.** *Expert Opin Drug Deliv,* 2006, vol. 3 (5), 629-40
- **QU, X.** *Biomacromolecules,* 2006, vol. 7 (12), 3452-9
- **UCHEGBU, I.F. et al.** *Int J Pharm,* 2001, vol. 224, 185-199
- **ALABI et al.** *Proc Natl Acad Sci USA.,* 06 August 2013, vol. 110 (32), 12881-6
- **ZHANG et al.** *Adv Mater.,* 06 September 2013, vol. 25 (33), 4641-5
- **JIANG et al.** *Nano Lett.,* 13 March 2013, vol. 13 (3), 1059-64
- **KARAGIANNIS et al.** *ACS Nano.,* 23 October 2012, vol. 6 (10), 8484-7
- **WHITEHEAD et al.** *ACS Nano.,* 28 August 2012, vol. 6 (8), 6922-9
- **LEE et al.** *Nat Nanotechnol.,* 03 June 2012, vol. 7 (6), 389-93
- **COELHO et al.** *N Engl J Med,* 2013, vol. 369, 819-29

- **TABERNERO et al.** *Cancer Discovery,* 2013, vol. 3 (4), 363-470
- **ROSIN et al.** *Molecular Therapy,* December 2011, vol. 19 (12), 1286-2200
- **CUTLER et al.** *J. Am. Chem. Soc.,* 2011, vol. 133, 9254-9257
- **HAO et al.** *Small,* 2011, vol. 7, 3158-3162
- **ZHANG et al.** *ACS Nano.,* 2011, vol. 5, 6962-6970
- **CUTLER et al.** *J. Am. Chem. Soc.,* 2012, vol. 134, 1376-1391
- **YOUNG et al.** *Nano Lett,* 2012, vol. 12, 3867-71
- **ZHENG et al.** *Proc. Natl. Acad. Sci. USA.,* 2012, vol. 109, 11975-80
- **MIRKIN.** *Nanomedicine,* 2012, vol. 7, 635-638
- **ZHANG et al.** *J. Am. Chem. Soc.,* 2012, vol. 134, 16488-1691
- **WEINTRAUB.** *Nature,* 2013, vol. 495, S14-S16
- **CHOI et al.** *Proc. Natl. Acad. Sci. USA.,* 2013, vol. 110 (19), 7625-7630
- **JENSEN et al.** *Sci. Transl. Med.,* 2013, vol. 5, 209ra152
- **MIRKIN et al.** *Small,* vol. 10, 186-192
- **SCHIFFELERS et al.** *Nucleic Acids Research,* 2004, vol. 32 (19
- **BARTLETT et al.** *PNAS,* 25 September 2007, vol. 104 (39
- **DAVIS et al.** *Nature,* 15 April 2010, vol. 464
- **JAMES E. ; DAHLMAN ; CARMEN BARNES et al.** *Nature Nanotechnology,* 11 May 2014
- **BARNES et al.** *Nature Nanotechnology,* 11 May 2014
- **ALVAREZ-ERVITI et al.** *Nat Biotechnol,* 2011, vol. 29, 341
- **EL-ANDALOUSSI et al.** *Nature Protocols,* 2012, vol. 7, 2112-2126
- **WAHLGREN et al.** *Nucleic Acids Research,* 2012, vol. 40 (17), e130
- **SPUCH ; NAVARRO.** *Journal of Drug Delivery,* 2011, vol. 2011, 12
- **MORRISSEY et al.** *Nature Biotechnology,* August 2005, vol. 23 (8
- **ZIMMERMAN et al.** *Nature Letters,* 04 May 2006, vol. 441
- **ZIMMERMAN et al.** *Nature,* 04 May 2006, vol. 441
- **LI.** *Gene Therapy,* 2012, vol. 19, 775-780
- **GEISBERT et al.** *Lancet,* 2010, vol. 375, 1896-905
- **JUDGE.** *J. Clin. Invest.,* 2009, vol. 119, 661-673
- *Advanced Drug Delivery Reviews,* 2012, vol. 64, 1730-1737
- **SEMPLE et al.** *Nature Niotechnology,* 02 February 2010, vol. 28, 172-177
- **JAYARAMAN.** *Angew. Chem. Int. Ed.,* 2012, vol. 51, 8529-8533
- **MICHAEL S D KORMANN et al.** *Expression of therapeutic proteins after delivery of chemically modified mRNA in mice: Nature Biotechnology,* 2011, vol. 29, 154-157
- **NOVOBRANTSEVA.** *Molecular Therapy-Nucleic Acids,* 2012, vol. 1, e4
- **SCHRUM et al.** *Delivery and Formulation of Engineered Nucleic Acids*
- **MAZZA et al.** *ACS Nano.,* 26 February 2013, vol. 7 (2), 1016-26
- **UCHEGBU ; SIEW.** *J Pharm Sci.,* 2013, vol. 102 (2), 305-10
- **LALATSA et al.** *J Control Release.,* 20 July 2012, vol. 161 (2), 523-36
- **LAWRENCE et al.** *Journal of the American Chemical Society,* 2007, vol. 129, 10110-10112
- **AKINC et al.** *Nat. Biotech.,* 2010, vol. 26, 561-569
- **MCNAUGHTON et al.** *Proc. Natl. Acad. Sci. USA,* 2009, vol. 106, 6111-6116
- **CRONICAN et al.** *ACS Chemical Biology,* 2010, vol. 5, 747-752
- **CRONICAN et al.** *Chemistry & Biology,* 2011, vol. 18, 833-838
- **THOMPSON et al.** *Methods in Enzymology,* 2012, vol. 503, 293-319
- **THOMPSON, D.B. et al.** *Chemistry & Biology,* 2012, vol. 19 (7), 831-843
- **KONERMANN et al.** *Nature,* 29 January 2015, vol. 517, 583-588
- **GERBASCH ; REDDY.** *Nature Biotech,* 06 April 2015
- **XIA CF ; BOADO RJ ; PARDRIDGE WM.** Antibody-mediated targeting of siRNA via the human insulin receptor using avidin-biotin technology. *Mol Pharm.,* May 2009, vol. 6 (3), 747-51
- **ZHANG Y ; SCHLACHETZKI F ; PARDRIDGE WM.** Global non-viral gene transfer to the primate brain following intravenous administration. *Mol Ther.,* January 2003, vol. 7 (1), 11-8
- **SCHROEDER, A. ; LEVINS, C. ; CORTEZ, C. ; LANGER, R. ; ANDERSON, D.** Lipid-based nano-therapeutics for siRNA delivery. *Journal of Internal Medicine,* 2010, vol. 267, 9-21
- **EL-ANDALOUSSI S et al.** Exosome-mediated delivery of siRNA in vitro and in vivo. *Nat Protoc.,* December 2012, vol. 7 (12), 2112-26
- **TANGRI S et al.** Rationally engineered therapeutic proteins with reduced immunogenicity. *J Immunol.,* 15 March 2005, vol. 174 (6), 3187-96
- Overview of principles of hybridization and the strategy of nucleic acid probe assay. **TIJSSEN.** Laboratory Techniques In Biochemistry And Molecular Biology-Hybridization With Nucleic Acid Probes. Elsevier, 1993

- **DEVEREUX et al.** Nucleic Acids Research. University of Wisconsin, 1984, vol. 12, 387
- **AUSUBEL et al.** *ibid,* 1999
- **ATSCHUL et al.** *J. Mol. Biol.,* 1990, 403-410
- **AUSUBEL et al.** *ibid,* 1999, 7-58, 7-60
- **DEVEREUX et al.** *Nuc. Acids Research,* 1984, vol. 12, 387
- **AUSUBEL et al.** Short Protocols in Molecular Biology. 1999
- **ALTSCHUL et al.** *J. Mol. Biol.,* 1990, 403-410
- **AUSUBEL et al.** *Short Protocols in Molecular Biology,* 1999, 7-58, 7-60
- *FEMS Microbiol Lett,* 1999, vol. 174 (2), 247-50
- *FEMS Microbiol Lett.,* 1999, vol. 177 (1), 187-8
- **HIGGINS DG ; SHARP PM.** *Gene,* 1988, vol. 73 (1), 237-244
- **LIVINGSTONE C.D. ; BARTON G.J.** Protein sequence alignments: a strategy for the hierarchical analysis of residue conservation. *Comput. Appl. Biosci.,* 1993, vol. 9, 745-756
- **TAYLOR W.R.** The classification of amino acid conservation. *J. Theor. Biol.,* 1986, vol. 119, 205-218
- **SIMON RJ et al.** *PNAS,* 1992, vol. 89 (20), 9367-9371
- **HORWELL DC.** *Trends Biotechnol.,* 1995, vol. 13 (4), 132-134
- **SAMBROOK ; FRITSCH ; MANIATIS.** MOLECULAR CLONING: A LABORATORY MANUAL. 1989
- CURRENT PROTOCOLS IN MOLECULAR BIOLOGY. 1987
- METHODS IN ENZYMOLOGY. Academic Press, Inc,
- A PRACTICAL APPROACH. 1995
- ANTIBODIES, A LABORATORY MANUAL, and ANIMAL CELL CULTURE. 1988
- **GOEDDEL.** GENE EXPRESSION TECHNOLOGY: METHODS IN ENZYMOLOGY. Academic Press, 1990
- **BOSHART et al.** *Cell,* 1985, vol. 41, 521-530
- *Mol. Cell. Biol.,* 1988, vol. 8 (1), 466-472
- *Proc. Natl. Acad. Sci. USA.,* 1981, vol. 78 (3), 1527-31
- **GOEDDEL.** GENE EXPRESSION TECHNOLOGY: METHODS IN ENZYMOLOGY. Academic Press, 1990, vol. 185
- **JOHNSON.** *Gene,* 1988, vol. 67, 31-40
- **AMRANN et al.** *Gene,* 1988, vol. 69, 301-315
- **STUDIER et al.** GENE EXPRESSION TECHNOLOGY: METHODS IN ENZYMOLOGY. Academic Press, 1990, vol. 185, 60-89
- **BALDARI et al.** *EMBO J.,* 1987, vol. 6, 229-234
- **KUIJAN ; HERSKOWITZ.** *Cell,* 1982, vol. 30, 933-943
- **SCHULTZ et al.** *Gene,* 1987, vol. 54, 113-123
- **SMITH et al.** *Mol. Cell. Biol.,* 1983, vol. 3, 2156-2165
- **LUCKLOW ; SUMMERS.** *Virology,* 1989, vol. 170, 31-39
- **SEED.** *Nature,* 1987, vol. 329, 840
- **KAUFMAN et al.** *EMBO J.,* 1987, vol. 6, 187-195
- **SAMBROOK et al.** MOLECULAR CLONING: A LABORATORY MANUAL. Cold Spring Harbor Laboratory, 1989
- **PINKERT et al.** *Genes Dev.,* 1987, vol. 1, 268-277
- **CALAME ; EATON.** *Adv. Immunol.,* 1988, vol. 43, 235-275
- **WINOTO ; BALTIMORE.** *EMBO J.,* 1989, vol. 8, 729-733
- **BANEIJI et al.** *Cell,* 1983, vol. 33, 729-740
- **QUEEN ; BALTIMORE.** *Cell,* 1983, vol. 33, 741-748
- **BYRNE ; RUDDLE.** *Proc. Natl. Acad. Sci. USA,* 1989, vol. 86, 5473-5477
- **EDLUND et al.** *Science,* 1985, vol. 230, 912-916
- **KESSEL ; GRUSS.** *Science,* 1990, vol. 249, 374-379
- **CAMPES ; TILGHMAN.** *Genes Dev.,* 1989, vol. 3, 537-546
- **ISHINO et al.** *J. Bacteriol.,* 1987, vol. 169, 5429-5433
- **NAKATA et al.** *J. Bacteriol.,* 1989, vol. 171, 3553-3556
- **GROENEN et al.** *Mol. Microbiol.,* 1993, vol. 10, 1057-1065
- **HOE et al.** *Emerg. Infect. Dis.,* 1999, vol. 5, 254-263
- **MASEPOHL et al.** *Biochim. Biophys. Acta,* 1996, vol. 1307, 26-30
- **MOJICA et al.** *Mol. Microbiol.,* 1995, vol. 17, 85-93
- **JANSSEN et al.** *OMICS J. Integ. Biol.,* 2002, vol. 6, 23-33
- **MOJICA et al.** *Mol. Microbiol.,* 2000, vol. 36, 244-246
- **VAN EMBDEN et al.** *J. Bacteriol.,* 2000, vol. 182, 2393-2401
- **JANSEN et al.** *Mol. Microbiol.,* 2002, vol. 43, 1565-1575
- **MOLE.** *Cell,* 15 January 2010, vol. 37 (1), 7
- **NAKAMURA, Y. et al.** Codon usage tabulated from the international DNA sequence databases: status for the year 2000. *Nucl. Acids Res.,* 2000, vol. 28, 292
- **ZUKER ; STIEGLER.** *Nucleic Acids Res.,* 1981, vol. 9, 133-148
- **GRUBER et al.** *Cell,* 2008, vol. 106 (1), 23-24
- **PA CARR ; GM CHURCH.** *Nature Biotechnology,* 2009, vol. 27 (12), 1151-62

- **ANDERSON.** *Science,* 1992, vol. 256, 808-813
- **NABEL ; FEIGNER.** *TIBTECH,* 1993, vol. 11, 211-217
- **MITANI ; CASKEY.** *TIBTECH,* 1993, vol. 11, 162-166
- **DILLON.** *TIBTECH,* 1993, vol. 11, 167-175
- **MILLER.** *Nature,* 1992, vol. 357, 455-460
- **VAN BRUNT.** *Biotechnology,* 1988, vol. 6 (10), 1149-1154
- **VIGNE.** *Restorative Neurology and Neuroscience,* 1995, vol. 8, 35-36
- **KREMER ; PERRICAUDET.** *British Medical Bulletin,* 1995, vol. 51 (1), 31-44
- **HADDADA et al.** Current Topics in Microbiology and Immunology. 1995
- **YU et al.** *Gene Therapy,* 1994, vol. 1, 13-26
- **CRYSTAL.** *Science,* 1995, vol. 270, 404-410
- **BLAESE et al.** *Cancer Gene Ther.,* 1995, vol. 2, 291-297
- **BEHR et al.** *Bioconjugate Chem.,* 1994, vol. 5, 382-389
- **REMY et al.** *Bioconjugate Chem.,* 1994, vol. 5, 647-654
- **GAO et al.** *Gene Therapy,* 1995, vol. 2, 710-722
- **AHMAD et al.** *Cancer Res.,* 1992, vol. 52, 4817-4820
- **BUCHSCHER et al.** *J. Virol.,* 1992, vol. 66, 2731-2739
- **JOHANN et al.** *J. Virol.,* 1992, vol. 66, 1635-1640
- **SOMMNERFELT et al.** *Virol.,* 1990, vol. 176, 58-59
- **WILSON et al.** *J. Virol.,* 1989, vol. 63, 2374-2378
- **MILLER et al.** *J. Virol.,* 1991, vol. 65, 2220-2224
- **WEST et al.** *Virology,* 1987, vol. 160, 38-47
- **KOTIN.** *Human Gene Therapy,* 1994, vol. 5, 793-801
- **MUZYCZKA.** *J. Clin. Invest.,* 1994, vol. 94, 1351
- **TRATSCHIN et al.** *Mol. Cell. Biol.,* 1985, vol. 5, 3251-3260
- **TRATSCHIN et al.** *Mol. Cell. Biol.,* 1984, vol. 4, 2072-2081
- **HERMONAT ; MUZYCZKA.** *PNAS,* 1984, vol. 81, 6466-6470
- **SAMULSKI et al.** *J. Virol.,* 1989, vol. 63, 03822-3828
- **NEKRASOV.** Plant genome editing made easy: targeted mutagenesis in model and crop plants using the CRISPR/Cas system. *Plant Methods,* 2013, vol. 9, 39
- **BROOKS.** Efficient gene editing in tomato in the first generation using the CRISPR/Cas9 system. *Plant Physiology,* September 2014
- **SHAN.** Targeted genome modification of crop plants using a CRISPR-Cas system. *Nature Biotechnology,* 2013, vol. 31, 686-688
- **FENG.** Efficient genome editing in plants using a CRISPR/Cas system. *Cell Research,* 20 August 2013, vol. 23, 1229-1232
- **XIE.** RNA-guided genome editing in plants using a CRISPR-Cas system. *Mol Plant.,* November 2013, vol. 6 (6), 1975-83
- **XU.** Gene targeting using the Agrobacterium tumefaciens-mediated CRISPR-Cas system in rice. *Rice,* 2014, vol. 7, 5
- **ZHOU et al.** Exploiting SNPs for biallelic CRISPR mutations in the outcrossing woody perennial Populus reveals 4-coumarate: CoA ligase specificity and Redundancy. *New Phytologist,* 2015
- **CALIANDO et al.** Targeted DNA degradation using a CRISPR device stably carried in the host genome. *NATURE COMMUNICATIONS,* vol. 6, 6989
- **MORRELL et al.** Crop genomics: advances and applications. *Nat Rev Genet.,* 29 December 2011, vol. 13 (2), 85-96
- **CHAN-HUI et al.** *Clinical Immunology,* 2003, 162-174
- Tables A and B. Disease specific information is available from McKusick-Nathans Institute of Genetic Medicine. Johns Hopkins University
- National Center for Biotechnology Information. National Library of Medicine
- **ROBERT D. WELLS ; TETSUO ASHIZAWA.** Genetic Instabilities and Neurological Diseases. Academic Press, 13 October 2011
- **MCIVOR EI ; POLAK U.** *Napierala M. RNA Biol.,* vol. 7 (5), 551-8
- Genetics of Epilepsy and Genetic Epilepsies. Mariani Foundation Paediatric Neurology. vol. 20, 2009
- Genetic Diseases of the Eye. Oxford University Press, 2012
- **MORRELL et al.** Crop genomics:advances and applications. *Nat Rev Genet.,* 29 December 2011, vol. 13 (2), 85-96
- **DING, Q. et al.** A TALEN genome-editing system for generating human stem cell-based disease models. *Cell Stem Cell,* 2013, vol. 12, 238-251
- **SOLDNER, F. et al.** Generation of isogenic pluripotent stem cells differing exclusively at two early onset Parkinson point mutations. *Cell,* 2011, vol. 146, 318-331
- **CARLSON, D.F. et al.** Efficient TALEN-mediated gene knockout in livestock. *Proc Natl Acad Sci U S A,* 2012, vol. 109, 17382-17387
- **GEURTS, A.M. et al.** Knockout Rats via Embryo Microinjection of Zinc-Finger Nucleases. *Science,* 2009, vol. 325, 433-433

- **TAKASU, Y. et al.** Targeted mutagenesis in the silkworm Bombyx mori using zinc finger nuclease mRNA injection. *Insect Biochem Molec,* 2010, vol. 40, 759-765
- **WATANABE, T. et al.** Non-transgenic genome modifications in a hemimetabolous insect using zinc-finger and TAL effector nucleases. *Nat Commun,* 2012, vol. 3
- **PORTEUS, M.H. ; BALTIMORE, D.** Chimeric nucleases stimulate gene targeting in human cells. *Science,* 2003, vol. 300, 763
- **MILLER, J.C. et al.** An improved zinc-finger nuclease architecture for highly specific genome editing. *Nat Biotechnol,* 2007, vol. 25, 778-785
- **SANDER, J.D. et al.** Selection-free zinc-finger-nuclease engineering by context-dependent assembly (CoDA). *Nat Methods,* 2011, vol. 8, 67-69
- **WOOD, A.J. et al.** Targeted genome editing across species using ZFNs and TALENs. *Science,* 2011, vol. 333, 307
- **CHRISTIAN, M. et al.** Targeting DNA double-strand breaks with TAL effector nucleases. *Genetics,* 2010, vol. 186, 757-761
- **ZHANG, F. et al.** Efficient construction of sequence-specific TAL effectors for modulating mammalian transcription. *Nat Biotechnol,* 2011, vol. 29, 149-153
- **MILLER, J.C. et al.** A TALE nuclease architecture for efficient genome editing. *Nat Biotechnol,* 2011, vol. 29, 143-148
- **REYON, D. et al.** FLASH assembly of TALENs for high-throughput genome editing. *Nat Biotechnol,* 2012, vol. 30, 460-465
- **BOCH, J. et al.** Breaking the code of DNA binding specificity of TAL-type III effectors. *Science,* 2009, vol. 326, 1509-1512
- **MOSCOU, M.J. ; BOGDANOVE, A.J.** A simple cipher governs DNA recognition by TAL effectors. *Science,* 2009, vol. 326, 1501
- **SANJANA, N.E. et al.** A transcription activator-like effector toolbox for genome engineering. *Nat Protoc,* 2012, vol. 7, 171-192
- **DEVEAU, H. ; GARNEAU, J.E ; MOINEAU, S.** CRISPR-Cas system and its role in phage-bacteria interactions. *Annu Rev Microbiol,* 2010, vol. 64, 475-493
- **HORVATH, P. ; BARRANGOU, R.** CRISPR-Cas, the immune system of bacteria and archaea. *Science,* 2010, vol. 327, 167-170
- **MAKAROVA, K.S. et al.** Evolution and classification of the CRISPR-Cas systems. *Nat Rev Microbiol,* 2011, vol. 9, 467-477
- **BHAYA, D. ; DAVISON, M. ; BARRANGOU, R.** CRISPR-Cas systems in bacteria and archaea: versatile small RNAs for adaptive defense and regulation. *Annu Rev Genet,* 2011, vol. 45, 273-297
- **CONG, L. et al.** Multiplex genome engineering using CRISPR-Cas systems. *Science,* 2013, vol. 339, 819-823
- **MALI, P. et al.** RNA-guided human genome engineering via Cas9. *Science,* 2013, vol. 339, 823-826
- **JINEK, M. et al.** RNA-programmed genome editing in human cells. *eLife,* 2013, vol. 2, e00471
- **CHO, S.W. ; KIM, S. ; KIM, J.M ; KIM, J.S.** Targeted genome engineering in human cells with the Cas9 RNA-guided endonuclease. *Nat Biotechnol,* 2013, vol. 31, 230-232
- **GARNEAU, J.E. et al.** The CRISPR-Cas bacterial immune system cleaves bacteriophage and plasmid DNA. *Nature,* 2010, vol. 468, 67-71
- **JINEK, M. et al.** A programmable dual-RNA-guided DNA endonuclease in adaptive bacterial immunity. *Science,* 2012, vol. 337, 816-821
- **GASIUNAS, G. ; BARRANGOU, R. ; HORVATH, P. ; SIKSNYS, V.** Cas9-crRNA ribonucleoprotein complex mediates specific DNA cleavage for adaptive immunity in bacteria. *Proc Natl Acad Sci U S A,* 2012, vol. 109, E2579-2586
- **URNOV, F.D. ; REBAR, E.J. ; HOLMES, M.C. ; ZHANG, H.S. ; GREGORY, P.D.** Genome editing with engineered zinc finger nucleases. *Nat Rev Genet,* 2010, vol. 11, 636-646
- **HSU, P.D ; ZHANG, F.** Dissecting neural function using targeted genome engineering technologies. *ACS Chem Neurosci,* 2012, vol. 3, 603-610
- **PEREZ, E.E. et al.** Establishment of HIV-1 resistance in CD4(+) T cells by genome editing using zinc-finger nucleases. *Nat Biotechnol,* 2008, vol. 26, 808-816
- **CONG, L. et al.** Multiplex Genome Engineering Using CRISPR-Cas Systems. *Science,* 2013, vol. 339, 819-823
- **CHEN, F.Q. et al.** High-frequency genome editing using ssDNA oligonucleotides with zinc-finger nucleases. *Nat Methods,* 2011, vol. 8, 753-U796
- **BEDELL, V.M. et al.** In vivo genome editing using a high-efficiency TALEN system. *Nature,* 2012, vol. 491, 114-U133
- **SALEH-GOHARI, N. ; HELLEDAY, T.** Conservative homologous recombination preferentially repairs DNA double-strand breaks in the S phase of the cell cycle in human cells. *Nucleic Acids Res,* 2004, vol. 32, 3683-3688
- **DELTCHEVA, E. et al.** CRISPR RNA maturation by trans-encoded small RNA and host factor RNase III. *Nature,* 2011, vol. 471, 602-607
- **SAPRANAUSKAS, R. et al.** The Streptococcus thermophilus CRISPR-Cas system provides immunity in Escherichia coli. *Nucleic Acids Res,* 2011, vol. 39, 9275-9282
- **HWANG, W.Y. et al.** Efficient genome editing in zebrafish using a CRISPR-Cas system. *Nat Biotechnol,* 2013, vol. 31, 227-229

- **WANG, H. et al.** One-Step Generation of Mice Carrying Mutations in Multiple Genes by CRISPR-Cas-Mediated Genome Engineering. *Cell,* 2013, vol. 153, 910-918
- **SHEN, B. et al.** Generation of gene-modified mice via Cas9/RNA-mediated gene targeting. *Cell Res,* 2013, vol. 23, 720-723
- **QI, L.S. et al.** Repurposing CRISPR as an RNA-guided platform for sequence-specific control of gene expression. *Cell,* 2013, vol. 152, 1173-1183
- **TUSCHL, T.** Expanding small RNA interference. *Nat Biotechnol,* 2002, vol. 20, 446-448
- **SMITHIES, O ; GREGG, R.G. ; BOGGS, S.S.** Koralewski, M.A. & Kucherlapati, R.S. Insertion of DNA sequences into the human chromosomal beta-globin locus by homologous recombination. *Nature,* 1985, vol. 317, 230-234
- **THOMAS, K.R. ; FOLGER, K.R. ; CAPECCHI, M.R.** High frequency targeting of genes to specific sites in the mammalian genome. *Cell,* 1986, vol. 44, 419-428
- **HASTY, P. ; RIVERA-PEREZ, J. ; BRADLEY, A.** The length of homology required for gene targeting in embryonic stem cells. *Mol Cell Biol,* 1991, vol. 11, 5586-5591
- **WU, S. ; YING, G.X. ; WU, Q. ; CAPECCHI, M.R.** A protocol for constructing gene targeting vectors: generating knockout mice for the cadherin family and beyond. *Nat Protoc,* 2008, vol. 3, 1056-1076
- **GUSCHIN, D.Y. et al.** A rapid and general assay for monitoring endogenous gene modification. *Methods Mol Biol,* 2010, vol. 649, 247-256
- **OLIVEIRA, T.Y. et al.** Translocation capture sequencing: a method for high throughput mapping of chromosomal rearrangements. *J Immunol Methods,* 2012, vol. 375, 176-181
- **BOGENHAGEN, D.F. ; BROWN, D.D.** Nucleotide sequences in Xenopus 5S DNA required for transcription termination. *Cell,* 1981, vol. 24, 261-270
- **BULTMANN, S. et al.** Targeted transcriptional activation of silent oct4 pluripotency gene by combining designer TALEs and inhibition of epigenetic modifiers. *Nucleic Acids Res,* 2012, vol. 40, 5368-5377
- **VALTON et al.** Overcoming transcription activator-like effector (TALE) DNA binding domain sensitivity to cytosine methylation. *J Biol Chem,* 2012, vol. 287, 38427-38432
- **MUSSOLINO, C. et al.** A novel TALE nuclease scaffold enables high genome editing activity in combination with low toxicity. *Nucleic acids research,* 2011, vol. 39, 9283-9293
- **BOBIS-WOZOWICZ, S. ; OSIAK, A. ; RAHMAN, S.H. ; CATHOMEN, T.** Targeted genome editing in pluripotent stem cells using zinc-finger nucleases. *Methods,* 2011, vol. 53, 339-346
- **JIANG, W. ; BIKARD, D. ; COX, D. ; ZHANG, F. ; MARRAFFINI, L.A.** RNA-guided editing of bacterial genomes using CRISPR-Cas systems. *Nat Biotechnol,* 2013, vol. 31, 233-239
- **MICHAELIS, L.M., MAUD.** Die kinetik der invertinwirkung. *Biochem.,* 1913
- **MAHFOUZ, M.M. et al.** De novo-engineered transcription activator-like effector (TALE) hybrid nuclease with novel DNA binding specificity creates double-strand breaks. *Proc Natl Acad Sci U S A,* 2011, vol. 108, 2623-2628
- **WILSON, E.B.** Probable inference, the law of succession, and statistical inference. *J Am Stat Assoc,* 1927, vol. 22, 209-212
- **LOPES, V.S.** Retinal gene therapy with a large MYO7A cDNA using adeno-assocaited virus. *Gene Ther,* 24 January 2013
- **KAPLITT, M.G. et al.** Safety and tolerability of gene therapy with an adeno-associated virus (AAV) borne GAD gene for Parkinson's disease: an open label, phase I trial. *Lancet.,* 23 June 2007, vol. 369 (9579), 2097-105
- **NATHWANI, A.C. et al.** Adenovirus-associated virus vector-mediated gene transfer in hemophilia B. *N Engl J Med.,* 10 December 2011, vol. 365 (25), 2357-65
- **GRAY SJ ; FOTI SB ; SCHWARTZ JW ; BACH-ABOINA L ; TAYLOR-BLAKE B ; COLEMAN J ; EHLERS MD ; ZYLKA MJ ; MCCOWN TJ ; SAMULSKI RJ.** Optimizing promoters for recombinant adeno-associated virus-mediated gene expression in the peripheral and central nervous system using self-complementary vectors. *Hum Gene Ther.,* September 2011, vol. 22 (9), 1143-53
- **LIU D ; FISCHER I.** Two alternative promoters direct neuron-specific expression of the rat microtubule-associated protein 1B gene. *J Neurosci.,* 15 August 1996, vol. 16 (16), 5026-36
- **LEVITT N. ; BRIGGS D. ; GIL A. ; PROUDFOOT N.J.** Definition of an efficient synthetic poly(A) site. *Genes Dev.,* 1989, vol. 3, 1019-1025
- **MCCLURE C ; COLE KL ; WULFF P ; KLUGMANN M ; MURRAY AJ.** Production and titering of recombinant adeno-associated viral vectors. *J Vis Exp.,* 27 November 2011, vol. 57, e3348
- **BANKER G ; GOSLIN K.** Developments in neuronal cell culture. *Nature,* 10 November 1988, vol. 336 (6195), 185-6
- **CHANG, N. ; SUN, C. ; GAO, L. ; ZHU, D. ; XU, X ; ZHU, X. ; XIONG, J.W. ; XI, J.J.** Genome editing with RNA-guided Cas9 nuclease in zebrafish embryos. *Cell research,* 2013, vol. 23, 465-472
- **DIANOV, G.L. ; HUBSCHER, U.** Mammalian base excision repair: the forgotten archangel. *Nucleic acids research,* 2013, vol. 41, 3483-3490

- **FRIEDLAND, A.E. ; TZUR, Y.B. ; ESVELT, K.M. ; COLAIACOVO, M.P. ; CHURCH, G.M. ; CALAR-CO, J.A.** Heritable genome editing in C. elegans via a CRISPR-Cas9 system. *Nature methods,* 2013, vol. 10, 741-743
- **FU, Y. ; FODEN, J.A. ; KHAYTER, C. ; MAEDER, M.L. ; REYON, D. ; JOUNG, J.K. ; SANDER, J.D.** High-frequency off-target mutagenesis induced by CRISPR-Cas nucleases in human cells. *Nature biotechnology,* 2013
- **GRATZ, S.J. ; CUMMINGS, A.M. ; NGUYEN, J.N. ; HAMM, D.C. ; DONOHUE, L.K. ; HARRISON, M.M. ; WILDONGER, J. ; O'CONNOR-GILES, K.M.** Genome Engineering of Drosophila with the CRISPR RNA-Guided Cas9 Nuclease. *Genetics,* 2013, vol. 194, 1029-1035
- **HSU, P.D. ; SCOTT, D.A ; WEINSTEIN, J.A. ; RAN, F.A. ; KONERMANN, S. ; AGARWALA, V. ; LI, Y. ; FINE, E.J. ; WU, X. ; SHALEM, O. et al.** DNA targeting specificity of RNA-guided Cas9 nucleases. *Nature biotechnology,* 2013
- **MALI, P. ; AACH, J. ; STRANGES, P.B. ; ESVELT, K.M. ; MOOSBURNER, M. ; KOSURI, S. ; YANG, L ; CHURCH, G.M.** CAS9 transcriptional activators for target specificity screening and paired nickases for cooperative genome engineering. *Nature biotechnology,* 2013
- **MARESCA, M. ; LIN, V.G. ; GUO, N. ; YANG, Y.** Obligate ligation-gated recombination (ObLiGaRe): custom-designed nuclease-mediated targeted integration through nonhomologous end joining. *Genome research,* 2013, vol. 23, 539-546
- **PATTANAYAK, V. ; LIN, S ; GUILINGER, J.P ; MA, E. ; DOUDNA, J.A. ; LIU, D.R.** High-throughput profiling of off-target DNA cleavage reveals RNA-programmed Cas9 nuclease specificity. *Nature biotechnology,* 2013
- **BARRANGOU, R. ; FREMAUX, C. ; DEVEAU, H ; RICHARDS, M. ; BOYAVAL, P. ; MOINEAU, S. ; HORVATH, P.** CRISPR provides acquired resistance against viruses in prokaryotes. *Science,* 2007, vol. 315 (5819), 1709-1712
- **BARRANGOU, R. ; MARRAFFINI, L. A.** CRISPR-Cas systems: Prokaryotes upgrade to adaptive immunity. *Mol Cell,* 2014, vol. 54 (2), 234-244
- **BOCH, J. ; BONAS, U.** Xanthomonas AvrBs3 family-type III effectors: discovery and function. *Annu Rev Phytopathol,* 2010, vol. 48, 419-436
- **CHYLINSKI, K. ; MAKAROVA, K. S. ; CHARPENTIER, E. ; KOONIN, E. V.** Classification and evolution of type II CRISPR-Cas systems. *Nucleic Acids Res,* 2014, vol. 42 (10), 6091-6105
- **CONG, L. ; RAN, F. A. ; COX, D. ; LIN, S. ; BARRETTO, R. ; HABIB, N. ; ZHANG, F.** Multiplex genome engineering using CRISPR/Cas systems. *Science,* 2013, vol. 339 (6121), 819-823
- **DELTCHEVA, E. ; CHYLINSKI, K. ; SHARMA, C. M. ; GONZALES, K. ; CHAO, Y. ; PIRZADA, Z. A. ; CHARPENTIER, E.** CRISPR RNA maturation by trans-encoded small RNA and host factor RNase III. *Nature,* 2011, vol. 471 (7340), 602-607
- **DIANOV, G. L. ; HUBSCHER, U.** Mammalian base excision repair: the forgotten archangel. *Nucleic Acids Res,* 2013, vol. 41 (6), 3483-3490
- **FU, Y. ; FODEN, J. A. ; KHAYTER, C. ; MAEDER, M. L ; REYON, D. ; JOUNG, J. K. ; SANDER, J. D.** High-frequency off-target mutagenesis induced by CRISPR-Cas nucleases in human cells. *Nat Biotechnol,* 2013, vol. 31 (9), 822-826
- **GARNEAU, J. E. ; DUPUIS, M. E. ; VILLION, M. ; ROMERO, D. A. ; BARRANGOU, R. ; BOYAVAL, P. ; MOINEAU, S.** The CRISPR/Cas bacterial immune system cleaves bacteriophage and plasmid DNA. *Nature,* 2010, vol. 468 (7320), 67-71
- **GASIUNAS, G. ; SINKUNAS, T. ; SIKSNYS, V.** Molecular mechanisms of CRISPR-mediated microbial immunity. *Cell Mol Life Sci,* 2014, vol. 71 (3), 449-465
- **HSU, P. D. ; LANDER, E. S. ; ZHANG, F.** Development and Applications of CRISPR-Cas9 for Genome Engineering. *Cell,* 2014, vol. 157 (6), 1262-1278
- **HSU, P. D. ; SCOTT, D. A. ; WEINSTEIN, J. A. ; RAN, F. A. ; KONERMANN, S. ; AGARWALA, V. ; ZHANG, F.** DNA targeting specificity of RNA-guided Cas9 nucleases. *Nat Biotechnol,* 2013, vol. 31 (9), 827-832
- **JINEK, M. ; CHYLINSKI, K. ; FONFARA, I ; HAUER, M. ; DOUDNA, J. A. ; CHARPENTIER, E.** A programmable dual-RNA-guided DNA endonuclease in adaptive bacterial immunity. *Science,* 2012, vol. 337 (6096), 816-821
- **KLUG, A.** The discovery of zinc fingers and their applications in gene regulation and genome manipulation. *Annu Rev Biochem,* 2010, vol. 79, 213-231
- **KUSCU, C. ; ARSLAN, S. ; SINGH, R. ; THORPE, J. ; ADLI, M.** Genome-wide analysis reveals characteristics of off-target sites bound by the Cas9 endonuclease. *Nat Biotechnol.,* 2014
- **MALI, P. ; AACH, J. ; STRANGES, P. B. ; ESVELT, K. M. ; MOOSBURNER, M. ; KOSURI, S. ; CHURCH, G. M.** CAS9 transcriptional activators for target specificity screening and paired nickases for cooperative genome engineering. *Nat Biotechnol,* 2013, vol. 31 (9), 833-838
- **MOJICA, F. J. ; DIEZ-VILLASENOR, C. ; GARCIA-MARTINEZ, J. ; ALMENDROS, C.** Short motif sequences determine the targets of the prokaryotic CRISPR defence system. *Microbiology,* 2009, vol. 155, 733-740

- **NISHIMASU, H ; RAN, F. A. ; HSU, P. D. ; KONER-MANN, S. ; SHEHATA, S. I. ; DOHMAE, N. ; NU-REKI, O.** Crystal structure of cas9 in complex with guide RNA and target DNA. *Cell,* 2014, vol. 156 (5), 935-949
- **PATTANAYAK, V. ; LIN, S. ; GUILINGER, J. P ; MA, E. ; DOUDNA, J. A. ; LIU, D. R.** High-throughput profiling of off-target DNA cleavage reveals RNA-programmed Cas9 nuclease specificity. *Nat Biotechnol,* 2013, vol. 31 (9), 839-843
- **RAN, F. A. ; HSU, P. D. ; LIN, C. Y. ; GOOTEN-BERG, J. S. ; KONERMANN, S. ; TREVINO, A. E. ; ZHANG, F.** Double nicking by RNA-guided CRISPR Cas9 for enhanced genome editing specificity. *Cell,* 2013, vol. 154 (6), 1380-1389
- **WANG, H. ; YANG, H. ; SHIVALILA, C. S. ; DAW-LATY, M. M. ; CHENG, A. W. ; ZHANG, F. ; JAE-NISCH, R.** One-step generation of mice carrying mutations in multiple genes by CRISPR/Cas-mediated genome engineering. *Cell,* 2013, vol. 153 (4), 910-918
- **WIEDENHEFT, B. ; STERNBERG, S. H. ; DOUD-NA, J. A.** RNA-guided genetic silencing systems in bacteria and archaea. *Nature,* 2012, vol. 482 (7385), 331-338
- **WU, X. ; SCOTT, D. A. ; KRIZ, A. J. ; CHIU, A. C. ; HSU, P. D. ; DADON, D. B. ; SHARP, P. A.** Genome-wide binding of the CRISPR endonuclease Cas9 in mammalian cells. *Nat Biotechnol.,* 2014
- **RAN et al.** *In vivo genome editing using Staphylococcus aureus Cas9*
- **RAN et al.** *Nature,* 01 April 2015